# FASCICULE DE BREVET EUROPEEN

(11) **EP 1 475 441 B1**
(45) Date de publication et mention de la délivrance du brevet: **08.07.2009**
(21) Numéro de dépôt: 04016071.5
(22) Date de dépôt: 28.10.1999
(51) Int. Cl.: C12N 15/31, C12N 1/21, C07K 14/22, C07K 16/12, A61K 38/16

(54) **Acides nucléiques et polypeptides spécifiques des souches pathogènes du genre Neisseria**
Spezifische Nukleinsäuren und Polypeptide aus krankheitserregenden Stämmen von Neisseria
Specific nucleic acids and polypeptides from pathogenic strains of Neisseria

(30) Priorité: 30.10.1998 FR 9813693
(43) Date de publication de la demande: 10.11.2004
(62) Demande divisionnaire de: 99950875.7
(73) Titulaire: Sanofi Pasteur, 69367 Lyon Cedex 07 (FR); INSTITUT NATIONAL DE LA SANTE ET DE LA RECHERCHE MEDICALE (INSERM), 75013 Paris (FR)
(72) Inventeur: Aujame, Luc, 69210 Fleurieux sur L'Arbresle (FR); Bouchardon, Annabelle, 69500 Bron (FR); Renauld-Mongenie, Geneviève, 69630 Chaponost (FR); Rokbi, Bachra, 69003 Lyon (FR); Nassif, Xavier, 75015 Paris (FR); Tinsley, Colin, 75015 Paris (FR); Perrin, Agnès, 75015 Paris (FR)
(74) Mandataire: Bernasconi, Jean Raymond

(56) Documents cités:
- EP-A- 0 839 909
- WO-A-98/02547
- TINSLEY C R ET AL: "ANALYSIS OF THE GENETIC DIFFERENCES BETWEEN NEISSERIA MENINGITIDIS AND NEISSERIA GONORRHOEAE: TWO CLOSELY RELATED BACTERIA EXPRESSING TWO DIFFERENT PATHOGENICITIES" PROCEEDINGS OF THE NATIONAL ACADEMY OF SCIENCES OF USA, vol. 93, no. 20, 1 octobre 1996 (1996-10-01), pages 11109-1114, XP002028346
- DATABASE UNIPROT [Online] EBI; HTTP://WWW.EBI.UNIPROT.ORG 1 juillet 1993 (1993-07-01), "Serotype-specific antigen 1 precursor of Pasteurella haemolytica" XP002299656 extrait de UNIPROT Database accession no. P31631
- TETTELIN H. ET AL: 'Complete genome sequence of Neisseria meningitidis serogroup B strain MC58' SCIENCE vol. 287, Mars 2000, pages 1809 - 1815
- TURNER D.P. ET AL: 'Autotransported serine protease A of Neisseria meningitidis: an immunogenic, surface-exposed outer membrane, and secreted protein.' INFECT. IMMUN. vol. 70, no. 8, Août 2008, pages 4447 - 4461
- BURRELL C.J. ET AL: 'Expression in Escherichia coli of hepatitis B virus DNA sequences cloned in plasmid pBR322.' NATURE vol. 279, no. 5708, 03 Mai 1979, pages 43 - 47

## Description

La présente invention a trait à des acides nucléiques codant pour des polypeptides spécifiques des souches pathogènes du genre *Neisseria* notamment utiles pour prévenir ou traiter une infection par *Neisseria meningitidis*.

D'une manière générale, les méningites sont soit d'origine virale, soit d'origine bactérienne. Les bactéries principalement responsables sont *Haemophilus influenzae* de type b, *Neisseria meningitidis* et *Streptococcus pneumoniae.* L'espèce *Neisseria meningitidis* est subdivisée en sérogroupes. selon la nature des polysaccharides capsulaires. Bien qu'il existe une douzain de sérogroupes, 90% des cas de méningites sont attribuables à trois sérogroupes : A, B et C.

Il existe des vaccins efficaces à base de polysaccharides capsulaires pour prévenir les méningites à *Neisseria meningitidis* sérogroupes A et C. Ces polysaccharides tels quels ne sont que peu ou pas immunogènes chez les enfants de moins de deux ans et n'induisent pas de mémoire immunitaire. Toutefois, ces inconvénients peuvent être surmontés en conjuguant ces polysaccharides à une protéine porteuse.

En revanche, le polysaccharide de *Neisseria meningitidis* sérogroupe B n'est pas ou peu immunogène chez l'homme, qu'il soit sous forme conjuguée ou non. Ainsi il apparaît hautement souhaitable de rechercher un vaccin à l'encontre des méningites causées par *Neisseria meningitidis,* notamment du sérogroupe B, autre qu'un vaccin à base de polysaccharide.

A cette fin, différentes protéines de la membrane externe de *N*. *meningitidis* ont déjà été proposées, telles que le récepteur membranaire de la transferrine humaine (WO 90/12591 et WO93/06861).

*Neisseria meningitidis* est génétiquement très proche de *Neisseria gonorrhoeae et Neisseria lactamica. N. gonorrhoeae* est surtout responsable d'infections localisées dans le tractus urogénital. Elle colonise la muqueuse génitale, puis traverse l'épithélium, envahit ensuite le sous-épithélium où elle se multiplie et est responsable d'une forte réaction inflammatoire. Par contre, N. *Jactamica* est considérée comme une espèce non pathogène.

Des séquences présentes chez *N. gonorrhoeae* et *N. meningitidis,* absentes de *N. lactamica* ont été divulguées dans la demande de brevet WO 98/02 547, mais cette demande de brevet antérieure ne localise pas et n'identifie pas les séquences codantes.

Les auteurs de la présente invention sont maintenant parvenus à identifier certains de ces gènes en recherchant dans le génome du méningoccoque, les cadres de lecture ouverts spécifiques des souches pathogènes du genre *Neisseria,* en mettant en oeuvre la stratégie suivante :

Certaines des séquences divulguées dans la demande de brevet WO98/02547 (désignées dans ladite demande antérieure SEQ ID n° 66, 67, 69, 70, 72 à 96, 98, et 99), ont été positionnées sur la séquence du génome de la souche *N. meningitidis* de sérogroupe B (ATCC 13090), disponible à partir de la banque Pathoseq® d'Incyte Pharmaceuticals ainsi que sur la séquence du génome de la souche *Neisseria meningitidis* Z2491 (Sanger Center). Ceci a permis d'identifier, dans le génome de *N. meningitidis* qui compte 2,3 Méga bases, 19 contigus représentant 220 000 paires de bases.

Les auteurs de la présente invention ont ensuite analysé, pour chacun des 19 contigs, la présence de phases de lecture ouvertes (." open reading frames-ORF "), contenant au moins 100 acides aminés (et par définition, bornées par un codon d'initiation et un codon stop), à l'aide, du logiciel Gene Jockey II sequence processor^{®} (Biosoft). Cette analyse a permis de sélectionner environ 400 ORFs candidates.

Les séquences de chacune de ces ORFs ont ensuite été analysées à l'aide du logiciel Codon Use^{®} (Conrad Halling), qui tient compte de la fréquence d'utilisation des codons chez *N. meningitidis.* N'ont été retenues que les ORFs dont les séquences présentent une fréquence d'utilisation maximale de ces codons. A l'issue de cette analyse, 197 ORFs candidates ont été retenues.

Les ORFs sélectionnées par cette double analyse ont été soumises à une recherche d'homologies sur l'ensemble des banques disponibles à l'aide du programme BLASTX^{®} depuis l'accès à la banque Pathoseq® d'Incyte Pharmaceuticals. Cette recherche d'homologies a permis d'exclure les ORFs codant *a priori* pour des protéines cytoplasmiques ou périplasmiques, en particulier des protéines du métabolisme. Les ORFs ont également été soumises à une analyse des motifs protéiques éventuels, à l'aide du programme Protean^{®} de DNA Star (Lasergene software).

Les auteurs de la présente invention ont ensuite recherché si les ORFs retenues à l'issue de l'étape précédente (au nombre de 118) étaient effectivement absentes de *N. lactamica,* comme le prévoyait la demande de l'art antérieur WO 98/02547.

A cette fin, une amplification par PCR a été mise en oeuvre. Cette amplification s'est révélée inopérante pour 78 des 118 ORFs testées. Seules les ORFs pour lesquelles l'amplification chez *N. lactamica* était négative (séquences appelées "lactamica") ont été retenues. Afin de vérifier que ces résultats négatifs n'étaient pas des " faux-négatifs ", les séquences lactamica⁻retenues ont été soumises à un contrôle par dot blot. A l'issue de cette étape, seules 23 ORFs ont été confirmées *N. meningitidis*⁺/ *N. lactamica⁻.*

Enfin, ces 23 ORFs ont été repositionnées dans leur ensemble sur le génome de *N. meningitidis* ATCC13090. Ceci a permis de mettre en évidence que trois ORFs préalablement éliminées sur la base de leur fonction protéique putative, apparaissaient localisées à proximité ou même encadrées par certaines des 23 ORFs *N. meningitidis*⁺/ *N. lactamica⁻.* Ces trois ORFs (SEQ ID n° 29, 35 et 37) ont été réintroduites dans l'étude, et il s'est avéré qu'elles étaient elles aussi *N. meningitidis*⁺/ *N. lactamica⁻.*

Les auteurs de la présente invention ont ensuite cherché à savoir si les ORFs identifiées à partir du génome de la souche *N. meningitidis* de sérogroupe B ATCC 13090 étaient aussi présentes dans les génomes de *N*. *meningitidis* Z2491 (Sanger Center) de sérogroupe A et de *N. gonorrhoeae* FA1090 (Advanced Center of Génome Technology, Oklahoma University).Puis ils ont comparé les séquences dérivées de ces différents génomes, par alignement multiple (Clustal, Infobiogen). Ceci a permis de redéfinir pour certaines des ORFs la position la plus probable des codons d'initiation et de fin de traduction. Les séquences des cadres de lecture ouverts issues de la souche ATCC13090 sont données dans les SEQ ID N° 1 - 51 (numéros impairs) [groupe I] et les séquences d'acides aminés qui en sont déduites, dans les SEQ ID N° 2 - 52 (numéros pairs) et SEQ ID N° 53 [groupe II] .

Les auteurs de la présente invention ont plus particulièrement trouvé que les ORFs qui, lorsqu'elles étaient issues de la souche ATCC 13090, étaient caractérisées par les séquences telles montrées dans les SEQ ID n° 19, 27, 39, 45, 47 et 49, étaient spécifiques de *Neisseria meningitidis* dans la mesure où il n'a pas tété possible de mettre en évidence des séquences identiques ou homologues dans le génome de *N. gonorrhoeae*. Ils ont aussi trouvé que l'ORF caractérisée par la séquences de souche telle que montrée dans le sec ID N° 39 était spécifique de *Neisseria meningitidis* de sérogroupe B.

La présente invention fournit donc un acide nucléique sous forme isolée codant pour un polypeptide, ou un fragment antigénique de celui-ci à l'exclusion des acides nucléiques divulgués dans les SEQ ID n° 70, 73, 74, 77, 80, 81, 87, 88, 89, 94, 95 et 98 de la demande WO 98/02547 (séquences annexées à la présente description et numérotées SEQ ID n°70A, 73A, 74A, 77A, 80A, 81A, 87A, 88A, 89A, 94A, 95A, 98A pour les distinguer des séquences de l'invention); ledit polypeptide ayant une séquence d'acides aminés qui est identique ou homologue à une séquence choisie parmi celles du groupe II. Ledit acide nucléique peut avoir une séquence nucléotidique choisie parmi celles du groupe I.

### OBJET DE L'INVENTION

L'objet de la présente invention est défini par les revendications 1 à 8 ci-jointes.

Les acides aminés encadrés dans la séquence SEQ ID N° 8 correspondent à la séquence signal, l'acide aminé en gras représente le premier acide aminé de la forme mature. La sequence d'acides aminés de la forme protéique mature est représenter dans la SEQ ID n° 53.

### DESCRIPTION DETAILLEE

Le terme « acide nucléique » inclut et signifie également ORF. gène, polynucléotide, ADN et ARN. Le terme « acide nucléique sous forme isolée » signifie un acide nucléique séparé de l'environnement biologique dans lequel il se trouve dans des conditions naturelles. Par exemple, une molécule d'ADN existe dans des conditions naturelles lorsqu'elle se trouve intégrée dans un génome ou bien lorsqu'elle fait partie d'une banque de gènes. Dans ce cas là, elle ne peut être sous forme isolée. Par contre, la même molécule séparée du génome par clonage (par exemple suite à une amplification par PCR) doit être considérée comme étant sous forme isolée. De manière typique, une molécule d'ADN sous forme isolée, ne contient pas les régions codantes qui lui sont contiguës en 5' et 3' dans le génome dont elle est issue. Les acides nucléiques sous forme isolée peuvent être intégrés dans des vecteurs (par exemple plasmides or vecteurs viraux ou bactériens) sans pour cela se départir de leur caractéristique d'être séparés de leur environnement naturel.

Dans le cadre de la présente invention, les termes de " polypeptide et " protéine " sont équivalents et interchangeables entre eux. Ils désignent n'importe quelle chaîne d'acides aminés, quelle qu'en soit sa longueur et ses modifications post-traductionnelles (par exemple, phosphorylation ou glycosylation).

Par " fragments antigéniques des polypeptides spécifiques des souches pathogènes du genre *Neisseria* ", on entend les polypeptides dérivés des polypeptides de l'invention tels que définis précédemment, par des délétions de parties desdits polypeptides sans destruction de l'antigénicité (par exemple, sans perte notable de l'activité antigénique) desdits polypeptides. L'antigénicité spécifique peut être déterminée en utilisant diverses méthodes connues de l'homme du métier, comme expliqué plus loin.

Ces fragments ont de préférence au moins 12 acides aminés de long, de préférence encore au moins 20 acides aminés de long, préférentiellement 50 acides aminés de long, de préférence encore 75 acides aminés de long, préférentiellement 100 acides aminés de long.

Ces fragments peuvent être utilisés pour révéler des épitopes qui peuvent être masqués chez les polypeptides parents. Ils sont également avantageux pour induire une réponse immune protectrice dépendante des lymphocytes T. Les délétions peuvent en effet permettre d'éliminer des régions immunodominantes de haute variabilité entre différentes souches.

De tels fragments peuvent être obtenus en utilisant des techniques standard connues de l'homme du métier (par exemple, Ausubel et al, Current Protocols in Molecular Biology, John Wiley & sons Inc, 1994), par exemple par PCR, RT-PCR, traitement par enzymes de restriction des molécules d'ADN clonées, ou par la méthode de Kunkel et al (Proc. Natl. Acad. Sci. USA (1985) 82:448).

Par " séquence d'acides aminés homologue ", on entend une séquence qui diffère d'une des séquences du groupe Il par substitution, délétion, et/ou insertion d'un ou plusieurs acides aminés, à des positions telles que ces modifications ne détruisent pas l'antigénicité spécifique du polypeptide en question.

Lesdites substitutions sont de préférence des substitutions conservatives, c'est-à-dire des substitutions d'acides aminés de même classe, tels que des substitutions d'acides aminés aux chaînes latérales non chargées (tels que l'asparagine, la glutamine, la serine, la thréonine, et la tyrosine), d'acides aminés aux chaînes latérales basiques (tels que la lysine, l'arginine, et l'histidine), d'acides aminés aux chaînes latérales acides (tels que l'acide aspartique et l'acide glutamique) ; d'acides aminés aux chaînes latérales apolaires (tels que la glycine, l'alanine, la valine, la leucine, l'isoleucine, la proline, la phénylalanine, la méthionine, le tryptophane, et la cystéine).

De manière avantageuse, une séquence d'acides aminés homologue possède un degré d'homologie (i.e., d'identité) d'au moins 75 % avec une des séquences du groupe 11 ; de préférence ce degré d'homologie est d'au moins 80 %, de manière tout à fait préférée, d'au moins 90 %. Les séquences d'acides aminés homologues incluent en particulier les séquences qui sont substantiellement identiques à une des séquences du groupe II. Par " séquence substantiellement identique " on signifie une séquence dont le degré d'homologie (*i.e.*, d'identité) avec une des séquences du groupe II est d'au moins 90%, avantageusement d'au moins 95%, de préférence d'au moins 97%, de manière tout à fait préférée d'au moins 99%. De plus, elle peut ne différer de la séquence de référence que par une majorité de substitutions conservatives.

Le degré d'homologie (aussi appelé degré d'identité) est généralement déterminé en utilisant un logiciel d'analyse de séquence (par exemple, Sequence Analysis Software Package of the Genetics Computer Group, University of Wisconsin Biotechnology Center, 1710 University Avenue, Madison, WI 53705). Des séquences d'acides aminés similaires sont alignées pour obtenir le maximum de degré d'homologie (*i.e*. identité). A cette fin, il peut être nécessaire d'introduire de manière artificielle des espaces (" gaps ") dans la séquence. Une fois l'alignement optimal réalisé, le degré d'homologie (*i.e.* identité) est établi par enregistrement de toutes les positions pour lesquelles les acides aminés des deux séquences comparées sont identiques, par rapport au nombre total de positions.

En d'autres termes, la présente description fournit un polypeptide sous forme isolée, ou un fragment de celui-ci, et ayant une séquence d'acides aminés identique ou homologue à une séquence choisie parmi les séquences du groupe II.

Par " séquences nucléotidiques homologues ", on entend des séquences qui diffèrent des séquences du groupe I par substitution d'un nucléotide ou plusieurs nucléotides, ou par délétion et/ou insertion d'un ou plusieurs codons, à des positions telles que ces séquences codent (i) toujours pour des polypeptide ayant les séquences du groupe II, par effet de la dégénérescence du code génétique ; ou bien (ii) pour des polypeptides possédant des séquences homologues tels que définies précédemment.

De manière avantageuse, une séquence nucléotidique homologue possède un degré d'homologie d'au moins 60 % avec une des séquences du groupe I ; de préférence ce degré d'homologie est d'au moins 80 %, de manière tout à fait préférée d'au moins 90 %.

De manière typique, une séquence nucléotidique homologue s'hybride spécifiquement aux séquences complémentaires des séquences du groupe 1 dans des conditions stringentes. La température à laquelle le test d'hybridation est mis en oeuvre constitue un facteur important influençant la stringence. De manière conventionnelle, cette température, dite température d'hybridation (Th), est choisie de 5 à 40°C, de préférence de 20 à 25°C au dessous de la température à laquelle 50% des brins appariés se séparent (Tm). De manière générale, on considère que des conditions de forte stringence sont remplies lorsque Th est inférieure à Tm de 5 à 25°C approximativement, par exemple de 5 à 10°C ou le plus souvent, de 20 à 25°C approximativement. Une stringence modérée s'établit lorsque Th est inférieure à Tm de 30 à 40°C.

Pour les séquences comprenant plus de 30 bases, la température Tm est définie par la relation : Tm = 81,5 + 0,41 (%G+C) + 16,6Log(concentration en cations) - 0,63(%formamide) - (600/nombre de bases). Ainsi, la force ionique a un impact majeur sur la valeur de Tm. La température Tm augmente de 16,6°C chaque fois que la concentration en cation monovalent augmente d'un facteur 10. L'addition de formamide dans le tampon d'hybridation fait par contre baisser la valeur de Tm. (Pour une référence complète voir Sambrook et al, Molecular Cloning, A laboratory manual, Cold Spring Harbor laboratory Press, 1989, pages 9.54-9.62).

De manière conventionnelle, les expériences d'hybridation sont conduites à une température de 60 à 68°C, par exemple à 65°C. A cette température, des conditions d'hybridation stringentes peuvent être par exemple mises en oeuvre en 6xSSC, avantageusement en 2xSSC ou 1xSSC, de préférence, en 0,5xSSC, 0,3xSSC ou O,1xSSC (en absence de formamide). Une solution de 1xSSC contient 0,15 M de NaCl et 0,015 de citrate de sodium.

C'est pourquoi, en d'autres termes, l'invention fournit un polynucléotide sous forme isolée, qui est capable de s'hybrider dans des conditions stringentes avec une molécule d'ADN ayant une des séquences nucléotidiques telles que montrées dans le groupe I tel que défini ci-dessus ou leurs complémentaires.

Une classe particulière de séquences homologues est constituée par celles que l'on rencontre dans la nature en vertu du phénomène extrêmement fréquent de la variation allélique. Une espèce bactérienne par exemple, *N. meningitidis* ou *N. gonorrhoeae*, est constituée par une grande variété de souches qui diffèrent entre elles par des variations mineures, dites variations alléliques. Ainsi, un polypeptide qui est présent dans différentes souches et qui bien évidemment remplit dans chacune d'entre elles, la même fonction biologique, peut posséder une séquence d'acides aminés qui n'est pas identique d'une souche à l'autre. En d'autres termes, les séquences issues de la variation allélique sont purement des séquences équivalentes ou alternatives à celles du groupe Il. La classe de séquences variantes alléliques de l'une des séquences du groupe Il est constituée par les séquences du polypeptide tel qu'il se trouve dans une espèce pathogène du genre *Neisseria* (par exemple *N. meningitidis* ou de *N. gonorrhoeae)* autre que la souche de *N. meningitidis* ATCC 13090. La fonction biologique qui est associée aux séquences variantes allèliques est la même que celle qui est associée à la séquence de référence. Les différences (substitution, délétion ou addition d'un ou plusieurs acides aminés) qu'elles présentent entre elles (y compris la séquence de référence) n'altèrent pas la fonction biologique du polypeptide. Par " fonction biologique ", on entend la fonction exercée par le polypeptide dans les cellules qui le produisent de manière naturelle.

La variation allélique s'exprime également au niveau des séquences codantes. Un polynucléotide codant pour un polypeptide possédant une séquence qui est une variante allélique de l'une des séquences du groupe I peut être facilement cloné par amplification de l'ADN génomique des souches d'espèces pathogènes du genre *Neisseria,* par exemple par PCR (réaction en chaîne par la polymérase), en utilisant des amorces oligonucléotidiques synthétiques capables de s'hybrider au niveau des extrémités 5' et 3' de la région codante. Les séquences de telles amorces peuvent facilement être établies par un homme du métier à partir des séquences nucléotidiques données dans les SEQ ID N° 1 - 51 (numéros impairs). Les amorces ont en général de 10 à 40 nucléotides, de préférence de 15 à 25 nucléotides.

C'est pourquoi, en d'autres termes, l'invention fournit une molécule d'ADN sous forme isolée qui peut être amplifiée et/ou clonée par PCR à partir du génome d'une souche de *Neisseria* pathogène en utilisant un couple d'amorces PCR 5' et 3' ; les séquences de ces amorces étant établies à partir d'une des séquences nucléotidiques telles que montrées dans le groupes I tel que, défini ci-dessus Un exemple est donné pour chaque couple d'amorces dans l'exemple I.1 ci-après.

La présente invention fournit plus particulièrement les variants alléliques ayant les séquences nucléotidiques SEQ ID n° 54 à 76 (numéros pairs) et les produits codés par ces séquences nucléotidiques, ayant les séquences d'acides aminés SEQ 10 n° 55 à 77 (numéros impairs).

Les polypeptides de l'invention peuvent être fusionnés à d'autres polypeptides, par exemple par traduction d'un gène hybride. Des vecteurs pour l'expression de polypeptides de fusion sont disponibles dans le commerce, comme les vecteurs pMal-c2 ou pMai-p2 de New England Biolabs, dans lesquels la protéine à laquelle peuvent être fusionnés les polypeptides de l'invention est une protéine de liaison au maltose, ou le système glutathion-S-transférase de Pharmacia, ou le système His-Tag de Novagen. De tels systèmes sont en particulier utiles pour une purification des polypeptides de l'invention. Les polypeptides de l'invention peuvent être fusionnés à des polypeptides présentant une activité d'adjuvant, comme par exemple la sous-unité B de la toxine cholérique ou de la toxine thermosensible de *E. coli.*

Les acides nucléiques de la présente invention peuvent être utilisés (i) dans un procédé de production des polypeptides codés par lesdits acides nucléiques dans un système hôte recombinant, (ii) pour la construction de vecteurs vaccinaux tels que des poxvirus, destinés à être utilisés dans des méthodes et des compositions de prévention et/ou de traitement d'une infection par les souches pathogènes de *Neisseria,* en particulier par *Neisseria meningitidis,* (iii) comme agent vaccinal sous une forme nue ou en association avec un véhicule favorisant le transfert aux cellules cible et, (iv) dans la construction de souches de *Neisseria* atténuées qui peuvent surexprimer un acide nucléique de invention ou l'exprimer sous une forme non toxique, mutée.

La présente invention fournit également (i) une cassette d'expression contenant un polynucléotide de l'invention placée sous le contrôle d'éléments permettant son expression, en particulier sous le contrôle d'un promoteur approprié ; (ii) un vecteur d'expression contenant ladite cassette d'expression ; (iii) une cellule hôte (procaryote ou eucaryote) transformée avec une cassette d'expression et/ou un vecteur d'expression tels que définis ci dessus, ainsi que (iv) une méthode pour obtenir un polypeptide codé par ledit polynucléotide de l'invention, comprenant la culture de ladite cellule transformée, dans des conditions permettant l'expression du polynucléotide de l'invention, et la récupération du polypeptide de la culture cellulaire.

Parmi les hôtes eucaryotes pouvant être utilisés, on peut notamment citer les cellules de levure (par exemple, *Saccharomyces cerevisiae* ou *Pichia Pastoris),* les cellules de mammifères (par exemple, COS1, NIH3T3, ou JEG3), des cellules d'arthropodes (par exemple, *Spodoptera frugiperda* (SF9)), et des cellules végétales. Parmi les hôtes procaryotes pouvant être utilisés, on peut notamment citer *E. coli*.

Le choix de la cassette d'expression dépend du système hôte choisi ainsi que des caractéristiques désirées pour le polypeptide exprimé. De manière générale, les cassettes d'expression incluent un promoteur qui est fonctionnel dans le système hôte sélectionné et qui peut être constitutif ou inductible ; un site de liaison au ribosome ; un codon d'initiation (ATG) ; si nécessaire une région codant pour un peptide signal ; une séquence nucléotidique de l'invention ; un codon stop , éventuellement une région 3' terminale (terminateur de traduction et/ou de transcription). Le cadre de lecture ouverte ("open reading frame ORF") constitué par la séquence nucléotidique de l'invention, seule ou associée à la région codant pour le peptide signal, est placé sous le contrôle du promoteur de telle sorte que la traduction et la transcription aient lieu dans le système hôte. Les promoteurs et les régions codant pour les peptides signal sont connus de l'homme du métier. Parmi ceux-ci, on peut notamment citer le promoteur de *Salmonella typhimurium* inductible par l'arabinose (promoteur araB) et qui est fonctionnel dans les bactéries Gram⁻ tel que *E. coli* (US 5,028,530 et Cagnon et al., Protein Engineering (1991) 4(7) : 843), le promoteur du gène du bactériophage T7 codant pour l'ARN polymérase, (US 4,952.496) ; le peptide signal OspA et RlpB (Takase et al, J. Bact. (1987) 169:5692).

Le polypeptide exprimé peut être recueilli sous une forme pratiquement purifiée à partir de l'extrait cellulaire ou du surnageant après centrifugation de la culture de cellules recombinantes. Le polypeptide recombinant peut être notamment purifié par des méthodes de purification par affinité à l'aide d'anticorps ou par toute autre méthode connue de l'homme du métier, telle que par fusion génétique avec un petit domaine de liaison.

Les acides nucléiques de l'invention peuvent également être utiles dans le domaine de la vaccination, soit en utilisant un hôte viral ou bactérien comme véhicule de libération de l'ADN, soit en administrant l'acide nucléique d'intérêt sous une forme libre.

La présente invention fournit également (i) un vecteur vaccinal contenant un acide nucléique de l'invention, placé sous le contrôle d'éléments permettant son expression ; (ii) une composition pharmaceutique contenant une quantité thérapeutiquement ou prophylactiquement efficace dudit vecteur vaccinal ; (iii) une méthode pour induire une réponse immune contre *Neisseria-* chez un vertébré, en particulier un mammifère, de préférence un humain, ladite méthode comprenant l'administration audit vertébré d'une quantité immunologiquement efficace dudit vecteur vaccinal pour provoquer une réponse immune, en particulier une réponse protectrice ou thérapeutique à *Neisseria meningitidis ;* (iv) une méthode pour prévenir et/ou traiter une infection par les souches pathogènes de *Neisseria,* en particulier par *Neisseria meningitidis,* qui comprend administration d'une quantité prophylactique ou thérapeutique dudit vecteur vaccinal de l'invention à un individu nécessitant un tel traitement.

En association avec les polypeptides de l'invention, le vecteur vaccinal tel que défini ci-dessus peut également comprendre des séquences nucléotidiques dont l'expression permet la stimulation de la réponse immune, telles que les séquences codant pour des cytokines.

Ledit vecteur vaccinal de l'invention peut être administré par n'importe quelle voie conventionnelle dans le domaine de la vaccination, en particulier par la voie parentérale (par exemple, sous-cutanée, intra-dermique, intra-musculaire, intraveineuse ou intra-péritonéale). Le dosage dépend de nombreux paramètres connus de l'homme du métier, tels que le vecteur lui-même, la voie d'administration, le poids, l'âge ou le sexe de l'animal ou de l'homme à vacciner.

La présente invention fournit également (i) une composition pharmaceutique contenant une quantité thérapeutiquement ou prophylactiquement efficace d'un polynucléotide de l'invention ; (ii) une méthode pour induire une réponse immune contre les souches pathogènes de *Neisseria,* en particulier par *Neisseria meningitidis* chez un vertébré par administration audit vertébré d'une quantité immunologiquement efficace dudit polynucléotide pour provoquer une réponse immune, en particulier une réponse immune protectrice envers les souches pathogènes de *Neisseria,* en particulier par *Neisseria meningitidis ;* et (iii) une méthode de prévention et de traitement à une infection par les souches pathogènes de *Neisseria,* en particulier par *Neisseria meningitidis,* par administration d'une quantité thérapeutique ou prophylactique dudit polynucléotide à un individu nécessitant un tel traitement.

Les polynucléotides de l'invention (ADN ou ARN) peuvent être administrés en tant que tels à un vertébré. Lorsqu'une molécule d'ADN de l'invention est utilisée, elle peut être sous la forme d'un plasmide incapable de se répliquer dans une cellule de vertébré et incapable d'intégrer le génome dudit vertébré. Ladite molécule d'ADN est typiquement placée sous le contrôle d'un promoteur adapté pour l'expression dans une cellule de vertébré. Ledit polynucléotide utilisé comme vaccin peut être formulé selon diverses méthodes connues de l'homme du métier. Ledit polynucléotide peut en particulier être utilisé sous une forme nue, exempt de tout véhicule favorisant le transfert à la cellule cible, tels que des liposomes anioniques, des lipides cationiques, des microparticules, par exemple des microparticules d'or, des agents de précipitation, par exemple du phosphate de calcium, ou tout autre agent facilitant la transfection. Dans ce cas, le polynucléotide peut être simplement dilué dans une solution physiologiquement acceptable, telle qu'une solution stérile ou une solution stérile tampon, en présence ou en l'absence d'un véhicule. Lorsqu'il est présent, ce véhicule peut être de préférence isotonique, hypotonique, ou faiblement hypertonique, et a une force ionique relativement faible. Il peut par exemple s'agir d'une solution de saccharose (par exemple une solution contenant 20 % de saccharose).

De manière alternative, un polynucléotide de l'invention peut être associé à des agents qui facilitent la transfection. Il peut être, entre autres , (i) associé à un agent chimique qui modifie la perméabilité cellulaire tel que la bupivacaïne (WO 94/16737) ; (ii) encapsulé dans des liposomes, éventuellement en présence de substances supplémentaires facilitant la transfection (WO 93/18759, WO 93119768, WO 94/25608 et WO 95/2397, WO 93/18759 et WO 93/19768) ; ou (iii) associé à des lipides cationiques ou des microparticules de silice, d'or ou de tungstène.

Lorsque les polynucléotides de l'invention recouvrent des microparticules, celles-ci peuvent être injectées par voie intradermique ou intraépidermique par la technique du canon à gènes, "gene gun" (US 4,945,050, U.S. No. 5,015,580 et WO 94/24263).

La quantité d'ADN à utiliser comme vaccin dépend notamment de la force du promoteur utilisé dans la construction de l'ADN, de l'immunogénicité du produit exprimé, de l'individu auquel cet ADN est administré, du mode d'administration et du type de formulation. De manière générale, une quantité thérapeutiquement ou prophylactiquement efficace variant d'environ 1 µg à environ 1 mg, de préférence d'environ 10 µg à environ 800 µg et, de manière préférentielle d'environ 25 µg à environ 250 µg, peut être administrée à des adultes humains.

Le polynucléotide de l'invention peut être administré par toute voie d'administration conventionnelle telle que notamment par voie parentérale. Le choix de la voie d'administration dépend en particulier de la formulation choisie. Un polynucléotide formulé en association avec la bupivacaïne est avantageusement administré au muscle. Lorsque des liposomes neutres ou anioniques ou un lipide cationique tel que DOTMA (chlorure de N-[1-(2,3-dioléyloxy)propyl]-N,N,N-triméthylammonium) ou DC-Chol (3 beta-(N-(N',N'-dimethyl aminomethane)-carbamoyl) cholesterol), sont utilisés, la formulation peut être avantageusement injectée par voie intraveineuse, intramusculaire, intradermique ou sous-cutanée. Un polynucléotide sous une forme nue peut de manière avantageuse être administré par voie intramusculaire, intradermique ou sous-cutanée.

Les séquences nucléotidiques de l'invention permettent la construction de sondes et d'amorces nucléotidiques spécifiques utiles en diagnostic. Lesdites sondes ou amorces sont des acides nucléiques ayant des séquences identiques ou homologues à des portions des séquences du groupe l ou à leurs séquences complémentaires.

De manière préférentielle, lesdites sondes contiennent d'environ 5 à environ 100, de préférence d'environ 10 à environ 80 nucléotides. Elles peuvent contenir des bases modifiées, les résidus sucre et phosphate pouvant être également modifiés ou substitués. Les sondes de l'invention peuvent être utilisées dans des tests de diagnostic, pour capturer ou détecter des polynucléotides spécifiques des souches pathogènes de *Neisseria.* De telles sondes de capture peuvent être de manière conventionnelle immobilisées sur un support solide directement ou indirectement, par liaison covalente ou par adsorption passive. Une sonde de détection peut être marquée notamment par un isotope radioactif, une enzyme telle que la peroxidase ou la phosphatase alcaline, ou des enzymes capables d'hydrolyser un substrat chromogène, fluorogène ou luminescent, ou encore par des composés eux-mêmes homogènes, fluorogènes ou luminescents, des analogues nucléotidiques ; ou la biotine.

Une amorce contient généralement d'environ 10 à environ 40 nucléotides, et peut être utilisée pour initier une polymérisation enzymatique de l'ADN dans un processus d'amplification (par exemple la PCR), dans un processus d'élongation ou dans une méthode de transcription inverse. Une amorce de l'invention peut être notamment une amorce telle que décrite dans l'exemple II.1 ci-après.

La présente invention fournit également :
(i) un réactif contenant une sonde de l'invention pour la détection et/ou l'identification de la présence des souches pathogènes de *Neisseria* dans un échantillon biologique ;
(ii) un procédé de détection et/ou d'identification de la présence des souches pathogènes de *Neisseria* dans un échantillon biologique ; ladite méthode comprenant les étapes consistant à a) extraire l'ADN ou l'ARN d'un échantillon biologique et le dénaturer ; b) exposer ledit ADN ou ledit ARN à une sonde de l'invention, dans des conditions d'hybridation stringentes, de manière à détecter l'hybridation ; et
(iii) une méthode de détection et/ou d'identification des souches pathogènes de *Neisseria* dans un échantillon biologique dans lequel l'ADN est extrait d'un échantillon biologique et est mis en présence d'au moins une et de préférence de deux amorces de l'invention et est amplifié par exemple par PCR.

Comme mentionné précédemment, les polypeptides produits par l'expression des séquences ORF identifiées sont utiles comme agents vaccinaux. L'antigénicité spécifique des polypeptides homologues des polypeptides de séquences du groupe II peut être évaluée en testant la réactivité croisée avec un antisérum dirigé contre les polypeptides de séquences du groupe II. Un antisérum hyper-immun monospécifique peut être produit contre un polypeptide de séquence du groupe Il purifié ou un polypeptide de fusion, par exemple un produit d'expression des systèmes MBP, GST, ou His-tag.

L'antigénicité spécifique peut être déterminée en utilisant diverses méthodes connues de l'homme du métier, en particulier les techniques de Western-Blot, Dot-Blot, et ELISA, décrites ci-dessous.

Dans la technique de Western-Blot, la préparation protéique à tester est soumise à une électrophorèse sur gel SDS-PAGE. Après transfert sur une membrane de nitrocellulose, le matériel est incubé avec un antisérum hyper-immmun monospécifique obtenu après avoir immunisé un animal avec le matériel réfèrent : c'est-à-dire dans le cas présent, avec un polypeptide ayant une séquence d'acides aminés du groupe Il. Cet antisérum est au préalable dilué dans un domaine de dilution allant d'environ 1:50 à 1:5000, de préférence d'environ 1:100 à 1:500. L'antigénicité spécifique est révélée lorsqu'une bande correspondant au produit présente une réactivité à l'une des dilutions ci-dessus.

Dans le test ELISA, on utilisée de préférence une préparation protéique purifiée, bien qu'un extrait cellulaire entier puisse être également utilisé. Environ 100 µl d'une préparation à environ 10 µg/ml sont distribués dans les puits d'une plaque. La plaque est incubée pendant deux heures à 37°C puis une nuit à 4°C. La plaque est ensuite lavée avec une solution saline de tampon phosphate (PBS) comprenant 0,05 % de Tween 20. Les puits sont saturés avec 250 µl de PBS contenant 1 % d'albumine de sérum bovin (BSA) pour empêcher la liaison non spécifique d'anticorps. Après une heure d'incubation à 37°C, la plaque est lavée avec le tampon PBS/Tween. L'antisérum est dilué en série dans du tampon PBS/Tween contenant 0,5 % BSA. 100 µl de cette dilutions sont ajoutés par puits. La plaque est incubée pendant 90 minutes à 37°C, lavée et évaluée selon les procédures standard. Par exemple, lorsque des anticorps spécifiques sont produits chez des lapins, un conjugué peroxydase de chèvre anti-lapin est ajouté au puits. L'incubation est réalisée pendant 90 minutes à 37°C et la plaque est ensuite lavée. La réaction est mesurée par colorimétrie (une réaction est positive lorsque la valeur de densité optique est de 1 si la dilution est d'au moins 1:50, de préférence d'au moins 1:500).

Dans le test Dot-Blot, on utilise de préférence une protéine purifiée, étant entendu qu'il est également possible d'utiliser un extrait cellulaire entier. Une solution protéique à environ 100 µg/ml est diluée deux fois en série dans un tampon Tris-HCl 50mM, pH : 7,5. 100 µl de chaque dilution sont appliqués à une membrane de nitrocellulose (appareil BioRad). Le tampon est enlevé en appliquant du vide au système. Les puits sont lavés par addition de 50 mM de tampon Tris-HCl (pH : 7,5) et la membrane est séchée à l'air. La membrane est ensuite saturée dans un tampon bloquant (50 mM Tris-HCl (pH : 7,5) 0,15M NaCl, 10 g/l de lait écrémé) et incubée avec une dilution d'antisérum allant d'environ 1:50 à 1:5000, de préférence à environ 1:500. La réaction est révélée conformément aux procédures standard. Par exemple, lorsque des anticorps spécifiques sont produits chez des lapins, un conjugué peroxydase de chèvre anti-lapin est ajouté aux puits. L'incubation est réalisée pendant 90 minutes à 37°C. La réaction est développée avec le substrat approprié et mesurée, par exemple par colorimétrie, par l'apparition d'une tache colorée (une réaction est positive lorsqu'une tache colorée apparaît en association avec une dilution d'au moins 1:50, de préférence d'au moins 1:500).

La présente invention fournit également (i) une composition pharmaceutique contenant une quantité thérapeutiquement ou prophylactiquement efficace d'un polypeptide de l'invention ; (ii) une méthode pour induire une réponse immune contre les souches pathogènes de *Neisseria* chez un vertébré, par administration audit vertébré d'une quantité immunogéniquement efficace d'un polypeptide de l'invention pour provoquer une réponse immune, en particulier une réponse immune protectrice contre les souches pathogènes de *Neisseria* ; et (iii) une méthode de prévention et/ou de traitement d'une infection par les souches pathogènes de *Neisseria,* par administration d'une quantité thérapeutique ou prophylactique d'un polypeptide de l'invention à un individu nécessitant un tel traitement.

Les compositions immunogènes de l'invention peuvent être administrées par n'importe quelle voie conventionnelle dans le domaine de la vaccination, en particulier par la voie parentérale (par exemple, sous-cutanée, intra-dermique, intra-musculaire, intraveineuse ou intra-péritonéale). Le choix de la voie d'administration dépend d'un certain nombre de paramètres tel que l'adjuvant associé au polypeptide.

Une composition de l'invention contient au moins un polypeptide tel que défini précédemment. Il peut également contenir au moins un antigène supplémentaire de *Neisseria meningitidis* et/ou *Neisseria gonorrhoeae.*

Les polypeptides de l'invention peuvent être formulés avec des liposomes, de préférence des liposomes neutres ou anioniques, des microsphères, des ISCOMS, des particules "virus-like" pour faciliter le transfert du polypeptide et/ou augmenter la réponse immune.

L'administration peut être réalisée par une dose unique ou par des doses répétées si nécessaire à des intervalles qui peuvent être déterminés par l'homme du métier.

Par exemple, une dose initiale peut être suivie de trois doses de rappel à des intervalles d'une ou plusieurs semaines ou d'un ou plusieurs mois. La dose appropriée dépend de nombreux paramètres incluant l'individu traité (adulte ou enfant), l'antigène vaccinal particulier, la voie d'administration et la fréquence d'administration, la présence ou l'absence ou encore le type d'adjuvant, et l'effet désiré (par exemple, protection et/ou traitement) et peut être déterminée par l'homme du métier. Si la voie d'administration est parentérale, la dose est préférentiellement inférieure à 1 mg, de préférence d'environ 100 µg. Les polypeptides et polynucléotides de l'invention utilisés en tant qu'agents vaccinaux, peuvent être utilisés de manière séquentielle, dans un procédé d'immunisation en plusieurs étapes. Par exemple, un vertébré peut être initialement sensibilisé avec un vecteur vaccinal de l'invention, tel qu'un poxvirus, par exemple par la voie parentérale, puis être stimulé deux fois avec le polypeptide codé par le vecteur vaccinal.

Un polypeptide de l'invention peut également être utile comme agent de diagnostic pour détecter la présence d'anticorps anti-*Neisseria meningitidis* et/ou anti-*Neisseria gonorrhoeae* dans un échantillon biologique tel qu'un échantillon de sang.

La présente invention fournit également des anticorps monospécifiques dirigés contre les polypeptides de l'invention.

Par "anticorps monospécifique" on entend un anticorps capable de réagir de manière spécifique avec un polypeptide de *Neisseria* de l'invention. De tels anticorps peuvent être polyclonaux ou monoclonaux, et peuvent être des anticorps recombinants par exemple chimériques (par exemple, constituées par une région variable d'origine murine associée à une région constante d'origine humaine), humanisés et/ou à chaîne unique. Lesdits anticorps peuvent être également sous la forme de fragments d'immunoglobuline par exemple de fragments F(ab)'2 ou Fab. Les anticorps de l'invention peuvent être de n'importe quel isotype par exemple IgA ou IgG, les anticorps polyclonaux pouvant être d'un isotype unique ou pouvant contenir un mélange de plusieurs isotypes.

Les anticorps de l'invention dirigés contre les polypeptides de l'invention, peuvent être produits et identifiés en utilisant des méthodes immunologiques standard, par exemple l'analyse de Western-Blot, un essai Dot-Blot, un test ELISA (Coligan et al, Current Protocols in Immunology (1994) John Wiley & Sons, Inc., New York, NY). Lesdits anticorps peuvent être utilisés dans des procédés de diagnostic pour détecter la présence d'un antigène de *Neisseria meningitidis* dans un échantillon tel que notamment un échantillon biologique (par exemple du sang).

Les anticorps de l'invention peuvent également être utilisés dans des procédés de chromatographie par affinité pour purifier un polypeptide de l'invention. Enfin, de tels anticorps peuvent également être utilisés dans des méthodes d'immunisation passive prophylactique ou thérapeutique.

La présente invention fournit également une méthode de diagnostic pour la détection de la présence de souches pathogènes de *Neisseria* dans un échantillon biologique comprenant la mise en contact dudit échantillon biologique avec un anticorps, ou un polypeptide de l'invention, de telle sorte qu'un complexe immun soit formé, et la détection dudit complexe indicatrice de souches pathogènes de *Neisseria* dans organisme dont provient l'échantillon. L'homme du métier comprend que le complexe immun est formé entre un composant de l'échantillon et l'anticorps ou le polypeptide de l'invention, toute substance non liée pouvant être éliminée préalablement à la détection du complexe.

Ainsi, un réactif de type polypeptide peut être utilisé pour la détection de la présence d'anticorps anti*-Neisseria meningitidis* et/ou *Neisseria gonorrhoeae* dans un échantillon, tandis qu'un anticorps de l'invention peut être utilisé comme réactif pour tester la présence de polypeptide de *Neisseria meningitidis* et/ou *Neisseria gonorrhoeae* dans un échantillon.

Pour une utilisation dans des applications de diagnostic, le réactif (par exemple l'anticorps ou le polypeptide de l'invention) peut être à l'état libre ou immobilisé sur un support solide, par des moyens directs ou indirects.

Les moyens directs incluent l'adsorption passive ou la liaison covalente entre le support et le réactif.

Par moyen indirect, on entend qu'une substance qui interagit avec ledit réactif est fixée au support solide. Par exemple, si un réactif de type polypeptide est utilisé, un anticorps qui se lie à celui-ci peut servir de substance anti-réactif, étant entendu que celle-ci se lie à un anticorps qui n'est pas impliqué dans la reconnaissance des anticorps dans les échantillons biologiques.

Parmi les moyens indirects pouvant être utilisés, on peut également citer le système ligand récepteur, une molécule telle qu'une vitamine pouvant être greffée sur le réactif de type polypeptide et le récepteur correspondant pouvant être immobilisé sur la phase solide. Ceci est illustré par le système biotine-streptavidine: On peut également ajouter au réactif une queue peptidique par génie chimique ou génie génétique, et immobiliser le produit greffé ou fusionné par adsorption passive ou liaison covalente avec la queue peptidique.

La présente invention fournit également un procédé de purification, à partir d'un échantillon biologique, d'un polypeptide de *Neisseria* de l'invention, par chromatographie d'affinité avec un anticorps monospécifique de l'invention. Ledit anticorps est de préférence d'isotype IgG.

Selon un exemple de réalisation, un échantillon biologique, de préférence dans une solution tampon, est appliqué à un matériel chromatographique, de préférence équilibré avec le tampon utilisé pour diluer l'échantillon biologique, de telle sorte que le polypeptide de l'invention (c'est-à-dire l'antigène) puisse adsorber sur le matériel. Les composants non liés sont lavés et l'antigène est ensuite élué avec un tampon d'élution approprié, tel qu'un tampon Glycine ou un tampon contenant un agent chaotropique, par exemple la guanidine HCl, ou une forte concentration en sel (par exemple, 3 M MgCl₂). Les fractions éluées sont recueillies et la présence d'antigène est détectée par exemple par mesure de l'absorbance à 280 nm.

La présente invention fournit également (i) une composition pharmaceutique contenant une quantité thérapeutiquement ou prophylactiquement efficace d'un anticorps monospécifique de l'invention et (ii) une méthode de prévention et/ou de traitement d'une infection par les souches pathogènes de *Neisseria,* par administration d'une quantité thérapeutique ou prophylactique d'un anticorps monospécifique de l'invention à un individu nécessitant un tel traitement.

A cette fin, l'anticorps monospécifique de l'invention est de préférence d'isotype IgG, et de préférence fixe le complément. Ledit anticorps monospécifique selon l'invention peut être administré seul ou en mélange avec au moins un autre anticorps monospécifique, spécifique d'un polypeptide de *Neisseria meningitidis* et/ou *Neisseria gonorrhoeae* différent, selon l'invention. La quantité d'anticorps peut être déterminée facilement par l'homme du métier. Par exemple, une administration quotidienne d'environ 100 à 1000 mg d'anticorps sur une semaine ou trois doses quotidiennes d'environ 100 à 1000 ring d'anticorps sur deux ou trois jours, peut être une posologie efficace.

L'efficacité thérapeutique ou prophylactique peut être évaluée en utilisant des méthodes standard connues de l'homme du métier, par exemple en mesurant l'induction d'une réponse immune ou l'induction d'une immunité protectrice et/ou thérapeutique (chez des souris ou des rats nouveau-nés); par évaluation de la charge bactérienne dans le liquide céphalo-rachidien). La protection peut être déterminée en comparant le degré d'infection de *Neisseria* à un groupe contrôle. La protection est mise en évidence lorsque l'infection est réduite par comparaison au groupe contrôle. Une telle évaluation peut être faite avec les polynucléotides, les vecteurs vaccinaux, les polypeptides ainsi que les anticorps selon l'invention. L'efficacité thérapeutique ou prophylactique d'un produit selon l'invention (polynucléotide ou polypeptide) peut être aussi évaluée en test de bactéricidie tel que décrit par Danve et al., Vaccine (1993) 11 (12) :1214, à l'encontre de la souche méningoccoque d'origine du polynucléotide' ou polypeptide utilisé. Dans le domaine des vaccins méningoccoques, le test de bactéricidie est en effet reconnu comme étant le test de référence à partir duquel on peut valablement prédire l'intérêt vaccinal d'un produit. En bref, on administre un produit selon l'invention à des animaux tels que le lapin afin d'obtenir un antisérum à l'encontre de ce produit. Puis cet antisérum est testé pour sa capacité de lyse. Le titre de bactéricidie d'un antisérum représente l'inverse de la dilution de cet antisérum pour lequel 50 % de la charge en méningoccoques est lysée. On considère que l'antisérum est bactéricide lorsque le titre est supérieur à 4 vis à vis de la souche méningoccoque d'origine du polynucléotide ou polypeptide utilisé. Dans ce cas-là, il est démontré que le produit à l'encontre duquel l'antisérum a été généré, est potentiellement intéressant d'un point de vue pharmaceutique.

Les exemples suivant illustrent l'invention sans en limiter la portée.

### EXEMPLES

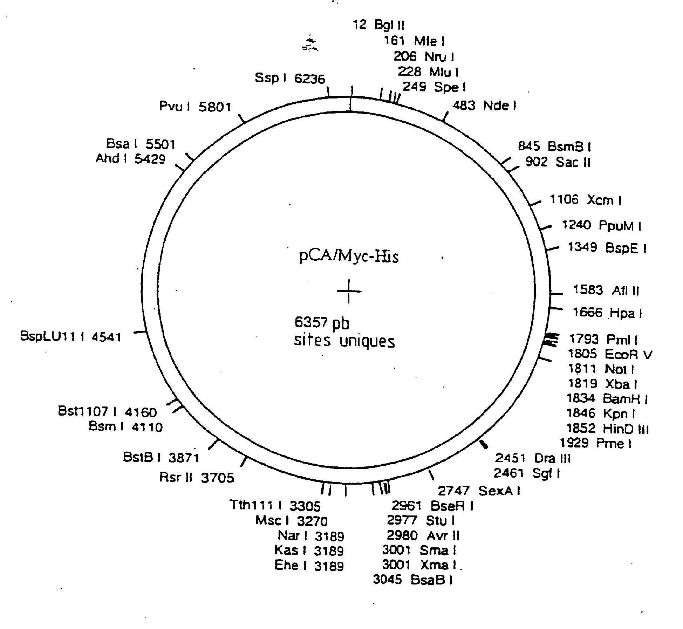

### Légende de la figure

La figure en annexe représente le vecteur pCAMyc-His utilisé comme vecteur de clonage.

### Détails de la stratégie d'identification des ORF :

Afin de sélectionner les séquences ORF spécifiques des souches pathogènes du genre *Neisseria,* une amplification par PCR est menée sur les séquences des 118 ORFs retenues après analyse par le logiciel Gene Jocke ^{®}, Codon Use^{®}, et la recherche d'homologies. Seules les séquences pour lesquelles l'amplification chez *N. lactamica* est négative (séquences appelées " lactamica⁻ ") sont retenues. Afin de vérifier que ces résultats négatifs ne sont pas dés " faux-négatifs ", les séquences lactamica⁻ retenues sont soumises à un dot blot.

### A - Amplification par PCR :

### A.1. Extraction des ADN génomique

Les ADN génomiques de l'ensemble des souches de Neisseria utilisées dans cette étude ont été préparés selon un protocole identique. Les souches de *N. meningitidis, N. lactamica, N. flava, N. subflava* et *N. mucosa* ont été cultivées sur boite de milieu MHA (Muller Hinton Agar, Difco), les *N. gonorrhoeae* ont été cultivées sur boite de milieu MHA supplémenté par 10% de sang cuit de cheval (Biomérieux) et 1% d'Isovitalex (Biomérieux). La culture se fait à 37°C pendant 18 h sous une atmosphère contenant 10% CO2 une nuit à 37°C. Puis les cellules sont récoltées, lavées dans du tampon phosphate PBS (pH 7.2) et l'ADN est extrait selon le protocole D du Kit " Rapid Prep genomic DNA isolation kit for cells and tissue " (Pharmacia Biotech).

Les ADN génomiques ont alors été contrôlés sur gel d'agarose pour leur intégrité et par réaction PCR pour leur pureté.

### A.2 Réaction de PCR pour cribler les ORFs absentes dans N. lactamica 2314 :

Une amplification PCR a été réalisée sur les ADN génomiques de la souche *N. meningitidis* ATCC 13090 et *N. lactamica* 2314 (ATCC 23970) selon le protocole suivant :

La réaction de PCR a été réalisée sur un volume de 50 µl avec 10 ng d'ADN génomique, 250 µM de chacun des dNTPs, 300 nM de chacune des amorces, Tampon Taq DNA polymerase 1X, et 2 u de Taq DNA Polymerase (Appligène).

Les cycles d'amplification sont :

| | | |
|---|---|---|
| 97°C | 45 secondes | 25 cycles |
| 56°C | 1 minute | 25 cycles |
| 72°C | 2.30 minutes | 25 cycles |

Pour chacune des ORFs analysées, des contrôles positifs et négatifs de la réaction PCR ont été réalisés. A ce stade, seules les ORFs *N. meningitidis* + et *N. lactamica* -sont retenues.

### B - Sélection des ORFs N. meningitidis⁺ N. lactamica⁻ par dot blot sur ADN génomique :

L'absence d'un produit d'amplification par PCR d'une ORF avec de l'ADN génomique de *N. lactamica* 2314 comme matrice, ne certifie pas l'absence de cette ORF dans le génome de *N. lactamica* 2314. En effet, une certaine variabilité dans la région où devraient s'hybrider les oligonucléotides peut être responsable de l'absence de produit amplifié pour une ORF donnée.

Dans ce contexte, une vérification supplémentaire est mise en oeuvre par dot blot sur ADN génomique en utilisant comme sonde les produits d'amplification génomique sur la souche de *N. meningitidis* correspondant à chacune des phases de lecture identifiées. Les filtres du dot blot contiennent de l'ADN génomique des souches suivantes : 2 souches de *N. lactamica* 8064, 2314, une souche de *N. flava* ATCC 30008, une souche de *N. mucosa* ATCC 9297, 3 souches de *N. meningitidis* de sérogroupe B ATCC13090, M982, B16B6, une souche de *N. meningitidis* de sérogroupe A Z2491, une souche de *N. meningitidis* de sérogroupe C (souche Z4182). 2 souches de *N. gonorrhoeae* MS11 et FA1090. Cette analyse en dot blot permet de valider l'absence de l'ORF dans *N. lactamica* 2314 et 8064 et elle est aussi indicatrice du degré de variabilité d'une ORF au sein des souches de *Neisseria.*

La technique de dot blot utilisée est la suivante. Environ 50ng d'ADN génomiques dénaturé 5 min à 100°C des différentes souches de *Neisseria* sont déposés sous vide sur une membrane de nitrocellulose Hybond N+ (Amersham) placée entre les machoires d'un appareil à dot blot (Bio-Rad). Puis l'ADN est fixé sur les membranes 5 min à un rayonnement UV 315nm.

Les membranes sont incubées dans un tampon de pré-hybridation (contenant de l'ADN de sperme de saumon dénaturé). Elles sont ensuite hybridées avec une sonde correspondant au produit d'amplification de l'ORF d'intérêt, marquée selon un protocole de marquage froid, comme le système 'DIG DNA labelling and détection kit' (Boehringer Mannheim).

L'ORF qui ne s'hybride pas sur l'ADN génomique de *N. lactamica* 2314 et 8064 est retenue définitivement comme candidat vaccinal potentiel.

### Exemple I : Clonage

### 1. Amplification par PCR

Chacune des ORFs a été amplifiée par PCR à partir de l'ADN génomique de *N. meningitidis* sérogroupe B (souche ATCC 13090), selon un protocole standard.

Deux oligonucléotides, amorces du côté N-terminal et du côté C-Terminal ont été définis pour chacune des séquences ORF de l'invention.

L'amorce du côté N-Terminal comprend un site de restriction enzymatique pour le clonage, une séquence Kozak CCACC pour l'initiation de la traduction (M. Kozak,J. Mol. Biol. 196 : 947-950), l'ATG de l'ORF potentielle et environ 17 bases spécifiques de la partie 5' de l'ORF.

L'amorce du côté C-Terminal a été définie de manière à ce que l'ORF clonée soit en fusion dans sa partie 3' avec une répétition de 8 histidines et un codon stop présents dans le vecteur derrière le site multiple de clonage, d'où l'insertion d'une base " A " pour garder la bonne phase de lecture après le colonage et la disparition du codon stop de l'ORF. L'amorce du côté C-Terminal comprend ainsi un site de restriction enzymatique pour le clonage, une base " A ", puis environ 20 bases spécifiques de la partie 3' du gène à partir du codon précédent le codon stop.

Après recherche des sites de restriction absents chez chacune des ORFs à l'aide du sous-programme MapDraw de DNASTAR (Lasergene Software), les sites de restriction Xbal en 5' et BglII en 3' sont utilisés pour l'ORF SEQ ID n°19. Pour l'ORF SEQ ID n°41, les sites SpeI en 5' et BgIII en 3' sont utilisés. Les sites de restriction Xbal en 5' et BamHI en 3' sont utilisés pour cloner les ORFs restantes.

Le mélange de PCR comprend pour un volume final de 100 µl, 10-50 ng ADN génomique, les amorces N-Terminale et C-Terminale à 200 nM chacun, les dNTPs à 250 µM chacun, le tampon PCR 1X (Composition du tampon PCR 10X : 200 mM Tris-HCl (pH8.8), 20 mM MgS04, 100 mM KCl, 100 mM (NH4)2SO4, 1% TritonX-100, 1mg/ml de sérumalbumine de boeuf exempte de nucléase) et 2.5 U de Polymerase.

L'amplification se fait comme suit :

| Etape | Température (°C) | Temps (min.) | Nombre de cycles |
|---|---|---|---|
| Dénaturation | 97 | 0.45 | 25 |
| Hybridation | cf tableau | 1 | 25 |
| Elongation | 72 | 1/kb ADN | 25 |

Les amorces utilisées et les conditions de PCR présentées dans le tableau ci-dessous, dans lequel "variant allélique N. g" signifie qu'un variant allélique est présent chez *Neisseria gonorrhoeae* et "variant allélique N. m A" signifie qu'un variant allélique est présent chez *Neisseria meningitidis* de sérogroupe A.

| **N° ORF (réf. interne)** | **SEQ ID n°** | **Amorce 5'** | **Amorce 3'** | **Polymérase** | **T° Hybridation** |
|---|---|---|---|---|---|
| 22 | 1-2 variant allélique N.g : 54, 55 | | | Tfu (Appligene) | 56°C |
| 41 | 3-4 | | | Tfu (Appligene) | 43°C |
| 42-43 | 5-6 variant allélique N.g. : 56, 57 | | | Tfu (Appligene) | 56°C |
| 47 | 7-8 variant allélique N.g. : 58, 59 | | | Tfu (Appligene) | 56°C |
| 55 | 9-10 variant allélique N.g. : 60, 61 | | | Pfu Turbo (Stratagene) | 56°C |
| 68 | 11-12 | | | Tfu (Appligene) | 56°C |
| 71 | 13-14 | | | Tfu (Appligene) | 56°C |
| 72 | 15-16 variant allélique N.mA. : 62,63 | | CGG GAT CCA TGC CGC TTG GGG GAG GC (SEQ ID n° 93) | Pfu Turbo (Stratagene) | 56°C |
| 73 | 17-18 variant allélique N.g : 64, 65 | | CGG GAT CCA CAG TTT GCC CGA CAT AC (SEQ ID n° 95) | Pfu Turbo (Stratagene) | 56°C |
| 74 | 19-20 | | | Pfu Turbo (Stralagene) | 56°C |
| 98 | 21-22 | | | Pfu Turbo (Stratagene) | 56°C |
| 116 | 23-24 variant allélique N.g : 66, 67 | | | Pfu Turbo (Shatagene) | 56°C |
| 122 | 25-26 | | | Pru Turbo (Stratagene) | 56°C |
| 125 | 27-28 | | | Pfu turbo (Stratagene) | 56° C |
| 128 | 29.30 variant allélique N.m A : 68, 69 | | | Tfu (Appligene) | 56° C |
| 152 | 31.32 variant allélique N.g:70,71 | | | Pfu turbo (Stratagene) | 56° C |
| 153 | 33-34 | | | Tfu (Appligene) | 56° C |
| 155 | 35-36 | | | Tfu (Appligene) | 56° C |
| 156 | 37-38 | | | Tfu (Appligene) | 56° C |
| 157 | 39-40 | | | Tfu (Appligene) | 56° C |
| 158 | 41-42 variant allélique N.mA : 72, 73 | | | Tfu (Appligene) | 56° C |
| 159 | 43-44 variant allélique N.mA :74, 75 | | | Tfu (Appligene) | 56° C |
| 161 | 45-46 | | | Tfu (Appligene) | 56° C |
| 163-1 | 47-48 | | | Pfu Turbo (Shatagene) | 56° C |
| 163-2 | 49-50 | | | Tfu (Appligene) | 56° C |
| 167-168 | 51-52 variant allélique N. g : 76. 77 | | | Tfu (Appligene) | 56° C |

### 2 - Clonage, transformation et sélection des recombinants

Le vecteur de clonage utilisé est le vecteur pCA/Myc-His ou pM1070 de 6.357 kb (cf figure), issu du plasmide pCDNA 3.1 (Invitrogen). Le pCA/Myc-His comprend notamment le promoteur ie1 de CMV (bases 249-902), l'intron A du gène ie1 de CMV (Chapman et al., 1991 Nucleic Acids Research, 19, 3979-3986), un site multiple de clonage (bases 1792-1852) avec les sites Pmll, EcoRV, Notl, Xbal, BamHI, KpnI et HindIII, une séquence codant pour une polyhistidine et un codon stop (bases 1908-1928), une séquence de terminaison 3'bgh (bases 1853-2197) et le gène de résistance à l'ampicilline pour la sélection des clones recombinants dans *E. coli.*

Après purification (Kit GeneClean Bio101), les produits d'amplification PCR sont digérés 2 heures à 37°C par les enzymes adéquates (XbaI-BamHI, XbaI-BgIII, ou SpeI-BgIII) dans un volume réactionnel final de 20 µl. Les produits de digestion sont alors ligués avec le vecteur pCA/Myc-His préalablement digéré par XbaI et BamHI selon le protocole de "Rapid DNA Ligation Kit" (Boehringer Mannheim). 15 µl de la ligature est utilisé pour transformer 100 µl de cellules compétentes *E. coli* XLI-blue (Novagen). Les cellules sont incubées 30 minutes dans la glace, 30 secondes à 42°C et 2 minutes dans la glace. Puis on ajoute 500 µl de milieu LB sans antibiotique et on incube 1 heure à 37°C. Ensuite 50 et 550 µl de la culture sont étalés sur des boites de milieu LB plus ampicilline (50 µg/ml concentration finale) et incubés une nuit à 37°C.

Le lendemain 36 colonies sont mises en culture dans 2 ml de LB plus ampicilline (50 µg/ml) et incubées une nuit à 37°C.

Le lendemain l'ADN plasmidique est extrait selon le protocole Qiagen mini-prep (Qiagen) et les recombinants sont identifiés par restriction enzymatique suivi d'une électrophorèse sur gel d'agarose. Les jonctions de clonage sont ensuite vérifiées par séquençage.

### Exemple II : Evaluation de l'activité protectrice des ORF de l'invention

### A. Préparation de l'ADN -destiné aux expériences d'immunisations :

Une colonie isolée d'un clone recombinant est utilisée pour inoculer une préculture en milieu LB+ ampicilline et 5 ml de cette préculture représente l'inoculat d'une culture de 2,5 litres en milieu LB+ ampicilline. Le protocole de purification pour préparer l'ADN plasmidique est celui décrit dans le Kit EndoFree Giga (Qiagen). L'ADN purifié est élué de la colonne de purification avec un tampon Tris-HCl 10 mM EDTA 1 mM pH 8 et conservé à - 20°C. Avant l'injection, le plasmide recombinant purifié est dilué à raison de 100 µg/ml avec de l'eau (de qualité pour préparation injectable) et la concentration en NaCl est amenée à 150 mM.

### B. Production d'un sérum polyclonal spécifique :

### B. 1. Hyperimunisation dans un modèle animal :

Le modèle animal utilisé est la souris ou le lapin. La voie d'administration de l'ADN injecté est la voie intramusculaire ou la voie intradermique. Les plasmides recombinants à injecter sont éventuellement appliqués sur des billes s'ils sont injectés chez l'animal avec un appareil gene gun (BioRad). Le protocole d'immunisation suit un schéma comportant deux injections à 3 semaines d'intervalles.

### B.2. Analyse de l'activité bactéricide des anticorps induits :

Dix jours après la dernière injection, les animaux sont saignés et les sera sont analysés avec le test de bactéricidie selon le protocole de Danve et al., Vaccine (1993) 11 (12) :1214. Brièvement, les sera sont incubés à différentes dilutions (raison 2) en présence de complément de lapin et de méningococoques cultivés en présence ou en absence d'un agent chélatant du fer. Le titre de bactéricidie d'un sérum représente l'inverse de la dilution de cet antisérum pour lequel 50% des bactéries sont lysées.

On considère que l'antisérum n'est pas bactéricide lorsque son titre est inférieur à 4 contre la souche homologue.

Quand le titre de bactéricidie correspond à une séroconversion d'un facteur 4 contre la souche homologue, l'activité bactéricide de l'antisérum est analysée vis-à-vis des autres souches de Neisseria pour mesurer l'étendue de la réactivité croisée de l'antisérum d'intérêt.

### Exempte III : Production de protéines recombinantes purifiées

### 1. Production recombinante de protéines

### a. Préparation des transformants :

Le produit de PCR obtenu est ensuite digéré à 37°C pendant deux heures avec des enzymes de restriction dans 20 µl de volume réactionnel. Le produit de digestion est ligaturé dans un plasmide pET28a (Novagen), clivé de manière similaire, qui est déphosphorylé avant la ligature par traitement avec la phosphatase alcaline intestinale de veau. Le gène de fusion construit de cette manière permet la purification par affinité en une étape de la protéine de fusion résultante en raison de la présence de résidus histidine à l'extrémité N-terminale de la protéine de fusion qui sont codés par ce vecteur.

La réaction de ligature (20 µl) est effectuée à 14°C une nuit, avant transformation de 100 µl de cellules compétentes fraîches *E. coli* XL1-blue (Novagen). Les cellules sont incubées sur de la glace pendant deux heures puis soumises à un choc thermique à 42°C pendant 30 secondes avant d'être remises dans la glace pendant 90 secondes. Les échantillons sont ensuite ajoutés à 1 ml de bouillon LB en l'absence de sélection et cultivés à 37°C pendant deux heures. Les cellules sont ensuite étalées sur du milieu gélosé LB additionné de kanamycine (50 µg/ml de concentration finale) à une dilution 10x, et sont incubées une nuit à 37°C. Le jour suivant, 50 colonies sont repiquées sur des boîtes secondaires et sont incubées à 37°C une nuit.

### b. Production de la protéine :

Les transformants stockés (10 µl) sont étalés sur des boîtes de sélection et cultivés une nuit à 37°C. Quelques cellules sont recueillies à partir de la boite utilisée comme inoculum pour une culture starter une nuit (3 ml) à 37°C. Le jour suivant, un échantillon (temps T=0) est recueilli et centrifugé à 14 000 tpm pendant 3 minutes. La culture starter est ensuite utilisée pour inoculer un milieu LB contenant de la kanamycine (100 µg/ml) à une dilution de 1:50 (densité optique de départ DO₆₀₀ = 0,05-0,1). Les cellules sont cultivées à une DO₆₀₀ de 1,0, un échantillon est recueilli pour une SDS-PAGE (échantillon de pré-induction) et la culture restante est induite avec 1 mM d'IPTG. Les cultures sont cultivées pendant quatre heures et des échantillons sont prélevés toutes les heures. La culture est centrifugée à 600 x g pendant 20 minutes à 4°C. Le surnageant est écarté et les culots sont resuspendus dans 50 mM de Tris-HCl (pH : 8,0), 2 mM EDTA, et recentrifugé. Le surnageant est écarté et les cellules sont stockées à -70°C.

### 2. Purification des protéines

Les culots obtenus à partir d'un litre de culture préparé selon l'exemple 1.4 précédent sont séchés et resuspendus dans 20 ml de 20 mM Tris-HCl (pH 8.0), 0.5 M NaCl, 5 mM Imidazole, refroidis dans la glace. Du lysozyme est ajoutée à une concentration de 0,1 mg/ml et la suspension est homogénéisée en utilisant un homogénéiseur à haute vitesse (Turrax) puis traitée avec un sonicateur (Branson, Sonofier 450). De la Benzonase (Merck) est utilisée à une concentration finale de 1 U/ml pour éliminer l'ADN. La suspension est centrifugée à 40 000 x g pendant 20 minutes et le surnageant est filtré à travers une membrane de 0,45 µm. Le surnageant est chargé sur une colonne IMAC (12 ml de résine) qui a été préparée en immobilisant des cations Ni⁺⁺ selon les recommandations du fabricant (Pharmacia). La colonne est lavée avec 10 volumes de colonnes de 20 mM Tris-HCl (pH 8.0), 0.5 M NaCl, 60 mM imidazole. La protéine recombinante est éluée avec six volumes de 20 mM Tris-HCl (pH : 7.9), 0.5 M NaCl, 500 mM Imidazole, 0.1% Zwittergent 3-14.

Le profil d'élution est contrôlé en mesurant l'absorbance des fractions à une densité optique de 280 nm. Une fraction aliquote est analysée sur un gel SDS-PAGE et colorée avec du bleu de Coomassie (Phast System - Pharmacia), et les fractions correspondant au pic de protéine sont ensuite regroupées et concentrées. Pour éliminer le tampon d'élution, la fraction est passée sur une colonne G24 Sephadex (Pharmacia), équilibrée dans du tampon PBS (pH : 7.4).La solution protéique est stérilisée par filtration à travers une membrane de 0,45 µm, et la concentration protéique est déterminée par la microméthode BCA (Pierce). La solution protéique est stockée à -70°C.

### Exemple IV : Production d'anticorps polyclonaux monospécifiques

### 1. Antisérum hyperimmun de lapin

On injecte à des lapins de Nouvelle Zélande à la fois par voie cutanée et par voie intraveineuse 100 µg (au total) du polypeptide purifié à l'exemple III, en présence d'adjuvant complet de Freund dans un volume total d'approximativement 2 ml. 21 et 42 jours après l'injection initiale, les doses de rappel qui sont identiques aux doses initiales, sont administrées de la même manière, à l'exception du fait que de l'adjuvant incomplet de Freund est utilisé. 15 jours après la dernière injection, le sérum de l'animal est recueilli, décomplémenté, et filtré à travers une membrane de 0,45 µm.

### 2. Liquide d'ascites hyperimmun de souris

On injecte à 10 souris par voie sous-cutanée 10-50 µg du polypeptide de fusion purifié obtenu à l'exemple II, en présence d'adjuvant complet de Freund, dans un volume d'approximativement 200 µl. 7 et 14 jours après l'injection initiale, des doses de rappel, qui sont identiques aux doses initiales, sont administrées de la même manière, à l'exception du fait que de l'adjuvant incomplet de Freund est utilisé. 21 et 28 jours après l'injection initiale, les souris reçoivent 50 µg de l'antigène seul par voie intrapéritonéale. Au 21^{ème} jour, on injecte également aux souris par voie intrapéritonéale, les cellules 180/TG CM26684 de sarcome (Lennette & Schmidt, Diagnostic procedures for viral, rickettsial, and chlamydial infections, (1979) 5th Ed. Washington DC, American Public Health Association). Les liquides d'ascites sont recueillis 10 à 13 jours après la première injection.

### Exemple V : Purification des polypeptides de l'invention par immunoaffinité

### 1. Purification d'IgG spécifique

Un sérum immun tel que préparé à l'exemple IV est appliqué à une colonne de protéine A Sépharose 4 Fast Flow (Pharmacia) équilibrée avec 100 mM Tris-HCl (pH: 8.0). La résine est lavée en appliquant 10 volumes de colonnes de 100 mM Tris-HCl et 10 volumes de 10 mM Tris-HCl (pH : 8,0) à la colonne. Les IgG sont éluées avec un tampon glycine de 0,1 M (pH : 3,0) et sont recueillies par fraction de 5 ml auxquelles on ajoute 0,25 ml de Tris-HCl 1 M (pH : 8,0). La densité optique de l'éluat est mesurée à 280 nm et les fractions contenant les IgG sont regroupées et si nécessaire stockées à -70°C.

### 2. Préparation de la colonne

Une quantité appropriée de gel Sépharose 4B activé par CNBr (1 g de gel séché fournissant approximativement 3,5 ml de gel hydraté, et la capacité du gel allant de 5 à 10 mg d'IgG couplée par ml de gel) fabriqué par Pharmacia (17-0430-01) et suspendue dans un tampon HCl de 1. mM et lavée avec un buchner par addition de petites quantités de tampon HCl 1 mM. Le volume total du tampon est de 200 ml par gramme de gel.

Les IgG purifiées sont dialysées pendant quatre heures à 20±5°C contre 5 volumes de tampon PBS de 500 mM (pH : 7,5). Puis elles sont diluées dans 500 mM de PBS (pH : 7,5) pour une concentration finale de 3 mg/ml.

Les IgG sont incubées avec le gel une nuit à 5±3°C, sous agitation. Le gel est tassé dans une colonne de chromatographie et lavé avec 2 volumes de colonne de tampon phosphate, 500 mM (pH : 7,5) puis un volume de tampon sodium 50 mM NaCl (pH : 7,5). Le gel est ensuite transféré dans un tube puis incubé avec 100 mM d'éthanolamine, (pH : 7,5) pendant 4 heures à température ambiante sous agitation, puis lavé deux fois avec deux volumes de colonnes de PBS. Le gel est ensuite stocké dans du PBS merthiolate à 1/10 000. La quantité d'IgG couplé au gel est déterminée en mesurant la densité optique à 280 nm de la solution d'IgG et de l'éluat direct.

### 3. Adsorption et élution de l'antigène.

Une solution d'antigène dans 50 mM Tris-HCl (pH : 8,0), 2 mM EDTA, par exemple, le surnageant obtenu à l'exemple III.5 après traitement par la Benzonase, centrifugation et filtration à travers une membrane de 0,45 µm, est appliquée à une colonne équilibrée avec 50 mM Tris-HCl (pH :8.0), 2 mM EDTA à une vitesse de flux d'environ 10 ml/heure. Puis, la colonne est lavée avec 20 volumes de 50 mM Tris-HCl (pH : 8,0), 2 mM EDTA. De manière alternative, l'adsorption peut être réalisée en " batch " qui est laissé une nuit à 5 ±3°C, sous agitation.

Le gel est lavé avec 2 à 6 volumes de tampon PBS 10 mM (pH : 6,8). L'antigène est élué avec un tampon glycine 100 mM (pH : 2,5). L'éluat est recueilli dans des fractions de 3 ml auquel on ajoute 150 µl de tampon PBS 1 mM (pH : 8,0). La densité optique est mesurée à 280 nm pour chaque fraction ; celles contenant l'antigène sont recueillies et stockées à -20°C.

Fragments du génome de *N. meningitidis* Z2491 décrits dans la demande de brevet WO98/02 547
(2) INFORMATIONS POUR LA SEQ ID NO: 70A:
   (i) CARACTERISTIQUES DE LA SEQUENCE:
      (A) LONGUEUR: 243 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   - (iv) ANTI-SENS: NON
   (xi) DESCRIPTION DE LA SEQUENCE: SEQ ID NO: 70A:
(2) INFORMATIONS POUR LA SEQ ID NO: 73A:
   (i) CARACTERISTIQUES DE LA SEQUENCE:
      (A) LONGUEUR: 120 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 73A:
(2) INFORMATIONS POUR LA SEQ ID NO: 74A:
   (i) CARACTERISTIQUES DE LA SEQUENCE:
      (A) LONGUEUR: 120 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 74A:
(2) INFORMATIONS POUR LA SEQ ID NO: 77A
   (i) CARACTERISTIQUES DE LA SEQUENCE::
      (A) LONGUEUR: 269 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 77A:
(2) INFORMATIONS POUR LA SEQ ID NO: 80A:
   (i) CARACTERISTIQUES DE LA SEQUENCE:
      (A) LONGUEUR: 207 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 80A:
(2) INFORMATIONS POUR LA SEQ ID NO: 81A :
   (i) CARACTERISTIQUES DE LA SEQUENCE:
      (A) LONGUEUR: 224 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 81A:
(2) INFORMATIONS POUR LA SEQ ID NO: 87A:
   (i) CARACTERISTIQUES DE LA SEQUENCE:
      (A) LONGUEUR: 273 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 87A:
(2) INFORMATIONS POUR LA SEQ ID NO: 88A:
   (i) CARACTERISTIQUES DE LA SEQUENCE:
      (A) LONGUEUR: 270 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 88A:
(2) INFORMATIONS POUR LA SEQ ID NO: 89A:
   (i) CARACTERISTIQUES DE LA SEQUENCE:
      (A) LONGUEUR: 267 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 89A:
(2) INFORMATIONS POUR LA SEQ ID NO: 94A:
   (i) CARACTERISTIQUES DE LA SEQUENCE:
      (A) LONGUEUR: 308 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 94A:
(2) INFORMATIONS POUR LA SEQ ID NO: 95A:
   (i) CARACTERISTIQUES DE LA SEQUENCE:
      (A) LONGUEUR: 286 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 95A :
(2) INFORMATIONS POUR LA SEQ ID NO: 98A :
   (i) CARACTERISTIQUES DE LA SEQUENCE:
      (A) LONGUEUR: 316 paires de bases
      (B) TYPE: nucléotide
      (C) NOMBRE DE BRINS: simple
      (D) CONFIGURATION: linéaire
   (ii) TYPE DE MOLECULE: ADN (génomique)
   (iii) HYPOTHETIQUE: NON
   (iv) ANTI-SENS: NON
   (xi) DESCRIPTION DE LA SEQUENCE: SEQ ID NO: 98A

### LISTE DE SEQUENCES

<110> Pasteur Mérieux Sérums et Vaccins INSERM
<120> Acides nucléiques et polypeptides spécifiques des souches pathogènes du genre Neisseria
<130> BET 99/0918
<140>
   <141>
<150> FR 98 13 693 <151> 1998-10-30
<160> 131
<170> PatentIn Ver. 2.1
<210> 1
   <211> 858
   <212> ADN
   <213> Neisseria meningitidis
<220>
   <221> CDS
   <222> (1) .. (855)
<400> 1
   atg tct gaa gaa aaa ttg aaa atg agt ttc gag cca acc gta atc gaa    48
   Met Ser Glu Glu Lys Leu Lys Met Ser Phe Glu Pro Thr Val Ile Glu
    1               5                  10                  15
   cat ttg ggt gta aag atg tat tcg cac act gtt cct gcg att gcc gag    96
   His Leu Gly Val Lys Met Tyr Ser His Thr Val Pro Ala Ile Ala Glu
               20                  25                  30
   ttg ata gcg aat gcc tac gat gca tgt gct acg gaa gtg gaa gtt agg   144
   Leu Ile Ala Asn Ala Tyr Asp Ala Cys Ala Thr Glu Val Glu Val Arg
           35                  40                  45
   tta ttc gat aaa ccg gag cat aaa atc gtt atc aaa gat aat ggt ata   192
   Leu Phe Asp Lys Pro Glu His Lys Ile Val Ile Lys Asp Asn Gly Ile
       50                  55                  60
   gga atg agc ttc gat gaa atc aat gat ttt tat ttg aga atc ggt cgg   240
   Gly Met Ser Phe Asp Glu Ile Asn Asp Phe Tyr Leu Arg Ile Gly Arg
   65                  70                  75                  80
   aac aga agg gaa gaa aaa caa gct tcc ccg tgc gga aga att cca acg   288
   Asn Arg Arg Glu Glu Lys Gln Ala Ser Pro Cys Gly Arg Ile Pro Thr
                   85                  90                  95
   ggt aaa aaa ggc ctt ggt aaa ttg gca tta ttc ggg ctt ggc aac aaa   336
   Gly Lys Lys Gly Leu Gly Lys Leu Ala Leu Phe Gly Leu Gly Asn Lys
               100                 105                 110
   att gaa att tct act atc cag gga aac gaa agg gtt act ttt act ttg   384
   Ile Glu Ile Ser Thr Ile Gln Gly Asn Glu Arg Val Thr Phe Thr Leu
               115                 120                 125
   gat tat gca gag att cga aga agc aag ggt att tat caa ccg gag ttt     432
   Asp Tyr Ala Glu Ile Arg Arg Ser Lys Gly Ile Tyr Gln Pro Glu Phe
       130                 135                 140
   cga aaa gaa tct gtt gaa tcc aat atc gaa agc ggg aca acc ata act     480
   Arg Lys Glu Ser Val Glu Ser Asn Ile Glu Ser Gly Thr Thr Ile Thr
   145                 150                 155                 160
   tta acc gaa ctg acg aaa aag caa gga tat ccg tta gat aat tat gta     528
   Leu Thr Glu Leu Thr Lys Lys Gln Gly Tyr Pro Leu Asp Asn Tyr Val
                   165                 170                 175
   gag cat ctt tcc cgc ttg ttt gat ttt ccg gct cag gat ttt aaa atc     576
   Glu His Leu Ser Arg Leu Phe Asp Phe Pro Ala Gln Asp Phe Lys Ile
               180                 185                 190
   aaa gta agc ttg aac ggc tct gaa cct aaa atc att gat gga aat cta     624
   Lys Val Ser Leu Asn Gly Ser Glu Pro Lys Ile Ile Asp Gly Asn Leu
           195                 200                 205
   aaa tat gat ctt gtt acc cca caa ttc gaa tgg gaa tac cag gat tta     672
   Lys Tyr Asp Leu Val Thr Pro Gln Phe Glu Trp Glu Tyr Gln Asp Leu
       210                 215                 220
   gca acc aat att tca tcg tta tct tca aaa ttc gaa cag tat gaa tac     720
   Ala Thr Asn Ile Ser Ser Leu Ser Ser Lys Phe Glu Gln Tyr Glu Tyr
   225                 230                 235                 240
   agc gga tta ata caa ggt aag ttc att aca acg gaa aaa cct tta aag     768
   Ser Gly Leu Ile Gln Gly Lys Phe Ile Thr Thr Glu Lys Pro Leu Lys
                   245                 250                 255
   aat aat atg aaa ggt att acc ttg ttt gcc aac ggc aga atg gta aat     816
   Asn Asn Met Lys Gly Ile Thr Leu Phe Ala Asn Gly Arg Met Val Asn
               260                 265                 270
   atg ccc gag ttt ttc act gat agc gaa tcc agc cat ttc taa             858
   Met Pro Glu Phe Phe Thr Asp Ser Glu Ser Ser His Phe
           275                 280                 285
<210> 2
   <211> 2855
   <212> PRT
   <213> Neisseria meningitidis
<400> 2
<210> 3
   <211> 1101
   <212> ADN
   <213> Neisseria meningitidis
<220>
   <221> CDS
   <222> (1) .. (1098)
<400> 3
   atg aaa cac tta ctc atc gat ttt gaa aac gtc cag ccg caa aac tta    48
   Met Lys His Leu Leu Ile Asp Phe Glu Asn Val Gln Pro Gln Asn Leu
   1               5                   10                  15
   gac aaa tta cta acc gaa aat acc cat att tgg cta ttt ata ggt gta    96
   Asp Lys Leu Leu Thr Glu Asn Thr His Ile Trp Leu Phe Ile Gly Val
               20                  25                  30
   tta cac aaa atg tta cct att agt ctg gtg caa tcc cta cta cgt ttc     144
   Leu His Lys Met Leu Pro Ile Ser Leu Val Gln Ser Leu Leu Arg Phe
           35                  40                  45
   ggc gaa cgt gtc cat ctt gtc cag tta caa aaa acg ggg aaa aac gca     192
   Gly Glu Arg Val His Leu Val Gln Leu Gln Lys Thr Gly Lys Asn Ala
       50                  55                  60
   ttg gat ttt tac ctg tcc tat tac ctc gga caa att acc gcc aca gac     240
   Leu Asp Phe Tyr Leu Ser Tyr Tyr Leu Gly Gln Ile Thr Ala Thr Asp
   65                  70                  75                  80
   ccc aat gcc caa atc ggc ata ctc tcg cgt gat gga gga tac gat gtt     288
   Pro Asn Ala Gln Ile Gly Ile Leu Ser Arg Asp Gly Gly Tyr Asp Val
                   85                  90                  95
   ctg gtc gaa cat att ttg aaa aac cac cag gcg aag ggt atc gtg cgc     336
   Leu Val Glu His Ile Leu Lys Asn His Gln Ala Lys Gly Ile Val Arg
               100                 105                 110
   cta gcc aat ata gat gaa gta caa cat cag aaa att gct acc gaa ccg     384
   Leu Ala Asn Ile Asp Glu Val Gln His Gln Lys Ile Ala Thr Glu Pro
           115                 120                 125
   ccg tca gca ttg ctg gaa aac act cct cag cct gaa acc acc ctc aaa     432
   Pro Ser Ala Leu Leu Glu Asn Thr Pro Gln Pro Glu Thr Thr Leu Lys
       130                 135                 140
   cca cag caa cca tta act tcc tat ttc caa gca gcc cta act gca ctg     480
   Pro Gln Gln Pro Leu Thr Ser Tyr Phe Gln Ala Ala Leu Thr Ala Leu
   145                 150                 155                 160
   cgc cgc ccc gac gct ttc cgc ccc tgc cgc ctg cat aac ctg cga caa     528
   Arg Arg Pro Asp Ala Phe Arg Pro Cys Arg Leu His Asn Leu Arg Gln
                   165                 170                 175
   aat ctg cgt aag cat att ttg agt gat ttg ttt aaa gaa aaa acc gat     576
   Asn Leu Arg Lys His Ile Leu Ser Asp Leu Phe Lys Glu Lys Thr Asp
               180                 185                 190
   gaa gaa tgc gaa ata acc act gct aac gtt atc aat aaa ctc aaa gca     624
   Glu Glu Cys Glu Ile Thr Thr Ala Asn Val Ile Asn Lys Leu Lys Ala
           195                 200                 205
   caa aac ttc atc agc att gat gaa cag gaa acc gtt tcc tac cat ctc     672
   Gln Asn Phe Ile Ser Ile Asp Glu Gln Glu Thr Val Ser Tyr His Leu
       210                 215                 220
   agt gat aat gat ttg tta caa aga atc caa cgc cat att tta agc caa     720
   Ser Asp Asn Asp Leu Leu Gln Arg Ile Gln Arg His Ile Leu Ser Gln
   225                 230                 235                 240
   cgt ccc aaa acc tac gct gat ttt caa gcc gtc gtg caa aac cga gca     768
   Arg Pro Lys Thr Tyr Ala Asp Phe Gln Ala Val Val Gln Asn Arg Ala
                   245                 250                 255
   gat gca ctt cac tta aca gtc ggt acc aac gac att caa tcc ttt gcg     816
   Asp Ala Leu His Leu Thr Val Gly Thr Asn Asp Ile Gln Ser Phe Ala
               260                 265                 270
   cga cat ttg cgc gac caa aac ctg atc cgc caa aac aat ggg aaa att      864
   Arg His Leu Arg Asp Gln Asn Leu Ile Arg Gln Asn Asn Gly Lys Ile
           275                 280                 285
   gaa tat gca ccg ttt act gaa cct aaa cca cag cca acg ccc aag cag      912
   Glu Tyr Ala Pro Phe Thr Glu Pro Lys Pro Gln Pro Thr Pro Lys Gln
       290                 295                 300
   cct aaa aaa acc gca tgg gaa cct gat gaa att att tgg aaa aaa gtg      960
   Pro Lys Lys Thr Ala Trp Glu Pro Asp Glu Ile Ile Trp Lys Lys Val
   305                 310                 315                 320
   att gcc gcg tta tcg tta aag aac cgt cct aat aaa acc aaa act tta     1008
   Ile Ala Ala Leu Ser Leu Lys Asn Arg Pro Asn Lys Thr Lys Thr Leu
                   325                 330                 335
   cgc aat aca atc cag gca ctc aca aaa tcc aat gca caa gaa act gac     1056
   Arg Asn Thr Ile Gln Ala Leu Thr Lys Ser Asn Ala Gln Glu Thr Asp
               340                 345                 350
   aaa ctg cta caa cat tta caa gat gac cca agt cct acg tat tga         1101
   Lys Leu Leu Gln His Leu Gln Asp Asp Pro Ser Pro Thr Tyr
           355                 360                 365
<210> 4
   <211> 366
   <212> PRT
   <213> Neisseria meningitidis
<400> 4
   Met Lys His Leu Leu Ile Asp Phe Glu Asn Val Gln Pro Gln Asn Leu
    1               5                  10                  15
   Asp Lys Leu Leu Thr Glu Asn Thr His Ile Trp Leu Phe Ile Gly Val
               20                  25                  30
   Leu His Lys Met Leu Pro Ile Ser Leu Val Gln Ser Leu Leu Arg Phe
           35                  40                  45
   Gly Glu Arg Val His Leu Val Gln Leu Gln Lys Thr Gly Lys Asn Ala
       50                  55                  60
   Leu Asp Phe Tyr Leu Ser Tyr Tyr Leu Gly Gln Ile Thr Ala Thr Asp
   65                  70                  75                  80
   Pro Asn Ala Gln Ile Gly Ile Leu Ser Arg Asp Gly Gly Tyr Asp Val
                   85                  90                  95
   Leu Val Glu His Ile Leu Lys Asn His Gln Ala Lys Gly Ile Val Arg
               100                 105                 110
   Leu Ala Asn Ile Asp Glu Val Gln His Gln Lys Ile Ala Thr Glu Pro
           115                 120                 125
   Pro Ser Ala Leu Leu Glu Asn Thr Pro Gln Pro Glu Thr Thr Leu Lys
       130                 135                 140
   Pro Gln Gln Pro Leu Thr Ser Tyr Phe Gln Ala Ala Leu Thr Ala Leu
   145 150 155 160
   Arg Arg Pro Asp Ala Phe Arg Pro Cys Arg Leu His Asn Leu Arg Gln
   165 170 175
   Asn Leu Arg Lys His Ile Leu Ser Asp Leu Phe Lys Glu Lys Thr Asp
   180 185 190
   Glu Glu Cys Glu Ile Thr Thr Ala Asn Val Ile Asn Lys Leu Lys Ala
   195 200 205
   Gln Asn Phe Ile Ser Ile Asp Glu Gln Glu Thr Val Ser Tyr His Leu
   210 215 220
   Ser Asp Asn Asp Leu Leu Gln Arg Ile Gln Arg His Ile Leu Ser Gln
   225 230 235 240
   Arg Pro Lys Thr Tyr Ala Asp Phe Gln Ala Val Val Gln Asn Arg Ala
   245 250 255
   Asp Ala Leu His Leu Thr Val Gly Thr Asn Asp Ile Gln Ser Phe Ala
   260 265 270
   Arg His Leu Arg Asp Gln Asn Leu Ile Arg Gln Asn Asn Gly Lys Ile
   275 280 285
   Glu Tyr Ala Pro Phe Thr Glu Pro Lys Pro Gln Pro Thr Pro Lys Gln
   290 295 300
   Pro Lys Lys Thr Ala Trp Glu Pro Asp Glu Ile Ile Trp Lys Lys Val
   305 310 315 320
   Ile Ala Ala Leu Ser Leu Lys Asn Arg Pro Asn Lys Thr Lys Thr Leu
   325 330 335
   Arg Asn Thr Ile Gln Ala Leu Thr Lys Ser Asn Ala Gln Glu Thr Asp
   340 345 350
   Lys Leu Leu Gln His Leu Gln Asp Asp Pro Ser Pro Thr Tyr
   355 360 365
<210> 5
   <211> 1572
   <212> ADN
   <213> Neisseria meningitidis
<220>
   <221> CDS
   <222> (1)..(1569)
<400> 5
   atg aaa aaa tcc ctt ttc gtt ctc ttt ctg tat tca tcc cta ctt acc    48
   Met Lys Lys Ser Leu Phe Val Leu Phe Leu Tyr Ser Ser Leu Leu Thr
    1               5                  10                  15
   gcc agc gaa atc gcc tat cgc ttt gta ttc gga att gaa acc tta ccg    96
   Ala Ser Glu Ile Ala Tyr Arg Phe Val Phe Gly Ile Glu Thr Leu Pro
               20                  25                  30
   gct gca aaa atg gca gaa acg ttt gcg ctg aca ttt atg att gct gcg    144
   Ala Ala Lys Met Ala Glu Thr Phe Ala Leu Thr Phe Met Ile Ala Ala
           35                  40                  45
   ctg tat ctg ttt gcg cgt tat aag gct tcg cgg ctg ctg att gcg gtg    192
   Leu Tyr Leu Phe Ala Arg Tyr Lys Ala Ser Arg Leu Leu Ile Ala Val
       50                  55                  60
   ttt ttc gcg ttc agc att att gcc aac aat gta cat tat gcg gtt tat    240
   Phe Phe Ala Phe Ser Ile Ile Ala Asn Asn Val His Tyr Ala Val Tyr
   65                  70                  75                  80
   caa agt tgg atg acg ggc atc aat tat tgg ctg atg ctg aaa gag att    288
   Gln Ser Trp Met Thr Gly Ile Asn Tyr Trp Leu Met Leu Lys Glu Ile
                   85                  90                  95
   acc gaa gtc ggc agt gcg ggc gcg tcg atg ttg gat aag ttg tgg ctg    336
   Thr Glu Val Gly Ser Ala Gly Ala Ser Met Leu Asp Lys Leu Trp Leu
               100                 105                 110
   cct gcg ttg tgg ggc gtg ttg gaa gtc atg ttg ttt tgc agc ctt gcc    384
   Pro Ala Leu Trp Gly Val Leu Glu Val Met Leu Phe Cys Ser Leu Ala
           115                 120                 125
   aag ttc cac cgt aag acg cat ttt tct gcc gat ata ctg ttt gcc ttc    432
   Lys Phe His Arg Lys Thr His Phe Ser Ala Asp Ile Leu Phe Ala Phe
       130                 135                 140
   cta atg ctg atg att ttc gtg cgt tcg ttc gac acg aaa caa gag cac    480
   Leu Met Leu Met Ile Phe Val Arg Ser Phe Asp Thr Lys Gln Glu His
   145                 150                 155                 160
   ggt att tcg ccc aaa ccg aca tac agc cgc atc aaa gcc aat tat ttc    528
   Gly Ile Ser Pro Lys Pro Thr Tyr Ser Arg Ile Lys Ala Asn Tyr Phe
                   165                 170                 175
   agc ttc ggt tat ttt gtc gga cgc gtg ttg ccg tat cag ttg ttt gat    576
   Ser Phe Gly Tyr Phe Val Gly Arg Val Leu Pro Tyr Gln Leu Phe Asp
               180                 185                 190
   tta agc agg att ccc gcc ttt aag cag cct gct cca agc aaa atc ggg    624
   Leu Ser Arg Ile Pro Ala Phe Lys Gln Pro Ala Pro Ser Lys Ile Gly
           195                 200                 205
   cag ggc agt gtt caa aat atc gtc ctg att atg ggc gaa agc gaa agc    672
   Gln Gly Ser Val Gln Asn Ile Val Leu Ile Met Gly Glu Ser Glu Ser
       210                 215                 220
   gcg gcg cat ttg aag ctg ttt ggc tac gga cgc gaa act tcg ccg ttt    720
   Ala Ala His Leu Lys Leu Phe Gly Tyr Gly Arg Glu Thr Ser Pro Phe
   225                 230                 235                 240
   tta acc cgg ctg tcg caa gcc gat ttt aag ccg att gtg aaa caa agt    768
   Leu Thr Arg Leu Ser Gln Ala Asp Phe Lys Pro Ile Val Lys Gln Ser
                   245                 250                 255
   tat tcc gca ggc ttt atg act gca gtg tcc ctg ccc agt ttt ttc aat    816
   Tyr Ser Ala Gly Phe Met Thr Ala Val Ser Leu Pro Ser Phe Phe Asn
               260                 265                 270
   gcg ata ccg cac gcc aac ggc ttg gaa caa atc agc ggc ggc gat act     864
   Ala Ile Pro His Ala Asn Gly Leu Glu Gln Ile Ser Gly Gly Asp Thr
           275                 280                 285
   aat atg ttc cgc ctc gcc aaa gag cag ggc tat gaa acg tat ttt tac     912
   Asn Met Phe Arg Leu Ala Lys Glu Gln Gly Tyr Glu Thr Tyr Phe Tyr
       290                 295                 300
   agc gca cag gcg gaa aac gag atg gcg att ttg aac tta atc ggt aag     960
   Ser Ala Gln Ala Glu Asn Glu Met Ala Ile Leu Asn Leu Ile Gly Lys
   305                 310                 315                 320
   aaa tgg ata gac cat ctg att cag ccg acg cag ctt ggc tac ggc aac    1008
   Lys Trp Ile Asp His Leu Ile Gln Pro Thr Gln Leu Gly Tyr Gly Asn
                   325                 330                 335
   ggc gac aat atg ccc gat gag aag ctg ctg ccg ctg ttc gac aaa atc    1056
   Gly Asp Asn Met Pro Asp Glu Lys Leu Leu Pro Leu Phe Asp Lys Ile
               340                 345                 350
   aat ttg cag cag ggc agg cat ttt atc gtg ttg cac caa cgt ggt tcg    1104
   Asn Leu Gln Gln Gly Arg His Phe Ile Val Leu His Gln Arg Gly Ser
           355                 360                 365
   cac gcc cca tac agc gca ttg ttg cag cct caa gat aaa gta ttc ggc    1152
   His Ala Pro Tyr Ser Ala Leu Leu Gln Pro Gln Asp Lys Val Phe Gly
       370                 375                 380
   gaa ctt att gtg gat aag tac gac aac acc atc cac aaa acc gac caa    1200
   Glu Leu Ile Val Asp Lys Tyr Asp Asn Thr Ile His Lys Thr Asp Gln
   385                 390                 395                 400
   atg att caa acc gta ttc gag cag ctg caa aag cag cct gac ggc aac    1248
   Met Ile Gln Thr Val Phe Glu Gln Leu Gln Lys Gln Pro Asp Gly Asn
                   405                 410                 415
   tgg ctg ttt gcc tat acc tcc gat cat ggc cag tat gtt cgc caa gat    1296
   Trp Leu Phe Ala Tyr Thr Ser Asp His Gly Gln Tyr Val Arg Gln Asp
               420                 425                 430
   atc tac aat caa ggc acg gtg cag ccc gac agc tat ctc gtg ccg ctg    1344
   Ile Tyr Asn Gln Gly Thr Val Gln Pro Asp Ser Tyr Leu Val Pro Leu
           435                 440                 445
   gtg ttg tac agc tcg aat aag gcc gtg caa cag gct gcc aac cag gct    1392
   Val Leu Tyr Ser Ser Asn Lys Ala Val Gln Gln Ala Ala Asn Gln Ala
       450                 455                 460
   ttt gcg cct tgc gag att gcc ttc cat cag cag ctt tca acg ttc ctg    1440
   Phe Ala Pro Cys Glu Ile Ala Phe His Gln Gln Leu Ser Thr Phe Leu
   465                 470                 475                 480
   att cac acg ttg ggc tac gat atg ccg gtt tca ggt tgt cgc gaa ggc    1488
   Ile His Thr Leu Gly Tyr Asp Met Pro Val Ser Gly Cys Arg Glu Gly
                   485                 490                 495
   tcg gta acg ggc aac ctg att acg ggt gat gca ggc agc ttg aac att    1536
   Ser Val Thr Gly Asn Leu Ile Thr Gly Asp Ala Gly Ser Leu Asn Ile
               500                 505                 510
   cgc gac ggc aag gcg gaa tat gtt tat ccg caa tga                  1572
   Arg Asp Gly Lys Ala Glu Tyr Val Tyr Pro Gln
           515                 520
<210> 6
   <211> 523
   <212> PRT
   <213> Neisseria meningitidis
<400> 6
   Met Lys Lys Ser Leu Phe Val Leu Phe Leu Tyr Ser Ser Leu Leu Thr
   1 5 10 15
   Ala Ser Glu Ile Ala Tyr Arg Phe Val Phe Gly Ile Glu Thr Leu Pro
   20 25 30
   Ala Ala Lys Met Ala Glu Thr Phe Ala Leu Thr Phe Met Ile Ala Ala
   35 40 45
   Leu Tyr Leu Phe Ala Arg Tyr Lys Ala Ser Arg Leu Leu Ile Ala Val
   50 55 60
   Phe Phe Ala Phe Ser Ile Ile Ala Asn Asn Val His Tyr Ala Val Tyr
   65 70 75 80
   Gln Ser Trp Met Thr Gly Ile Asn Tyr Trp Leu Met Leu Lys Glu Ile
   85 90 95
   Thr Glu Val Gly Ser Ala Gly Ala Ser Met Leu Asp Lys Leu Trp Leu
   100 105 110
   Pro Ala Leu Trp Gly Val Leu Glu Val Met Leu Phe Cys Ser Leu Ala
   115 120 125
   Lys Phe His Arg Lys Thr His Phe Ser Ala Asp Ile Leu Phe Ala Phe
   130 135 140
   Leu Met Leu Met Ile Phe Val Arg Ser Phe Asp Thr Lys Gln Glu His
   145 150 155 160
   Gly Ile Ser Pro Lys Pro Thr Tyr Ser Arg Ile Lys Ala Asn Tyr Phe
   165 170 175
   Ser Phe Gly Tyr Phe Val Gly Arg Val Leu Pro Tyr Gln Leu Phe Asp
   180 185 190
   Leu Ser Arg Ile Pro Ala Phe Lys Gln Pro Ala Pro Ser Lys Ile Gly
   195 200 205
   Gln Gly Ser Val Gln Asn Ile Val Leu Ile Met Gly Glu Ser Glu Ser
   210 215 220
   Ala Ala His Leu Lys Leu Phe Gly Tyr Gly Arg Glu Thr Ser Pro Phe
   225 230 235 240
   Leu Thr Arg Leu Ser Gln Ala Asp Phe Lys Pro Ile Val Lys Gln Ser
   245 250 255
   Tyr Ser Ala Gly Phe Met Thr Ala Val Ser Leu Pro Ser Phe Phe Asn
   260 265 270
   Ala Ile Pro His Ala Asn Gly Leu Glu Gln Ile Ser Gly Gly Asp Thr
   275 280 285
   Asn Met Phe Arg Leu Ala Lys Glu Gln Gly Tyr Glu Thr Tyr Phe Tyr
   290 295 300
   Ser Ala Gln Ala Glu Asn Glu Met Ala Ile Leu Asn Leu Ile Gly Lys
   305 310 315 320
   Lys Trp Ile Asp His Leu Ile Gln Pro Thr Gln Leu Gly Tyr Gly Asn
   325 330 335
   Gly Asp Asn Met Pro Asp Glu Lys Leu Leu Pro Leu Phe Asp Lys Ile
   340 345 350
   Asn Leu Gln Gln Gly Arg His Phe Ile Val Leu His Gln Arg Gly Ser
   355 360 365
   His Ala Pro Tyr Ser Ala Leu Leu Gln Pro Gln Asp Lys Val Phe Gly
   370 375 380
   Glu Leu Ile Val Asp Lys Tyr Asp Asn Thr Ile His Lys Thr Asp Gln
   385 390 395 400
   Met Ile Gln Thr Val Phe Glu Gln Leu Gln Lys Gln Pro Asp Gly Asn
   405 410 415
   Trp Leu Phe Ala Tyr Thr Ser Asp His Gly Gln Tyr Val Arg Gln Asp
   420 425 430
   Ile Tyr Asn Gln Gly Thr Val Gln Pro Asp Ser Tyr Leu Val Pro Leu
   435 440 445
   Val Leu Tyr Ser Ser Asn Lys Ala Val Gln Gln Ala Ala Asn Gln Ala
   450 455 460
   Phe Ala Pro Cys Glu Ile Ala Phe His Gln Gln Leu Ser Thr Phe Leu
   465 470 475 480
   Ile His Thr Leu Gly Tyr Asp Met Pro Val Ser Gly Cys Arg Glu Gly
   485 490 495
   Ser Val Thr Gly Asn Leu Ile Thr Gly Asp Ala Gly Ser Leu Asn Ile
   500 505 510
   Arg Asp Gly Lys Ala Glu Tyr Val Tyr Pro Gln
   515 520
<210> 7
   <211> 3204
   <212> ADN
   <213> Neisseria meningitidis
<220>
   <221> CDS
   <222> (1)..(3201)
<400> 7
   atg cga acg acc cca acc ttc cct aca aaa act ttc aaa ccg gct gcc      48
   Met Arg Thr Thr Pro Thr Phe Pro Thr Lys Thr Phe Lys Pro Ala Ala
    1               5                  10                  15
   atg gcg tta gct gtt gca aca aca ctt tct gcc tgc tta ggc ggc ggc      96
   Met Ala Leu Ala Val Ala Thr Thr Leu Ser Ala Cys Leu Gly Gly Gly
               20                  25                  30
   ggc ggc act tct gcg ccc gac ttc aat gca ggc ggc acc ggt atc ggc     144
   Gly Gly Thr Ser Ala Pro Asp Phe Asn Ala Gly Gly Thr Gly Ile Gly
           35                  40                  45
   agc aac agc aga gca aca aca gcg aaa tca gca gca gta tct tac gcc     192
   Ser Asn Ser Arg Ala Thr Thr Ala Lys Ser Ala Ala Val Ser Tyr Ala
       50                  55                  60
   ggt atc aag aac gaa atg tgc aaa gac aga agc atg ctc tgt gcc ggt     240
   Gly Ile Lys Asn Glu Met Cys Lys Asp Arg Ser Met Leu Cys Ala Gly
   65                  70                  75                  80
   cgg gat gac gtt gcg gtt aca gac agg gat gcc aaa atc aat gcc ccc     288
   Arg Asp Asp Val Ala Val Thr Asp Arg Asp Ala Lys Ile Asn Ala Pro
                   85                  90                  95
   ccc ccg aat ctg cat acc gga gac ttt aca aac cca aat gac gca tac     336
   Pro Pro Asn Leu His Thr Gly Asp Phe Thr Asn Pro Asn Asp Ala Tyr
               100                 105                 110
   aag aat ttg atc aac ctc aaa cct gca att gaa gca ggc tat aca gga     384
   Lys Asn Leu Ile Asn Leu Lys Pro Ala Ile Glu Ala Gly Tyr Thr Gly
           115                 120                 125
   cgc ggg gta gag gta ggt atc gtc gat aca ggc gaa tcc gtc ggc agc     432
   Arg Gly Val Glu Val Gly Ile Val Asp Thr Gly Glu Ser Val Gly Ser
       130                 135                 140
   ata tcc ttt ccc gaa ctg tat ggc aga aaa gaa cac ggc tat aac gaa     480
   Ile Ser Phe Pro Glu Leu Tyr Gly Arg Lys Glu His Gly Tyr Asn Glu
   145                 150                 155                 160
   aat tac aaa aac tat acg gcg tat atg cgg aag gaa gcg cct gaa gac     528
   Asn Tyr Lys Asn Tyr Thr Ala Tyr Met Arg Lys Glu Ala Pro Glu Asp
                   165                 170                 175
   gga ggc ggt aaa gac att aaa gct tct ttc gac gat gag gcc gtt ata     576
   Gly Gly Gly Lys Asp Ile Lys Ala Ser Phe Asp Asp Glu Ala Val Ile
               180                 185                 190
   gag act gaa gca aag ccg acg gat atc cgc cac gta aaa gaa atc gga     624
   Glu Thr Glu Ala Lys Pro Thr Asp Ile Arg His Val Lys Glu Ile Gly
           195                 200                 205
   cac atc gat gtg gtc tcc cat att att ggc ggg cgt tcc gtg gac ggc     672
   His Ile Asp Val Val Ser His Ile Ile Gly Gly Arg Ser Val Asp Gly
       210                 215                 220
   aga cct gca ggc ggt att gcg ccc gat gcg acg cta cac ata atg aat     720
   Arg Pro Ala Gly Gly Ile Ala Pro Asp Ala Thr Leu His Ile Met Asn
   225                 230                 235                 240
   acg cat gat gga acc aag aac gaa ata atg tct gca gcc atc cgc aat     768
   Thr His Asp Gly Thr Lys Asn Glu Ile Met Ser Ala Ala Ile Arg Asn
                   245                 250                 255
   gca tgg gtc aag ctg ggc gaa cgt ggc gtg cgc atc gtc aat aac agt     816
   Ala Trp Val Lys Leu Gly Glu Arg Gly Val Arg Ile Val Asn Asn Ser
               260                 265                 270
   ttt gga aca aca tcg agg gca ggc act gcc gac cat ttc caa ata gcc     864
   Phe Gly Thr Thr Ser Arg Ala Gly Thr Ala Asp His Phe Gln Ile Ala
           275                 280                 285
   aat tcg gag gag cag tac cgc caa gcg ttg ctc gcc tat tcc ggc ggt     912
   Asn Ser Glu Glu Gln Tyr Arg Gln Ala Leu Leu Ala Tyr Ser Gly Gly
       290                 295                 300
   gat aaa aca gac gag ggt atc cgc ctg atg caa cag agc gat tac ggc     960
   Asp Lys Thr Asp Glu Gly Ile Arg Leu Met Gln Gln Ser Asp Tyr Gly
   305                 310                 315                 320
   aac ttg tcc tac cac atc cgt aat aaa aac atg ctt ttc att ttt tcg     1008
   Asn Leu Ser Tyr His Ile Arg Asn Lys Asn Met Leu Phe Ile Phe Ser
                   325                 330                 335
   gca agc aat gac gca caa gct cag ccc aac aca ctg acc cta ttg cca     1056
   Ala Ser Asn Asp Ala Gln Ala Gln Pro Asn Thr Leu Thr Leu Leu Pro
               340                 345                 350
   ttt tat gaa aaa gat gct caa aaa ggc att atc aca gtc gca ggc gta     1104
   Phe Tyr Glu Lys Asp Ala Gln Lys Gly Ile Ile Thr Val Ala Gly Val
           355                 360                 365
   gac cgc agt gga gaa aag ttc aat ggc tcc aac cat tgc gga att act     1152
   Asp Arg Ser Gly Glu Lys Phe Asn Gly Ser Asn His Cys Gly Ile Thr
       370                 375                 380
   gcc atg tgg tgc cta tcg gca ccc tat gaa gca agc gtc cgt ttc acc     1200
   Ala Met Trp Cys Leu Ser Ala Pro Tyr Glu Ala Ser Val Arg Phe Thr
   385                 390                 395                 400
   cgt aca aac ccg att caa att gcc gga aca tcc ttt tcc gca ccc atc     1248
   Arg Thr Asn Pro Ile Gln Ile Ala Gly Thr Ser Phe Ser Ala Pro Ile
                   405                 410                 415
   gta acc ggc acg gcg gct ctg ctg ctg cag aaa tac ccg tgg atg agc     1296
   Val Thr Gly Thr Ala Ala Leu Leu Leu Gln Lys Tyr Pro Trp Met Ser
               420                 425                 430
   aac gac aac ctg cgt acc acg ctg ctg aca acg gct cag gac atc ggt     1344
   Asn Asp Asn Leu Arg Thr Thr Leu Leu Thr Thr Ala Gln Asp Ile Gly
           435                 440                 445
   gca gtc ggc gtg gac agc aag ttc ggc tgg gga ctg ctg gat gcg ggt     1392
   Ala Val Gly Val Asp Ser Lys Phe Gly Trp Gly Leu Leu Asp Ala Gly
       450                 455                 460
   aag gcc atg aac gga ccc gcg tcc ttt ccg ttc ggc gac ttt acc gcc    1440
   Lys Ala Met Asn Gly Pro Ala Ser Phe Pro Phe Gly Asp Phe Thr Ala
   465                 470                 475                 480
   gat acg aaa ggt aca tcc gat att gcc tac tcc ttc cgt aac gac att    1488
   Asp Thr Lys Gly Thr Ser Asp Ile Ala Tyr Ser Phe Arg Asn Asp Ile
                   485                 490                 495
   tca ggc acg ggc ggc ctg atc aaa aaa ggc ggc agc caa ctg caa ctg    1536
   Ser Gly Thr Gly Gly Leu Ile Lys Lys Gly Gly Ser Gln Leu Gln Leu
               500                 505                 510
   cac ggc aac aac acc tat acg ggc aaa acc att atc gaa ggc ggt tcg    1584
   His Gly Asn Asn Thr Tyr Thr Gly Lys Thr Ile Ile Glu Gly Gly Ser
           515                 520                 525
   ctg gtg ttg tac ggc aac aac aaa tcg gat atg cgc gtc gaa acc aaa    1632
   Leu Val Leu Tyr Gly Asn Asn Lys Ser Asp Met Arg Val Glu Thr Lys
       530                 535                 540
   ggt gcg ctg att tat aac ggg gcg gca tcc ggc ggt agc ctg aac agc    1680
   Gly Ala Leu Ile Tyr Asn Gly Ala Ala Ser Gly Gly Ser Leu Asn Ser
   545                 550                 555                 560
   gac ggc att gtc tat ctg gca gat acc gac cga tcc ggc gca aac gaa    1728
   Asp Gly Ile Val Tyr Leu Ala Asp Thr Asp Arg Ser Gly Ala Asn Glu
                   565                 570                 575
   acc gtg cac atc aaa ggc gat ctg cag ctg ggc ggc gaa ggt acg ctg    1776
   Thr Val His Ile Lys Gly Asp Leu Gln Leu Gly Gly Glu Gly Thr Leu
               580                 585                 590
   tac aca cgt ttg ggc aaa ctg ctg aaa gtg gac ggt acg gcg atg acc    1824
   Tyr Thr Arg Leu Gly Lys Leu Leu Lys Val Asp Gly Thr Ala Met Thr
           595                 600                 605
   ggc ggc aag ctg tac atg tcg gca cgc ggc aaa ggg gca ggc tat ctc    1872
   Gly Gly Lys Leu Tyr Met Ser Ala Arg Gly Lys Gly Ala Gly Tyr Leu
       610                 615                 620
   aac cgt acc gga caa cgt gtt ccc ttc ctg agt gcc gcc aaa atc ggg    1920
   Asn Arg Thr Gly Gln Arg Val Pro Phe Leu Ser Ala Ala Lys Ile Gly
   625                 630                 635                 640
   cgg gat tat tct ttc ttc aca aac atc gaa acc gac ggt ggt ctg ctg    1968
   Arg Asp Tyr Ser Phe Phe Thr Asn Ile Glu Thr Asp Gly Gly Leu Leu
                   645                 650                 655
   gct tcc ctc gac agc gtc gaa aaa aca gcg ggc agt gaa ggc gac acg    2016
   Ala Ser Leu Asp Ser Val Glu Lys Thr Ala Gly Ser Glu Gly Asp Thr
               660                 665                 670
   ctg tcc tat tat gtc cgt cgc ggc aat gcg gca cgg act gct tcg gca    2064
   Leu Ser Tyr Tyr Val Arg Arg Gly Asn Ala Ala Arg Thr Ala Ser Ala
           675                 680                 685
   gcg gca cat tcc gcg ccc gcc ggt ctg aaa cac gcc gta gaa cag ggc    2112
   Ala Ala His Ser Ala Pro Ala Gly Leu Lys His Ala Val Glu Gln Gly
       690                 695                 700
   ggc agc aat ctg gaa aac ctg atg gtc gaa ctg gat gcc tcc gaa tca    2160
   Gly Ser Asn Leu Glu Asn Leu Met Val Glu Leu Asp Ala Ser Glu Ser
   705                 710                 715                 720
   tcc gca aca ccc gag acg gtt gaa act gcg gcc gcc gac cgc aca gat    2208
   Ser Ala Thr Pro Glu Thr Val Glu Thr Ala Ala Ala Asp Arg Thr Asp
                   725                 730                 735
   atg ccg ggc atc cgc ccc tac ggc gca act ttc cgc gca gcg gca gcc    2256
   Met Pro Gly Ile Arg Pro Tyr Gly Ala Thr Phe Arg Ala Ala Ala Ala
               740                 745                 750
   gta cag cat gcg aat gcc gcc gac ggt gta cgc atc ttc aac agt ctc    2304
   Val Gln His Ala Asn Ala Ala Asp Gly Val Arg Ile Phe Asn Ser Leu
           755                 760                 765
   gcc gct acc gtc tat gcc gac agt acc gcc gcc cat gcc gat atg cag    2352
   Ala Ala Thr Val Tyr Ala Asp Ser Thr Ala Ala His Ala Asp Met Gln
       770                 775                 780
   gga cgc cgg ctg aaa gcc gta tcg gac ggg ttg gac cac aac gct acg    2400
   Gly Arg Arg Leu Lys Ala Val Ser Asp Gly Leu Asp His Asn Ala Thr
   785                 790                 795                 800
   ggt ctg cgc gtc atc gcg caa acc caa cag gac ggt gga acg tgg gaa    2448
   Gly Leu Arg Val Ile Ala Gln Thr Gln Gln Asp Gly Gly Thr Trp Glu
                   805                 810                 815
   cag ggc ggt gtt gaa ggc aaa atg cgc ggc agt acc caa acc gtc ggc    2496
   Gln Gly Gly Val Glu Gly Lys Met Arg Gly Ser Thr Gln Thr Val Gly
               820                 825                 830
   att gcc gcg aaa acc ggc gaa aat acg aca gca gcc gcc aca ctg ggc    2544
   Ile Ala Ala Lys Thr Gly Glu Asn Thr Thr Ala Ala Ala Thr Leu Gly
           835                 840                 845
   atg gga cac agc aca tgg agc gaa aac agt gca aat gca aaa acc gac    2592
   Met Gly His Ser Thr Trp Ser Glu Asn Ser Ala Asn Ala Lys Thr Asp
       850                 855                 860
   agc att agt ctg ttt gca ggc ata cgg cac gat gcg ggc gat atc ggc    2640
   Ser Ile Ser Leu Phe Ala Gly Ile Arg His Asp Ala Gly Asp Ile Gly
   865                 870                 875                 880
   tat ctc aaa ggc ctg ttc tcc tac gga cgc tac aaa aac agc atc agc    2688
   Tyr Leu Lys Gly Leu Phe Ser Tyr Gly Arg Tyr Lys Asn Ser Ile Ser
                   885                 890                 895
   cgc agc acc ggt gcg gac gaa cat gcg gaa ggc agc gtc aac ggc acg    2736
   Arg Ser Thr Gly Ala Asp Glu His Ala Glu Gly Ser Val Asn Gly Thr
               900                 905                 910
   ctg atg cag ctg ggc gca ctg ggc ggt gtc aac gtt ccg ttt gcc gca    2784
   Leu Met Gln Leu Gly Ala Leu Gly Gly Val Asn Val Pro Phe Ala Ala
           915                 920                 925
   acg gga gat ttg acg gtc gaa ggc ggt ctg cgc tac gac ctg ctc aaa    2832
   Thr Gly Asp Leu Thr Val Glu Gly Gly Leu Arg Tyr Asp Leu Leu Lys
       930                 935                 940
   cag gat gca ttc gcc gaa aaa ggc agt gct ttg ggc tgg agc ggc aac    2880
   Gln Asp Ala Phe Ala Glu Lys Gly Ser Ala Leu Gly Trp Ser Gly Asn
   945                 950                 955                 960
   agc ctc act gaa ggc aca ctg gtc gga ctc gcg ggt ctg aag ctg tcg    2928
   Ser Leu Thr Glu Gly Thr Leu Val Gly Leu Ala Gly Leu Lys Leu Ser
                   965                 970                 975
   caa ccc ttg agc gat aaa gcc gtc ctg ttt gca acg gcg ggc gtg gaa    2976
   Gln Pro Leu Ser Asp Lys Ala Val Leu Phe Ala Thr Ala Gly Val Glu
               980                 985                 990
   cgc gac ctg aac gga cgc gac tac acg gta acg ggc ggc ttt acc ggc    3024
   Arg Asp Leu Asn Gly Arg Asp Tyr Thr Val Thr Gly Gly Phe Thr Gly
           995                 1000                1005
   gcg act gca gca acc ggc aag acg ggg gca cgc aat atg ccg cac acc    3072
   Ala Thr Ala Ala Thr Gly Lys Thr Gly Ala Arg Asn Met Pro His Thr
       1010                1015                1020
   cgc ctg gtt gcc ggt ctg ggc gcg gat gtc gaa ttc ggc aac ggc tgg    3120
   Arg Leu Val Ala Gly Leu Gly Ala Asp Val Glu Phe Gly Asn Gly Trp
   1025                1030                1035                1040
   aac ggc ttg gca cgt tac agc tac gcc ggt tcc aaa cag tac ggc aac    3168
   Asn Gly Leu Ala Arg Tyr Ser Tyr Ala Gly Ser Lys Gln Tyr Gly Asn
                   1045                1050                1055
   cac agc gga cga gtc ggc gta ggc tac cgg ttc tga                    3204
   His Ser Gly Arg Val Gly Val Gly Tyr Arg Phe
               1060                1065
<210> 8
   <211> 1067
   <212> PRT
   <213> Neisseria meningitidis
<400> 8
   Met Arg Thr Thr Pro Thr Phe Pro Thr Lys Thr Phe Lys Pro Ala Ala
    1               5                  10                  15
   Met Ala Leu Ala Val Ala Thr Thr Leu Ser Ala Cys Leu Gly Gly Gly
                20                  25                  30
   Gly Gly Thr Ser Ala Pro Asp Phe Asn Ala Gly Gly Thr Gly Ile Gly
            35                  40                  45
   Ser Asn Ser Arg Ala Thr Thr Ala Lys Ser Ala Ala Val Ser Tyr Ala
        50                  55                  60
   Gly Ile Lys Asn Glu Met Cys Lys Asp Arg Ser Met Leu Cys Ala Gly
    65                  70                  75                  80
   Arg Asp Asp Val Ala Val Thr Asp Arg Asp Ala Lys Ile Asn Ala Pro
                    85                  90                  95
   Pro Pro Asn Leu His Thr Gly Asp Phe Thr Asn Pro Asn Asp Ala Tyr
               100                 105                 110
   Lys Asn Leu Ile Asn Leu Lys Pro Ala Ile Glu Ala Gly Tyr Thr Gly
           115                 120                 125
   Arg Gly Val Glu Val Gly Ile Val Asp Thr Gly Glu Ser Val Gly Ser
       130                 135                 140
   Ile Ser Phe Pro Glu Leu Tyr Gly Arg Lys Glu His Gly Tyr Asn Glu
   145                 150                 155                 160
   Asn Tyr Lys Asn Tyr Thr Ala Tyr Met Arg Lys Glu Ala Pro Glu Asp
                   165                 170                 175
   Gly Gly Gly Lys Asp Ile Lys Ala Ser Phe Asp Asp Glu Ala Val Ile
               180                 185                 190
   Glu Thr Glu Ala Lys Pro Thr Asp Ile Arg His Val Lys Glu Ile Gly
           195                 200                 205
   His Ile Asp Val Val Ser His Ile Ile Gly Gly Arg Ser Val Asp Gly
       210                 215                 220
   Arg Pro Ala Gly Gly Ile Ala Pro Asp Ala Thr Leu His Ile Met Asn
   225                 230                 235                 240
   Thr His Asp Gly Thr Lys Asn Glu Ile Met Ser Ala Ala Ile Arg Asn
                   245                 250                 255
   Ala Trp Val Lys Leu Gly Glu Arg Gly Val Arg Ile Val Asn Asn Ser
               260                 265                 270
   Phe Gly Thr Thr Ser Arg Ala Gly Thr Ala Asp His Phe Gln Ile Ala
           275                 280                 285
   Asn Ser Glu Glu Gln Tyr Arg Gln Ala Leu Leu Ala Tyr Ser Gly Gly
       290                 295                 300
   Asp Lys Thr Asp Glu Gly Ile Arg Leu Met Gln Gln Ser Asp Tyr Gly
   305                 310                 315                 320
   Asn Leu Ser Tyr His Ile Arg Asn Lys Asn Met Leu Phe Ile Phe Ser
                   325                 330                 335
   Ala Ser Asn Asp Ala Gln Ala Gln Pro Asn Thr Leu Thr Leu Leu Pro
               340                 345                 350
   Phe Tyr Glu Lys Asp Ala Gln Lys Gly Ile Ile Thr Val Ala Gly Val
           355                 360                 365
   Asp Arg Ser Gly Glu Lys Phe Asn Gly Ser Asn His Cys Gly Ile Thr
       370                 375                 380
   Ala Met Trp Cys Leu Ser Ala Pro Tyr Glu Ala Ser Val Arg Phe Thr
   385                 390                 395                 400
   Arg Thr Asn Pro Ile Gln Ile Ala Gly Thr Ser Phe Ser Ala Pro Ile
                   405                 410                 415
   Val Thr Gly Thr Ala Ala Leu Leu Leu Gln Lys Tyr Pro Trp Met Ser
               420                 425                 430
   Asn Asp Asn Leu Arg Thr Thr Leu Leu Thr Thr Ala Gln Asp Ile Gly
           435                 440                 445
   Ala Val Gly Val Asp Ser Lys Phe Gly Trp Gly Leu Leu Asp Ala Gly
       450                 455                 460
   Lys Ala Met Asn Gly Pro Ala Ser Phe Pro Phe Gly Asp Phe Thr Ala
   465                 470                 475                 480
   Asp Thr Lys Gly Thr Ser Asp Ile Ala Tyr Ser Phe Arg Asn Asp Ile
                   485                 490                 495
   Ser Gly Thr Gly Gly Leu Ile Lys Lys Gly Gly Ser Gln Leu Gln Leu
               500                 505                 510
   His Gly Asn Asn Thr Tyr Thr Gly Lys Thr Ile Ile Glu Gly Gly Ser
           515                 520                 525
   Leu Val Leu Tyr Gly Asn Asn Lys Ser Asp Met Arg Val Glu Thr Lys
       530                 535                 540
   Gly Ala Leu Ile Tyr Asn Gly Ala Ala Ser Gly Gly Ser Leu Asn Ser
   545                 550                 555                 560
   Asp Gly Ile Val Tyr Leu Ala Asp Thr Asp Arg Ser Gly Ala Asn Glu
                   565                 570                 575
   Thr Val His Ile Lys Gly Asp Leu Gln Leu Gly Gly Glu Gly Thr Leu
               580                 585                 590
   Tyr Thr Arg Leu Gly Lys Leu Leu Lys Val Asp Gly Thr Ala Met Thr
           595                 600                 605
   Gly Gly Lys Leu Tyr Met Ser Ala Arg Gly Lys Gly Ala Gly Tyr Leu
       610                 615                 620
   Asn Arg Thr Gly Gln Arg Val Pro Phe Leu Ser Ala Ala Lys Ile Gly
   625                 630                 635                 640
   Arg Asp Tyr Ser Phe Phe Thr Asn Ile Glu Thr Asp Gly Gly Leu Leu
                   645                 650                 655
   Ala Ser Leu Asp Ser Val Glu Lys Thr Ala Gly Ser Glu Gly Asp Thr
               660                 665                 670
   Leu Ser Tyr Tyr Val Arg Arg Gly Asn Ala Ala Arg Thr Ala Ser Ala
           675                 680                 685
   Ala Ala His Ser Ala Pro Ala Gly Leu Lys His Ala Val Glu Gln Gly
       690                 695                 700
   Gly Ser Asn Leu Glu Asn Leu Met Val Glu Leu Asp Ala Ser Glu Ser
   705                 710                 715                 720
   Ser Ala Thr Pro Glu Thr Val Glu Thr Ala Ala Ala Asp Arg Thr Asp
                   725                 730                 735
   Met Pro Gly Ile Arg Pro Tyr Gly Ala Thr Phe Arg Ala Ala Ala Ala
               740                 745                 750
   Val Gln His Ala Asn Ala Ala Asp Gly Val Arg Ile Phe Asn Ser Leu
           755                 760                 765
   Ala Ala Thr Val Tyr Ala Asp Ser Thr Ala Ala His Ala Asp Met Gln
       770                 775                 780
   Gly Arg Arg Leu Lys Ala Val Ser Asp Gly Leu Asp His Asn Ala Thr
   785                 790                 795                 800
   Gly Leu Arg Val Ile Ala Gln Thr Gln Gln Asp Gly Gly Thr Trp Glu
                   805                 810                 815
   Gln Gly Gly Val Glu Gly Lys Met Arg Gly Ser Thr Gln Thr Val Gly
               820                 825                 830
   Ile Ala Ala Lys Thr Gly Glu Asn Thr Thr Ala Ala Ala Thr Leu Gly
           835                 840                 845
   Met Gly His Ser Thr Trp Ser Glu Asn Ser Ala Asn Ala Lys Thr Asp
       850                 855                 860
   Ser Ile Ser Leu Phe Ala Gly Ile Arg His Asp Ala Gly Asp Ile Gly
   865                 870                 875                 880
   Tyr Leu Lys Gly Leu Phe Ser Tyr Gly Arg Tyr Lys Asn Ser Ile Ser
                   885                 890                 895
   Arg Ser Thr Gly Ala Asp Glu His Ala Glu Gly Ser Val Asn Gly Thr
               900                 905                 910
   Leu Met Gln Leu Gly Ala Leu Gly Gly Val Asn Val Pro Phe Ala Ala
           915                 920                 925
   Thr Gly Asp Leu Thr Val Glu Gly Gly Leu Arg Tyr Asp Leu Leu Lys
       930                 935                 940
   Gln Asp Ala Phe Ala Glu Lys Gly Ser Ala Leu Gly Trp Ser Gly Asn
   945                 950                 955                 960
   Ser Leu Thr Glu Gly Thr Leu Val Gly Leu Ala Gly Leu Lys Leu Ser
                   965                 970                 975
   Gln Pro Leu Ser Asp Lys Ala Val Leu Phe Ala Thr Ala Gly Val Glu
               980                 985                 990
   Arg Asp Leu Asn Gly Arg Asp Tyr Thr Val Thr Gly Gly Phe Thr Gly
           995                 1000                1005
   Ala Thr Ala Ala Thr Gly Lys Thr Gly Ala Arg Asn Met Pro His Thr
       1010                1015                1020
   Arg Leu Val Ala Gly Leu Gly Ala Asp Val Glu Phe Gly Asn Gly Trp
   025                 1030                1035                1040
   Asn Gly Leu Ala Arg Tyr Ser Tyr Ala Gly Ser Lys Gln Tyr Gly Asn
                   1045                1050                1055
   His Ser Gly Arg Val Gly Val Gly Tyr Arg Phe
               1060                1065
<210> 9
   <211> 339
   <212> ADN
   <213> Neisseria meningitidis
<220>
   <221> CDS
   <222> (1) .. (336)
<400> 9
   atg aac aca cgc atc atc gtt tcg gct gcg ttc gtt gcg ttg gca tta      48
   Met Asn Thr Arg Ile Ile Val Ser Ala Ala Phe Val Ala Leu Ala Leu
   1               5                   10                  15
   gca ggt tgc ggc tca atc aat aat gta acc gtt tcc gac cag aaa ctt      96
   Ala Gly Cys Gly Ser Ile Asn Asn Val Thr Val Ser Asp Gln Lys Leu
               20                  25                  30
   cag gaa cgt gcc gcg ttt gcc ttg ggc gtc agc caa aat gcc gta aaa      144
   Gln Glu Arg Ala Ala Phe Ala Leu Gly Val Ser Gln Asn Ala Val Lys
           35                  40                  45
   atc agc aac cgc agc aat gaa agc ata cgc atc aac ttt acc gca act      192
   Ile Ser Asn Arg Ser Asn Glu Ser Ile Arg Ile Asn Phe Thr Ala Thr
       50                  55                  60
   gtg ggt aag cgc gtg agc caa tgc tat gtt acc agt gta atc agc aca      240
   Val Gly Lys Arg Val Ser Gln Cys Tyr Val Thr Ser Val Ile Ser Thr
   65                  70                  75                  80
   atc ggc gtt acc act tcc gat gca att tgt ttg gga ggc gga acg cac      288
   Ile Gly Val Thr Thr Ser Asp Ala Ile Cys Leu Gly Gly Gly Thr His
                   85                  90                  95
   aaa ggc aaa agt caa tgc aat gct ttg ctt aaa gcg gca ggc cgt tgc      336
   Lys Gly Lys Ser Gln Cys Asn Ala Leu Leu Lys Ala Ala Gly Arg Cys
               100                 105                 110
   taa                                                                   339
<210> 10
   <211> 112
   <212> PRT
   <213> Neisseria meningitidis
<400> 10
   Met Asn Thr Arg Ile Ile Val Ser Ala Ala Phe Val Ala Leu Ala Leu
   1 5 10 15
   Ala Gly Cys Gly Ser Ile Asn Asn Val Thr Val Ser Asp Gln Lys Leu
   20 25 30
   Gln Glu Arg Ala Ala Phe Ala Leu Gly Val Ser Gln Asn Ala Val Lys
   35 40 45
   Ile Ser Asn Arg Ser Asn Glu Ser Ile Arg Ile Asn Phe Thr Ala Thr
   50 55 60
   Val Gly Lys Arg Val Ser Gln Cys Tyr Val Thr Ser Val Ile Ser Thr
   65 70 75 80
   Ile Gly Val Thr Thr Ser Asp Ala Ile Cys Leu Gly Gly Gly Thr His
   85 90 95
   Lys Gly Lys Ser Gln Cys Asn Ala Leu Leu Lys Ala Ala Gly Arg Cys
   100 105 110
<210> 11
   <211> 1314
   <212> ADN
   <213> Neisseria meningitidis
<220>
   <221> CDS
   <222> (1)..(1311)
<400> 11
   atg ctg acg ttt atc gga ctg ctg att atc ggg gtc atc gta tgg ctg      48
   Met Leu Thr Phe Ile Gly Leu Leu Ile Ile Gly Val Ile Val Trp Leu
     1               5                  10                  15
   ctg ctg acg gaa aaa gtg tcg ccc atc atc gca tta atc ttg gtg ccg      96
   Leu Leu Thr Glu Lys Val Ser Pro Ile Ile Ala Leu Ile Leu Val Pro
               20                  25                  30
   ctg ttt ggg gcg ttg ctg gcg ggg ttt gat gta tcc caa tta aaa gaa     144
   Leu Phe Gly Ala Leu Leu Ala Gly Phe Asp Val Ser Gln Leu Lys Glu
           35                  40                  45
   ttt tat tcg ggc ggc acc aaa tcg gtg atg cag att gtg att atg ttt     192
   Phe Tyr Ser Gly Gly Thr Lys Ser Val Met Gln Ile Val Ile Met Phe
       50                  55                  60
   atg ttt tcc att ttg ttt ttt gga atc atg aac gat gtg ggg ctg ttc     240
   Met Phe Ser Ile Leu Phe Phe Gly Ile Met Asn Asp Val Gly Leu Phe
   65                  70                  75                  80
   cgt ccg atg ata ggc ggt ttg att aag ctg act cgg ggt aat atc gtg     288
   Arg Pro Met Ile Gly Gly Leu Ile Lys Leu Thr Arg Gly Asn Ile Val
                   85                  90                  95
   gca gtg agt gtg ggg acg gtc ttg gtg tcg gtg gtg gcg cag ttg gac     336
   Ala Val Ser Val Gly Thr Val Leu Val Ser Val Val Ala Gln Leu Asp
               100                 105                 110
   ggg gcg ggt gcg acg acg ttt tta ttg gtc gtc ccc gcc ctt ttg ccg     384
   Gly Ala Gly Ala Thr Thr Phe Leu Leu Val Val Pro Ala Leu Leu Pro
           115                 120                 125
   ctt tac aag cgt ctg cat atg aat cct tac ctg ctg ttt ttg ctg ctg     432
   Leu Tyr Lys Arg Leu His Met Asn Pro Tyr Leu Leu Phe Leu Leu Leu
       130                 135                 140
   act tcc agt gcg gga ttg att aac ctt ctg ccg tgg ggc ggg ccg acc     480
   Thr Ser Ser Ala Gly Leu Ile Asn Leu Leu Pro Trp Gly Gly Pro Thr
   145                 150                 155                 160
   ggg cgg gtt gca agc gtg ttg ggc gca gat gtg ggc gaa ttg tat aaa      528
   Gly Arg Val Ala Ser Val Leu Gly Ala Asp Val Gly Glu Leu Tyr Lys
                   165                 170                 175
   cct ttg ttg acg gtg caa att atc ggt gtg gtg ttt atc ctt gcg ctg      576
   Pro Leu Leu Thr Val Gln Ile Ile Gly Val Val Phe Ile Leu Ala Leu
               180                 185                 190
   tcc ctg ctt ttg ggt gtg cgt gaa aaa agg cgg att gtc cgg gag ttg      624
   Ser Leu Leu Leu Gly Val Arg Glu Lys Arg Arg Ile Val Arg Glu Leu
           195                 200                 205
   ggc gcg ttg ccc gcc gtg gcg gat ttg ata aag ccg gtg cct ttg tcg      672
   Gly Ala Leu Pro Ala Val Ala Asp Leu Ile Lys Pro Val Pro Leu Ser
       210                 215                 220
   gaa gaa gaa caa aaa ttg gcg cgt ccg aaa ctg ttt tgg tgg aat gtc      720
   Glu Glu Glu Gln Lys Leu Ala Arg Pro Lys Leu Phe Trp Trp Asn Val
   225                 230                 235                 240
   ctg ctg ttt ttg gcg gcg atg agc ctg ctt ttt tcg ggc atc ttc ccg      768
   Leu Leu Phe Leu Ala Ala Met Ser Leu Leu Phe Ser Gly Ile Phe Pro
                   245                 250                 255
   ccg ggt tat gta ttt atg ctg gct gca acg gcg gcg ttg ctt ttg aat      816
   Pro Gly Tyr Val Phe Met Leu Ala Ala Thr Ala Ala Leu Leu Leu Asn
               260                 265                 270
   tac cgc agc ccg cag gaa cag atg gag cgg att tat gcc cac gcc ggc      864
   Tyr Arg Ser Pro Gln Glu Gln Met Glu Arg Ile Tyr Ala His Ala Gly
           275                 280                 285
   ggc gcg gtg atg atg gcg tcc att att ttg gcg gca ggt acg ttt ttg      912
   Gly Ala Val Met Met Ala Ser Ile Ile Leu Ala Ala Gly Thr Phe Leu
       290                 295                 300
   ggg att ttg aag ggt gcg ggg atg ttg gac gcg att tcc aaa gac att      960
   Gly Ile Leu Lys Gly Ala Gly Met Leu Asp Ala Ile Ser Lys Asp Ile
   305                 310                 315                 320
   gtg cat atc ctg ccg gac gcg ctg ctg cct tat ctg cat att gcc atc     1008
   Val His Ile Leu Pro Asp Ala Leu Leu Pro Tyr Leu His Ile Ala Ile
                   325                 330                 335
   ggt gtg ttg ggc att ccg ctt gag ttg gtt ttg agt acg gac gct tat     1056
   Gly Val Leu Gly Ile Pro Leu Glu Leu Val Leu Ser Thr Asp Ala Tyr
               340                 345                 350
   tat ttc gga ctg ttt ccg att gtg gag cag att acc tcg cag gcg ggc     1104
   Tyr Phe Gly Leu Phe Pro Ile Val Glu Gln Ile Thr Ser Gln Ala Gly
           355                 360                 365
   gtg gcg ccc gaa gca gca ggt tat gcg atg ttg atc ggc agt atc gtc     1152
   Val Ala Pro Glu Ala Ala Gly Tyr Ala Met Leu Ile Gly Ser Ile Val
       370                 375                 380
   ggc act ttt gtt acg ccg ctt tcg ccg gct ttg tgg atg ggc ttg ggt     1200
   Gly Thr Phe Val Thr Pro Leu Ser Pro Ala Leu Trp Met Gly Leu Gly
   385                 390                 395                 400
   ttg gcg aaa ttg tcg atg ggc aaa cac atc cgt tat tcg ttt ttt tgg    1248
   Leu Ala Lys Leu Ser Met Gly Lys His Ile Arg Tyr Ser Phe Phe Trp
                   405                 410                 415
   gcg tgg ggt ttg tcg ctg gcg ata ttg gcc agt tcg ata gcg gca gga    1296
   Ala Trp Gly Leu Ser Leu Ala Ile Leu Ala Ser Ser Ile Ala Ala Gly
               420                 425                 430
   atc gtg cct ctg ccg taa                                            1314
   Ile Val Pro Leu Pro
           435
<210> 12
   <211> 437
   <212> PRT
   <213> Neisseria meningitidis
<400> 12
   Met Leu Thr Phe Ile Gly Leu Leu Ile Ile Gly Val Ile Val Trp Leu
   1               5                   10                  15
   Leu Leu Thr Glu Lys Val Ser Pro Ile Ile Ala Leu Ile Leu Val Pro
               20                  25                  30
   Leu Phe Gly Ala Leu Leu Ala Gly Phe Asp Val Ser Gln Leu Lys Glu
           35                  40                  45
   Phe Tyr Ser Gly Gly Thr Lys Ser Val Met Gln Ile Val Ile Met Phe
       50                  55                  60
   Met Phe Ser Ile Leu Phe Phe Gly Ile Met Asn Asp Val Gly Leu Phe
   65                  70                  75                  80
   Arg Pro Met Ile Gly Gly Leu Ile Lys Leu Thr Arg Gly Asn Ile Val
                   85                  90                  95
   Ala Val Ser Val Gly Thr Val Leu Val Ser Val Val Ala Gln Leu Asp
               100                 105                 110
   Gly Ala Gly Ala Thr Thr Phe Leu Leu Val Val Pro Ala Leu Leu Pro
           115                 120                 125
   Leu Tyr Lys Arg Leu His Met Asn Pro Tyr Leu Leu Phe Leu Leu Leu
       130                 135                 140
   Thr Ser Ser Ala Gly Leu Ile Asn Leu Leu Pro Trp Gly Gly Pro Thr
   145                 150                 155                 160
   Gly Arg Val Ala Ser Val Leu Gly Ala Asp Val Gly Glu Leu Tyr Lys
                   165                 170                 175
   Pro Leu Leu Thr Val Gln Ile Ile Gly Val Val Phe Ile Leu Ala Leu
               180                 185                 190
   Ser Leu Leu Leu Gly Val Arg Glu Lys Arg Arg Ile Val Arg Glu Leu
           195                 200                 205
   Gly Ala Leu Pro Ala Val Ala Asp Leu Ile Lys Pro Val Pro Leu Ser
   210 215 220
   Glu Glu Glu Gln Lys Leu Ala Arg Pro Lys Leu Phe Trp Trp Asn Val
   225 230 235 240
   Leu Leu Phe Leu Ala Ala Met Ser Leu Leu Phe Ser Gly Ile Phe Pro
   245 250 255
   Pro Gly Tyr Val Phe Met Leu Ala Ala Thr Ala Ala Leu Leu Leu Asn
   260 265 270
   Tyr Arg Ser Pro Gln Glu Gln Met Glu Arg Ile Tyr Ala His Ala Gly
   275 280 285
   Gly Ala Val Met Met Ala Ser Ile Ile Leu Ala Ala Gly Thr Phe Leu
   290 295 300
   Gly Ile Leu Lys Gly Ala Gly Met Leu Asp Ala Ile Ser Lys Asp Ile
   305 310 315 320
   Val His Ile Leu Pro Asp Ala Leu Leu Pro Tyr Leu His Ile Ala Ile
   325 330 335
   Gly Val Leu Gly Ile Pro Leu Glu Leu Val Leu Ser Thr Asp Ala Tyr
   340 345 350
   Tyr Phe Gly Leu Phe Pro Ile Val Glu Gln Ile Thr Ser Gln Ala Gly
   355 360 365
   Val Ala Pro Glu Ala Ala Gly Tyr Ala Met Leu Ile Gly Ser Ile Val
   370 375 380
   Gly Thr Phe Val Thr Pro Leu Ser Pro Ala Leu Trp Met Gly Leu Gly
   385 390 395 400
   Leu Ala Lys Leu Ser Met Gly Lys His Ile Arg Tyr Ser Phe Phe Trp
   405 410 415
   Ala Trp Gly Leu Ser Leu Ala Ile Leu Ala Ser Ser Ile Ala Ala Gly
   420 425 430
   Ile Val Pro Leu Pro
   435
<210> 13
   <211> 1155
   <212> ADN
   <Z13> Neisseria meningitidis
<220>
   <221> CDS
   <222> (1)..(1152)
<400> 13
   atg ggc atc cat ctc gac ttc ggc att agt cct aaa acg ttc cga cag 48
   Met Gly Ile His Leu Asp Phe Gly Ile Ser Pro Lys Thr Phe Arg Gln
    1                   5                       10                      15
   act tat ctg tat caa aag ccc aag ctc ttt aaa gga gcg gtt cgg aat    96
   Thr Tyr Leu Tyr Gln Lys Pro Lys Leu Phe Lys Gly Ala Val Arg Asn
                   20                  25                  30
   ctc gaa gcc gca tct tgt aaa tat atc aac gag ata tac caa cga gca    144
   Leu Glu Ala Ala Ser Cys Lys Tyr Ile Asn Glu Ile Tyr Gln Arg Ala
               35                  40                  45
   gac cca acc gca ccg ctg ttt cat ctg cgt aaa aaa ggc gca atc gtt    192
   Asp Pro Thr Ala Pro Leu Phe His Leu Arg Lys Lys Gly Ala Ile Val
           50                  55                  60
   cct aaa gaa gaa tac gtc gaa agt ttc gac gat ttg ggc aaa act cgc    240
   Pro Lys Glu Glu Tyr Val Glu Ser Phe Asp Asp Leu Gly Lys Thr Arg
    65                  70                  75                  80
   tac cgt ttt att aaa tcc gtt atc tac gaa cat atg aag aat ggt gcg    288
   Tyr Arg Phe Ile Lys Ser Val Ile Tyr Glu His Met Lys Asn Gly Ala
                        85                  90                  95
   tcg tta gtc tat aac cat att aac aac gag ccg ttt tca gac cat atc    336
   Ser Leu Val Tyr Asn His Ile Asn Asn Glu Pro Phe Ser Asp His Ile
                   100                 105                 110
   gcc cgt caa gtc gcc cgc ttt gcc ggc gca cat act att gtt agt gga    384
   Ala Arg Gln Val Ala Arg Phe Ala Gly Ala His Thr Ile Val Ser Gly
               115                 120                 125
   tat ctt gct ttt ggc agc gac gaa tct tat aaa aac cat tgg gat acc    432
   Tyr Leu Ala Phe Gly Ser Asp Glu Ser Tyr Lys Asn His Trp Asp Thr
           130                 135                 140
   cgc gat gtg tat gcc atc cag ctt ttc ggc aag aaa cgt tgg caa ctt    480
   Arg Asp Val Tyr Ala Ile Gln Leu Phe Gly Lys Lys Arg Trp Gln Leu
   145                 150                 155                 160
   act gcc cct gat ttc cct atg cca ttg tat atg caa cag act aaa gat    528
   Thr Ala Pro Asp Phe Pro Met Pro Leu Tyr Met Gln Gln Thr Lys Asp
                       165                 170                 175
   act gat att tcc att cct gaa cat atc gat atg gat att atc ctt gaa    576
   Thr Asp Ile Ser Ile Pro Glu His Ile Asp Met Asp Ile Ile Leu Glu
                   180                 185                 190
   gca ggt gat gtc ctc tac atc cca cgc ggt tgg tgg cac aga cct atc    624
   Ala Gly Asp Val Leu Tyr Ile Pro Arg Gly Trp Trp His Arg Pro Ile
               195                 200                 205
   ccg ctc ggc tgt gaa acc ttc cac ttc gct gtc ggt acc ttc ccg ccc    672
   Pro Leu Gly Cys Glu Thr Phe His Phe Ala Val Gly Thr Phe Pro Pro
           210                 215                 220
   aac ggc tat aat tac ctc gag tgg cta atg aag aaa ttc ccc acg ata    720
   Asn Gly Tyr Asn Tyr Leu Glu Trp Leu Met Lys Lys Phe Pro Thr Ile
   225                 230                 235                 240
   gaa agt ctg cgc cac agt ttc tca gac tgg gag caa gat agg acg cgt    768
   Glu Ser Leu Arg His Ser Phe Ser Asp Trp Glu Gln Asp Arg Thr Arg
                   245                     250                     255
   atc aac gat act gcc gca caa att gct gcc atg att gcc gac ccc gtc    816
   Ile Asn Asp Thr Ala Ala Gln Ile Ala Ala Met Ile Ala Asp Pro Val
               260                     265                     270
   aat tac gaa gcc ttc agt gaa gac ttc ctc ggc aaa gaa cgc acc gat    864
   Asn Tyr Glu Ala Phe Ser Glu Asp Phe Leu Gly Lys Glu Arg Thr Asp
           275                     280                     285
   acc gct ttt cat ctc gaa cag ttc gcg aat ccc aac gct act ccg ctt    912
   Thr Ala Phe His Leu Glu Gln Phe Ala Asn Pro Asn Ala Thr Pro Leu
       290                     295                     300
   tca gac gac gtc agg ttg aga cta aat gcc aat aat ttg gat acg ttg    960
   Ser Asp Asp Val Arg Leu Arg Leu Asn Ala Asn Asn Leu Asp Thr Leu
   305                     310                     315                     320
   gaa aag gga tat ttg att ggg aat ggg atg aag ata agc gta gat gaa    1008
   Glu Lys Gly Tyr Leu Ile Gly Asn Gly Met Lys Ile Ser Val Asp Glu
                   325                     330                     335
   ttg ggg aaa aaa gtg tta gaa cac atc ggt aag aat gaa ccg tta ttg    1056
   Leu Gly Lys Lys Val Leu Glu His Ile Gly Lys Asn Glu Pro Leu Leu
               340                     345                     350
   ttg aaa aat cta ctg gtt aac ttc aat cag gga aaa cat gaa gaa gtt    1104
   Leu Lys Asn Leu Leu Val Asn Phe Asn Gln Gly Lys His Glu Glu Val
           355                     360                     365
   agg aag ttg att tat cag ttg ata gag tta gat ttt ctg gaa ctt ttg    1152
   Arg Lys Leu Ile Tyr Gln Leu Ile Glu Leu Asp Phe Leu Glu Leu Leu
       370                     375                     380
   tga                                                                1155
<210> 14
   <211> 384
   <212> PRT
   <213> Neisseria meningitidis
<400> 14
   Met Gly Ile His Leu Asp Phe Gly Ile Ser Pro Lys Thr Phe Arg Gln
   1 5 10 15
   Thr Tyr Leu Tyr Gln Lys Pro Lys Leu Phe Lys Gly Ala Val Arg Asn
   20 25 30
   Leu Glu Ala Ala Ser Cys Lys Tyr Ile Asn Glu Ile Tyr Gln Arg Ala
   35 40 45
   Asp Pro Thr Ala Pro Leu Phe His Leu Arg Lys Lys Gly Ala Ile Val
   50 55 60
   Pro Lys Glu Glu Tyr Val Glu Ser Phe Asp Asp Leu Gly Lys Thr Arg
   65 70 75 80
   Tyr Arg Phe Ile Lys Ser Val Ile Tyr Glu His Met Lys Asn Gly Ala
   85 90 95
   Ser Leu Val Tyr Asn His Ile Asn Asn Glu Pro Phe Ser Asp His Ile
   100 105 110
   Ala Arg Gln Val Ala Arg Phe Ala Gly Ala His Thr Ile Val Ser Gly
   115 120 125
   Tyr Leu Ala Phe Gly Ser Asp Glu Ser Tyr Lys Asn His Trp Asp Thr
   130 135 140
   Arg Asp Val Tyr Ala Ile Gln Leu Phe Gly Lys Lys Arg Trp Gln Leu
   145 150 155 160
   Thr Ala Pro Asp Phe Pro Met Pro Leu Tyr Met Gln Gln Thr Lys Asp
   165 170 175
   Thr Asp Ile Ser Ile Pro Glu His Ile Asp Met Asp Ile Ile Leu Glu
   180 185 190
   Ala Gly Asp Val Leu Tyr Ile Pro Arg Gly Trp Trp His Arg Pro Ile
   195 200 205
   Pro Leu Gly Cys Glu Thr Phe His Phe Ala Val Gly Thr Phe Pro Pro
   210 215 220
   Asn Gly Tyr Asn Tyr Leu Glu Trp Leu Met Lys Lys Phe Pro Thr Ile
   225 230 235 240
   Glu Ser Leu Arg His Ser Phe Ser Asp Trp Glu Gln Asp Arg Thr Arg
   245 250 255
   Ile Asn Asp Thr Ala Ala Gln Ile Ala Ala Met Ile Ala Asp Pro Val
   260 265 270
   Asn Tyr Glu Ala Phe Ser Glu Asp Phe Leu Gly Lys Glu Arg Thr Asp
   275 280 285
   Thr Ala Phe His Leu Glu Gln Phe Ala Asn Pro Asn Ala Thr Pro Leu
   290 295 300
   Ser Asp Asp Val Arg Leu Arg Leu Asn Ala Asn Asn Leu Asp Thr Leu
   305 310 315 320
   Glu Lys Gly Tyr Leu Ile Gly Asn Gly Met Lys Ile Ser Val Asp Glu
   325 330 335
   Leu Gly Lys Lys Val Leu Glu His Ile Gly Lys Asn Glu Pro Leu Leu
   340 345 350
   Leu Lys Asn Leu Leu Val Asn Phe Asn Gln Gly Lys His Glu Glu Val
   355 360 365
   Arg Lys Leu Ile Tyr Gln Leu Ile Glu Leu Asp Phe Leu Glu Leu Leu
   370 375 380
<210> 15
   <211> 717
   <212> ADN
   <213> Neisseria meningitidis
<220>
   <221> CDS
   <222> (1) .. (714)
<400> 15
   atg aat aga ccc aag caa ccc ttc ttc cgt ccc gaa gtc gcc gtt gcc      48
   Met Asn Arg Pro Lys Gln Pro Phe Phe Arg Pro Glu Val Ala Val Ala
   1               5                   10                  15
   cgc caa acc agc ctg acg ggt aaa gtg att ctg aca cga ccg ttg tca      96
   Arg Gln Thr Ser Leu Thr Gly Lys Val Ile Leu Thr Arg Pro Leu Ser
               20                  25                  30
   ttt tcc cta tgg acg aca ttt gca tcg ata tct gcg tta ttg att atc      144
   Phe Ser Leu Trp Thr Thr Phe Ala Ser Ile Ser Ala Leu Leu Ile Ile
           35                  40                  45
   ctg ttt ttg ata ttt ggt aac tat acg cga aag aca aca gtg gag gga      192
   Leu Phe Leu Ile Phe Gly Asn Tyr Thr Arg Lys Thr Thr Val Glu Gly
       50                  55                  60
   caa att tta cct gca tcg ggc gta atc agg gtg tat gca ccg gat acg      240
   Gln Ile Leu Pro Ala Ser Gly Val Ile Arg Val Tyr Ala Pro Asp Thr
   65                  70                  75                  80
   ggg aca att aca gcg aaa ttc gtg gaa gat gga gaa aag gtt aag gct      288
   Gly Thr Ile Thr Ala Lys Phe Val Glu Asp Gly Glu Lys Val Lys Ala
                   85                  90                  95
   ggc gac aag cta ttt gcg ctt tcg acc tca cgt ttc ggc gca gga gat      336
   Gly Asp Lys Leu Phe Ala Leu Ser Thr Ser Arg Phe Gly Ala Gly Asp
               100                 105                 110
   agc gtg cag cag cag ttg aaa acg gag gca gtt ttg aag aaa acg ttg      384
   Ser Val Gln Gln Gln Leu Lys Thr Glu Ala Val Leu Lys Lys Thr Leu
           115                 120                 125
   gca gaa cag gaa ctg ggt cgt ctg aag ctg ata cac ggg aat gaa acg      432
   Ala Glu Gln Glu Leu Gly Arg Leu Lys Leu Ile His Gly Asn Glu Thr
       130                 135                 140
   cgc agc ctt aaa gca act gtc gaa cgt ttg gaa aac cag aaa ctc cat      480
   Arg Ser Leu Lys Ala Thr Val Glu Arg Leu Glu Asn Gln Lys Leu His
   145                 150                 155                 160
   att tcg caa cag ata gac ggt cag aaa agg cgc att aga ctt gcg gaa      528
   Ile Ser Gln Gln Ile Asp Gly Gln Lys Arg Arg Ile Arg Leu Ala Glu
                   165                 170                 175
   gaa atg ttg cag aaa tat cgt ttc cta tcc gcc aat gat gca gtg cca      576
   Glu Met Leu Gln Lys Tyr Arg Phe Leu Ser Ala Asn Asp Ala Val Pro
               180                 185                 190
   aaa caa gaa atg atg aat gtc aag gca gag ctt tta gag cag aaa gcc      624
   Lys Gln Glu Met Met Asn Val Lys Ala Glu Leu Leu Glu Gln Lys Ala
           195                 200                 205
   aaa ctt gat gcc tac cgc cga gaa gaa gtc ggg ctg ctt cag gaa atc      672
   Lys Leu Asp Ala Tyr Arg Arg Glu Glu Val Gly Leu Leu Gln Glu Ile
       210                 215                 220
   cgc acg cag aat ctg aca ttg gcc agc ctc ccc caa gcg gca tga        717
   Arg Thr Gln Asn Leu Thr Leu Ala Ser Leu Pro Gln Ala Ala
   225                 230                 235
<210> 16
   <211> 238
   <212> PRT
   <213> Neisseria meningitidis
<400> 16
   Met Asn Arg Pro Lys Gln Pro Phe Phe Arg Pro Glu Val Ala Val Ala
   1 5 10 15
   Arg Gln Thr Ser Leu Thr Gly Lys Val Ile Leu Thr Arg Pro Leu Ser
   20 25 30
   Phe Ser Leu Trp Thr Thr Phe Ala Ser Ile Ser Ala Leu Leu Ile Ile
   35 40 45
   Leu Phe Leu Ile Phe Gly Asn Tyr Thr Arg Lys Thr Thr Val Glu Gly
   50 55 60
   Gln Ile Leu Pro Ala Ser Gly Val Ile Arg Val Tyr Ala Pro Asp Thr
   65 70 75 80
   Gly Thr Ile Thr Ala Lys Phe Val Glu Asp Gly Glu Lys Val Lys Ala
   85 90 95
   Gly Asp Lys Leu Phe Ala Leu Ser Thr Ser Arg Phe Gly Ala Gly Asp
   100 105 110
   Ser Val Gln Gln Gln Leu Lys Thr Glu Ala Val Leu Lys Lys Thr Leu
   115 120 125
   Ala Glu Gln Glu Leu Gly Arg Leu Lys Leu Ile His Gly Asn Glu Thr
   130 135 140
   Arg Ser Leu Lys Ala Thr Val Glu Arg Leu Glu Asn Gln Lys Leu His
   145 150 155 160
   Ile Ser Gln Gln Ile Asp Gly Gln Lys Arg Arg Ile Arg Leu Ala Glu
   165 170 175
   Glu Met Leu Gln Lys Tyr Arg Phe Leu Ser Ala Asn Asp Ala Val Pro
   180 185 190
   Lys Gln Glu Met Met Asn Val Lys Ala Glu Leu Leu Glu Gln Lys Ala
   195 200 205
   Lys Leu Asp Ala Tyr Arg Arg Glu Glu Val Gly Leu Leu Gln Glu Ile
   210 215 220
   Arg Thr Gln Asn Leu Thr Leu Ala Ser Leu Pro Gln Ala Ala
   225 230 235
<210> 17
   <211> 690
   <212> ADN
   <213> Neisseria meningitidis
<220>
   <221> CDS
   <222> (1)..(687)
<400> 17
   atg atg aat gtc gag gca gag ctt tta gag cag aaa gcc aaa ctt gat      48
   Met Met Asn Val Glu Ala Glu Leu Leu Glu Gln Lys Ala Lys Leu Asp
     1               5                  10                  15
   gcc tac ggc cga gaa gaa gcc ggg ctg ctt cag gaa atc cgc acg cag      96
   Ala Tyr Gly Arg Glu Glu Ala Gly Leu Leu Gln Glu Ile Arg Thr Gln
                20                  25                  30
   aat ctg aca ttg gcc agc ctc ccc aaa cgg cat gag aca gaa caa agc     144
   Asn Leu Thr Leu Ala Ser Leu Pro Lys Arg His Glu Thr Glu Gln Ser
            35                  40                  45
   cag ctt gaa cgc acc atg gcc gat att tct caa gaa gtt ttg gat ttt     192
   Gln Leu Glu Arg Thr Met Ala Asp Ile Ser Gln Glu Val Leu Asp Phe
        50                  55                  60
   gaa atg cgc tct gaa caa atc atc cgt gca gga cgg tcg ggt tat ata     240
   Glu Met Arg Ser Glu Gln Ile Ile Arg Ala Gly Arg Ser Gly Tyr Ile
    65                  70                  75                  80
   gca ata ccg aac gtc gaa gtc gga cag cag gtt gat cct tcc aaa ctg     288
   Ala Ile Pro Asn Val Glu Val Gly Gln Gln Val Asp Pro Ser Lys Leu
                    85                  90                  95
   ctc ttg agc att gtt ccc gaa cgt acc gag cta tat gcc cat cta tat     336
   Leu Leu Ser Ile Val Pro Glu Arg Thr Glu Leu Tyr Ala His Leu Tyr
               100                 105                 110
   atc ccc agc agt gca gca ggc ttt atc aag ccg aaa gac aag gtt gtc     384
   Ile Pro Ser Ser Ala Ala Gly Phe Ile Lys Pro Lys Asp Lys Val Val
           115                 120                 125
   cta cgt tat cag gca tat ccc tat caa aaa ttc ggg ctt gct tcc ggc     432
   Leu Arg Tyr Gln Ala Tyr Pro Tyr Gln Lys Phe Gly Leu Ala Ser Gly
       130                 135                 140
   agt gtc gta tca gta gca aaa acg gca ctg ggc aga cag gaa ttg tcg     480
   Ser Val Val Ser Val Ala Lys Thr Ala Leu Gly Arg Gln Glu Leu Ser
   145                 150                 155                 160
   gga ttg ggc atg gta tcc tcc gat ttg gcg aag agc aac gaa cct gtt     528
   Gly Leu Gly Met Val Ser Ser Asp Leu Ala Lys Ser Asn Glu Pro Val
                   165                 170                 175
   tat ctc gtg aaa ata aaa ccc gac aaa cca acc atc act gca tac ggt     576
   Tyr Leu Val Lys Ile Lys Pro Asp Lys Pro Thr Ile Thr Ala Tyr Gly
               180                 185                 190
   gag gaa aaa ccg ctg caa atc ggc atg acg ttg gaa gca gac atc ctg     624
   Glu Glu Lys Pro Leu Gln Ile Gly Met Thr Leu Glu Ala Asp Ile Leu
   195 200 205
   cac gag aaa cgg cgg ctg tac gaa tgg gta ttg gag ctg att tat agt 672
   His Glu Lys Arg Arg Leu Tyr Glu Trp Val Leu Glu Leu Ile Tyr Ser
   210 215 220
   atg tcg ggc aaa ctg taa 690
   Met Ser Gly Lys Leu
   225
<210> 18
   <211> 229
   <212> PRT
   <213> Neisseria meningitidis
<400> 18
   Met Met Asn Val Glu Ala Glu Leu Leu Glu Gln Lys Ala Lys Leu Asp
   1               5                   10                  15
   Ala Tyr Gly Arg Glu Glu Ala Gly Leu Leu Gln Glu Ile Arg Thr Gln
               20                  25                  30
   Asn Leu Thr Leu Ala Ser Leu Pro Lys Arg His Glu Thr Glu Gln Ser
           35                  40                  45
   Gln Leu Glu Arg Thr Met Ala Asp Ile Ser Gln Glu Val Leu Asp Phe
       50                  55                  60
   Glu Met Arg Ser Glu Gln Ile Ile Arg Ala Gly Arg Ser Gly Tyr Ile
   65                  70                  75                  80
   Ala Ile Pro Asn Val Glu Val Gly Gln Gln Val Asp Pro Ser Lys Leu
                   85                  90                  95
   Leu Leu Ser Ile Val Pro Glu Arg Thr Glu Leu Tyr Ala His Leu Tyr
               100                 105                 110
   Ile Pro Ser Ser Ala Ala Gly Phe Ile Lys Pro Lys Asp Lys Val Val
           115                 120                 125
   Leu Arg Tyr Gln Ala Tyr Pro Tyr Gln Lys Phe Gly Leu Ala Ser Gly
       130                 135                 140
   Ser Val Val Ser Val Ala Lys Thr Ala Leu Gly Arg Gln Glu Leu Ser
   145                 150                 155                 160
   Gly Leu Gly Met Val Ser Ser Asp Leu Ala Lys Ser Asn Glu Pro Val
                   165                 170                 175
   Tyr Leu Val Lys Ile Lys Pro Asp Lys Pro Thr Ile Thr Ala Tyr Gly
               180                 185                 190
   Glu Glu Lys Pro Leu Gln Ile Gly Met Thr Leu Glu Ala Asp Ile Leu
           195                 200                 205
   His Glu Lys Arg Arg Leu Tyr Glu Trp Val Leu Glu Leu Ile Tyr Ser
       210                 215                 220
   Met Ser Gly Lys Leu
   225
<210> 19
   <211> 1743
   <212> ADN
   <213> Neisseria meningitidis
<220>
   <221> CDS
   <222> (1).. (1740)
<400> 19
   atg aaa ttt ttt cct gct cca tgt ctg ttg gtt atc ctg gct gtc ata      48
   Met Lys Phe Phe Pro Ala Pro Cys Leu Leu Val Ile Leu Ala Val Ile
     1               5                  10                  15
   ccc ctt aaa acc tta gct gcc gat gaa aac gat gca gaa ctt atc cgt      96
   Pro Leu Lys Thr Leu Ala Ala Asp Glu Asn Asp Ala Glu Leu Ile Arg
                20                  25                  30
   tcc atg cag cgt cag cag cac ata gat gct gaa ttg tta act gat gca     144
   Ser Met Gln Arg Gln Gln His Ile Asp Ala Glu Leu Leu Thr Asp Ala
            35                  40                  45
   aat gtc cgt ttc gag caa cca ttg gag aag aac aat tat gtc ctg agt     192
   Asn Val Arg Phe Glu Gln Pro Leu Glu Lys Asn Asn Tyr Val Leu Ser
        50                  55                  60
   gaa gat gaa aca ccg tgt act cgg gta aat tac att agt tta gat gat     240
   Glu Asp Glu Thr Pro Cys Thr Arg Val Asn Tyr Ile Ser Leu Asp Asp
    65                  70                  75                  80
   aag acg gcg cgc aaa ttt tct ttt ctt cct tct gtg ctc atg aaa gaa     288
   Lys Thr Ala Arg Lys Phe Ser Phe Leu Pro Ser Val Leu Met Lys Glu
                    85                  90                  95
   aca gct ttt aaa act ggg atg tgt tta ggt tcc aat aat ttg agc agg     336
   Thr Ala Phe Lys Thr Gly Met Cys Leu Gly Ser Asn Asn Leu Ser Arg
               100                 105                 110
   cta caa aaa gcc gcg caa cag ata ctg att gtg cgt ggc tac ctc act     384
   Leu Gln Lys Ala Ala Gln Gln Ile Leu Ile Val Arg Gly Tyr Leu Thr
           115                 120                 125
   tcc caa gct att atc caa cca cag aat atg gat tcg gga att ctg aaa     432
   Ser Gln Ala Ile Ile Gln Pro Gln Asn Met Asp Ser Gly Ile Leu Lys
       130                 135                 140
   tta cgg gta tca gca ggc gaa atc agg gat atc cgc tat gaa gaa aaa     480
   Leu Arg Val Ser Ala Gly Glu Ile Arg Asp Ile Arg Tyr Glu Glu Lys
   145                 150                 155                 160
   cgg gat gcg aag tct gcc gag ggc agt att agt gca ttc aat aac aaa     528
   Arg Asp Ala Lys Ser Ala Glu Gly Ser Ile Ser Ala Phe Asn Asn Lys
                   165                 170                 175
   ctt ccc tta tat agg aac aaa att ctc aat ctt cgc gat gta gag cag     576
   Leu Pro Leu Tyr Arg Asn Lys Ile Leu Asn Leu Arg Asp Val Glu Gln
               180                 185                 190
   ggc ttg gaa aac ctg cgt cgt ttg ccg agt gtt aaa aca gat att cag      624
   Gly Leu Glu Asn Leu Arg Arg Leu Pro Ser Val Lys Thr Asp Ile Gln
           195                 200                 205
   att ata ccg tcc gaa gaa gaa ggc aaa agc gat tta cag atc aaa tgg      672
   Ile Ile Pro Ser Glu Glu Glu Gly Lys Ser Asp Leu Gln Ile Lys Trp
       210                 215                 220
   cag cag aat aaa ccc ata cgg ttc agt atc ggt ata gat gat gcg ggc      720
   Gln Gln Asn Lys Pro Ile Arg Phe Ser Ile Gly Ile Asp Asp Ala Gly
   225                 230                 235                 240
   ggc aaa acg acc ggc aaa tat caa gga aat gtc gct tta tcg tcc gat      768
   Gly Lys Thr Thr Gly Lys Tyr Gln Gly Asn Val Ala Leu Ser Ser Asp
                   245                 250                 255
   aac cct ttg ggc tta agc gat tcg ttt tat gtt tca tat gga cgc ggt      816
   Asn Pro Leu Gly Leu Ser Asp Ser Phe Tyr Val Ser Tyr Gly Arg Gly
               260                 265                 270
   ttg gtg cac aaa acg gac ttg act gct gcc acc ggt acg gaa act gaa      864
   Leu Val His Lys Thr Asp Leu Thr Ala Ala Thr Gly Thr Glu Thr Glu
           275                 280                 285
   agc gga tcc aga agt tac agc gtg cat tat tcg gtg ccc gta aaa aaa      912
   Ser Gly Ser Arg Ser Tyr Ser Val His Tyr Ser Val Pro Val Lys Lys
       290                 295                 300
   tgg ctg ttt tct ttt aat cac aat gga cat cgt tac cac gaa gca acc      960
   Trp Leu Phe Ser Phe Asn His Asn Gly His Arg Tyr His Glu Ala Thr
   305                 310                 315                 320
   gaa ggc tat tcc gtc aat tac gat tac aac ggc aaa caa tat cag agc     1008
   Glu Gly Tyr Ser Val Asn Tyr Asp Tyr Asn Gly Lys Gln Tyr Gln Ser
                   325                 330                 335
   agc ctg gcc gcc gag cgc atg ctt tgg ccc ccc agc ttt cct caa act     1056
   Ser Leu Ala Ala Glu Arg Met Leu Trp Pro Pro Ser Phe Pro Gln Thr
               340                 345                 350
   tca gtc cga atg aaa tta tgg aca cgc caa acc tat aaa tac atc gac     1104
   Ser Val Arg Met Lys Leu Trp Thr Arg Gln Thr Tyr Lys Tyr Ile Asp
           355                 360                 365
   gat gcc gaa atc gaa gtg caa cgc cgc cgc tct gca ggc tgg gaa gcc     1152
   Asp Ala Glu Ile Glu Val Gln Arg Arg Arg Ser Ala Gly Trp Glu Ala
       370                 375                 380
   gaa ttg cgc cac cgt gct tac ctc cac cgt tgg cag ctt gac ggc aag     1200
   Glu Leu Arg His Arg Ala Tyr Leu His Arg Trp Gln Leu Asp Gly Lys
   385                 390                 395                 400
   ttg tct tac aaa cgc ggg acc ggc atg cgc caa agt atg ccc gca cct     1248
   Leu Ser Tyr Lys Arg Gly Thr Gly Met Arg Gln Ser Met Pro Ala Pro
                   405                 410                 415
   gaa gaa aac ggc ggc ggt act att cca gcc aca tcc cgt atg aaa atc     1296
   Glu Glu Asn Gly Gly Gly Thr Ile Pro Ala Thr Ser Arg Met Lys Ile
               420                 425                 430
   ata acc gcc gga ttg gat gca gcg gcc ccg tct atg ttg ggc aaa cag      1344
   Ile Thr Ala Gly Leu Asp Ala Ala Ala Pro Ser Met Leu Gly Lys Gln
           435                 440                 445
   cag ttt ttc tac gca acc gcc att caa gct caa tgg aac aaa acg cct      1392
   Gln Phe Phe Tyr Ala Thr Ala Ile Gln Ala Gln Trp Asn Lys Thr Pro
       450                 455                 460
   ttg gtt gcc caa gac aag ttg tct atc ggc agc cgc tac acc gtt cgc      1440
   Leu Val Ala Gln Asp Lys Leu Ser Ile Gly Ser Arg Tyr Thr Val Arg
   465                 470                 475                 480
   gga ttt gat ggg gag cag agt ctt ttc gga gag cga ggt ttc tac tgg      1488
   Gly Phe Asp Gly Glu Gln Ser Leu Phe Gly Glu Arg Gly Phe Tyr Trp
                   485                 490                 495
   cag aat act tta act tgg tat ttt cat ccg aac cat cag ttc tat ctc      1536
   Gln Asn Thr Leu Thr Trp Tyr Phe His Pro Asn His Gln Phe Tyr Leu
               500                 505                 510
   ggt gcg gac tat ggc cgc gta tct ggc gaa agt gca caa tat gta tcg      1584
   Gly Ala Asp Tyr Gly Arg Val Ser Gly Glu Ser Ala Gln Tyr Val Ser
           515                 520                 525
   ggc aag cag ctg atg ggt gca gtg gtc ggc ttc aga gga ggg cat aaa      1632
   Gly Lys Gln Leu Met Gly Ala Val Val Gly Phe Arg Gly Gly His Lys
       530                 535                 540
   gta ggc ggt atg ttt gct tat gat ctg ttt gcc ggc aag ccg ctt cat      1680
   Val Gly Gly Met Phe Ala Tyr Asp Leu Phe Ala Gly Lys Pro Leu His
   545                 550                 555                 560
   aaa ccc aaa ggc ttt cag acg acc aac acc gtt tac ggc ttc aac ttg      1728
   Lys Pro Lys Gly Phe Gln Thr Thr Asn Thr Val Tyr Gly Phe Asn Leu
                   565                 570                 575
   aat tac agt ttc taa                                                  1743
   Asn Tyr Ser Phe
               580
<210> 20
   <211> 580
   <212> PRT
   <213> Neisseria meningitidis
<400> 20
   Met Lys Phe Phe Pro Ala Pro Cys Leu Leu Val Ile Leu Ala Val Ile
     1               5                  10                  15
   Pro Leu Lys Thr Leu Ala Ala Asp Glu Asn Asp Ala Glu Leu Ile Arg
                20                  25                  30
   Ser Met Gln Arg Gln Gln His Ile Asp Ala Glu Leu Leu Thr Asp Ala
            35                  40                  45
   Asn Val Arg Phe Glu Gln Pro Leu Glu Lys Asn Asn Tyr Val Leu Ser
   50                  55                  60
   Glu Asp Glu Thr Pro Cys Thr Arg Val Asn Tyr Ile Ser Leu Asp Asp
   65                  70                  75                  80
   Lys Thr Ala Arg Lys Phe Ser Phe Leu Pro Ser Val Leu Met Lys Glu
                   85                  90                  95
   Thr Ala Phe Lys Thr Gly Met Cys Leu Gly Ser Asn Asn Leu Ser Arg
               100                 105                 110
   Leu Gln Lys Ala Ala Gln Gln Ile Leu Ile Val Arg Gly Tyr Leu Thr
           115                 120                 125
   Ser Gln Ala Ile Ile Gln Pro Gln Asn Met Asp Ser Gly Ile Leu Lys
       130                 135                 140
   Leu Arg Val Ser Ala Gly Glu Ile Arg Asp Ile Arg Tyr Glu Glu Lys
   145                 150                 155                 160
   Arg Asp Ala Lys Ser Ala Glu Gly Ser Ile Ser Ala Phe Asn Asn Lys
                   165                 170                 175
   Leu Pro Leu Tyr Arg Asn Lys Ile Leu Asn Leu Arg Asp Val Glu Gln
               180                 185                 190
   Gly Leu Glu Asn Leu Arg Arg Leu Pro Ser Val Lys Thr Asp Ile Gln
           195                 200                 205
   Ile Ile Pro Ser Glu Glu Glu Gly Lys Ser Asp Leu Gln Ile Lys Trp
       210                 215                 220
   Gln Gln Asn Lys Pro Ile Arg Phe Ser Ile Gly Ile Asp Asp Ala Gly
   225                 230                 235                 240
   Gly Lys Thr Thr Gly Lys Tyr Gln Gly Asn Val Ala Leu Ser Ser Asp
                   245                 250                 255
   Asn Pro Leu Gly Leu Ser Asp Ser Phe Tyr Val Ser Tyr Gly Arg Gly
               260                 265                 270
   Leu Val His Lys Thr Asp Leu Thr Ala Ala Thr Gly Thr Glu Thr Glu
           275                 280                 285
   Ser Gly Ser Arg Ser Tyr Ser Val His Tyr Ser Val Pro Val Lys Lys
       290                 295                 300
   Trp Leu Phe Ser Phe Asn His Asn Gly His Arg Tyr His Glu Ala Thr
   305                 310                 315                 320
   Glu Gly Tyr Ser Val Asn Tyr Asp Tyr Asn Gly Lys Gln Tyr Gln Ser
                   325                 330                 335
   Ser Leu Ala Ala Glu Arg Met Leu Trp Pro Pro Ser Phe Pro Gln Thr
               340                 345                 350
   Ser Val Arg Met Lys Leu Trp Thr Arg Gln Thr Tyr Lys Tyr Ile Asp
           355                 360                 365
   Asp Ala Glu Ile Glu Val Gln Arg Arg Arg Ser Ala Gly Trp Glu Ala
   370 375 380
   Glu Leu Arg His Arg Ala Tyr Leu His Arg Trp Gln Leu Asp Gly Lys
   385 390 395 400
   Leu Ser Tyr Lys Arg Gly Thr Gly Met Arg Gln Ser Met Pro Ala Pro
   405 410 415
   Glu Glu Asn Gly Gly Gly Thr Ile Pro Ala Thr Ser Arg Met Lys Ile
   420 425 430
   Ile Thr Ala Gly Leu Asp Ala Ala Ala Pro Ser Met Leu Gly Lys Gln
   435 440 445
   Gln Phe Phe Tyr Ala Thr Ala Ile Gln Ala Gln Trp Asn Lys Thr Pro
   450 455 460
   Leu Val Ala Gln Asp Lys Leu Ser Ile Gly Ser Arg Tyr Thr Val Arg
   465 470 475 480
   Gly Phe Asp Gly Glu Gln Ser Leu Phe Gly Glu Arg Gly Phe Tyr Trp
   485 490 495
   Gln Asn Thr Leu Thr Trp Tyr Phe His Pro Asn His Gln Phe Tyr Leu
   500 505 510
   Gly Ala Asp Tyr Gly Arg Val Ser Gly Glu Ser Ala Gln Tyr Val Ser
   515 520 525
   Gly Lys Gln Leu Met Gly Ala Val Val Gly Phe Arg Gly Gly His Lys
   530 535 540
   Val Gly Gly Met Phe Ala Tyr Asp Leu Phe Ala Gly Lys Pro Leu His
   545 550 555 560
   Lys Pro Lys Gly Phe Gln Thr Thr Asn Thr Val Tyr Gly Phe Asn Leu
   565 570 575
   Asn Tyr Ser Phe
   580
<210> 21
   <211> 411
   <212> ADN
   <213> Neisseria meningitidis
<220>
   <221> CDS
   <222> (1) .. (408)
<400> 21
   atg att gaa ttt gtc cga gcc aaa aaa cgg ctg ctt tgg gca ttt gtg     48
   Met Ile Glu Phe Val Arg Ala Lys Lys Arg Leu Leu Trp Ala Phe Val
     1               5                  10                  15
   ctt ttg ctt gtg tgg acg tgc ggt tac cga tac gcc gcc gac aag gcc     96
   Leu Leu Leu Val Trp Thr Cys Gly Tyr Arg Tyr Ala Ala Asp Lys Ala
                20                  25                  30
   gaa gcg aaa caa acc gcc ctg att gcc acc tat cgg cat tct tct atg    144
   Glu Ala Lys Gln Thr Ala Leu Ile Ala Thr Tyr Arg His Ser Ser Met
            35                  40                  45
   gtt gcg gcg gaa caa tac gcc ttg cag ctt aaa aaa gcg cag gac gaa    192
   Val Ala Ala Glu Gln Tyr Ala Leu Gln Leu Lys Lys Ala Gln Asp Glu
        50                  55                  60
   agg cag cgg tgg tac gac ttt tcc caa aaa caa gga aga aag ccc gtg    240
   Arg Gln Arg Trp Tyr Asp Phe Ser Gln Lys Gln Gly Arg Lys Pro Val
    65                  70                  75                  80
   aaa aaa cag tat ccg ccg caa acg aaa aaa gcc ggc tat ctg aaa acc    288
   Lys Lys Gln Tyr Pro Pro Gln Thr Lys Lys Ala Gly Tyr Leu Lys Thr
                    85                  90                  95
   aag gaa gaa ctg ctt gcg gaa ttg gct tgc ctt aaa gcg gaa atg gct    336
   Lys Glu Glu Leu Leu Ala Glu Leu Ala Cys Leu Lys Ala Glu Met Ala
               100                 105                 110
   gcc cta aaa aag ctc gat gcc tta atc tat ggg aaa gaa gtg cgg cag    384
   Ala Leu Lys Lys Leu Asp Ala Leu Ile Tyr Gly Lys Glu Val Arg Gln
           115                 120                 125
   aaa gaa cgc aac tcg tcg cag ggt taa                                411
   Lys Glu Arg Asn Ser Ser Gln Gly
       130                 135
<210> 22
   <211> 136
   <212> PRT
   <213> Neisseria meningitidis
<400> 22
   Met Ile Glu Phe Val Arg Ala Lys Lys Arg Leu Leu Trp Ala Phe Val
   1 5 10 15
   Leu Leu Leu Val Trp Thr Cys Gly Tyr Arg Tyr Ala Ala Asp Lys Ala
   20 25 30
   Glu Ala Lys Gln Thr Ala Leu Ile Ala Thr Tyr Arg His Ser Ser Met
   35 40 45
   Val Ala Ala Glu Gln Tyr Ala Leu Gln Leu Lys Lys Ala Gln Asp Glu
   50 55 60
   Arg Gln Arg Trp Tyr Asp Phe Ser Gln Lys Gln Gly Arg Lys Pro Val
   65 70 75 80
   Lys Lys Gln Tyr Pro Pro Gln Thr Lys Lys Ala Gly Tyr Leu Lys Thr
   85 90 95
   Lys Glu Glu Leu Leu Ala Glu Leu Ala Cys Leu Lys Ala Glu Met Ala
   100 105 110
   Ala Leu Lys Lys Leu Asp Ala Leu Ile Tyr Gly Lys Glu Val Arg Gln
   115 120 125
   Lys Glu Arg Asn Ser Ser Gln Gly
   130 135
<210> 23
   <211> 924
   <212> ADN
   <213> Neisseria meningitidis
<220>
   <221> CDS
   <222> (1) .. (921)
<400> 23
   atg caa tac agc aca ctg gca gga caa acc gac aac tcc ctc gtt tcc     48
   Met Gln Tyr Ser Thr Leu Ala Gly Gln Thr Asp Asn Ser Leu Val Ser
    1               5                  10                  15
   aat aat ttc ggg ttt ttg cgc ctg ccg ctt aat ttt atg ccg tat gaa     96
   Asn Asn Phe Gly Phe Leu Arg Leu Pro Leu Asn Phe Met Pro Tyr Glu
               20                  25                  30
   agt cat gcc gat tgg gtt att acc ggc gtg cct tat gat atg gcg gtt    144
   Ser His Ala Asp Trp Val Ile Thr Gly Val Pro Tyr Asp Met Ala Val
           35                  40                  45
   tca ggg cgt tcc ggc gcg cgt ttc ggt cct gaa gcc atc cgg cgc gcc    192
   Ser Gly Arg Ser Gly Ala Arg Phe Gly Pro Glu Ala Ile Arg Arg Ala
       50                  55                  60
   tcc gtc aac ctc gct tgg gag cac cgc agg ttt cca tgg aca ttt gat    240
   Ser Val Asn Leu Ala Trp Glu His Arg Arg Phe Pro Trp Thr Phe Asp
   65                  70                  75                  80
   gtg cgc gaa cgc ctg aac att att gat tgc ggc gac ttg gtt ttt tct    288
   Val Arg Glu Arg Leu Asn Ile Ile Asp Cys Gly Asp Leu Val Phe Ser
                   85                  90                  95
   ttt ggc gac agc agg gat ttt gtc gaa aaa atg gaa gcg cac gcc ggc    336
   Phe Gly Asp Ser Arg Asp Phe Val Glu Lys Met Glu Ala His Ala Gly
               100                 105                 110
   aaa tta ctt tct tcc ggc aaa cgc tgt ttg agt ttg ggc ggc gac cat    384
   Lys Leu Leu Ser Ser Gly Lys Arg Cys Leu Ser Leu Gly Gly Asp His
           115                 120                 125
   ttc att acc ctc ccg ttg ttg cgc gcc cac gcc cgc tat ttc ggc aaa    432
   Phe Ile Thr Leu Pro Leu Leu Arg Ala His Ala Arg Tyr Phe Gly Lys
       130                 135                 140
   ctc gca ctg att cat ttt gac gcg cac acc gac acc tac gac aac ggc    480
   Leu Ala Leu Ile His Phe Asp Ala His Thr Asp Thr Tyr Asp Asn Gly
   145                 150                 155                 160
   agc gaa tac gac cac ggt acg atg ttc tat acc gcc ccc aag gaa ggc    528
   Ser Glu Tyr Asp His Gly Thr Met Phe Tyr Thr Ala Pro Lys Glu Gly
                   165                 170                 175
   ctc atc gac ccg tcc cgt tcc gta caa atc ggc ata cgt acc gaa cac    576
   Leu Ile Asp Pro Ser Arg Ser Val Gln Ile Gly Ile Arg Thr Glu His
               180                 185                 190
   agt aaa aaa ttg cct ttt act gtg ttg acc gcc ccc caa gtt aat gaa      624
   Ser Lys Lys Leu Pro Phe Thr Val Leu Thr Ala Pro Gln Val Asn Glu
           195                 200                 205
   gac agt gtt gaa gag acc gtc cgt aaa atc aaa gaa acc gtc ggc aat      672
   Asp Ser Val Glu Glu Thr Val Arg Lys Ile Lys Glu Thr Val Gly Asn
       210                 215                 220
   atg ccc gtt tac ctg act ttc gac ata gac tgc ctc gac ccg tcg ttc      720
   Met Pro Val Tyr Leu Thr Phe Asp Ile Asp Cys Leu Asp Pro Ser Phe
   225                 230                 235                 240
   gcc ccc ggg acc ggt acg ccc gta tgc ggc ggc ttg agc agc gac agg      768
   Ala Pro Gly Thr Gly Thr Pro Val Cys Gly Gly Leu Ser Ser Asp Arg
                   245                 250                 255
   gca tta aaa atc cta cgt ggg ctg acg gat ctc gac atc gtc ggt atg      816
   Ala Leu Lys Ile Leu Arg Gly Leu Thr Asp Leu Asp Ile Val Gly Met
               260                 265                 270
   gat gtt gta gaa gtt gcc ccc tct tac gac caa tcc gac att acc gct      864
   Asp Val Val Glu Val Ala Pro Ser Tyr Asp Gln Ser Asp Ile Thr Ala
           275                 280                 285
   ttg gcc ggc gcc aca att gcc ttg gaa atg ctt tac ctt caa ggt gcg      912
   Leu Ala Gly Ala Thr Ile Ala Leu Glu Met Leu Tyr Leu Gln Gly Ala
       290                 295                 300
   aaa aag gac tga                                                      924
   Lys Lys Asp
   305
<210> 24
   <211> 307
   <212> PRT
   <213> Neisseria meningitidis
<400> 24
   Met Gln Tyr Ser Thr Leu Ala Gly Gln Thr Asp Asn Ser Leu Val Ser
   1 5 10 15
   Asn Asn Phe Gly Phe Leu Arg Leu Pro Leu Asn Phe Met Pro Tyr Glu
   20 25 30
   Ser His Ala Asp Trp Val Ile Thr Gly Val Pro Tyr Asp Met Ala Val
   35 40 45
   Ser Gly Arg Ser Gly Ala Arg Phe Gly Pro Glu Ala Ile Arg Arg Ala
   50 55 60
   Ser Val Asn Leu Ala Trp Glu His Arg Arg Phe Pro Trp Thr Phe Asp
   65 70 75 80
   Val Arg Glu Arg Leu Asn Ile Ile Asp Cys Gly Asp Leu Val Phe Ser
   85 90 95
   Phe Gly Asp Ser Arg Asp Phe Val Glu Lys Met Glu Ala His Ala Gly
   100 105 110
   Lys Leu Leu Ser Ser Gly Lys Arg Cys Leu Ser Leu Gly Gly Asp His
   115 120 125
   Phe Ile Thr Leu Pro Leu Leu Arg Ala His Ala Arg Tyr Phe Gly Lys
   130 135 140
   Leu Ala Leu Ile His Phe Asp Ala His Thr Asp Thr Tyr Asp Asn Gly
   145 150 155 160
   Ser Glu Tyr Asp His Gly Thr Met Phe Tyr Thr Ala Pro Lys Glu Gly
   165 170 175
   Leu Ile Asp Pro Ser Arg Ser Val Gln Ile Gly Ile Arg Thr Glu His
   180 185 190
   Ser Lys Lys Leu Pro Phe Thr Val Leu Thr Ala Pro Gln Val Asn Glu
   195 200 205
   Asp Ser Val Glu Glu Thr Val Arg Lys Ile Lys Glu Thr Val Gly Asn
   210 215 220
   Met Pro Val Tyr Leu Thr Phe Asp Ile Asp Cys Leu Asp Pro Ser Phe
   225 230 235 240
   Ala Pro Gly Thr Gly Thr Pro Val Cys Gly Gly Leu Ser Ser Asp Arg
   245 250 255
   Ala Leu Lys Ile Leu Arg Gly Leu Thr Asp Leu Asp Ile Val Gly Met
   260 265 270
   Asp Val Val Glu Val Ala Pro Ser Tyr Asp Gln Ser Asp Ile Thr Ala
   275 280 285
   Leu Ala Gly Ala Thr Ile Ala Leu Glu Met Leu Tyr Leu Gln Gly Ala
   290 295 300
   Lys Lys Asp
   305
<210> 25
   <211> 426
   <212> ADN
   <213> Neisseria meningitidis
<220>
   <221> CDS
   <222> (1)..(423)
<400> 25
   atg gag cag tcg ggc aaa ttc agt tgg tct gcg gca gct ttt tgg gac      48
   Met Glu Gln Ser Gly Lys Phe Ser Trp Ser Ala Ala Ala Phe Trp Asp
   1               5                   10                  15
   att ccc tac ccc gtc acc agg cgg att gcc tca agt ttg tat tcg acc      96
   Ile Pro Tyr Pro Val Thr Arg Arg Ile Ala Ser Ser Leu Tyr Ser Thr
               20                  25                  30
   gaa tat ttt gtc gta tgc ttt ctg cgt ttg atg cca ctc tct ccg tgt      144
   Glu Tyr Phe Val Val Cys Phe Leu Arg Leu Met Pro Leu Ser Pro Cys
           35                  40                  45
   aat ctg tat ttt gtc acc cat ctg cgt acc aat gaa tcg gaa ata gaa      192
   Asn Leu Tyr Phe Val Thr His Leu Arg Thr Asn Glu Ser Glu Ile Glu
       50                  55                  60
   aga tgg tct gct gtt ccc tgc caa ata gta ttg aac gac ggc aag tcg      240
   Arg Trp Ser Ala Val Pro Cys Gln Ile Val Leu Asn Asp Gly Lys Ser
   65                  70                  75                  80
   gaa ttc ggc gga ttc gca ttt gaa gtg caa ctt tcc cta aca gaa aaa      288
   Glu Phe Gly Gly Phe Ala Phe Glu Val Gln Leu Ser Leu Thr Glu Lys
                   85                  90                  95
   ggc cag tat gcg gta gca tac gac ctt tcc tgc aag aaa gat tgc cat      336
   Gly Gln Tyr Ala Val Ala Tyr Asp Leu Ser Cys Lys Lys Asp Cys His
               100                 105                 110
   gag cta cac gca act gac cca agg cga acg ata cca cat cca ata cct      384
   Glu Leu His Ala Thr Asp Pro Arg Arg Thr Ile Pro His Pro Ile Pro
           115                 120                 125
   gtc ccg cca ctg cac cgt cac cga aat cgc caa aca gct taa              426
   Val Pro Pro Leu His Arg His Arg Asn Arg Gln Thr Ala
       130                 135                 140
<210> 26
   <211> 141
   <212> PRT
   <213> Neisseria meningitidis
<400> 26
   Met Glu Gln Ser Gly Lys Phe Ser Trp Ser Ala Ala Ala Phe Trp Asp
   1 5 10 15
   Ile Pro Tyr Pro Val Thr Arg Arg Ile Ala Ser Ser Leu Tyr Ser Thr
   20 25 30
   Glu Tyr Phe Val Val Cys Phe Leu Arg Leu Met Pro Leu Ser Pro Cys
   35 40 45
   Asn Leu Tyr Phe Val Thr His Leu Arg Thr Asn Glu Ser Glu Ile Glu
   50 55 60
   Arg Trp Ser Ala Val Pro Cys Gln Ile Val Leu Asn Asp Gly Lys Ser
   65 70 75 80
   Glu Phe Gly Gly Phe Ala Phe Glu Val Gln Leu Ser Leu Thr Glu Lys
   85 90 95
   Gly Gln Tyr Ala Val Ala Tyr Asp Leu Ser Cys Lys Lys Asp Cys His
   100 105 110
   Glu Leu His Ala Thr Asp Pro Arg Arg Thr Ile Pro His Pro Ile Pro
   115 120 125
   Val Pro Pro Leu His Arg His Arg Asn Arg Gln Thr Ala
   130 135 140
<210> 27
   <211> 351
   <212> ADN
   <213> Neisseria meningitidis
<220>
   <221> CDS
   <222> (1) .. (348)
<400> 27
   atg caa aac ggc ggg gga aag att tac cag acg gcg gac aat gtg gaa      48
   Met Gln Asn Gly Gly Gly Lys Ile Tyr Gln Thr Ala Asp Asn Val Glu
     1               5                  10                  15
   ggg att atg ctg ttg aag gta gta cct gag cgt acc gtt tcg gca gat      96
   Gly Ile Met Leu Leu Lys Val Val Pro Glu Arg Thr Val Ser Ala Asp
                20                  25                  30
   gca aaa acc aga gac ccg atg tgg gac aat gcg gct tta cag acc agc     144
   Ala Lys Thr Arg Asp Pro Met Trp Asp Asn Ala Ala Leu Gln Thr Ser
            35                  40                  45
   gaa ggc gta aat ttt att gct cgt ttc cta gga ttt ttt agc gat ggg     192
   Glu Gly Val Asn Phe Ile Ala Arg Phe Leu Gly Phe Phe Ser Asp Gly
        50                  55                  60
   gaa tac cgc tat gtg gat gtc ctg caa ccc aac cat tcc gat att att     240
   Glu Tyr Arg Tyr Val Asp Val Leu Gln Pro Asn His Ser Asp Ile Ile
    65                  70                  75                  80
   cgg tat tca ggt aaa gat ttt ccg cta aat caa ata ctt aac cat ata     288
   Arg Tyr Ser Gly Lys Asp Phe Pro Leu Asn Gln Ile Leu Asn His Ile
                    85                  90                  95
   cac ccc gcc cgt tat gcg gta acg ttc gaa aac aat gtc gat tcc aag     336
   His Pro Ala Arg Tyr Ala Val Thr Phe Glu Asn Asn Val Asp Ser Lys
               100                 105                 110
   ctg cgc agg cac tga                                                 351
   Leu Arg Arg His
           115
<210> 28
   <211> 116
   <212> PRT
   <213> Neisseria meningitidis
<400> 28
   Met Gln Asn Gly Gly Gly Lys Ile Tyr Gln Thr Ala Asp Asn Val Glu
     1               5                  10                  15
   Gly Ile Met Leu Leu Lys Val Val Pro Glu Arg Thr Val Ser Ala Asp
                20                  25                  30
   Ala Lys Thr Arg Asp Pro Met Trp Asp Asn Ala Ala Leu Gln Thr Ser
   35 40 45
   Glu Gly Val Asn Phe Ile Ala Arg Phe Leu Gly Phe Phe Ser Asp Gly
   50 55 60
   Glu Tyr Arg Tyr Val Asp Val Leu Gln Pro Asn His Ser Asp Ile Ile
   65 70 75 80
   Arg Tyr Ser Gly Lys Asp Phe Pro Leu Asn Gln Ile Leu Asn His Ile
   85 90 95
   His Pro Ala Arg Tyr Ala Val Thr Phe Glu Asn Asn Val Asp Ser Lys
   100 105 110
   Leu Arg Arg His
   115
<210> 29
   <211> 1404
   <212> ADN
   <213> Neisseria meningitidis
<220>
   <221> CDS
   <222> (1)..(1401)
<400> 29
   atg aca ttg ctc aat cta atg ata atg caa gat tac ggt att tcc gtt      48
   Met Thr Leu Leu Asn Leu Met Ile Met Gln Asp Tyr Gly Ile Ser Val
     1               5                  10                  15
   tgc ctg aca ctg acg ccc tat ttg caa cat gaa cta ttt tcg gct atg      96
   Cys Leu Thr Leu Thr Pro Tyr Leu Gln His Glu Leu Phe Ser Ala Met
                20                  25                  30
   aaa tcc tat ttt tcc aaa tat atc cta ccc gtt tca ctt ttt acc ttg     144
   Lys Ser Tyr Phe Ser Lys Tyr Ile Leu Pro Val Ser Leu Phe Thr Leu
            35                  40                  45
   cca cta tcc ctt tcc cca tcc gtt tcg gct ttt acg ctg cct gaa gca     192
   Pro Leu Ser Leu Ser Pro Ser Val Ser Ala Phe Thr Leu Pro Glu Ala
        50                  55                  60
   tgg cgg gcg gcg cag caa cat tcg gct gat ttt caa gcg tcc cat tac     240
   Trp Arg Ala Ala Gln Gln His Ser Ala Asp Phe Gln Ala Ser His Tyr
    65                  70                  75                  80
   cag cgt gat gca gtg cgc gca cgg caa caa caa gcc aag gcc gca ttc     288
   Gln Arg Asp Ala Val Arg Ala Arg Gln Gln Gln Ala Lys Ala Ala Phe
                    85                  90                  95
   ctt ccc cat gta tcc gcc aat gcc agc tac cag cgc cag ccg cca tcg     336
   Leu Pro His Val Ser Ala Asn Ala Ser Tyr Gln Arg Gln Pro Pro Ser
               100                 105                 110
   att tct tcc acc cgc gaa aca cag gga tgg agc gtg cag gtg gga caa     384
   Ile Ser Ser Thr Arg Glu Thr Gln Gly Trp Ser Val Gln Val Gly Gln
           115                 120                 125
   acc tta ttt gac gct gcc aaa ttt gca caa tac cgc caa agc agg ttc     432
   Thr Leu Phe Asp Ala Ala Lys Phe Ala Gln Tyr Arg Gln Ser Arg Phe
       130                 135                 140
   gat acg cag gct gca gaa cag cgt ttc gat gcg gca cgc gaa gaa ttg     480
   Asp Thr Gln Ala Ala Glu Gln Arg Phe Asp Ala Ala Arg Glu Glu Leu
   145                 150                 155                 160
   ctg ttg aaa gtt gcc gaa agt tat ttc aac gtt tta ctc agc cga gac     528
   Leu Leu Lys Val Ala Glu Ser Tyr Phe Asn Val Leu Leu Ser Arg Asp
                   165                 170                 175
   acc gtt gcc gcc cat gcg gcg gaa aaa gag gct tat gcc cag cag gta     576
   Thr Val Ala Ala His Ala Ala Glu Lys Glu Ala Tyr Ala Gln Gln Val
               180                 185                 190
   agg cag gcg cag gct tta ttc aat aaa ggt gct gcc acc gcg ctg gat     624
   Arg Gln Ala Gln Ala Leu Phe Asn Lys Gly Ala Ala Thr Ala Leu Asp
           195                 200                 205
   att cac gaa gcc aaa gcc ggt tac gac aat gcc ctg gcc caa gaa atc     672
   Ile His Glu Ala Lys Ala Gly Tyr Asp Asn Ala Leu Ala Gln Glu Ile
       210                 215                 220
   gcc gta ttg gct gag aaa caa acc tat gaa aac cag ttg aac gac tac     720
   Ala Val Leu Ala Glu Lys Gln Thr Tyr Glu Asn Gln Leu Asn Asp Tyr
   225                 230                 235                 240
   acc gac ctg gat agc aaa caa atc gag gcc ata gat acc gcc aac ctg     768
   Thr Asp Leu Asp Ser Lys Gln Ile Glu Ala Ile Asp Thr Ala Asn Leu
                   245                 250                 255
   ttg gca cgc tat ctg ccc aag ctg gaa cgt tac agt ctg gat gaa tgg     816
   Leu Ala Arg Tyr Leu Pro Lys Leu Glu Arg Tyr Ser Leu Asp Glu Trp
               260                 265                 270
   cag cgc att gcc tta tcc aac aat cat gaa tac cgg atg cag cag ctt     864
   Gln Arg Ile Ala Leu Ser Asn Asn His Glu Tyr Arg Met Gln Gln Leu
           275                 280                 285
   gcc ctg caa agc agc gga cag gcg ctt cgg gca gca cag aac agc cgc     912
   Ala Leu Gln Ser Ser Gly Gln Ala Leu Arg Ala Ala Gln Asn Ser Arg
       290                 295                 300
   tat ccc acc gtt tct gcc cat gtc ggc tat cag aat aac ctc tac act     960
   Tyr Pro Thr Val Ser Ala His Val Gly Tyr Gln Asn Asn Leu Tyr Thr
   305                 310                 315                 320
   tca tct gcg cag aat aat gac tac cac tat cgg ggc aaa ggg atg agc     1008
   Ser Ser Ala Gln Asn Asn Asp Tyr His Tyr Arg Gly Lys Gly Met Ser
                   325                 330                 335
   gtc ggc gta cag ttg aat ttg ccg ctt tat acc ggc gga gaa ttg tcg     1056
   Val Gly Val Gln Leu Asn Leu Pro Leu Tyr Thr Gly Gly Glu Leu Ser
               340                 345                 350
   ggc aaa atc cat gaa gcc gaa gcg caa tac ggg gcc gcc gaa gca cag     1104
   Gly Lys Ile His Glu Ala Glu Ala Gln Tyr Gly Ala Ala Glu Ala Gln
           355                 360                 365
   ctg acc gca acc gag cgg cac atc aaa ctc gcc gta cgc cag gct tat    1152
   Leu Thr Ala Thr Glu Arg His Ile Lys Leu Ala Val Arg Gln Ala Tyr
       370                 375                 380
   acc gaa agc ggt gcg gcg cgt tac caa atc atg gcg caa gaa cgg gtt    1200
   Thr Glu Ser Gly Ala Ala Arg Tyr Gln Ile Met Ala Gln Glu Arg Val
   385                 390                 395                 400
   ttg gaa agc agc cgt ttg aaa ctg aaa tcg acc gaa acc ggc caa caa    1248
   Leu Glu Ser Ser Arg Leu Lys Leu Lys Ser Thr Glu Thr Gly Gln Gln
                   405                 410                 415
   tac ggc atc cgc aac cgg ctg gaa gta ata cgg gcg cgg cag gaa gtc    1296
   Tyr Gly Ile Arg Asn Arg Leu Glu Val Ile Arg Ala Arg Gln Glu Val
               420                 425                 430
   gcc caa gca gaa cag aaa ctg gct caa gca cgg tat aaa ttc atg ctg    1344
   Ala Gln Ala Glu Gln Lys Leu Ala Gln Ala Arg Tyr Lys Phe Met Leu
           435                 440                 445
   gct tat ttg cgc ttg gtg aaa gag agc ggg tta ggg ttg gaa acg gta    1392
   Ala Tyr Leu Arg Leu Val Lys Glu Ser Gly Leu Gly Leu Glu Thr Val
       450                 455                 460
   ttt gcg gaa taa                                                    1404
   Phe Ala Glu
   465
<210> 30
   <211> 467
   <212> PRT
   <213> Neisseria meningitidis
<400> 30
   Met Thr Leu Leu Asn Leu Met Ile Met Gln Asp Tyr Gly Ile Ser Val
    1               5                  10                  15
   Cys Leu Thr Leu Thr Pro Tyr Leu Gln His Glu Leu Phe Ser Ala Met
               20                  25                  30
   Lys Ser Tyr Phe Ser Lys Tyr Ile Leu Pro Val Ser Leu Phe Thr Leu
           35                  40                  45
   Pro Leu Ser Leu Ser Pro Ser Val Ser Ala Phe Thr Leu Pro Glu Ala
       50                  55                  60
   Trp Arg Ala Ala Gln Gln His Ser Ala Asp Phe Gln Ala Ser His Tyr
   65                  70                  75                  80
   Gln Arg Asp Ala Val Arg Ala Arg Gln Gln Gln Ala Lys Ala Ala Phe
                   85                  90                  95
   Leu Pro His Val Ser Ala Asn Ala Ser Tyr Gln Arg Gln Pro Pro Ser
               100                 105                 110
   Ile Ser Ser Thr Arg Glu Thr Gln Gly Trp Ser Val Gln Val Gly Gln
   115 120 125
   Thr Leu Phe Asp Ala Ala Lys Phe Ala Gln Tyr Arg Gln Ser Arg Phe
   130 135 140
   Asp Thr Gln Ala Ala Glu Gln Arg Phe Asp Ala Ala Arg Glu Glu Leu
   145 150 155 160
   Leu Leu Lys Val Ala Glu Ser Tyr Phe Asn Val Leu Leu Ser Arg Asp
   165 170 175
   Thr Val Ala Ala His Ala Ala Glu Lys Glu Ala Tyr Ala Gln Gln Val
   180 185 190
   Arg Gln Ala Gln Ala Leu Phe Asn Lys Gly Ala Ala Thr Ala Leu Asp
   195 200 205
   Ile His Glu Ala Lys Ala Gly Tyr Asp Asn Ala Leu Ala Gln Glu Ile
   210 215 220
   Ala Val Leu Ala Glu Lys Gln Thr Tyr Glu Asn Gln Leu Asn Asp Tyr
   225 230 235 240
   Thr Asp Leu Asp Ser Lys Gln Ile Glu Ala Ile Asp Thr Ala Asn Leu
   245 250 255
   Leu Ala Arg Tyr Leu Pro Lys Leu Glu Arg Tyr Ser Leu Asp Glu Trp
   260 265 270
   Gln Arg Ile Ala Leu Ser Asn Asn His Glu Tyr Arg Met Gln Gln Leu
   275 280 285
   Ala Leu Gln Ser Ser Gly Gln Ala Leu Arg Ala Ala Gln Asn Ser Arg
   290 295 300
   Tyr Pro Thr Val Ser Ala His Val Gly Tyr Gln Asn Asn Leu Tyr Thr
   305 310 315 320
   Ser Ser Ala Gln Asn Asn Asp Tyr His Tyr Arg Gly Lys Gly Met Ser
   325 330 335
   Val Gly Val Gln Leu Asn Leu Pro Leu Tyr Thr Gly Gly Glu Leu Ser
   340 345 350
   Gly Lys Ile His Glu Ala Glu Ala Gln Tyr Gly Ala Ala Glu Ala Gln
   355 360 365
   Leu Thr Ala Thr Glu Arg His Ile Lys Leu Ala Val Arg Gln Ala Tyr
   370 375 380
   Thr Glu Ser Gly Ala Ala Arg Tyr Gln Ile Met Ala Gln Glu Arg Val
   385 390 395 400
   Leu Glu Ser Ser Arg Leu Lys Leu Lys Ser Thr Glu Thr Gly Gln Gln
   405 410 415
   Tyr Gly Ile Arg Asn Arg Leu Glu Val Ile Arg Ala Arg Gln Glu Val
   420 425 430
   Ala Gln Ala Glu Gln Lys Leu Ala Gln Ala Arg Tyr Lys Phe Met Leu
   435 440 445
   Ala Tyr Leu Arg Leu Val Lys Glu Ser Gly Leu Gly Leu Glu Thr Val
   450 455 460
   Phe Ala Glu
   465
<210> 31
   <211> 696
   <212> ADN
   <213> Neisseria meningitidis
<220>
   <221> CDS
   <222> (1)..(693)
<400> 31
   atg aaa caa tcc gcc cga ata aaa aat atg gat cag aca tta aaa aat     48
   Met Lys Gln Ser Ala Arg Ile Lys Asn Met Asp Gln Thr Leu Lys Asn
     1               5                  10                  15
   aca ttg ggc att tgc gcg ctt tta gcc ttt tgt ttt ggc gcg gcc atc     96
   Thr Leu Gly Ile Cys Ala Leu Leu Ala Phe Cys Phe Gly Ala Ala Ile
                    20                  25                  30
   gca tca ggt tat cac ttg gaa tat gaa tac ggc tac cgt tat tct gcc    144
   Ala Ser Gly Tyr His Leu Glu Tyr Glu Tyr Gly Tyr Arg Tyr Ser Ala
                35                  40                  45
   gtg ggt gct ttg gct tcg gtt gta ttt tta tta tta ttg gca cgc ggt    192
   Val Gly Ala Leu Ala Ser Val Val Phe Leu Leu Leu Leu Ala Arg Gly
        50                  55                  60
   ttc ccg cgc gtt tct tca gtt gtt tta ctg att tac gtc ggc aca acc    240
   Phe Pro Arg Val Ser Ser Val Val Leu Leu Ile Tyr Val Gly Thr Thr
    65                  70                  75                  80
   gcc cta tat ttg ccg gtc ggc tgg ctg tat ggt gcg ccg tct tat cag    288
   Ala Leu Tyr Leu Pro Val Gly Trp Leu Tyr Gly Ala Pro Ser Tyr Gln
                    85                  90                  95
   ata gtc ggt tcg ata ttg gaa agc aat cct gcc gag gcg cgt gaa ttt    336
   Ile Val Gly Ser Ile Leu Glu Ser Asn Pro Ala Glu Ala Arg Glu Phe
               100                 105                 110
   gtc ggc aat ctt ccc ggg tcg ctt tat ttt gtg cag gca tta ttt ttc    384
   Val Gly Asn Leu Pro Gly Ser Leu Tyr Phe Val Gln Ala Leu Phe Phe
           115                 120                 125
   att ttt ggc ttg aca gtt tgg aga tat tgt gta tcg ggg ggg gta ttt    432
   Ile Phe Gly Leu Thr Val Trp Arg Tyr Cys Val Ser Gly Gly Val Phe
       130                 135                 140
   gct gac gta aaa aac tat aaa cgc cgc agc aaa ata tgg ctg act ata    480
   Ala Asp Val Lys Asn Tyr Lys Arg Arg Ser Lys Ile Trp Leu Thr Ile
   145                 150                 155                 160
   tta ttg act ttg att ttg tcc tgc gcg gtg atg gat aaa atc gcc agc    528
   Leu Leu Thr Leu Ile Leu Ser Cys Ala Val Met Asp Lys Ile Ala Ser
                   165                 170                 175
   gat aaa gat ttg cga gaa cct gat gcc ggc ctg ttg ttg aat att ttc    576
   Asp Lys Asp Leu Arg Glu Pro Asp Ala Gly Leu Leu Leu Asn Ile Phe
               180                 185                 190
   gac ctg tat tac gat ttg gct tcc gcg ccg gca cca ata tgt cgc caa    624
   Asp Leu Tyr Tyr Asp Leu Ala Ser Ala Pro Ala Pro Ile Cys Arg Gln
           195                 200                 205
   gcg cgc cca cat ttt gga agc agc aaa aaa agc gtc aac atg gca tat    672
   Ala Arg Pro His Phe Gly Ser Ser Lys Lys Ser Val Asn Met Ala Tyr
       210                 215                 220
   ccg tca tgt tgc gcc caa gta taa                                    696
   Pro Ser Cys Cys Ala Gln Val
   225                 230
<210> 32
   <211> 231
   <212> PRT
   <213> Neisseria meningitidis
<400> 32
   Met Lys Gln Ser Ala Arg Ile Lys Asn Met Asp Gln Thr Leu Lys Asn
   1 5 10 15
   Thr Leu Gly Ile Cys Ala Leu Leu Ala Phe Cys Phe Gly Ala Ala Ile
   20 25 30
   Ala Ser Gly Tyr His Leu Glu Tyr Glu Tyr Gly Tyr Arg Tyr Ser Ala
   35 40 45
   Val Gly Ala Leu Ala Ser Val Val Phe Leu Leu Leu Leu Ala Arg Gly
   50 55 60
   Phe Pro Arg Val Ser Ser Val Val Leu Leu Ile Tyr Val Gly Thr Thr
   65 70 75 80
   Ala Leu Tyr Leu Pro Val Gly Trp Leu Tyr Gly Ala Pro Ser Tyr Gln
   85 90 95
   Ile Val Gly Ser Ile Leu Glu Ser Asn Pro Ala Glu Ala Arg Glu Phe
   100 105 110
   Val Gly Asn Leu Pro Gly Ser Leu Tyr Phe Val Gln Ala Leu Phe Phe
   115 120 125
   Ile Phe Gly Leu Thr Val Trp Arg Tyr Cys Val Ser Gly Gly Val Phe
   130 135 140
   Ala Asp Val Lys Asn Tyr Lys Arg Arg Ser Lys Ile Trp Leu Thr Ile
   145 150 155 160
   Leu Leu Thr Leu Ile Leu Ser Cys Ala Val Met Asp Lys Ile Ala Ser
   165 170 175
   Asp Lys Asp Leu Arg Glu Pro Asp Ala Gly Leu Leu Leu Asn Ile Phe
   180 185 190
   Asp Leu Tyr Tyr Asp Leu Ala Ser Ala Pro Ala Pro Ile Cys Arg Gln
   195 200 205
   Ala Arg Pro His Phe Gly Ser Ser Lys Lys Ser Val Asn Met Ala Tyr
   210 215 220
   Pro Ser Cys Cys Ala Gln Val
   225 230
<210> 33
   <211> 909
   <212> ADN
   <213> Neisseria meningitidis
<220>
   <221> CDS
   <222> (1) .. (906)
<400> 33
   atg aat gtt tac ggt ttc cca ttg ccc gat acg cct ttt ttg agt cgg    48
   Met Asn Val Tyr Gly Phe Pro Leu Pro Asp Thr Pro Phe Leu Ser Arg
    1               5                  10                  15
   acc aaa ggg ctg ttg ata aac ggt tac cat ttc acc gcc cac gcg acg    96
   Thr Lys Gly Leu Leu Ile Asn Gly Tyr His Phe Thr Ala His Ala Thr
               20                  25                  30
   aat ctt tcg ctg ccg cag act ttg ggg ctg ccg gga gag ccg aac aat   144
   Asn Leu Ser Leu Pro Gln Thr Leu Gly Leu Pro Gly Glu Pro Asn Asn
           35                  40                  45
   aac att gtc agc ttg gcg aag cag gcg ggt ttt cgg acg gcg tgg ctg   192
   Asn Ile Val Ser Leu Ala Lys Gln Ala Gly Phe Arg Thr Ala Trp Leu
       50                  55                  60
   tct aat caa gga atg ttg ggg cat ttt gcc aac gaa att tcc acc tat   240
   Ser Asn Gln Gly Met Leu Gly His Phe Ala Asn Glu Ile Ser Thr Tyr
   65                  70                  75                      80
   gcc cta cgc agc gat tat ccg tgg ttt acc caa agg ggt gat tat ggc   288
   Ala Leu Arg Ser Asp Tyr Pro Trp Phe Thr Gln Arg Gly Asp Tyr Gly
                   85                  90                  95
   aaa agc gcg ggg ttg agc gac cgc ctt ttg ttg ccg gcg ttc aaa cgg   336
   Lys Ser Ala Gly Leu Ser Asp Arg Leu Leu Leu Pro Ala Phe Lys Arg
               100                 105                 110
   gtt ttg ata gga aat gca ggc acg aag cct cgg ctg att gtg atg cac   384
   Val Leu Ile Gly Asn Ala Gly Thr Lys Pro Arg Leu Ile Val Met His
           115                 120                 125
   ctg atg ggt tcg cac agt gat ttt tgc aca cgt ttg gat aag gat gcg   432
   Leu Met Gly Ser His Ser Asp Phe Cys Thr Arg Leu Asp Lys Asp Ala
       130                 135                 140
   cgg cgg ttt cag tat caa act gaa aaa ata tcc tgc tat gtt tcc acc     480
   Arg Arg Phe Gln Tyr Gln Thr Glu Lys Ile Ser Cys Tyr Val Ser Thr
   145                 150                 155                 160
   atc gcg caa acc gat aaa ttt tta gaa gat aca gtt aag ata ttg aat     528
   Ile Ala Gln Thr Asp Lys Phe Leu Glu Asp Thr Val Lys Ile Leu Asn
                   165                 170                 175
   gaa aat aaa gaa agc tgg tct ttg gtt tac ttt tcc gac cac ggt ttg     576
   Glu Asn Lys Glu Ser Trp Ser Leu Val Tyr Phe Ser Asp His Gly Leu
               180                 185                 190
   atg cat gtc ggt aaa ggc ggc gag cga acg ttg aca cat ggt gcg tgg     624
   Met His Val Gly Lys Gly Gly Glu Arg Thr Leu Thr His Gly Ala Trp
           195                 200                 205
   aag cgt caa agc tac ggc gtg ccg ctg gtt aaa att tcg tcc gat gac     672
   Lys Arg Gln Ser Tyr Gly Val Pro Leu Val Lys Ile Ser Ser Asp Asp
       210                 215                 220
   acg cgg cgc gaa atg att aaa gtg agg cgc agc gcg ttt aat ttt tta     720
   Thr Arg Arg Glu Met Ile Lys Val Arg Arg Ser Ala Phe Asn Phe Leu
   225                 230                 235                 240
   cgc gga ttc ggc agt tgg acg ggt atc gaa acc gac gag ttg ccc gat     768
   Arg Gly Phe Gly Ser Trp Thr Gly Ile Glu Thr Asp Glu Leu Pro Asp
                   245                 250                 255
   gac ggc tat gat ttt tgg ggg aat gtt ccc gat gtg cag ggc gaa ggc     816
   Asp Gly Tyr Asp Phe Trp Gly Asn Val Pro Asp Val Gln Gly Glu Gly
               260                 265                 270
   aat aac ctt gcc ttt atc gac gga ctg ccc gac gac ccc gcg ccg tgg     864
   Asn Asn Leu Ala Phe Ile Asp Gly Leu Pro Asp Asp Pro Ala Pro Trp
           275                 280                 285
   tat gcg gga aaa ggc aaa tcg act aaa aat acg tct aaa aaa tga         909
   Tyr Ala Gly Lys Gly Lys Ser Thr Lys Asn Thr Ser Lys Lys
       290                 295                 300
<210> 34
   <211> 302
   <212> PRT
   <213> Neisseria meningitidis
<400> 34
   Met Asn Val Tyr Gly Phe Pro Leu Pro Asp Thr Pro Phe Leu Ser Arg
   1 5 10 15
   Thr Lys Gly Leu Leu Ile Asn Gly Tyr His Phe Thr Ala His Ala Thr
   20 25 30
   Asn Leu Ser Leu Pro Gln Thr Leu Gly Leu Pro Gly Glu Pro Asn Asn
   35 40 45
   Asn Ile Val Ser Leu Ala Lys Gln Ala Gly Phe Arg Thr Ala Trp Leu
   50 55 60
   Ser Asn Gln Gly Met Leu Gly His Phe Ala Asn Glu Ile Ser Thr Tyr
   65 70 75 80
   Ala Leu Arg Ser Asp Tyr Pro Trp Phe Thr Gln Arg Gly Asp Tyr Gly
   85 90 95
   Lys Ser Ala Gly Leu Ser Asp Arg Leu Leu Leu Pro Ala Phe Lys Arg
   100 105 110
   Val Leu Ile Gly Asn Ala Gly Thr Lys Pro Arg Leu Ile Val Met His
   115 120 125
   Leu Met Gly Ser His Ser Asp Phe Cys Thr Arg Leu Asp Lys Asp Ala
   130 135 140
   Arg Arg Phe Gln Tyr Gln Thr Glu Lys Ile Ser Cys Tyr Val Ser Thr
   145 150 155 160
   Ile Ala Gln Thr Asp Lys Phe Leu Glu Asp Thr Val Lys Ile Leu Asn
   165 170 175
   Glu Asn Lys Glu Ser Trp Ser Leu Val Tyr Phe Ser Asp His Gly Leu
   180 185 190
   Met His Val Gly Lys Gly Gly Glu Arg Thr Leu Thr His Gly Ala Trp
   195 200 205
   Lys Arg Gln Ser Tyr Gly Val Pro Leu Val Lys Ile Ser Ser Asp Asp
   210 215 220
   Thr Arg Arg Glu Met Ile Lys Val Arg Arg Ser Ala Phe Asn Phe Leu
   225 230 235 240
   Arg Gly Phe Gly Ser Trp Thr Gly Ile Glu Thr Asp Glu Leu Pro Asp
   245 250 255
   Asp Gly Tyr Asp Phe Trp Gly Asn Val Pro Asp Val Gln Gly Glu Gly
   260 265 270
   Asn Asn Leu Ala Phe Ile Asp Gly Leu Pro Asp Asp Pro Ala Pro Trp
   275 280 285
   Tyr Ala Gly Lys Gly Lys Ser Thr Lys Asn Thr Ser Lys Lys
   290 295 300
<210> 35
   <211> 864
   <212> ADN
   <213> Neisseria meningitidis
<220>
   <221> CDS
   <222> (1) .. (861)
<400> 35
   atg atg agt caa cac tct gcc gga gca cgt ttc cgc caa gcc gtg aaa    48
   Met Met Ser Gln His Ser Ala Gly Ala Arg Phe Arg Gln Ala Val Lys
     1               5                  10                  15
   gaa tcg aat ccg ctt gcc gtc gcc ggt tgc gtc aat gct tat ttt gca    96
   Glu Ser Asn Pro Leu Ala Val Ala Gly Cys Val Asn Ala Tyr Phe Ala
               20                  25                  30
   cga ttg gcc acc caa agc ggt ttc aaa gcc atc tat ctg tcc ggc ggc   144
   Arg Leu Ala Thr Gln Ser Gly Phe Lys Ala Ile Tyr Leu Ser Gly Gly
           35                  40                  45
   ggc gtg gca gcc tgt tct tgc ggt atc cct gat ttg ggc att acc aca   192
   Gly Val Ala Ala Cys Ser Cys Gly Ile Pro Asp Leu Gly Ile Thr Thr
       50                  55                  60
   atg gaa gat gtg ctg atc gac gca cga cgc att acg gac aac gtg gat   240
   Met Glu Asp Val Leu Ile Asp Ala Arg Arg Ile Thr Asp Asn Val Asp
   65                  70                  75                  80
   acg cct ctg ctg gtg gac atc gat gtg ggt tgg ggc ggt gca ttc aat   288
   Thr Pro Leu Leu Val Asp Ile Asp Val Gly Trp Gly Gly Ala Phe Asn
                   85                  90                  95
   att gcc cgt acc att cgc aac ttt gaa cgc gcc ggt gtt gca gcg gtt   336
   Ile Ala Arg Thr Ile Arg Asn Phe Glu Arg Ala Gly Val Ala Ala Val
               100                 105                 110
   cac atc gaa gat cag gta gcg caa aaa cgc tgc ggc cac cgt ccg aac   384
   His Ile Glu Asp Gln Val Ala Gln Lys Arg Cys Gly His Arg Pro Asn
           115                 120                 125
   aaa gcc att gta tct aaa gat gaa atg gtc gac cgt atc aaa gct gcc   432
   Lys Ala Ile Val Ser Lys Asp Glu Met Val Asp Arg Ile Lys Ala Ala
       130                 135                 140
   gta gat gcg cgc gtt gat gag aac ttc gtg att atg gcg cgt acc gat   480
   Val Asp Ala Arg Val Asp Glu Asn Phe Val Ile Met Ala Arg Thr Asp
   145                 150                 155                 160
   gcg ctg gcg gta gaa ggt ttg gat gcc gct atc gaa cgc gcc caa gct   528
   Ala Leu Ala Val Glu Gly Leu Asp Ala Ala Ile Glu Arg Ala Gln Ala
                   165                 170                 175
   tgt gtc gaa gcc ggt gcg gac atg att ttc cct gaa gcc atg acc gat   576
   Cys Val Glu Ala Gly Ala Asp Met Ile Phe Pro Glu Ala Met Thr Asp
               180                 185                 190
   ttg aac atg tac cgc caa ttt gca gat gcg gtg aaa gtg ccc gtg ttg   624
   Leu Asn Met Tyr Arg Gln Phe Ala Asp Ala Val Lys Val Pro Val Leu
           195                 200                 205
   gcg aac att acc gag ttt ggt tcc act ccg ctt tat acc caa agc gag   672
   Ala Asn Ile Thr Glu Phe Gly Ser Thr Pro Leu Tyr Thr Gln Ser Glu
       210                 215                 220
   ctg gct gaa aac ggc gtg tcg ctg gtg ctg tat ccg ctg tca tcg ttc   720
   Leu Ala Glu Asn Gly Val Ser Leu Val Leu Tyr Pro Leu Ser Ser Phe
   225                 230                 235                 240
   cgt gca gca agc aaa gcc gct ctg aat gtt tac gaa gcg att atg cgc   768
   Arg Ala Ala Ser Lys Ala Ala Leu Asn Val Tyr Glu Ala Ile Met Arg
                   245                 250                 255
   gat ggc act tca ggc ggc ggt ggt gga cag tat gca aac ccg tgc cga    816
   Asp Gly Thr Ser Gly Gly Gly Gly Gly Gln Tyr Ala Asn Pro Cys Arg
               260                 265                 270
   gct gta cga gca tct gaa cta tca tgc ctt cga gca aaa act gga taa    864
   Ala Val Arg Ala Ser Glu Leu Ser Cys Leu Arg Ala Lys Thr Gly
           275                 280                 285
<210> 36
   <211> 287
   <212> PRT
   <213> Neisseria meningitidis
<400> 36
   Met Met Ser Gln His Ser Ala Gly Ala Arg Phe Arg Gln Ala Val Lys
   1 5 10 15
   Glu Ser Asn Pro Leu Ala Val Ala Gly Cys Val Asn Ala Tyr Phe Ala
   20 25 30
   Arg Leu Ala Thr Gln Ser Gly Phe Lys Ala Ile Tyr Leu Ser Gly Gly
   35 40 45
   Gly Val Ala Ala Cys Ser Cys Gly Ile Pro Asp Leu Gly Ile Thr Thr
   50 55 60
   Met Glu Asp Val Leu Ile Asp Ala Arg Arg Ile Thr Asp Asn Val Asp
   65 70 75 80
   Thr Pro Leu Leu Val Asp Ile Asp Val Gly Trp Gly Gly Ala Phe Asn
   85 90 95
   Ile Ala Arg Thr Ile Arg Asn Phe Glu Arg Ala Gly Val Ala Ala Val
   100 105 110
   His Ile Glu Asp Gln Val Ala Gln Lys Arg Cys Gly His Arg Pro Asn
   115 120 125
   Lys Ala Ile Val Ser Lys Asp Glu Met Val Asp Arg Ile Lys Ala Ala
   130 135 140
   Val Asp Ala Arg Val Asp Glu Asn Phe Val Ile Met Ala Arg Thr Asp
   145 150 155 160
   Ala Leu Ala Val Glu Gly Leu Asp Ala Ala Ile Glu Arg Ala Gln Ala
   165 170 175
   Cys Val Glu Ala Gly Ala Asp Met Ile Phe Pro Glu Ala Met Thr Asp
   180 185 190
   Leu Asn Met Tyr Arg Gln Phe Ala Asp Ala Val Lys Val Pro Val Leu
   195 200 205
   Ala Asn Ile Thr Glu Phe Gly Ser Thr Pro Leu Tyr Thr Gln Ser Glu
   210 215 220
   Leu Ala Glu Asn Gly Val Ser Leu Val Leu Tyr Pro Leu Ser Ser Phe
   225 230 235 240
   Arg Ala Ala Ser Lys Ala Ala Leu Asn Val Tyr Glu Ala Ile Met Arg
   245 250 255
   Asp Gly Thr Ser Gly Gly Gly Gly Gly Gln Tyr Ala Asn Pro Cys Arg
   260 265 270
   Ala Val Arg Ala Ser Glu Leu Ser Cys Leu Arg Ala Lys Thr Gly
   275 280 285
<210> 37
   <211> 921
   <212> ADN
   <213> Neisseria meningitidis
<220>
   <221> CDS
   <222> (1) .. (918)
<400> 37
   atg cct tcg agc aaa aac tgg ata aat tgt ttc aaa aat gat tta ccg      48
   Met Pro Ser Ser Lys Asn Trp Ile Asn Cys Phe Lys Asn Asp Leu Pro
   1               5                   10                  15
   ctt tca gac tgc ctt tca aca aat ccg cat cgg tcg tct gaa aac ccg      96
   Leu Ser Asp Cys Leu Ser Thr Asn Pro His Arg Ser Ser Glu Asn Pro
               20                  25                  30
   aaa ccc ata aaa aca caa agg aga aat acc atg act gaa act act caa      144
   Lys Pro Ile Lys Thr Gln Arg Arg Asn Thr Met Thr Glu Thr Thr Gln
           35                  40                  45
   acc ccg acc ctc aaa cct aaa aaa tcc gtt gcg ctt tct ggc gtt gcg      192
   Thr Pro Thr Leu Lys Pro Lys Lys Ser Val Ala Leu Ser Gly Val Ala
       50                  55                  60
   gcc ggt aat acc gct ttg tgt acc gtt ggc cgt acc ggc aac gat ttg      240
   Ala Gly Asn Thr Ala Leu Cys Thr Val Gly Arg Thr Gly Asn Asp Leu
   65                  70                  75                  80
   agc tat cgc ggt tac gac att ctg gat ttg gca caa aaa tgt gag ttt      288
   Ser Tyr Arg Gly Tyr Asp Ile Leu Asp Leu Ala Gln Lys Cys Glu Phe
                   85                  90                  95
   gaa gaa gtt gcc cac ctg ctg att cac ggc cat tta ccc aac aaa ttc      336
   Glu Glu Val Ala His Leu Leu Ile His Gly His Leu Pro Asn Lys Phe
               100                 105                 110
   gag ctg gcc gct tat aaa gcc aag ctc aaa tcc atg cgc ggc ctg cct      384
   Glu Leu Ala Ala Tyr Lys Ala Lys Leu Lys Ser Met Arg Gly Leu Pro
           115                 120                 125
   atc cgt gtg att aaa gtt ttg gaa agc ctg cct gca cat acc cat ccg      432
   Ile Arg Val Ile Lys Val Leu Glu Ser Leu Pro Ala His Thr His Pro
       130                 135                 140
   atg gac gtg atg cgt acc ggc gta tcc atg ctg ggc tgt gtt cat cct      480
   Met Asp Val Met Arg Thr Gly Val Ser Met Leu Gly Cys Val His Pro
   145                 150                 155                 160
   gaa cgt gaa ggc cat ccg gaa agc gaa gcg cgc gac att gcc gac aaa     528
   Glu Arg Glu Gly His Pro Glu Ser Glu Ala Arg Asp Ile Ala Asp Lys
                   165                     170                 175
   ctg atc gcc agc ctc ggc agt atc ctc ttg tac tgg tat caa tat tcg     576
   Leu Ile Ala Ser Leu Gly Ser Ile Leu Leu Tyr Trp Tyr Gln Tyr Ser
               180                 185                 190
   cac aac ggc aaa cgc att gaa gtt gaa agc gaa gaa gag acc atc ggc     624
   His Asn Gly Lys Arg Ile Glu Val Glu Ser Glu Glu Glu Thr Ile Gly
           195                 200                 205
   ggt cat ttc ctg cac ctg ttg cac ggc aaa cgc cca agc gaa tca cac     672
   Gly His Phe Leu His Leu Leu His Gly Lys Arg Pro Ser Glu Ser His
       210                 215                 220
   atc aaa gcc atg cac gtt tca ctg att ctg tat gcc gaa cac gag ttc     720
   Ile Lys Ala Met His Val Ser Leu Ile Leu Tyr Ala Glu His Glu Phe
   225                 230                 235                 240
   aac gct tct acc ttt acc gcc cgc gtg atc gcc ggt aca ggc tct gat     768
   Asn Ala Ser Thr Phe Thr Ala Arg Val Ile Ala Gly Thr Gly Ser Asp
                   245                 250                 255
   atg tac tcc agc att acc gga gca atc ggc gcg ttg aaa ggt ccg aaa     816
   Met Tyr Ser Ser Ile Thr Gly Ala Ile Gly Ala Leu Lys Gly Pro Lys
               260                 265                 270
   cac ggc ggc gcg aac gaa ggg ctt acg ata ttc aaa aac gct acc gca     864
   His Gly Gly Ala Asn Glu Gly Leu Thr Ile Phe Lys Asn Ala Thr Ala
           275                 280                 285
   atg ccg acg aag ccg aag ccg aca tcc gcg aac gca tcg gcc gca aag     912
   Met Pro Thr Lys Pro Lys Pro Thr Ser Ala Asn Ala Ser Ala Ala Lys
       290                 295                 300
   aaa tcg tga                                                         921
   Lys Ser
   305
<210> 38
   <211> 306
   <212> PRT
   <213> Neisseria meningitidis
<400> 38
   Met Pro Ser Ser Lys Asn Trp Ile Asn Cys Phe Lys Asn Asp Leu Pro
   1 5 10 15
   Leu Ser Asp Cys Leu Ser Thr Asn Pro His Arg Ser Ser Glu Asn Pro
   20 25 30
   Lys Pro Ile Lys Thr Gln Arg Arg Asn Thr Met Thr Glu Thr Thr Gln
   35 40 45
   Thr Pro Thr Leu Lys Pro Lys Lys Ser Val Ala Leu Ser Gly Val Ala
   50 55 60
   Ala Gly Asn Thr Ala Leu Cys Thr Val Gly Arg Thr Gly Asn Asp Leu
   65 70 75 80
   Ser Tyr Arg Gly Tyr Asp Ile Leu Asp Leu Ala Gln Lys Cys Glu Phe
   85 90 95
   Glu Glu Val Ala His Leu Leu Ile His Gly His Leu Pro Asn Lys Phe
   100 105 110
   Glu Leu Ala Ala Tyr Lys Ala Lys Leu Lys Ser Met Arg Gly Leu Pro
   115 120 125
   Ile Arg Val Ile Lys Val Leu Glu Ser Leu Pro Ala His Thr His Pro
   130 135 140
   Met Asp Val Met Arg Thr Gly Val Ser Met Leu Gly Cys Val His Pro
   145 150 155 160
   Glu Arg Glu Gly His Pro Glu Ser Glu Ala Arg Asp Ile Ala Asp Lys
   165 170 175
   Leu Ile Ala Ser Leu Gly Ser Ile Leu Leu Tyr Trp Tyr Gln Tyr Ser
   180 185 190
   His Asn Gly Lys Arg Ile Glu Val Glu Ser Glu Glu Glu Thr Ile Gly
   195 200 205
   Gly His Phe Leu His Leu Leu His Gly Lys Arg Pro Ser Glu Ser His
   210 215 220
   Ile Lys Ala Met His Val Ser Leu Ile Leu Tyr Ala Glu His Glu Phe
   225 230 235 240
   Asn Ala Ser Thr Phe Thr Ala Arg Val Ile Ala Gly Thr Gly Ser Asp
   245 250 255
   Met Tyr Ser Ser Ile Thr Gly Ala Ile Gly Ala Leu Lys Gly Pro Lys
   260 265 270
   His Gly Gly Ala Asn Glu Gly Leu Thr Ile Phe Lys Asn Ala Thr Ala
   275 280 285
   Met Pro Thr Lys Pro Lys Pro Thr Ser Ala Asn Ala Ser Ala Ala Lys
   290 295 300
   Lys Ser
   305
<210> 39
   <211> 945
   <212> ADN
   <213> Neisseria meningitidis
<220>
   <221> CDS
   <222> (1) .. (942)
<400> 39
   atg cac cta tgt gga aag tat tat gga gta aat atg aag ctg cgt gat      48
   Met His Leu Cys Gly Lys Tyr Tyr Gly Val Asn Met Lys Leu Arg Asp
    1               5                  10                  15
   tta ctg atg gga ata ttc ttg gca gtt tct gcg gcc ctt ctg aat gca      96
   Leu Leu Met Gly Ile Phe Leu Ala Val Ser Ala Ala Leu Leu Asn Ala
               20                  25                  30
   acc atc ggc ata ttc agc aag ata ttg atg gag cag ggc ttg tct gtt     144
   Thr Ile Gly Ile Phe Ser Lys Ile Leu Met Glu Gln Gly Leu Ser Val
           35                  40                  45
   cag cat att gca ttt ttg aaa act ttg aca ggt ttc gtg ttt atc agc     192
   Gln His Ile Ala Phe Leu Lys Thr Leu Thr Gly Phe Val Phe Ile Ser
       50                  55                  60
   att ttg ctt tgc cgt acc ggt ttt acc aga cag att gcg gat att tca     240
   Ile Leu Leu Cys Arg Thr Gly Phe Thr Arg Gln Ile Ala Asp Ile Ser
   65                  70                  75                  80
   aga aag aaa gag gca att ttg ccg ttg ctg tta aaa gta gca att tgt     288
   Arg Lys Lys Glu Ala Ile Leu Pro Leu Leu Leu Lys Val Ala Ile Cys
                   85                  90                  95
   gct ttt ttc gga att tat acg ttg ttt ttc ttt gaa acc aca gct tat     336
   Ala Phe Phe Gly Ile Tyr Thr Leu Phe Phe Phe Glu Thr Thr Ala Tyr
               100                 105                 110
   caa tat ggc aat gct gcg aat gta gta gtt gta tta atg gca tcg gct     384
   Gln Tyr Gly Asn Ala Ala Asn Val Val Val Val Leu Met Ala Ser Ala
           115                 120                 125
   gcc gta tct gcc ttg ata ttg gac agc ata ctg tta gat gaa cgt att     432
   Ala Val Ser Ala Leu Ile Leu Asp Ser Ile Leu Leu Asp Glu Arg Ile
       130                 135                 140
   tgc att tct tca gtc gtc ggt gtg ggt ttg gca gta ttg ggg atc gca     480
   Cys Ile Ser Ser Val Val Gly Val Gly Leu Ala Val Leu Gly Ile Ala
   145                 150                 155                 160
   atg att tct tgg act gga gaa gga agt tta ggg ttg att ctg aat gcc     528
   Met Ile Ser Trp Thr Gly Glu Gly Ser Leu Gly Leu Ile Leu Asn Ala
                   165                 170                 175
   gca ctg gcg ggc tcg ggc tac ggt tgt ttt tcc gtt ttg att aag aaa     576
   Ala Leu Ala Gly Ser Gly Tyr Gly Cys Phe Ser Val Leu Ile Lys Lys
               180                 185                 190
   ttc ggc cta aac ggc ggt att tat ttg aca cgg ata ttg atg ttt ttt     624
   Phe Gly Leu Asn Gly Gly Ile Tyr Leu Thr Arg Ile Leu Met Phe Phe
           195                 200                 205
   gga agt att ttt ttg ttt atc cct tca ttg gaa ggt att gag gat ata     672
   Gly Ser Ile Phe Leu Phe Ile Pro Ser Leu Glu Gly Ile Glu Asp Ile
       210                 215                 220
   cat tgg caa tgg tct ttt att ccg cca ctc ttg gca ttg tct tta ttg     720
   His Trp Gln Trp Ser Phe Ile Pro Pro Leu Leu Ala Leu Ser Leu Leu
   225                 230                 235                 240
   ccg acg att tta gga ttt tat tgt aca act aaa gca ttg gat tat ttg 768
   Pro Thr Ile Leu Gly Phe Tyr Cys Thr Thr Lys Ala Leu Asp Tyr Leu
   245 250 255
   agt gct gcg aag gta cag gta act gaa ttg gcc gag cca ttg ttt gct 816
   Ser Ala Ala Lys Val Gln Val Thr Glu Leu Ala Glu Pro Leu Phe Ala
   260 265 270
   gcc gta ctg gct tgg ttg ttt ttg aat gaa ata ccg gaa gga cgc ttc 864
   Ala Val Leu Ala Trp Leu Phe Leu Asn Glu Ile Pro Glu Gly Arg Phe
   275 280 285
   ttt gtc ggc gcc att ctg att att gcc ggt att gtg tct atc aat ggg 912
   Phe Val Gly Ala Ile Leu Ile Ile Ala Gly Ile Val Ser Ile Asn Gly
   290 295 300
   ctg tat cga cca ttg ttg aag cga att gaa taa 945
   Leu Tyr Arg Pro Leu Leu Lys Arg Ile Glu
   305 310
<210> 40
   <211> 314
   <212> PRT
   <213> Neisseria meningitidis
<400> 40
   Met His Leu Cys Gly Lys Tyr Tyr Gly Val Asn Met Lys Leu Arg Asp
   1               5                   10                  15
   Leu Leu Met Gly Ile Phe Leu Ala Val Ser Ala Ala Leu Leu Asn Ala
               20                  25                  30
   Thr Ile Gly Ile Phe Ser Lys Ile Leu Met Glu Gln Gly Leu Ser Val
           35                  40                  45
   Gln His Ile Ala Phe Leu Lys Thr Leu Thr Gly Phe Val Phe Ile Ser
       50                  55                  60
   Ile Leu Leu Cys Arg Thr Gly Phe Thr Arg Gln Ile Ala Asp Ile Ser
   65                  70                  75                  80
   Arg Lys Lys Glu Ala Ile Leu Pro Leu Leu Leu Lys Val Ala Ile Cys
                   85                  90                  95
   Ala Phe Phe Gly Ile Tyr Thr Leu Phe Phe Phe Glu Thr Thr Ala Tyr
               100                 105                 110
   Gln Tyr Gly Asn Ala Ala Asn Val Val Val Val Leu Met Ala Ser Ala
           115                 120                 125
   Ala Val Ser Ala Leu Ile Leu Asp Ser Ile Leu Leu Asp Glu Arg Ile
       130                 135                 140
   Cys Ile Ser Ser Val Val Gly Val Gly Leu Ala Val Leu Gly Ile Ala
   145                 150                 155                 160
   Met Ile Ser Trp Thr Gly Glu Gly Ser Leu Gly Leu Ile Leu Asn Ala
                   165                 170                 175
   Ala Leu Ala Gly Ser Gly Tyr Gly Cys Phe Ser Val Leu Ile Lys Lys
               180                 185                 190
   Phe Gly Leu Asn Gly Gly Ile Tyr Leu Thr Arg Ile Leu Met Phe Phe
           195                 200                 205
   Gly Ser Ile Phe Leu Phe Ile Pro Ser Leu Glu Gly Ile Glu Asp Ile
       210                 215                 220
   His Trp Gln Trp Ser Phe Ile Pro Pro Leu Leu Ala Leu Ser Leu Leu
   225                 230                 235                 240
   Pro Thr Ile Leu Gly Phe Tyr Cys Thr Thr Lys Ala Leu Asp Tyr Leu
                   245                 250                 255
   Ser Ala Ala Lys Val Gln Val Thr Glu Leu Ala Glu Pro Leu Phe Ala
               260                 265                 270
   Ala Val Leu Ala Trp Leu Phe Leu Asn Glu Ile Pro Glu Gly Arg Phe
           275                 280                 285
   Phe Val Gly Ala Ile Leu Ile Ile Ala Gly Ile Val Ser Ile Asn Gly
       290                 295                 300
   Leu Tyr Arg Pro Leu Leu Lys Arg Ile Glu
   305                 310
<210> 41
   <211> 2610
   <212> ADN
   <213> Neisseria meningitidis
<220>
   <221> CDS
   <222> (1) .. (2607)
<400> 41
   atg gct gcc aac caa cgt tac cgc aaa ccg ctg ccc ggt acg gat ttg      48
   Met Ala Ala Asn Gln Arg Tyr Arg Lys Pro Leu Pro Gly Thr Asp Leu
     1               5                  10                  15
   gaa tac tac gac gcg cgt gcg gcg tgt gag gac atc aag ccc ggc tct      96
   Glu Tyr Tyr Asp Ala Arg Ala Ala Cys Glu Asp Ile Lys Pro Gly Ser
                20                  25                  30
   tac gac aag ctg cct tac acg agc cgc att ttg gcg gag aat ttg gtc     144
   Tyr Asp Lys Leu Pro Tyr Thr Ser Arg Ile Leu Ala Glu Asn Leu Val
            35                  40                  45
   aac cgc gcg gac aaa gtc gat ttg ccg acg ctg caa agc tgg ctg ggt     192
   Asn Arg Ala Asp Lys Val Asp Leu Pro Thr Leu Gln Ser Trp Leu Gly
        50                  55                  60
   cag ctg att gag gga aaa cag gaa atc gac ttt cct tgg tat ccg gcg     240
   Gln Leu Ile Glu Gly Lys Gln Glu Ile Asp Phe Pro Trp Tyr Pro Ala
    65                  70                  75                  80
   cgg gtg gtg tgc cac gat att ctg ggg cag acc gcg ttg gtg gat ttg     288
   Arg Val Val Cys His Asp Ile Leu Gly Gln Thr Ala Leu Val Asp Leu
                   85                  90                  95
   gca ggt ctg cgc gat gcg att gcc gaa aaa ggc ggc gat cct gcc aaa    336
   Ala Gly Leu Arg Asp Ala Ile Ala Glu Lys Gly Gly Asp Pro Ala Lys
               100                 105                 110
   gtg aat ccg gtg gtt gca aaa ccc agc ttc atc gtc gac cac tct ctg    384
   Val Asn Pro Val Val Ala Lys Pro Ser Phe Ile Val Asp His Ser Leu
           115                 120                 125
   gcc gtt gaa tgc ggc ggc tac gac ccc gat gcc ttc cgc aaa aac cgc    432
   Ala Val Glu Cys Gly Gly Tyr Asp Pro Asp Ala Phe Arg Lys Asn Arg
       130                 135                 140
   caa atc gaa gac aga cgt aac gaa gac cgt ttc cac ttc atc aac tgg    480
   Gln Ile Glu Asp Arg Arg Asn Glu Asp Arg Phe His Phe Ile Asn Trp
   145                 150                 155                 160
   aca aaa acc gca ttt gaa aat gtg gac gtg att ccg gcg ggc aac ggc    528
   Thr Lys Thr Ala Phe Glu Asn Val Asp Val Ile Pro Ala Gly Asn Gly
                   165                 170                 175
   atc atg cac caa atc aat cta gaa aaa atg tcg ccc gtc gtc caa gtc    576
   Ile Met His Gln Ile Asn Leu Glu Lys Met Ser Pro Val Val Gln Val
               180                 185                 190
   aaa aac ggc gtg gcg ttc ccc gat acc tgc gtc ggc acg gat tcg cac    624
   Lys Asn Gly Val Ala Phe Pro Asp Thr Cys Val Gly Thr Asp Ser His
           195                 200                 205
   acg ccg cac gtc gat gcg ctg ggc gtg att tcc gtg ggc gtg ggc gga    672
   Thr Pro His Val Asp Ala Leu Gly Val Ile Ser Val Gly Val Gly Gly
       210                 215                 220
   ttg gaa gcg gaa acc gtg atg ctg ggt cgc gcg tcc atg atg cgc ctg    720
   Leu Glu Ala Glu Thr Val Met Leu Gly Arg Ala Ser Met Met Arg Leu
   225                 230                 235                 240
   ccc gat att gtc ggc gtt gag ctg aac ggc aaa cgg cag gcg ggc att    768
   Pro Asp Ile Val Gly Val Glu Leu Asn Gly Lys Arg Gln Ala Gly Ile
                   245                 250                 255
   acg gcg acg gat att gtg ttg gca ctg acc gag ttt ctg cgc aaa gaa    816
   Thr Ala Thr Asp Ile Val Leu Ala Leu Thr Glu Phe Leu Arg Lys Glu
               260                 265                 270
   cgc gtg gtc ggg gcg ttt gtc gaa ttc ttc ggc gag ggc gcg aga agc    864
   Arg Val Val Gly Ala Phe Val Glu Phe Phe Gly Glu Gly Ala Arg Ser
           275                 280                 285
   ctg tct atc ggc gac cgc gcg acc att tcc aac atg acg ccg gag ttc    912
   Leu Ser Ile Gly Asp Arg Ala Thr Ile Ser Asn Met Thr Pro Glu Phe
       290                 295                 300
   ggc gcg act gcc gcg atg ttc gct att gat gag caa acc att gat tat    960
   Gly Ala Thr Ala Ala Met Phe Ala Ile Asp Glu Gln Thr Ile Asp Tyr
   305                 310                 315                 320
   ttg aaa ctg acc gga cgc gac gac gcg cag gtg aaa ttg gtg gaa acc   1008
   Leu Lys Leu Thr Gly Arg Asp Asp Ala Gln Val Lys Leu Val Glu Thr
                   325                 330                 335
   tac gcc aaa acc gca ggc tta tgg gca gat gcc ttg aaa acc gcc gtt    1056
   Tyr Ala Lys Thr Ala Gly Leu Trp Ala Asp Ala Leu Lys Thr Ala Val
               340                 345                 350
   tat ccg cgc gtt ttg aaa ttt gat ttg agc agc gta acg cgc aat atg    1104
   Tyr Pro Arg Val Leu Lys Phe Asp Leu Ser Ser Val Thr Arg Asn Met
           355                 360                 365
   gca ggc ccg agc aac ccg cac gcg cgt ttt gcg acc gcc gat ttg gcc    1152
   Ala Gly Pro Ser Asn Pro His Ala Arg Phe Ala Thr Ala Asp Leu Ala
       370                 375                 380
   agc aaa ggc ttg gct aaa cct tac gaa gag cct tca gac ggc caa atg    1200
   Ser Lys Gly Leu Ala Lys Pro Tyr Glu Glu Pro Ser Asp Gly Gln Met
   385                 390                 395                 400
   ccc gac ggc gcg gtc atc atc gcc gcg att acc agt tgc acc aac act    1248
   Pro Asp Gly Ala Val Ile Ile Ala Ala Ile Thr Ser Cys Thr Asn Thr
                   405                 410                 415
   tcc aac ccg cgc aac gtt gtt gcc gcc gcg ctc ttg gcg cgc aac gcc    1296
   Ser Asn Pro Arg Asn Val Val Ala Ala Ala Leu Leu Ala Arg Asn Ala
               420                 425                 430
   aac tgc ttc ggg ctg aaa cgc aaa ccg tgg gtc aaa acc tcg ttt gcc    1344
   Asn Cys Phe Gly Leu Lys Arg Lys Pro Trp Val Lys Thr Ser Phe Ala
           435                 440                 445
   ccc ggt tcg aaa gtg gcg gaa att tat ttg aaa gaa gca ggc ctg ctg    1392
   Pro Gly Ser Lys Val Ala Glu Ile Tyr Leu Lys Glu Ala Gly Leu Leu
       450                 455                 460
   ccc gaa atg gaa aaa ctc ggc ttc ggt atc gtc gcc ttc gcc tgc acc    1440
   Pro Glu Met Glu Lys Leu Gly Phe Gly Ile Val Ala Phe Ala Cys Thr
   465                 470                 475                 480
   acc tgc aac ggc atg agt ggc gcg ctg gat ccg aaa atc cag aaa gaa    1488
   Thr Cys Asn Gly Met Ser Gly Ala Leu Asp Pro Lys Ile Gln Lys Glu
                   485                 490                 495
   atc atc gac cgc gat ttg tac gcc acc gcc gta tta tca ggc aac cgc    1536
   Ile Ile Asp Arg Asp Leu Tyr Ala Thr Ala Val Leu Ser Gly Asn Arg
               500                 505                 510
   aac ttc gac ggc cgt gtc cat ccg tat gcg aaa cag gct ttc ctc gct    1584
   Asn Phe Asp Gly Arg Val His Pro Tyr Ala Lys Gln Ala Phe Leu Ala
           515                 520                 525
   tcg cct ccg ttg gtc gtt gcc tac gcg ctg gca ggc agt atc cgt ttc    1632
   Ser Pro Pro Leu Val Val Ala Tyr Ala Leu Ala Gly Ser Ile Arg Phe
       530                 535                 540
   gat att gaa aac gac gta ctc ggc gtt gca gac ggc aag gaa atc cgc    1680
   Asp Ile Glu Asn Asp Val Leu Gly Val Ala Asp Gly Lys Glu Ile Arg
   545                 550                 555                 560
   ctg aaa gac att tgg cct gcc gat gaa gaa atc gat gcc gtc gtt gcc    1728
   Leu Lys Asp Ile Trp Pro Ala Asp Glu Glu Ile Asp Ala Val Val Ala
                   565                 570                 575
   gaa tat gtg aaa ccg cag cag ttc cgc gat gtg tat gta ccg atg ttc    1776
   Glu Tyr Val Lys Pro Gln Gln Phe Arg Asp Val Tyr Val Pro Met Phe
               580                 585                 590
   gac acc ggc aca gcg caa aaa gca cct agt ccg ctg tac gat tgg cgt    1824
   Asp Thr Gly Thr Ala Gln Lys Ala Pro Ser Pro Leu Tyr Asp Trp Arg
           595                 600                 605
   ccg atg tcc acc tac atc cgc cgt ccg cct tac tgg gaa ggc gcg ctg    1872
   Pro Met Ser Thr Tyr Ile Arg Arg Pro Pro Tyr Trp Glu Gly Ala Leu
       610                 615                 620
   gca ggg gaa cgc aca tta aga ggt atg cgt ccg ctg gcg att ttg ccc    1920
   Ala Gly Glu Arg Thr Leu Arg Gly Met Arg Pro Leu Ala Ile Leu Pro
   625                 630                 635                 640
   gac aac atc acc acc gac cac ctc tcg ccg tcc aat gcg att ttg gcc    1968
   Asp Asn Ile Thr Thr Asp His Leu Ser Pro Ser Asn Ala Ile Leu Ala
                   645                 650                 655
   gtc agt gcc gca ggc gag tat ttg gcg aaa atg ggt ttg cct gaa gaa    2016
   Val Ser Ala Ala Gly Glu Tyr Leu Ala Lys Met Gly Leu Pro Glu Glu
               660                 665                 670
   gac ttc aac tct tac gca acc cac cgc ggc gac cac ttg acc gcc caa    2064
   Asp Phe Asn Ser Tyr Ala Thr His Arg Gly Asp His Leu Thr Ala Gln
           675                 680                 685
   cgc gct acc ttc gcc aat ccg aaa ctg ttt aac gaa atg gtg aaa aac    2112
   Arg Ala Thr Phe Ala Asn Pro Lys Leu Phe Asn Glu Met Val Lys Asn
       690                 695                 700
   gaa gac ggc agc gtg cgc caa ggc tcg ttc gcc cgc gtc gaa ccc gaa    2160
   Glu Asp Gly Ser Val Arg Gln Gly Ser Phe Ala Arg Val Glu Pro Glu
   705                 710                 715                 720
   ggc gaa acc atg cgc atg tgg gaa gcc atc gaa acc tat atg aac cgc    2208
   Gly Glu Thr Met Arg Met Trp Glu Ala Ile Glu Thr Tyr Met Asn Arg
                   725                 730                 735
   aaa cag ccg ctc atc atc att gcc ggt gcg gac tat ggt caa ggc tca    2256
   Lys Gln Pro Leu Ile Ile Ile Ala Gly Ala Asp Tyr Gly Gln Gly Ser
               740                 745                 750
   agc cgc gac tgg gct gca aaa ggc gta cgc ctc gcc ggc gta gaa gcg    2304
   Ser Arg Asp Trp Ala Ala Lys Gly Val Arg Leu Ala Gly Val Glu Ala
           755                 760                 765
   att gtt gcc gaa ggc ttc gag cgt atc cac cgc acc aac ctt atc ggc    2352
   Ile Val Ala Glu Gly Phe Glu Arg Ile His Arg Thr Asn Leu Ile Gly
       770                 775                 780
   atg ggc gtg ttg ccg ctg cag ttc aaa ccc gac acc aac cgc cat acc    2400
   Met Gly Val Leu Pro Leu Gln Phe Lys Pro Asp Thr Asn Arg His Thr
   785                 790                 795                 800
   ctg caa ctg gac ggt acg gaa acc tac gac gtg gtc ggc gaa cgc aca    2448
   Leu Gln Leu Asp Gly Thr Glu Thr Tyr Asp Val Val Gly Glu Arg Thr
                   805                 810                 815
   ccg cgc tgc gac ctg acc ctc gtg att cac cgt aaa aac ggc gaa acc      2496
   Pro Arg Cys Asp Leu Thr Leu Val Ile His Arg Lys Asn Gly Glu Thr
               820                 825                 830
   gtc gaa gtt ccc gtt acc tgc cgc ctc gat act gca gaa gaa gta ttg      2544
   Val Glu Val Pro Val Thr Cys Arg Leu Asp Thr Ala Glu Glu Val Leu
           835                 840                 845
   gta tat gaa gcc ggc ggc gtg ttg caa cgg ttt gca cag gat ttt ttg      2592
   Val Tyr Glu Ala Gly Gly Val Leu Gln Arg Phe Ala Gln Asp Phe Leu
       850                 855                 860
   gaa ggg aac gcg gct tag                                              2610
   Glu Gly Asn Ala Ala
   865
<210> 42
   <211> 869
   <212> PRT
   <213> Neisseria meningitidis
<400> 42
   Met Ala Ala Asn Gln Arg Tyr Arg Lys Pro Leu Pro Gly Thr Asp Leu
   1 5 10 15
   Glu Tyr Tyr Asp Ala Arg Ala Ala Cys Glu Asp Ile Lys Pro Gly Ser
   20 25 30
   Tyr Asp Lys Leu Pro Tyr Thr Ser Arg Ile Leu Ala Glu Asn Leu Val
   35 40 45
   Asn Arg Ala Asp Lys Val Asp Leu Pro Thr Leu Gln Ser Trp Leu Gly
   50 55 60
   Gln Leu Ile Glu Gly Lys Gln Glu Ile Asp Phe Pro Trp Tyr Pro Ala
   65 70 75 80
   Arg Val Val Cys His Asp Ile Leu Gly Gln Thr Ala Leu Val Asp Leu
   85 90 95
   Ala Gly Leu Arg Asp Ala Ile Ala Glu Lys Gly Gly Asp Pro Ala Lys
   100 105 110
   Val Asn Pro Val Val Ala Lys Pro Ser Phe Ile Val Asp His Ser Leu
   115 120 125
   Ala Val Glu Cys Gly Gly Tyr Asp Pro Asp Ala Phe Arg Lys Asn Arg
   130 135 140
   Gln Ile Glu Asp Arg Arg Asn Glu Asp Arg Phe His Phe Ile Asn Trp
   145 150 155 160
   Thr Lys Thr Ala Phe Glu Asn Val Asp Val Ile Pro Ala Gly Asn Gly
   165 170 175
   Ile Met His Gln Ile Asn Leu Glu Lys Met Ser Pro Val Val Gln Val
               180                 185                 190
   Lys Asn Gly Val Ala Phe Pro Asp Thr Cys Val Gly Thr Asp Ser His
           195                 200                 205
   Thr Pro His Val Asp Ala Leu Gly Val Ile Ser Val Gly Val Gly Gly
       210                 215                 220
   Leu Glu Ala Glu Thr Val Met Leu Gly Arg Ala Ser Met Met Arg Leu
   225                 230                 235                 240
   Pro Asp Ile Val Gly Val Glu Leu Asn Gly Lys Arg Gln Ala Gly Ile
                   245                 250                 255
   Thr Ala Thr Asp Ile Val Leu Ala Leu Thr Glu Phe Leu Arg Lys Glu
               260                 265                 270
   Arg Val Val Gly Ala Phe Val Glu Phe Phe Gly Glu Gly Ala Arg Ser
           275                 280                 285
   Leu Ser Ile Gly Asp Arg Ala Thr Ile Ser Asn Met Thr Pro Glu Phe
       290                 295                 300
   Gly Ala Thr Ala Ala Met Phe Ala Ile Asp Glu Gln Thr Ile Asp Tyr
   305                 310                 315                 320
   Leu Lys Leu Thr Gly Arg Asp Asp Ala Gln Val Lys Leu Val Glu Thr
                   325                 330                 335
   Tyr Ala Lys Thr Ala Gly Leu Trp Ala Asp Ala Leu Lys Thr Ala Val
               340                 345                 350
   Tyr Pro Arg Val Leu Lys Phe Asp Leu Ser Ser Val Thr Arg Asn Met
           355                 360                 365
   Ala Gly Pro Ser Asn Pro His Ala Arg Phe Ala Thr Ala Asp Leu Ala
       370                 375                 380
   Ser Lys Gly Leu Ala Lys Pro Tyr Glu Glu Pro Ser Asp Gly Gln Met
   385                 390                 395                 400
   Pro Asp Gly Ala Val Ile Ile Ala Ala Ile Thr Ser Cys Thr Asn Thr
                   405                 410                 415
   Ser Asn Pro Arg Asn Val Val Ala Ala Ala Leu Leu Ala Arg Asn Ala
               420                 425                 430
   Asn Cys Phe Gly Leu Lys Arg Lys Pro Trp Val Lys Thr Ser Phe Ala
           435                 440                 445
   Pro Gly Ser Lys Val Ala Glu Ile Tyr Leu Lys Glu Ala Gly Leu Leu
       450                 455                 460
   Pro Glu Met Glu Lys Leu Gly Phe Gly Ile Val Ala Phe Ala Cys Thr
   465                 470                 475                 480
   Thr Cys Asn Gly Met Ser Gly Ala Leu Asp Pro Lys Ile Gln Lys Glu
                   485                 490                 495
   Ile Ile Asp Arg Asp Leu Tyr Ala Thr Ala Val Leu Ser Gly Asn Arg
               500                     505                     510
   Asn Phe Asp Gly Arg Val His Pro Tyr Ala Lys Gln Ala Phe Leu Ala
           515                 520                 525
   Ser Pro Pro Leu Val Val Ala Tyr Ala Leu Ala Gly Ser Ile Arg Phe
       530                 535                 540
   Asp Ile Glu Asn Asp Val Leu Gly Val Ala Asp Gly Lys Glu Ile Arg
   545                 550                 555                 560
   Leu Lys Asp Ile Trp Pro Ala Asp Glu Glu Ile Asp Ala Val Val Ala
                   565                 570                 575
   Glu Tyr Val Lys Pro Gln Gln Phe Arg Asp Val Tyr Val Pro Met Phe
               580                 585                 590
   Asp Thr Gly Thr Ala Gln Lys Ala Pro Ser Pro Leu Tyr Asp Trp Arg
           595                 600                 605
   Pro Met Ser Thr Tyr Ile Arg Arg Pro Pro Tyr Trp Glu Gly Ala Leu
       610                 615                 620
   Ala Gly Glu Arg Thr Leu Arg Gly Met Arg Pro Leu Ala Ile Leu Pro
   625                 630                 635                 640
   Asp Asn Ile Thr Thr Asp His Leu Ser Pro Ser Asn Ala Ile Leu Ala
                   645                 650                 655
   Val Ser Ala Ala Gly Glu Tyr Leu Ala Lys Met Gly Leu Pro Glu Glu
               660                 665                 670
   Asp Phe Asn Ser Tyr Ala Thr His Arg Gly Asp His Leu Thr Ala Gln
           675                 680                 685
   Arg Ala Thr Phe Ala Asn Pro Lys Leu Phe Asn Glu Met Val Lys Asn
       690                 695                 700
   Glu Asp Gly Ser Val Arg Gln Gly Ser Phe Ala Arg Val Glu Pro Glu
   705                 710                 715                 720
   Gly Glu Thr Met Arg Met Trp Glu Ala Ile Glu Thr Tyr Met Asn Arg
                   725                 730                 735
   Lys Gln Pro Leu Ile Ile Ile Ala Gly Ala Asp Tyr Gly Gln Gly Ser
               740                 745                 750
   Ser Arg Asp Trp Ala Ala Lys Gly Val Arg Leu Ala Gly Val Glu Ala
           755                 760                 765
   Ile Val Ala Glu Gly Phe Glu Arg Ile His Arg Thr Asn Leu Ile Gly
       770                 775                 780
   Met Gly Val Leu Pro Leu Gln Phe Lys Pro Asp Thr Asn Arg His Thr
   785                 790                 795                 800
   Leu Gln Leu Asp Gly Thr Glu Thr Tyr Asp Val Val Gly Glu Arg Thr
                   805                 810                 815
   Pro Arg Cys Asp Leu Thr Leu Val Ile His Arg Lys Asn Gly Glu Thr
                   820                   825                   830
   Val Glu Val Pro Val Thr Cys Arg Leu Asp Thr Ala Glu Glu Val Leu
           835                     840                     845
   Val Tyr Glu Ala Gly Gly Val Leu Gln Arg Phe Ala Gln Asp Phe Leu
       850                     855                     860
   Glu Gly Asn Ala Ala
   865
<210> 43
   <211> 1170
   <212> ADN
   <213 > Neisseria meningitidis
<220>
   <221> CDS
   <222> (1) .. (1167)
<400> 43
   atg ccg caa att aaa att ccc gcc gtt tac tac cgt ggc ggt aca tca      48
   Met Pro Gln Ile Lys Ile Pro Ala Val Tyr Tyr Arg Gly Gly Thr Ser
   1               5                   10                  15
   aaa ggc gtg ttt ttc aaa cgt tcc gac ctg ccc gag gcg gcg cgg gaa      96
   Lys Gly Val Phe Phe Lys Arg Ser Asp Leu Pro Glu Ala Ala Arg Glu
               20                  25                  30
   gcg gga agc gca cgc gac aaa atc ctc ttg cgc gta ctc ggc agc ccg     144
   Ala Gly Ser Ala Arg Asp Lys Ile Leu Leu Arg Val Leu Gly Ser Pro
           35                  40                  45
   gac ccc tac ggc aag cag ata gac ggt ttg ggc aac gcc agt tcg tcc     192
   Asp Pro Tyr Gly Lys Gln Ile Asp Gly Leu Gly Asn Ala Ser Ser Ser
       50                  55                  60
   acc agc aaa gcc gtg att ttg gac aag tcc gaa cgc acc gat cac gat     240
   Thr Ser Lys Ala Val Ile Leu Asp Lys Ser Glu Arg Thr Asp His Asp
   65                  70                  75                  80
   gtc gat tac ctt ttc ggg caa gtt tcc atc gac aaa cct ttt gtc gat     288
   Val Asp Tyr Leu Phe Gly Gln Val Ser Ile Asp Lys Pro Phe Val Asp
                   85                  90                  95
   tgg agt ggc aac tgc ggc aac ctc acc gcc gcc gtg ggc gca ttt gcc     336
   Trp Ser Gly Asn Cys Gly Asn Leu Thr Ala Ala Val Gly Ala Phe Ala
               100                 105                 110
   atc gag caa ggc ttg gtc gat aaa tcc aaa atc cct tca gac ggc ccg     384
   Ile Glu Gln Gly Leu Val Asp Lys Ser Lys Ile Pro Ser Asp Gly Pro
           115                 120                 125
   tgt acc gtc aaa atc tgg cag aaa aac atc ggc aaa acc att att gcc     432
   Cys Thr Val Lys Ile Trp Gln Lys Asn Ile Gly Lys Thr Ile Ile Ala
       130                 135                 140
   cat gta ccg atg caa aac ggc gca gtt ttg gaa aca ggc gat ttt gag     480
   His Val Pro Met Gln Asn Gly Ala Val Leu Glu Thr Gly Asp Phe Glu
   145                 150                 155                 160
   ctc gac ggc gta acg ttc ccg gca gcc gaa gta caa atc gaa ttt ctt      528
   Leu Asp Gly Val Thr Phe Pro Ala Ala Glu Val Gln Ile Glu Phe Leu
                   165                 170                 175
   gat cca gcc gac ggc gaa ggc agt atg ttc cca acc ggc aat ttg gtc      576
   Asp Pro Ala Asp Gly Glu Gly Ser Met Phe Pro Thr Gly Asn Leu Val
               180                 185                 190
   gat gaa att gat gtg ccg aat ata ggc cgt ttg aaa gcc acg ctc atc      624
   Asp Glu Ile Asp Val Pro Asn Ile Gly Arg Leu Lys Ala Thr Leu Ile
           195                 200                 205
   aac gcg ggc att ccg acc gtt ttc ctg aat gcc gcc gac ttg ggc tac      672
   Asn Ala Gly Ile Pro Thr Val Phe Leu Asn Ala Ala Asp Leu Gly Tyr
       210                 215                 220
   acg ggc aaa gag ttg caa gac gac atc aac aac gat gcc gca gct ttg      720
   Thr Gly Lys Glu Leu Gln Asp Asp Ile Asn Asn Asp Ala Ala Ala Leu
   225                 230                 235                 240
   gaa aaa ttc gag aaa atc cgc gct tac ggt gcg ctg aaa atg ggt cta      768
   Glu Lys Phe Glu Lys Ile Arg Ala Tyr Gly Ala Leu Lys Met Gly Leu
                   245                 250                 255
   atc agc gac gta tcc gaa gct gcc gcc cgc gcg cac acg ccg aaa gtc      816
   Ile Ser Asp Val Ser Glu Ala Ala Ala Arg Ala His Thr Pro Lys Val
               260                 265                 270
   gcc ttc gtc gcg ccc gcc gcc gat tac acc gcc tcc agt ggc aaa acc      864
   Ala Phe Val Ala Pro Ala Ala Asp Tyr Thr Ala Ser Ser Gly Lys Thr
           275                 280                 285
   gtg aat gcc gcc gac atc gat ttg ctg gta cgc gcc ctg agc atg ggc      912
   Val Asn Ala Ala Asp Ile Asp Leu Leu Val Arg Ala Leu Ser Met Gly
       290                 295                 300
   aaa ttg cac cac gcg atg atg ggt acc gcc tct gtt gcc att gcg acc      960
   Lys Leu His His Ala Met Met Gly Thr Ala Ser Val Ala Ile Ala Thr
   305                 310                 315                 320
   gcc gcc gcc gtg ccc ggt acg ctg gtc aac ctt gcc gca ggg gcg gga     1008
   Ala Ala Ala Val Pro Gly Thr Leu Val Asn Leu Ala Ala Gly Ala Gly
                   325                 330                 335
   acg cgt aaa gaa gtg cgc ttc ggg cat cct tcc ggc aca ttg cgc gtc     1056
   Thr Arg Lys Glu Val Arg Phe Gly His Pro Ser Gly Thr Leu Arg Val
               340                 345                 350
   ggt gca gcc gcc gaa tgt cag gac gga caa tgg acg gcc acc aaa gcg     1104
   Gly Ala Ala Ala Glu Cys Gln Asp Gly Gln Trp Thr Ala Thr Lys Ala
           355                 360                 365
   gtt atg agc cgc agc gca cgc gtg atg atg gaa ggt tgg gtc agg gtg     1152
   Val Met Ser Arg Ser Ala Arg Val Met Met Glu Gly Trp Val Arg Val
       370                 375                 380
   ccg gaa gat tgt ttt taa                                             1170
   Pro Glu Asp Cys Phe
   385
<210> 44
   <211> 389
   <212> PRT
   <213> Neisseria meningitidis
<400> 44
   Met Pro Gln Ile Lys Ile Pro Ala Val Tyr Tyr Arg Gly Gly Thr Ser
   1 5 10 15
   Lys Gly Val Phe Phe Lys Arg Ser Asp Leu Pro Glu Ala Ala Arg Glu
   20 25 30
   Ala Gly Ser Ala Arg Asp Lys Ile Leu Leu Arg Val Leu Gly Ser Pro
   35 40 45
   Asp Pro Tyr Gly Lys Gln Ile Asp Gly Leu Gly Asn Ala Ser Ser Ser
   50 55 60
   Thr Ser Lys Ala Val Ile Leu Asp Lys Ser Glu Arg Thr Asp His Asp
   65 70 75 80
   Val Asp Tyr Leu Phe Gly Gln Val Ser Ile Asp Lys Pro Phe Val Asp
   85 90 95
   Trp Ser Gly Asn Cys Gly Asn Leu Thr Ala Ala Val Gly Ala Phe Ala
   100 105 110
   Ile Glu Gln Gly Leu Val Asp Lys Ser Lys Ile Pro Ser Asp Gly Pro
   115 120 125
   Cys Thr Val Lys Ile Trp Gln Lys Asn Ile Gly Lys Thr Ile Ile Ala
   130 135 140
   His Val Pro Met Gln Asn Gly Ala Val Leu Glu Thr Gly Asp Phe Glu
   145 150 155 160
   Leu Asp Gly Val Thr Phe Pro Ala Ala Glu Val Gln Ile Glu Phe Leu
   165 170 175
   Asp Pro Ala Asp Gly Glu Gly Ser Met Phe Pro Thr Gly Asn Leu Val
   180 185 190
   Asp Glu Ile Asp Val Pro Asn Ile Gly Arg Leu Lys Ala Thr Leu Ile
   195 200 205
   Asn Ala Gly Ile Pro Thr Val Phe Leu Asn Ala Ala Asp Leu Gly Tyr
   210 215 220
   Thr Gly Lys Glu Leu Gln Asp Asp Ile Asn Asn Asp Ala Ala Ala Leu
   225 230 235 240
   Glu Lys Phe Glu Lys Ile Arg Ala Tyr Gly Ala Leu Lys Met Gly Leu
   245 250 255
   Ile Ser Asp Val Ser Glu Ala Ala Ala Arg Ala His Thr Pro Lys Val
   260 265 270
   Ala Phe Val Ala Pro Ala Ala Asp Tyr Thr Ala Ser Ser Gly Lys Thr
           275                 280                 285
   Val Asn Ala Ala Asp Ile Asp Leu Leu Val Arg Ala Leu Ser Met Gly
       290                 295                 300
   Lys Leu His His Ala Met Met Gly Thr Ala Ser Val Ala Ile Ala Thr
   305                 310                 315                 320
   Ala Ala Ala Val Pro Gly Thr Leu Val Asn Leu Ala Ala Gly Ala Gly
                   325                 330                 335
   Thr Arg Lys Glu Val Arg Phe Gly His Pro Ser Gly Thr Leu Arg Val
               340                 345                 350
   Gly Ala Ala Ala Glu Cys Gln Asp Gly Gln Trp Thr Ala Thr Lys Ala
           355                 360                 365
   Val Met Ser Arg Ser Ala Arg Val Met Met Glu Gly Trp Val Arg Val
       370                 375                 380
   Pro Glu Asp Cys Phe
   385
<210> 45
   <211> 954
   <212> ADN
   <213> Neisseria meningitidis
<220>
   <221> CDS <222>
<400> 45
   atg cgc acg ccg ttt tgt tgg gca tac gcc aat gcc gcc cga ata tcg      48
   Met Arg Thr Pro Phe Cys Trp Ala Tyr Ala Asn Ala Ala Arg Ile Ser
   1               5                   10                  15
   gca atg ctg ccg gcg tgt tgg gcg cag gcg atg ttg gcc gaa gta atc      96
   Ala Met Leu Pro Ala Cys Trp Ala Gln Ala Met Leu Ala Glu Val Ile
               20                  25                  30
   agc tgc aac aag gct tcg tcg ctg ccg cag cct tcg gcg aga tcg gcg     144
   Ser Cys Asn Lys Ala Ser Ser Leu Pro Gln Pro Ser Ala Arg Ser Ala
           35                  40                  45
   ttt aaa tca acc tgc ttc atg ggt gat tct ccg tat ttg gtt cag ata     192
   Phe Lys Ser Thr Cys Phe Met Gly Asp Ser Pro Tyr Leu Val Gln Ile
       50                  55                  60
   gac ttg gtt ttt gcg ccg cag ggc ggt ggc ttc ttt caa gcc gat tat     240
   Asp Leu Val Phe Ala Pro Gln Gly Gly Gly Phe Phe Gln Ala Asp Tyr
   65                  70                  75                  80
   ttt gaa ttt gac ttt gct gcc gaa gcg cac ctg tgc cag cct gcc caa     288
   Phe Glu Phe Asp Phe Ala Ala Glu Ala His Leu Cys Gln Pro Ala Gln
                   85                  90                  95
   atc ggc ggc ggc aac ggt agc gat ttt cgg ata acc gcc ggt ggt ttg     336
   Ile Gly Gly Gly Asn Gly Ser Asp Phe Arg Ile Thr Ala Gly Gly Leu
               100                 105                 110
   cgc atc ggc cag cag gat aat cgg ttt gcc gcc ggg cgg cac ctg cac     384
   Arg Ile Gly Gln Gln Asp Asn Arg Phe Ala Ala Gly Arg His Leu His
           115                 120                 125
   ggt tcc tgc ctg aac agc gtg gga cag cat ttc caa agg ttg cga cag     432
   Gly Ser Cys Leu Asn Ser Val Gly Gln His Phe Gln Arg Leu Arg Gln
       130                 135                 140
   ggt cag cgg ctg tcc gtc gaa gcg gta gcc cat gcg gtt gct atc gct     480
   Gly Gln Arg Leu Ser Val Glu Ala Val Ala His Ala Val Ala Ile Ala
   145                 150                 155                 160
   ttg cag cgt cca cgt ttc ccg ttc cag att cag acg ccc ttt ttc act     528
   Leu Gln Arg Pro Arg Phe Pro Phe Gln Ile Gln Thr Pro Phe Phe Thr
                   165                 170                 175
   gaa agc ggc ata ttc cga cga agg aac aag gtg gat ggt atc ggt aaa     576
   Glu Ser Gly Ile Phe Arg Arg Arg Asn Lys Val Asp Gly Ile Gly Lys
               180                 185                 190
   cgg tat cgg ggc aat gcc gac ttt gga caa ttc ctg cgc acc ttt gcc     624
   Arg Tyr Arg Gly Asn Ala Asp Phe Gly Gln Phe Leu Arg Thr Phe Ala
           195                 200                 205
   gat ggg gag ata atc gcc ttt ttg cag cat tct gcc ctg atg gcc gcc     672
   Asp Gly Glu Ile Ile Ala Phe Leu Gln His Ser Ala Leu Met Ala Ala
       210                 215                 220
   gaa acc ggc ttt cag gtc ggt gct tct cga acc cat cac ttc cgg cac     720
   Glu Thr Gly Phe Gln Val Gly Ala Ser Arg Thr His His Phe Arg His
   225                 230                 235                 240
   atc aaa tcc gcc cgc cac gca cac ata gcc gta cat gcc ctg cac ggc     768
   Ile Lys Ser Ala Arg His Ala His Ile Ala Val His Ala Leu His Gly
                   245                 250                 255
   acg cac cat ttt caa ggt ctg ccc ttt gcg ggc ggt ata acg cca ata     816
   Thr His His Phe Gln Gly Leu Pro Phe Ala Gly Gly Ile Thr Pro Ile
               260                 265                 270
   cga ata gac cgg ttc gcc gtc caa ttc cgc ctg ata cac ggc acc ggt     864
   Arg Ile Asp Arg Phe Ala Val Gln Phe Arg Leu Ile His Gly Thr Gly
           275                 280                 285
   gag aca aaa cgg cgt atc ccg ttc aaa cac cag cat tat ccc gcc caa     912
   Glu Thr Lys Arg Arg Ile Pro Phe Lys His Gln His Tyr Pro Ala Gln
       290                 295                 300
   agc gat ttc gat tgc ggc cgt gcc ttc gtc gtt gcc caa taa             954
   Ser Asp Phe Asp Cys Gly Arg Ala Phe Val Val Ala Gln
   305                 310                 315
<210> 46
   <211> 317
   <212> PRT
   <213> Neisseria meningitidis
<400> 46
   Met Arg Thr Pro Phe Cys Trp Ala Tyr Ala Asn Ala Ala Arg Ile Ser
   1               5                   10                  15
   Ala Met Leu Pro Ala Cys Trp Ala Gln Ala Met Leu Ala Glu Val Ile
               20                  25                  30
   Ser Cys Asn Lys Ala Ser Ser Leu Pro Gln Pro Ser Ala Arg Ser Ala
           35                  40                  45
   Phe Lys Ser Thr Cys Phe Met Gly Asp Ser Pro Tyr Leu Val Gln Ile
       50                  55                  60
   Asp Leu Val Phe Ala Pro Gln Gly Gly Gly Phe Phe Gln Ala Asp Tyr
   65                  70                  75                  80
   Phe Glu Phe Asp Phe Ala Ala Glu Ala His Leu Cys Gln Pro Ala Gln
                   85                  90                  95
   Ile Gly Gly Gly Asn Gly Ser Asp Phe Arg Ile Thr Ala Gly Gly Leu
               100                 105                 110
   Arg Ile Gly Gln Gln Asp Asn Arg Phe Ala Ala Gly Arg His Leu His
           115                 120                 125
   Gly Ser Cys Leu Asn Ser Val Gly Gln His Phe Gln Arg Leu Arg Gln
       130                 135                 140
   Gly Gln Arg Leu Ser Val Glu Ala Val Ala His Ala Val Ala Ile Ala
   145                 150                 155                 160
   Leu Gln Arg Pro Arg Phe Pro Phe Gln Ile Gln Thr Pro Phe Phe Thr
                   165                 170                 175
   Glu Ser Gly Ile Phe Arg Arg Arg Asn Lys Val Asp Gly Ile Gly Lys
               180                 185                 190
   Arg Tyr Arg Gly Asn Ala Asp Phe Gly Gln Phe Leu Arg Thr Phe Ala
           195                 200                 205
   Asp Gly Glu Ile Ile Ala Phe Leu Gln His Ser Ala Leu Met Ala Ala
       210                 215                 220
   Glu Thr Gly Phe Gln Val Gly Ala Ser Arg Thr His His Phe Arg His
   225                 230                 235                 240
   Ile Lys Ser Ala Arg His Ala His Ile Ala Val His Ala Leu His Gly
                   245                 250                 255
   Thr His His Phe Gln Gly Leu Pro Phe Ala Gly Gly Ile Thr Pro Ile
               260                 265                 270
   Arg Ile Asp Arg Phe Ala Val Gln Phe Arg Leu Ile His Gly Thr Gly
           275                 280                 285
   Glu Thr Lys Arg Arg Ile Pro Phe Lys His Gln His Tyr Pro Ala Gln
       290                 295                 300
   Ser Asp Phe Asp Cys Gly Arg Ala Phe Val Val Ala Gln
   305 310 315
<210> 47
   <211> 648
   <212> ADN
   <213> Neisseria meningitidis
<220>
   <221> CDS
   <222> (1) .. (645)
<400> 47
   atg aga ata gag atc aca cca atc agc gaa tcc gct ttg gtc tgc cga    48
   Met Arg Ile Glu Ile Thr Pro Ile Ser Glu Ser Ala Leu Val Cys Arg
   1               5                   10                  15
   ctg aat gcg cct tcc gaa ctg ggc aaa cag caa aag ttg tgg gcg ttt    96
   Leu Asn Ala Pro Ser Glu Leu Gly Lys Gln Gln Lys Leu Trp Ala Phe
               20                  25                  30
   gcc gct gcg ctc ggg cag cac gac agg att gag gaa gtg gtg gtc ggc    144
   Ala Ala Ala Leu Gly Gln His Asp Arg Ile Glu Glu Val Val Val Gly
           35                  40                  45
   atg aac aat ctg acc gtg ttc acc cgt ttc gat acc gat ttg gcg acg    192
   Met Asn Asn Leu Thr Val Phe Thr Arg Phe Asp Thr Asp Leu Ala Thr
       50                  55                  60
   ctt gcc gat gaa ttg caa tat gtg tgg gaa cac acc gcc gtt aca gac    240
   Leu Ala Asp Glu Leu Gln Tyr Val Trp Glu His Thr Ala Val Thr Asp
   65                  70                  75                  80
   cat cag ggc aaa ctg gtg gaa att ccc gtc tgc tac ggc ggc gaa tac    288
   His Gln Gly Lys Leu Val Glu Ile Pro Val Cys Tyr Gly Gly Glu Tyr
                   85                  90                  95
   ggc ccg gat ttg gcg gaa gtc gct gct ttc cat cag acg gtt att tcc    336
   Gly Pro Asp Leu Ala Glu Val Ala Ala Phe His Gln Thr Val Ile Ser
               100                 105                 110
   gaa atc gtc cgc cgc cat acg gcg caa act tat acc gta ttt atg atg    384
   Glu Ile Val Arg Arg His Thr Ala Gln Thr Tyr Thr Val Phe Met Met
           115                 120                 125
   ggc ttc cag cct ggt ttc cct tat ctg ggc ggc ttg ccc gaa gca ttg    432
   Gly Phe Gln Pro Gly Phe Pro Tyr Leu Gly Gly Leu Pro Glu Ala Leu
       130                 135                 140
   cac acg ccc cgc cgt gcc gtg ccg aga acg tcc gtt cct gcc ggt tcg    480
   His Thr Pro Arg Arg Ala Val Pro Arg Thr Ser Val Pro Ala Gly Ser
   145                 150                 155                 160
   gtc ggt atc ggc ggc agt cag acc ggt gtg tat ccg ttc gct tcg ccc    528
   Val Gly Ile Gly Gly Ser Gln Thr Gly Val Tyr Pro Phe Ala Ser Pro
                   165                 170                 175
   ggc ggc tgg cag att atc ggc aga acc gaa tta ccc ttg ttc cga gcc    576
   Gly Gly Trp Gln Ile Ile Gly Arg Thr Glu Leu Pro Leu Phe Arg Ala
               180                 185                 190
   gat ttg aat ccg ccg acc ctg ctg gcg gcg ggt gac caa gtc cgc ttt    624
   Asp Leu Asn Pro Pro Thr Leu Leu Ala Ala Gly Asp Gln Val Arg Phe
           195                 200                 205
   gtt gca gaa agg att gag cca tga                                    648
   Val Ala Glu Arg Ile Glu Pro
       210                 215
<210> 48
   <211> 215
   <212> PRT
   <213> Neisseria meningitidis
<400> 48
   Met Arg Ile Glu Ile Thr Pro Ile Ser Glu Ser Ala Leu Val Cys Arg
   1 5 10 15
   Leu Asn Ala Pro Ser Glu Leu Gly Lys Gln Gln Lys Leu Trp Ala Phe
   20 25 30
   Ala Ala Ala Leu Gly Gln His Asp Arg Ile Glu Glu Val Val Val Gly
   35 40 45
   Met Asn Asn Leu Thr Val Phe Thr Arg Phe Asp Thr Asp Leu Ala Thr
   50 55 60
   Leu Ala Asp Glu Leu Gln Tyr Val Trp Glu His Thr Ala Val Thr Asp
   65 70 75 80
   His Gln Gly Lys Leu Val Glu Ile Pro Val Cys Tyr Gly Gly Glu Tyr
   85 90 95
   Gly Pro Asp Leu Ala Glu Val Ala Ala Phe His Gln Thr Val Ile Ser
   100 105 110
   Glu Ile Val Arg Arg His Thr Ala Gln Thr Tyr Thr Val Phe Met Met
   115 120 125
   Gly Phe Gln Pro Gly Phe Pro Tyr Leu Gly Gly Leu Pro Glu Ala Leu
   130 135 140
   His Thr Pro Arg Arg Ala Val Pro Arg Thr Ser Val Pro Ala Gly Ser
   145 150 155 160
   Val Gly Ile Gly Gly Ser Gln Thr Gly Val Tyr Pro Phe Ala Ser Pro
   165 170 175
   Gly Gly Trp Gln Ile Ile Gly Arg Thr Glu Leu Pro Leu Phe Arg Ala
   180 185 190
   Asp Leu Asn Pro Pro Thr Leu Leu Ala Ala Gly Asp Gln Val Arg Phe
   195 200 205
   Val Ala Glu Arg Ile Glu Pro
   210 215
<210> 49
   <211> 930
   <212> ADN
   <213> Neisseria meningitidis
<220>
   <221> CDS
   <222> (1) .. (927)
<400> 49
   atg att cac gtt tcg gca gtg cag gca ccg gcg cat att cag gat acc      48
   Met Ile His Val Ser Ala Val Gln Ala Pro Ala His Ile Gln Asp Thr
    1               5                  10                  15
   gga cgc tac gga cac cgg cgt tac ggc atc ggt cat gcc ggt gcg atg      96
   Gly Arg Tyr Gly His Arg Arg Tyr Gly Ile Gly His Ala Gly Ala Met
               20                  25                  30
   gac acg gtt gct ttg gcg gcg ggt aat att tta ttg ggc aac gac gaa     144
   Asp Thr Val Ala Leu Ala Ala Gly Asn Ile Leu Leu Gly Asn Asp Glu
           35                  40                  45
   ggc acg gcc gca atc gaa atc gct ttg ggc ggg ata atg ctg gtg ttt     192
   Gly Thr Ala Ala Ile Glu Ile Ala Leu Gly Gly Ile Met Leu Val Phe
       50                  55                  60
   gaa cgg gat acg ccg ttt tgt ctc acc ggt gcc gtg tat cag gcg gaa     240
   Glu Arg Asp Thr Pro Phe Cys Leu Thr Gly Ala Val Tyr Gln Ala Glu
   65                  70                  75                  80
   ttg gac ggc gaa ccg gtc tat tcg tat tgg cgt tat acc gcc cgc aaa     288
   Leu Asp Gly Glu Pro Val Tyr Ser Tyr Trp Arg Tyr Thr Ala Arg Lys
                   85                  90                  95
   ggg cag acc ttg aaa atg gtg cgt gcc gtg cag ggc atg tac ggc tat     336
   Gly Gln Thr Leu Lys Met Val Arg Ala Val Gln Gly Met Tyr Gly Tyr
               100                 105                 110
   gtg tgc gtg gcg ggc gga ttt gat gtg ccg gaa gtg atg ggt tcg aga     384
   Val Cys Val Ala Gly Gly Phe Asp Val Pro Glu Val Met Gly Ser Arg
           115                 120                 125
   agc acc gac ctg aaa gcc ggt ttc ggc ggc cat cag ggc aga atg ctg     432
   Ser Thr Asp Leu Lys Ala Gly Phe Gly Gly His Gln Gly Arg Met Leu
       130                 135                 140
   caa aaa ggc gat tat ctc ccc atc ggc aaa ggt gcg cag gaa ttg tcc     480
   Gln Lys Gly Asp Tyr Leu Pro Ile Gly Lys Gly Ala Gln Glu Leu Ser
   145                 150                 155                 160
   aaa gtc ggc att gcc ccg ata ccg ttt acc gat acc atc cac ctt gtt     528
   Lys Val Gly Ile Ala Pro Ile Pro Phe Thr Asp Thr Ile His Leu Val
                   165                 170                 175
   cct tcg tcg gaa tat gcc gct ttc agt gaa aaa ggg cgt ctg aat ctg     576
   Pro Ser Ser Glu Tyr Ala Ala Phe Ser Glu Lys Gly Arg Leu Asn Leu
               180                 185                 190
   gaa cgg gaa acg tgg acg ctg caa agc gat agc aac cgc atg ggc tac     624
   Glu Arg Glu Thr Trp Thr Leu Gln Ser Asp Ser Asn Arg Met Gly Tyr
           195                 200                 205
   cgc ttc gac gga cag ccg ctg acc ctg tcg caa cct ttg gaa atg ctg     672
   Arg Phe Asp Gly Gln Pro Leu Thr Leu Ser Gln Pro Leu Glu Met Leu
       210                 215                 220
   tcc cac gct gtt cag gca gga acc gtg cag gtg ccg ccc ggc ggc aaa     720
   Ser His Ala Val Gln Ala Gly Thr Val Gln Val Pro Pro Gly Gly Lys
   225                 230                 235                 240
   ccg att atc ctg ctg gcc gat gcg caa acc acc ggc ggt tat ccg aaa     768
   Pro Ile Ile Leu Leu Ala Asp Ala Gln Thr Thr Gly Gly Tyr Pro Lys
                   245                 250                 255
   atc gct acc gtt gcc gcc gcc gat ttg ggc agg ctg gca cag gtg cgc     816
   Ile Ala Thr Val Ala Ala Ala Asp Leu Gly Arg Leu Ala Gln Val Arg
               260                 265                 270
   ttc ggc agc aaa gtc aaa ttc aaa ata atc ggc ttg aaa gaa gcc acc     864
   Phe Gly Ser Lys Val Lys Phe Lys Ile Ile Gly Leu Lys Glu Ala Thr
           275                 280                 285
   gcc ctg cgg cgc aaa aac caa gtc tat ctg aac caa ata cgg aga atc     912
   Ala Leu Arg Arg Lys Asn Gln Val Tyr Leu Asn Gln Ile Arg Arg Ile
       290                 295                 300
   acc cat gaa gca ggt tga                                             930
   Thr His Glu Ala Gly
   305
<210> 50
   <211> 309
   <212> PRT
   <213> Neisseria meningitidis
<400> 50
   Met Ile His Val Ser Ala Val Gln Ala Pro Ala His Ile Gln Asp Thr
   1               5                   10                  15
   Gly Arg Tyr Gly His Arg Arg Tyr Gly Ile Gly His Ala Gly Ala Met
               20                  25                  30
   Asp Thr Val Ala Leu Ala Ala Gly Asn Ile Leu Leu Gly Asn Asp Glu
           35                  40                  45
   Gly Thr Ala Ala Ile Glu Ile Ala Leu Gly Gly Ile Met Leu Val Phe
       50                  55                  60
   Glu Arg Asp Thr Pro Phe Cys Leu Thr Gly Ala Val Tyr Gln Ala Glu
   65                  70                  75                  80
   Leu Asp Gly Glu Pro Val Tyr Ser Tyr Trp Arg Tyr Thr Ala Arg Lys
                   85                  90                  95
   Gly Gln Thr Leu Lys Met Val Arg Ala Val Gln Gly Met Tyr Gly Tyr
               100                 105                 110
   Val Cys Val Ala Gly Gly Phe Asp Val Pro Glu Val Met Gly Ser Arg
   115 120 125
   Ser Thr Asp Leu Lys Ala Gly Phe Gly Gly His Gln Gly Arg Met Leu
   130 135 140
   Gln Lys Gly Asp Tyr Leu Pro Ile Gly Lys Gly Ala Gln Glu Leu Ser
   145 150 155 160
   Lys Val Gly Ile Ala Pro Ile Pro Phe Thr Asp Thr Ile His Leu Val
   165 170 175
   Pro Ser Ser Glu Tyr Ala Ala Phe Ser Glu Lys Gly Arg Leu Asn Leu
   180 185 190
   Glu Arg Glu Thr Trp Thr Leu Gln Ser Asp Ser Asn Arg Met Gly Tyr
   195 200 205
   Arg Phe Asp Gly Gln Pro Leu Thr Leu Ser Gln Pro Leu Glu Met Leu
   210 215 220
   Ser His Ala Val Gln Ala Gly Thr Val Gln Val Pro Pro Gly Gly Lys
   225 230 235 240
   Pro Ile Ile Leu Leu Ala Asp Ala Gln Thr Thr Gly Gly Tyr Pro Lys
   245 250 255
   Ile Ala Thr Val Ala Ala Ala Asp Leu Gly Arg Leu Ala Gln Val Arg
   260 265 270
   Phe Gly Ser Lys Val Lys Phe Lys Ile Ile Gly Leu Lys Glu Ala Thr
   275 280 285
   Ala Leu Arg Arg Lys Asn Gln Val Tyr Leu Asn Gln Ile Arg Arg Ile
   290 295 300
   Thr His Glu Ala Gly
   305
<210> 51
   <211> 2094
   <212> ADN
   <213> Neisseria meningitidis
<220>
   <221> CDS
   <222> (1) .. (2091)
<400> 51
   atg aat tcg acc gca agt aaa acc ctg aaa gga ttg tcg ctg gtg ttt 48
   Met Asn Ser Thr Ala Ser Lys Thr Leu Lys Gly Leu Ser Leu Val Phe
   1 5 10 15
   ttc gcc tct gga ttc tgc gcc ctg att tac cag gtc agc tgg cag agg 96
   Phe Ala Ser Gly Phe Cys Ala Leu Ile Tyr Gln Val Ser Trp Gln Arg
   20 25 30
   ctt cta ttc agt cac ata ggt atc gat ttg agt tcg att act gtc att      144
   Leu Leu Phe Ser His Ile Gly Ile Asp Leu Ser Ser Ile Thr Val Ile
           35                  40                  45
   att tct gta ttt atg gtc ggc ttg ggt gta ggt gcg tat ttc ggt gga      192
   Ile Ser Val Phe Met Val Gly Leu Gly Val Gly Ala Tyr Phe Gly Gly
       50                  55                  60
   cgc att gct gac cgt ttt cct tca agt atc atc ccc ctg ttt tgc atc      240
   Arg Ile Ala Asp Arg Phe Pro Ser Ser Ile Ile Pro Leu Phe Cys Ile
   65                  70                  75                  80
   gct gaa gta tcc atc ggt ctg ttc ggt ttg gta agc agg ggt ctg att      288
   Ala Glu Val Ser Ile Gly Leu Phe Gly Leu Val Ser Arg Gly Leu Ile
                   85                  90                  95
   tcc ggc ttg ggg cat ctt tta gtt gag gct gat ttg ccc atc atc gct      336
   Ser Gly Leu Gly His Leu Leu Val Glu Ala Asp Leu Pro Ile Ile Ala
               100                 105                 110
   gct gcc aat ttc ctc tta ttg ctg ctt cct acc ttt atg atg ggc gcg      384
   Ala Ala Asn Phe Leu Leu Leu Leu Leu Pro Thr Phe Met Met Gly Ala
           115                 120                 125
   acc ttg ccc ttg ctg acc tgt ttt ttt aac cgg aaa ata cat aat gtt      432
   Thr Leu Pro Leu Leu Thr Cys Phe Phe Asn Arg Lys Ile His Asn Val
       130                 135                 140
   ggc gag tct atc ggt acc tta tat ttt ttc aac act ttg ggt gcg gca      480
   Gly Glu Ser Ile Gly Thr Leu Tyr Phe Phe Asn Thr Leu Gly Ala Ala
   145                 150                 155                 160
   ctc gga tcg ctt gcc gcc gcc gaa ttt ttc tac gtc ttt ttt acc ctc      528
   Leu Gly Ser Leu Ala Ala Ala Glu Phe Phe Tyr Val Phe Phe Thr Leu
                   165                 170                 175
   tcc caa acc att gcg ctg aca gcc tgc ttt aac ctt ctg att gct gct      576
   Ser Gln Thr Ile Ala Leu Thr Ala Cys Phe Asn Leu Leu Ile Ala Ala
               180                 185                 190
   tca gta tgc tgc gtt aca gaa agg atg gat ata gtg aac act aaa ccg      624
   Ser Val Cys Cys Val Thr Glu Arg Met Asp Ile Val Asn Thr Lys Pro
           195                 200                 205
   aat act agt ttg att tat atg ctt tct ttc ctt agc ggc tta ttg agc      672
   Asn Thr Ser Leu Ile Tyr Met Leu Ser Phe Leu Ser Gly Leu Leu Ser
       210                 215                 220
   ttg ggt ata gaa gtc ttg tgg gta agg atg ttt tcg ttc gca gca cag      720
   Leu Gly Ile Glu Val Leu Trp Val Arg Met Phe Ser Phe Ala Ala Gln
   225                 230                 235                 240
   tcc gtg cct cag gca ttt tca ttt act ctt gcc tat ttt ctg acc ggt      768
   Ser Val Pro Gln Ala Phe Ser Phe Thr Leu Ala Tyr Phe Leu Thr Gly
                   245                 250                 255
   atc gcc gtc ggc gcg tat ttt ggc aaa cgg att tgc cgc agc cgc ttt      816
   Ile Ala Val Gly Ala Tyr Phe Gly Lys Arg Ile Cys Arg Ser Arg Phe
               260                 265                 270
   gtt gat att ccc ttt atc ggg cag tgc ttc ttg tgg gcg ggt att gcc     864
   Val Asp Ile Pro Phe Ile Gly Gln Cys Phe Leu Trp Ala Gly Ile Ala
           275                     280                     285
   gac ttt ttg att ttg ggt gct gcg tgg ttg ttg acg ggt ttt tcc ggc     912
   Asp Phe Leu Ile Leu Gly Ala Ala Trp Leu Leu Thr Gly Phe Ser Gly
       290                     295                     300
   ttc gtc cac cac gcc ggt atc ttc att acc ctg tct gcc gtc gtc aga     960
   Phe Val His His Ala Gly Ile Phe Ile Thr Leu Ser Ala Val Val Arg
   305                 310                     315                 320
   ggg ttg att ttc ccg ctc gta cac cat gtg ggt acg gat ggc aac aaa    1008
   Gly Leu Ile Phe Pro Leu Val His His Val Gly Thr Asp Gly Asn Lys
                   325                     330                 335
   tcc gga cga cag gtt tcc aat gtt tat ttc gcc aac gtt gcc ggc agt    1056
   Ser Gly Arg Gln Val Ser Asn Val Tyr Phe Ala Asn Val Ala Gly Ser
               340                 345                     350
   gca ttg ggt ccg gtc ctt atc ggc ttt gtg ata ctt gat ttc ttg tcc    1104
   Ala Leu Gly Pro Val Leu Ile Gly Phe Val Ile Leu Asp Phe Leu Ser
           355                     360                     365
   acc caa cag att tac ctg ctc atc tgt ttg att tct gct gct gtc cct    1152
   Thr Gln Gln Ile Tyr Leu Leu Ile Cys Leu Ile Ser Ala Ala Val Pro
       370                     375                     380
   ttg ttt tgt aca ctg ttc caa aaa agt ctc cga ctg aat gca gtg tcg    1200
   Leu Phe Cys Thr Leu Phe Gln Lys Ser Leu Arg Leu Asn Ala Val Ser
   385                 390                     395                 400
   gta gca gtt tcc cta atg ttc ggc atc ctc atg ttc cta ctg ccg gat    1248
   Val Ala Val Ser Leu Met Phe Gly Ile Leu Met Phe Leu Leu Pro Asp
                   405                     410                 415
   tct gtc ttt caa aat att gct gac cgt ccg gat cgg ctg att gaa aac    1296
   Ser Val Phe Gln Asn Ile Ala Asp Arg Pro Asp Arg Leu Ile Glu Asn
               420                 425                     430
   aaa cac ggc att gtt gcg gtt tac cat aga gat ggt gat aag gtt gtt    1344
   Lys His Gly Ile Val Ala Val Tyr His Arg Asp Gly Asp Lys Val Val
           435                     440                     445
   tat ggg gcg aat gta tac gac ggc gca tac aat acc gat gta ttc aat    1392
   Tyr Gly Ala Asn Val Tyr Asp Gly Ala Tyr Asn Thr Asp Val Phe Asn
       450                     455                     460
   agt gtc aac ggc atc gaa cgt gcc tat ctg cta ccc tcc ctg aag tct    1440
   Ser Val Asn Gly Ile Glu Arg Ala Tyr Leu Leu Pro Ser Leu Lys Ser
   465                 470                     475                 480
   ggc ata cgc cgc att ttc gtc gtt gga ttg agt aca ggt tcg tgg gcg    1488
   Gly Ile Arg Arg Ile Phe Val Val Gly Leu Ser Thr Gly Ser Trp Ala
                   485                     490                 495
   cgc gtc ttg tct gcc att ccg gaa atg cag tcg atg atc gtt gcg gaa    1536
   Arg Val Leu Ser Ala Ile Pro Glu Met Gln Ser Met Ile Val Ala Glu
               500                     505                     510
   atc aat ccg gca tac cgt agc ctt atc gcg gac gag ccg caa atc gcc    1584
   Ile Asn Pro Ala Tyr Arg Ser Leu Ile Ala Asp Glu Pro Gln Ile Ala
           515                 520                 525
   ccg ctt ttg cag gac aaa cgt gtt gaa att gta ttg gat gac ggt agg    1632
   Pro Leu Leu Gln Asp Lys Arg Val Glu Ile Val Leu Asp Asp Gly Arg
       530                 535                 540
   aaa tgg ctg cgt cgc cat cct gat gaa aaa ttc gac ctg att ttg atg    1680
   Lys Trp Leu Arg Arg His Pro Asp Glu Lys Phe Asp Leu Ile Leu Met
   545                 550                 555                 560
   aat acg act tgg tac tgg cgt gcc tat tcc acc aac ctg ttg agt gcg    1728
   Asn Thr Thr Trp Tyr Trp Arg Ala Tyr Ser Thr Asn Leu Leu Ser Ala
                   565                 570                 575
   gaa ttt tta aaa cag gtg caa agc cac ctt acc ccg gat ggt att gta    1776
   Glu Phe Leu Lys Gln Val Gln Ser His Leu Thr Pro Asp Gly Ile Val
               580                 585                 590
   atg ttt aat acc acg cac agc ccg cat gct ttt gct acc gcc gta cac    1824
   Met Phe Asn Thr Thr His Ser Pro His Ala Phe Ala Thr Ala Val His
           595                 600                 605
   agt att ccc tat gca tac cgc tat ggg cat atg gta gtc ggc tcg gca    1872
   Ser Ile Pro Tyr Ala Tyr Arg Tyr Gly His Met Val Val Gly Ser Ala
       610                 615                 620
   acc ccg gta gtt ttc cct aat aaa gaa ctg ctc aag caa cgt ctc tcc    1920
   Thr Pro Val Val Phe Pro Asn Lys Glu Leu Leu Lys Gln Arg Leu Ser
   625                 630                 635                 640
   cgg ttg att tgg ccg gaa agc ggc agg cac gta ttt gac agc agc acc    1968
   Arg Leu Ile Trp Pro Glu Ser Gly Arg His Val Phe Asp Ser Ser Thr
                   645                 650                 655
   gtg gat gct gca gca caa aag gtt gtc tct cgt atg ctg att cag atg    2016
   Val Asp Ala Ala Ala Gln Lys Val Val Ser Arg Met Leu Ile Gln Met
               660                 665                 670
   acg gaa cct tcg gct ggg gcg gaa gtc att acc gac gat aat atg att    2064
   Thr Glu Pro Ser Ala Gly Ala Glu Val Ile Thr Asp Asp Asn Met Ile
           675                 680                 685
   gta gaa tac aaa tac ggc aga ggg att taa                            2094
   Val Glu Tyr Lys Tyr Gly Arg Gly Ile
       690                 695
<210> 52
   <211> 697
   <212> PRT
   <213> Neisseria meningitidis
<400> 52
   Met Asn Ser Thr Ala Ser Lys Thr Leu Lys Gly Leu Ser Leu Val Phe
   1 5 10 15
   Phe Ala Ser Gly Phe Cys Ala Leu Ile Tyr Gln Val Ser Trp Gln Arg
   20 25 30
   Leu Leu Phe Ser His Ile Gly Ile Asp Leu Ser Ser Ile Thr Val Ile
   35 40 45
   Ile Ser Val Phe Met Val Gly Leu Gly Val Gly Ala Tyr Phe Gly Gly
   50 55 60
   Arg Ile Ala Asp Arg Phe Pro Ser Ser Ile Ile Pro Leu Phe Cys Ile
   65 70 75 80
   Ala Glu Val Ser Ile Gly Leu Phe Gly Leu Val Ser Arg Gly Leu Ile
   85 90 95
   Ser Gly Leu Gly His Leu Leu Val Glu Ala Asp Leu Pro Ile Ile Ala
   100 105 110
   Ala Ala Asn Phe Leu Leu Leu Leu Leu Pro Thr Phe Met Met Gly Ala
   115 120 125
   Thr Leu Pro Leu Leu Thr Cys Phe Phe Asn Arg Lys Ile His Asn Val
   130 135 140
   Gly Glu Ser Ile Gly Thr Leu Tyr Phe Phe Asn Thr Leu Gly Ala Ala
   145 150 155 160
   Leu Gly Ser Leu Ala Ala Ala Glu Phe Phe Tyr Val Phe Phe Thr Leu
   165 170 175
   Ser Gln Thr Ile Ala Leu Thr Ala Cys Phe Asn Leu Leu Ile Ala Ala
   180 185 190
   Ser Val Cys Cys Val Thr Glu Arg Met Asp Ile Val Asn Thr Lys Pro
   195 200 205
   Asn Thr Ser Leu Ile Tyr Met Leu Ser Phe Leu Ser Gly Leu Leu Ser
   210 215 220
   Leu Gly Ile Glu Val Leu Trp Val Arg Met Phe Ser Phe Ala Ala Gln
   225 230 235 240
   Ser Val Pro Gln Ala Phe Ser Phe Thr Leu Ala Tyr Phe Leu Thr Gly
   245 250 255
   Ile Ala Val Gly Ala Tyr Phe Gly Lys Arg Ile Cys Arg Ser Arg Phe
   260 265 270
   Val Asp Ile Pro Phe Ile Gly Gln Cys Phe Leu Trp Ala Gly Ile Ala
   275 280 285
   Asp Phe Leu Ile Leu Gly Ala Ala Trp Leu Leu Thr Gly Phe Ser Gly
   290 295 300
   Phe Val His His Ala Gly Ile Phe Ile Thr Leu Ser Ala Val Val Arg
   305 310 315 320
   Gly Leu Ile Phe Pro Leu Val His His Val Gly Thr Asp Gly Asn Lys
   325 330 335
   Ser Gly Arg Gln Val Ser Asn Val Tyr Phe Ala Asn Val Ala Gly Ser
   340 345 350
   Ala Leu Gly Pro Val Leu Ile Gly Phe Val Ile Leu Asp Phe Leu Ser
   355 360 365
   Thr Gln Gln Ile Tyr Leu Leu Ile Cys Leu Ile Ser Ala Ala Val Pro
   370 375 380
   Leu Phe Cys Thr Leu Phe Gln Lys Ser Leu Arg Leu Asn Ala Val Ser
   385 390 395 400
   Val Ala Val Ser Leu Met Phe Gly Ile Leu Met Phe Leu Leu Pro Asp
   405 410 415
   Ser Val Phe Gln Asn Ile Ala Asp Arg Pro Asp Arg Leu Ile Glu Asn
   420 425 430
   Lys His Gly Ile Val Ala Val Tyr His Arg Asp Gly Asp Lys Val Val
   435 440 445
   Tyr Gly Ala Asn Val Tyr Asp Gly Ala Tyr Asn Thr Asp Val Phe Asn
   450 455 460
   Ser Val Asn Gly Ile Glu Arg Ala Tyr Leu Leu Pro Ser Leu Lys Ser
   465 470 475 480
   Gly Ile Arg Arg Ile Phe Val Val Gly Leu Ser Thr Gly Ser Trp Ala
   485 490 495
   Arg Val Leu Ser Ala Ile Pro Glu Met Gln Ser Met Ile Val Ala Glu
   500 505 510
   Ile Asn Pro Ala Tyr Arg Ser Leu Ile Ala Asp Glu Pro Gln Ile Ala
   515 520 525
   Pro Leu Leu Gln Asp Lys Arg Val Glu Ile Val Leu Asp Asp Gly Arg
   530 535 540
   Lys Trp Leu Arg Arg His Pro Asp Glu Lys Phe Asp Leu Ile Leu Met
   545 550 555 560
   Asn Thr Thr Trp Tyr Trp Arg Ala Tyr Ser Thr Asn Leu Leu Ser Ala
   565 570 575
   Glu Phe Leu Lys Gln Val Gln Ser His Leu Thr Pro Asp Gly Ile Val
   580 585 590
   Met Phe Asn Thr Thr His Ser Pro His Ala Phe Ala Thr Ala Val His
   595 600 605
   Ser Ile Pro Tyr Ala Tyr Arg Tyr Gly His Met Val Val Gly Ser Ala
   610 615 620
   Thr Pro Val Val Phe Pro Asn Lys Glu Leu Leu Lys Gln Arg Leu Ser
   625 630 635 640
   Arg Leu Ile Trp Pro Glu Ser Gly Arg His Val Phe Asp Ser Ser Thr
   645 650 655
   Val Asp Ala Ala Ala Gln Lys Val Val Ser Arg Met Leu Ile Gln Met
   660 665 670
   Thr Glu Pro Ser Ala Gly Ala Glu Val Ile Thr Asp Asp Asn Met Ile
   675 680 685
   Val Glu Tyr Lys Tyr Gly Arg Gly Ile
   690 695
<210> 53
   <211> 1040
   <212> PRT
   <213> Neisseria meningitidis
<400> 53
   Cys Leu Gly Gly Gly Gly Gly Thr Ser Ala Pro Asp Phe Asn Ala Gly
   1 5 10 15
   Gly Thr Gly Ile Gly Ser Asn Ser Arg Ala Thr Thr Ala Lys Ser Ala
   20 25 30
   Ala Val Ser Tyr Ala Gly Ile Lys Asn Glu Met Cys Lys Asp Arg Ser
   35 40 45
   Met Leu Cys Ala Gly Arg Asp Asp Val Ala Val Thr Asp Arg Asp Ala
   50 55 60
   Lys Ile Asn Ala Pro Pro Pro Asn Leu His Thr Gly Asp Phe Thr Asn
   65 70 75 80
   Pro Asn Asp Ala Tyr Lys Asn Leu Ile Asn Leu Lys Pro Ala Ile Glu
   85 90 95
   Ala Gly Tyr Thr Gly Arg Gly Val Glu Val Gly Ile Val Asp Thr Gly
   100 105 110
   Glu Ser Val Gly Ser Ile Ser Phe Pro Glu Leu Tyr Gly Arg Lys Glu
   115 120 125
   His Gly Tyr Asn Glu Asn Tyr Lys Asn Tyr Thr Ala Tyr Met Arg Lys
   130 135 140
   Glu Ala Pro Glu Asp Gly Gly Gly Lys Asp Ile Lys Ala Ser Phe Asp
   145 150 155 160
   Asp Glu Ala Val Ile Glu Thr Glu Ala Lys Pro Thr Asp Ile Arg His
   165 170 175
   Val Lys Glu Ile Gly His Ile Asp Val Val Ser His Ile Ile Gly Gly
   180 185 190
   Arg Ser Val Asp Gly Arg Pro Ala Gly Gly Ile Ala Pro Asp Ala Thr
   195 200 205
   Leu His Ile Met Asn Thr His Asp Gly Thr Lys Asn Glu Ile Met Ser
   210 215 220
   Ala Ala Ile Arg Asn Ala Trp Val Lys Leu Gly Glu Arg Gly Val Arg
   225 230 235 240
   Ile Val Asn Asn Ser Phe Gly Thr Thr Ser Arg Ala Gly Thr Ala Asp
   245 250 255
   His Phe Gln Ile Ala Asn Ser Glu Glu Gln Tyr Arg Gln Ala Leu Leu
   260 265 270
   Ala Tyr Ser Gly Gly Asp Lys Thr Asp Glu Gly Ile Arg Leu Met Gln
   275 280 285
   Gln Ser Asp Tyr Gly Asn Leu Ser Tyr His Ile Arg Asn Lys Asn Met
   290 295 300
   Leu Phe Ile Phe Ser Ala Ser Asn Asp Ala Gln Ala Gln Pro Asn Thr
   305 310 315 320
   Leu Thr Leu Leu Pro Phe Tyr Glu Lys Asp Ala Gln Lys Gly Ile Ile
   325 330 335
   Thr Val Ala Gly Val Asp Arg Ser Gly Glu Lys Phe Asn Gly Ser Asn
   340 345 350
   His Cys Gly Ile Thr Ala Met Trp Cys Leu Ser Ala Pro Tyr Glu Ala
   355 360 365
   Ser Val Arg Phe Thr Arg Thr Asn Pro Ile Gln Ile Ala Gly Thr Ser
   370 375 380
   Phe Ser Ala Pro Ile Val Thr Gly Thr Ala Ala Leu Leu Leu Gln Lys
   385 390 395 400
   Tyr Pro Trp Met Ser Asn Asp Asn Leu Arg Thr Thr Leu Leu Thr Thr
   405 410 415
   Ala Gln Asp Ile Gly Ala Val Gly Val Asp Ser Lys Phe Gly Trp Gly
   420 425 430
   Leu Leu Asp Ala Gly Lys Ala Met Asn Gly Pro Ala Ser Phe Pro Phe
   435 440 445
   Gly Asp Phe Thr Ala Asp Thr Lys Gly Thr Ser Asp Ile Ala Tyr Ser
   450 455 460
   Phe Arg Asn Asp Ile Ser Gly Thr Gly Gly Leu Ile Lys Lys Gly Gly
   465 470 475 480
   Ser Gln Leu Gln Leu His Gly Asn Asn Thr Tyr Thr Gly Lys Thr Ile
   485 490 495
   Ile Glu Gly Gly Ser Leu Val Leu Tyr Gly Asn Asn Lys Ser Asp Met
   500 505 510
   Arg Val Glu Thr Lys Gly Ala Leu Ile Tyr Asn Gly Ala Ala Ser Gly
   515 520 525
   Gly Ser Leu Asn Ser Asp Gly Ile Val Tyr Leu Ala Asp Thr Asp Arg
   530 535 540
   Ser Gly Ala Asn Glu Thr Val His Ile Lys Gly Asp Leu Gln Leu Gly
   545 550 555 560
   Gly Glu Gly Thr Leu Tyr Thr Arg Leu Gly Lys Leu Leu Lys Val Asp
   565 570 575
   Gly Thr Ala Met Thr Gly Gly Lys Leu Tyr Met Ser Ala Arg Gly Lys
   580 585 590
   Gly Ala Gly Tyr Leu Asn Arg Thr Gly Gln Arg Val Pro Phe Leu Ser
   595 600 605
   Ala Ala Lys Ile Gly Arg Asp Tyr Ser Phe Phe Thr Asn Ile Glu Thr
   610 615 620
   Asp Gly Gly Leu Leu Ala Ser Leu Asp Ser Val Glu Lys Thr Ala Gly
   625 630 635 640
   Ser Glu Gly Asp Thr Leu Ser Tyr Tyr Val Arg Arg Gly Asn Ala Ala
   645 650 655
   Arg Thr Ala Ser Ala Ala Ala His Ser Ala Pro Ala Gly Leu Lys His
   660 665 670
   Ala Val Glu Gln Gly Gly Ser Asn Leu Glu Asn Leu Met Val Glu Leu
   675 680 685
   Asp Ala Ser Glu Ser Ser Ala Thr Pro Glu Thr Val Glu Thr Ala Ala
   690 695 700
   Ala Asp Arg Thr Asp Met Pro Gly Ile Arg Pro Tyr Gly Ala Thr Phe
   705 710 715 720
   Arg Ala Ala Ala Ala Val Gln His Ala Asn Ala Ala Asp Gly Val Arg
   725 730 735
   Ile Phe Asn Ser Leu Ala Ala Thr Val Tyr Ala Asp Ser Thr Ala Ala
   740 745 750
   His Ala Asp Met Gln Gly Arg Arg Leu Lys Ala Val Ser Asp Gly Leu
   755 760 765
   Asp His Asn Ala Thr Gly Leu Arg Val Ile Ala Gln Thr Gln Gln Asp
   770 775 780
   Gly Gly Thr Trp Glu Gln Gly Gly Val Glu Gly Lys Met Arg Gly Ser
   785 790 795 800
   Thr Gln Thr Val Gly Ile Ala Ala Lys Thr Gly Glu Asn Thr Thr Ala
   805 810 815
   Ala Ala Thr Leu Gly Met Gly His Ser Thr Trp Ser Glu Asn Ser Ala
   820 825 830
   Asn Ala Lys Thr Asp Ser Ile Ser Leu Phe Ala Gly Ile Arg His Asp
   835 840 845
   Ala Gly Asp Ile Gly Tyr Leu Lys Gly Leu Phe Ser Tyr Gly Arg Tyr
   850 855 860
   Lys Asn Ser Ile Ser Arg Ser Thr Gly Ala Asp Glu His Ala Glu Gly
   865 870 875 880
   Ser Val Asn Gly Thr Leu Met Gln Leu Gly Ala Leu Gly Gly Val Asn
   885 890 895
   Val Pro Phe Ala Ala Thr Gly Asp Leu Thr Val Glu Gly Gly Leu Arg
   900 905 910
   Tyr Asp Leu Leu Lys Gln Asp Ala Phe Ala Glu Lys Gly Ser Ala Leu
   915 920 925
   Gly Trp Ser Gly Asn Ser Leu Thr Glu Gly Thr Leu Val Gly Leu Ala
   930 935 940
   Gly Leu Lys Leu Ser Gln Pro Leu Ser Asp Lys Ala Val Leu Phe Ala
   945 950 955 960
   Thr Ala Gly Val Glu Arg Asp Leu Asn Gly Arg Asp Tyr Thr Val Thr
   965 970 975
   Gly Gly Phe Thr Gly Ala Thr Ala Ala Thr Gly Lys Thr Gly Ala Arg
   980 985 990
   Asn Met Pro His Thr Arg Leu Val Ala Gly Leu Gly Ala Asp Val Glu
   995 1000 1005
   Phe Gly Asn Gly Trp Asn Gly Leu Ala Arg Tyr Ser Tyr Ala Gly Ser
   1010 1015 1020
   Lys Gln Tyr Gly Asn His Ser Gly Arg Val Gly Val Gly Tyr Arg Phe
   1025 1030 1035 1040
<210> 54
   <211> 858
   <212> ADN
   <213> Neisseria gonorrhoeae
<220>
   <221> CDS
   <222> (1)..(855)
<400> 54
   atg tct gaa gaa aaa ttg aaa atg agt ttc gag cca acc gta atc gaa 48
   Met Ser Glu Glu Lys Leu Lys Met Ser Phe Glu Pro Thr Val Ile Glu
   1 5 10 15
   cat ttg ggt gta aag atg tat tcg cac act gtt cct gcg att gcc gag 96
   His Leu Gly Val Lys Met Tyr Ser His Thr Val Pro Ala Ile Ala Glu
   20 25 30
   ttg ata gcg aat gcc tac gat gca tgt gct acg gaa gtg gaa gtt agg 144
   Leu Ile Ala Asn Ala Tyr Asp Ala Cys Ala Thr Glu Val Glu Val Arg
   35 40 45
   tta ttc gat aaa ccg gag cat aaa atc gtt att aaa gat aat ggc ata 192
   Leu Phe Asp Lys Pro Glu His Lys Ile Val Ile Lys Asp Asn Gly Ile
   50 55 60
   gga atg agc ttc gat gaa atc aat gat ttt tat ttg aga atc ggt cgg    240
   Gly Met Ser Phe Asp Glu Ile Asn Asp Phe Tyr Leu Arg Ile Gly Arg
    65                  70                  75                  80
   aac aga agg gaa gaa aaa caa gcc tcc ccg tgc gga aga att cca acg    288
   Asn Arg Arg Glu Glu Lys Gln Ala Ser Pro Cys Gly Arg Ile Pro Thr
                    85                  90                  95
   ggt aaa aaa ggt ctt ggt aaa ttg gca tta ttc agg ctt ggc aac aaa    336
   Gly Lys Lys Gly Leu Gly Lys Leu Ala Leu Phe Arg Leu Gly Asn Lys
               100                 105                 110
   atc gaa atc tct act atc caa gga aac gaa cgg gtt act ttt act ttg    384
   Ile Glu Ile Ser Thr Ile Gln Gly Asn Glu Arg Val Thr Phe Thr Leu
           115                 120                 125
   gat tat gca gag att aaa aaa agt gag cgt att tat caa ccg gag ttt    432
   Asp Tyr Ala Glu Ile Lys Lys Ser Glu Arg Ile Tyr Gln Pro Glu Phe
       130                 135                 140
   cag aaa gag tct gtt aaa ccc aat acc gaa aac gga aca act ata act    480
   Gln Lys Glu Ser Val Lys Pro Asn Thr Glu Asn Gly Thr Thr Ile Thr
   145                 150                 155                 160
   tta acc gag ctg acg aaa aaa caa gga tac ccg tta gat aat tat gtg    528
   Leu Thr Glu Leu Thr Lys Lys Gln Gly Tyr Pro Leu Asp Asn Tyr Val
                   165                 170                 175
   ggg cat ctt tcc cgt tta ttt gat ttt ccg gct cag gat ttt aaa atc    576
   Gly His Leu Ser Arg Leu Phe Asp Phe Pro Ala Gln Asp Phe Lys Ile
               180                 185                 190
   aaa gta agc ttg aac ggc tcg gaa cca aga atc att gac gga aac cta    624
   Lys Val Ser Leu Asn Gly Ser Glu Pro Arg Ile Ile Asp Gly Asn Leu
           195                 200                 205
   aaa tat aat ctt gtt acc cca caa ttc gaa tgg gaa tac cag gat cta    672
   Lys Tyr Asn Leu Val Thr Pro Gln Phe Glu Trp Glu Tyr Gln Asp Leu
       210                 215                 220
   gca acc aat att tca tcg tta tct tca aaa ttc gaa cag tat gaa tac    720
   Ala Thr Asn Ile Ser Ser Leu Ser Ser Lys Phe Glu Gln Tyr Glu Tyr
   225                 230                 235                 240
   agc gga tta ata caa ggt aag ttc att aca acg gaa aaa cct tta aag    768
   Ser Gly Leu Ile Gln Gly Lys Phe Ile Thr Thr Glu Lys Pro Leu Lys
                   245                 250                 255
   aat aat atg aaa ggt att acc ttg ttt gcc aac ggc aga atg gta aat    816
   Asn Asn Met Lys Gly Ile Thr Leu Phe Ala Asn Gly Arg Met Val Asn
               260                 265                 270
   atg ccc gag ttt ttc act gat agc gaa tcc agc cat ttc taa            858
   Met Pro Glu Phe Phe Thr Asp Ser Glu Ser Ser His Phe
           275                 280                 285
<210> 55
   <211> 285
   <212> PRT
   <213> Neisseria gonorrhoeae
<400> 55
   Met Ser Glu Glu Lys Leu Lys Met Ser Phe Glu Pro Thr Val Ile Glu
   1               5                   10                  15
   His Leu Gly Val Lys Met Tyr Ser His Thr Val Pro Ala Ile Ala Glu
               20                  25                  30
   Leu Ile Ala Asn Ala Tyr Asp Ala Cys Ala Thr Glu Val Glu Val Arg
           35                  40                  45
   Leu Phe Asp Lys Pro Glu His Lys Ile Val Ile Lys Asp Asn Gly Ile
       50                  55                  60
   Gly Met Ser Phe Asp Glu Ile Asn Asp Phe Tyr Leu Arg Ile Gly Arg
   65                  70                  75                  80
   Asn Arg Arg Glu Glu Lys Gln Ala Ser Pro Cys Gly Arg Ile Pro Thr
                   85                  90                  95
   Gly Lys Lys Gly Leu Gly Lys Leu Ala Leu Phe Arg Leu Gly Asn Lys
               100                 105                 110
   Ile Glu Ile Ser Thr Ile Gln Gly Asn Glu Arg Val Thr Phe Thr Leu
           115                 120                 125
   Asp Tyr Ala Glu Ile Lys Lys Ser Glu Arg Ile Tyr Gln Pro Glu Phe
       130                 135                 140
   Gln Lys Glu Ser Val Lys Pro Asn Thr Glu Asn Gly Thr Thr Ile Thr
   145                 150                 155                 160
   Leu Thr Glu Leu Thr Lys Lys Gln Gly Tyr Pro Leu Asp Asn Tyr Val
                   165                 170                 175
   Gly His Leu Ser Arg Leu Phe Asp Phe Pro Ala Gln Asp Phe Lys Ile
               180                 185                 190
   Lys Val Ser Leu Asn Gly Ser Glu Pro Arg Ile Ile Asp Gly Asn Leu
           195                 200                 205
   Lys Tyr Asn Leu Val Thr Pro Gln Phe Glu Trp Glu Tyr Gln Asp Leu
       210                 215                 220
   Ala Thr Asn Ile Ser Ser Leu Ser Ser Lys Phe Glu Gln Tyr Glu Tyr
   225                 230                 235                 240
   Ser Gly Leu Ile Gln Gly Lys Phe Ile Thr Thr Glu Lys Pro Leu Lys
                   245                 250                 255
   Asn Asn Met Lys Gly Ile Thr Leu Phe Ala Asn Gly Arg Met Val Asn
               260                 265                 270
   Met Pro Glu Phe Phe Thr Asp Ser Glu Ser Ser His Phe
           275                 280                 285
<210> 56
   <211> 1575
   <212> ADN
   <213> Neisseria gonorrhoeae
<220>
   <221> CDS
   <222> (1)..(1572)
<400> 56
   atg aaa aaa tcc ctt ttc gtt ctc ttt ctg tat tca tcc cta ctt acc      48
   Met Lys Lys Ser Leu Phe Val Leu Phe Leu Tyr Ser Ser Leu Leu Thr
    1               5                  10                  15
   gcc agc gaa atc gcc tat cgc ttt gta ttc gga att gaa acc tta ccg      96
   Ala Ser Glu Ile Ala Tyr Arg Phe Val Phe Gly Ile Glu Thr Leu Pro
               20                  25                  30
   gct gca aaa atg gcg gaa acg ttt gcg ctg aca ttt atg att gct gcg     144
   Ala Ala Lys Met Ala Glu Thr Phe Ala Leu Thr Phe Met Ile Ala Ala
           35                  40                  45
   ctg tat ctg ttt gcg cgt tat aag gct tcg cgg ctg ctg att gcg gtg     192
   Leu Tyr Leu Phe Ala Arg Tyr Lys Ala Ser Arg Leu Leu Ile Ala Val
       50                  55                  60
   ttt ttc gcg ttc agc atg att gcc aac aat gtg cat tac gcg gtt tat     240
   Phe Phe Ala Phe Ser Met Ile Ala Asn Asn Val His Tyr Ala Val Tyr
   65                  70                  75                  80
   caa agc tgg atg acg ggt att aac tat tgg ctg atg ctg aaa gag gtt     288
   Gln Ser Trp Met Thr Gly Ile Asn Tyr Trp Leu Met Leu Lys Glu Val
                   85                  90                  95
   acc gaa gtc ggc agc gcg ggc gcg tcg atg ttg gat aag ttg tgg ctg     336
   Thr Glu Val Gly Ser Ala Gly Ala Ser Met Leu Asp Lys Leu Trp Leu
               100                 105                 110
   cct gct ttg tgg ggc gtg gcg gaa gtc atg ttg ttt tgc agc ctt gcc     384
   Pro Ala Leu Trp Gly Val Ala Glu Val Met Leu Phe Cys Ser Leu Ala
           115                 120                 125
   aag ttc cgc cgt aag acg cat ttt tct gcc gat ata ctg ttt gcc ttc     432
   Lys Phe Arg Arg Lys Thr His Phe Ser Ala Asp Ile Leu Phe Ala Phe
       130                 135                 140
   cta atg ctg atg att ttc gtg cgt tcg ttc gac acg aaa caa gag cac     480
   Leu Met Leu Met Ile Phe Val Arg Ser Phe Asp Thr Lys Gln Glu His
   145                 150                 155                 160
   ggt att tcg ccc aaa ccg aca tac agc cgc atc aaa gcc aat tat ttc     528
   Gly Ile Ser Pro Lys Pro Thr Tyr Ser Arg Ile Lys Ala Asn Tyr Phe
                   165                 170                 175
   agc ttc ggt tat ttt gtc ggg cgc gtg ttg ccg tat cag ttg ttt gat     576
   Ser Phe Gly Tyr Phe Val Gly Arg Val Leu Pro Tyr Gln Leu Phe Asp
               180                 185                 190
   tta agc aag atc cct gtg ttc aaa cag cct gct cca agc aaa atc ggg     624
   Leu Ser Lys Ile Pro Val Phe Lys Gln Pro Ala Pro Ser Lys Ile Gly
           195                 200                 205
   caa ggc agt att caa aat atc gtc ctg att atg ggc gaa agc gaa agc      672
   Gln Gly Ser Ile Gln Asn Ile Val Leu Ile Met Gly Glu Ser Glu Ser
       210                 215                 220
   gcg gcg cat ttg aaa ttg ttt ggt tac ggg cgc gaa act tcg ccg ttt      720
   Ala Ala His Leu Lys Leu Phe Gly Tyr Gly Arg Glu Thr Ser Pro Phe
   225                 230                 235                 240
   tta acc cgg ctg tcg caa gcc gat ttt aag ccg att gtg aaa caa agt      768
   Leu Thr Arg Leu Ser Gln Ala Asp Phe Lys Pro Ile Val Lys Gln Ser
                   245                 250                 255
   tat tcc gca ggc ttt atg acg gca gta tcc ctg ccc agt ttc ttt aac      816
   Tyr Ser Ala Gly Phe Met Thr Ala Val Ser Leu Pro Ser Phe Phe Asn
               260                 265                 270
   gtc ata ccg cac gcc aac ggc ttg gaa caa atc agc ggc ggc gat acc      864
   Val Ile Pro His Ala Asn Gly Leu Glu Gln Ile Ser Gly Gly Asp Thr
           275                 280                 285
   aat atg ttc cgc ctc gcc aaa gag cag ggc tat gaa acg tat ttt tac      912
   Asn Met Phe Arg Leu Ala Lys Glu Gln Gly Tyr Glu Thr Tyr Phe Tyr
       290                 295                 300
   agt gcc cag gct gaa aac caa atg gca att ttg aac tta atc ggt aag      960
   Ser Ala Gln Ala Glu Asn Gln Met Ala Ile Leu Asn Leu Ile Gly Lys
   305                 310                 315                 320
   aaa tgg ata gac cat ctg att cag ccg acg caa ctt ggc tac ggc aac     1008
   Lys Trp Ile Asp His Leu Ile Gln Pro Thr Gln Leu Gly Tyr Gly Asn
                   325                 330                 335
   ggc gac aat atg ccc gat gag aag ctg ctg ccg ttg ttc gac aaa atc     1056
   Gly Asp Asn Met Pro Asp Glu Lys Leu Leu Pro Leu Phe Asp Lys Ile
               340                 345                 350
   aat ttg cag cag ggc agg cat ttt atc gtg ttg cac caa cgc ggt tcg     1104
   Asn Leu Gln Gln Gly Arg His Phe Ile Val Leu His Gln Arg Gly Ser
           355                 360                 365
   cac gcc cca tac ggc gca ttg ttg cag cct caa gat aaa gta ttc ggc     1152
   His Ala Pro Tyr Gly Ala Leu Leu Gln Pro Gln Asp Lys Val Phe Gly
       370                 375                 380
   gaa gcc gat att gtg gat aag tac gac aac acc atc cac aaa acc gac     1200
   Glu Ala Asp Ile Val Asp Lys Tyr Asp Asn Thr Ile His Lys Thr Asp
   385                 390                 395                 400
   caa atg att caa acc gta ttc gag cag ctg caa aag cag cct gac ggc     1248
   Gln Met Ile Gln Thr Val Phe Glu Gln Leu Gln Lys Gln Pro Asp Gly
                   405                 410                 415
   aac tgg ctg ttt gcc tat acc tcc gat cat ggc cag tat gtg cgc caa     1296
   Asn Trp Leu Phe Ala Tyr Thr Ser Asp His Gly Gln Tyr Val Arg Gln
               420                 425                 430
   gat atc tac aat caa ggc acg gtg cag ccc gac agc tat att gtg cct     1344
   Asp Ile Tyr Asn Gln Gly Thr Val Gln Pro Asp Ser Tyr Ile Val Pro
   435 440 445
   ctg gtt ttg tac agc ccg gat aag gcc gtg caa cag gct gcc aac cag 1392
   Leu Val Leu Tyr Ser Pro Asp Lys Ala Val Gln Gln Ala Ala Asn Gln
   450 455 460
   gct ttt gcg cct tgc gag att gcc ttc cat cag cag ctt tca acg ttc 1440
   Ala Phe Ala Pro Cys Glu Ile Ala Phe His Gln Gln Leu Ser Thr Phe
   465 470 475 480
   ctg att cac acg ttg ggc tac gat atg ccg gtt tca ggt tgt cgc gaa 1488
   Leu Ile His Thr Leu Gly Tyr Asp Met Pro Val Ser Gly Cys Arg Glu
   485 490 495
   ggc tcg gta aca ggc aac ctg att acg ggc gat gca ggc agc ttg aac 1536
   Gly Ser Val Thr Gly Asn Leu Ile Thr Gly Asp Ala Gly Ser Leu Asn
   500 505 510
   att cgc aac ggc aag gcg gaa tat gtt tat ccg caa taa 1575
   Ile Arg Asn Gly Lys Ala Glu Tyr Val Tyr Pro Gln
   515 520
<210> 57
   <211> 524
   <212> PRT
   <213> Neisseria gonorrhoeae
<400> 57
   Met Lys Lys Ser Leu Phe Val Leu Phe Leu Tyr Ser Ser Leu Leu Thr
    1               5                  10                  15
   Ala Ser Glu Ile Ala Tyr Arg Phe Val Phe Gly Ile Glu Thr Leu Pro
               20                  25                  30
   Ala Ala Lys Met Ala Glu Thr Phe Ala Leu Thr Phe Met Ile Ala Ala
           35                  40                  45
   Leu Tyr Leu Phe Ala Arg Tyr Lys Ala Ser Arg Leu Leu Ile Ala Val
       50                  55                  60
   Phe Phe Ala Phe Ser Met Ile Ala Asn Asn Val His Tyr Ala Val Tyr
   65                  70                  75                  80
   Gln Ser Trp Met Thr Gly Ile Asn Tyr Trp Leu Met Leu Lys Glu Val
                   85                  90                  95
   Thr Glu Val Gly Ser Ala Gly Ala Ser Met Leu Asp Lys Leu Trp Leu
               100                 105                 110
   Pro Ala Leu Trp Gly Val Ala Glu Val Met Leu Phe Cys Ser Leu Ala
           115                 120                 125
   Lys Phe Arg Arg Lys Thr His Phe Ser Ala Asp Ile Leu Phe Ala Phe
       130                 135                 140
   Leu Met Leu Met Ile Phe Val Arg Ser Phe Asp Thr Lys Gln Glu His
   145                 150                 155                 160
   Gly Ile Ser Pro Lys Pro Thr Tyr Ser Arg Ile Lys Ala Asn Tyr Phe
   165 170 175
   Ser Phe Gly Tyr Phe Val Gly Arg Val Leu Pro Tyr Gln Leu Phe Asp
   180 185 190
   Leu Ser Lys Ile Pro Val Phe Lys Gln Pro Ala Pro Ser Lys Ile Gly
   195 200 205
   Gln Gly Ser Ile Gln Asn Ile Val Leu Ile Met Gly Glu Ser Glu Ser
   210 215 220
   Ala Ala His Leu Lys Leu Phe Gly Tyr Gly Arg Glu Thr Ser Pro Phe
   225 230 235 240
   Leu Thr Arg Leu Ser Gln Ala Asp Phe Lys Pro Ile Val Lys Gln Ser
   245 250 255
   Tyr Ser Ala Gly Phe Met Thr Ala Val Ser Leu Pro Ser Phe Phe Asn
   260 265 270
   Val Ile Pro His Ala Asn Gly Leu Glu Gln Ile Ser Gly Gly Asp Thr
   275 280 285
   Asn Met Phe Arg Leu Ala Lys Glu Gln Gly Tyr Glu Thr Tyr Phe Tyr
   290 295 300
   Ser Ala Gln Ala Glu Asn Gln Met Ala Ile Leu Asn Leu Ile Gly Lys
   305 310 315 320
   Lys Trp Ile Asp His Leu Ile Gln Pro Thr Gln Leu Gly Tyr Gly Asn
   325 330 335
   Gly Asp Asn Met Pro Asp Glu Lys Leu Leu Pro Leu Phe Asp Lys Ile
   340 345 350
   Asn Leu Gln Gln Gly Arg His Phe Ile Val Leu His Gln Arg Gly Ser
   355 360 365
   His Ala Pro Tyr Gly Ala Leu Leu Gln Pro Gln Asp Lys Val Phe Gly
   370 375 380
   Glu Ala Asp Ile Val Asp Lys Tyr Asp Asn Thr Ile His Lys Thr Asp
   385 390 395 400
   Gln Met Ile Gln Thr Val Phe Glu Gln Leu Gln Lys Gln Pro Asp Gly
   405 410 415
   Asn Trp Leu Phe Ala Tyr Thr Ser Asp His Gly Gln Tyr Val Arg Gln
   420 425 430
   Asp Ile Tyr Asn Gln Gly Thr Val Gln Pro Asp Ser Tyr Ile Val Pro
   435 440 445
   Leu Val Leu Tyr Ser Pro Asp Lys Ala Val Gln Gln Ala Ala Asn Gln
   450 455 460
   Ala Phe Ala Pro Cys Glu Ile Ala Phe His Gln Gln Leu Ser Thr Phe
   465 470 475 480
   Leu Ile His Thr Leu Gly Tyr Asp Met Pro Val Ser Gly Cys Arg Glu
   485 490 495
   Gly Ser Val Thr Gly Asn Leu Ile Thr Gly Asp Ala Gly Ser Leu Asn
   500 505 510
   Ile Arg Asn Gly Lys Ala Glu Tyr Val Tyr Pro Gln
   515 520
<210> 58
   <211> 1314
   <212> ADN
   <213> Neisseria gonorrhoeae
<220>
   <221> CDS
   <222> (1)..(1311)
<400> 58
   atg ctg acg ttt atc gga ttg ctg att atc ggg gtc atc gta tgg ctg    48
   Met Leu Thr Phe Ile Gly Leu Leu Ile Ile Gly Val Ile Val Trp Leu
    1               5                  10                  15
   ttg ctg acg gaa aaa gtg tcg ccc atc atc gca tta atc ttg gtg ccg    96
   Leu Leu Thr Glu Lys Val Ser Pro Ile Ile Ala Leu Ile Leu Val Pro
               20                  25                  30
   ctg att ggg gcg ttg ctg gcg ggg ttt gat gta tcc caa tta aaa gaa   144
   Leu Ile Gly Ala Leu Leu Ala Gly Phe Asp Val Ser Gln Leu Lys Glu
           35                  40                  45
   ttt tat tcg ggc ggc acg aaa tcg gtg acg cag att gtg att atg ttt   192
   Phe Tyr Ser Gly Gly Thr Lys Ser Val Thr Gln Ile Val Ile Met Phe
       50                  55                  60
   atg ttt tcc att ttg ttt ttt gga atc atg aac gat gtg ggg ctg ttc   240
   Met Phe Ser Ile Leu Phe Phe Gly Ile Met Asn Asp Val Gly Leu Phe
   65                  70                  75                  80
   cgt ccg atg ata ggc ggt ttg att aag ctg act cgg ggt aat atc gtg   288
   Arg Pro Met Ile Gly Gly Leu Ile Lys Leu Thr Arg Gly Asn Ile Val
                   85                  90                  95
   gca gtg agt gtg ggg acg gtc ttg gtg tcg gtg gtg gca cag ttg gac   336
   Ala Val Ser Val Gly Thr Val Leu Val Ser Val Val Ala Gln Leu Asp
               100                 105                 110
   ggg gcg ggc gcg acg acg ttt tta tcg gtc gtc ccc gcc ctt ttg ccg   384
   Gly Ala Gly Ala Thr Thr Phe Leu Ser Val Val Pro Ala Leu Leu Pro
           115                 120                 125
   ctt tac aag cgt ctg cat atg aat cct tac ctg ctg ttt ttg ctg ctg   432
   Leu Tyr Lys Arg Leu His Met Asn Pro Tyr Leu Leu Phe Leu Leu Leu
       130                 135                 140
   act tcc agc gcg ggg cta atc aac ctt ttg ccg cgg ggc ggg ccg atc   480
   Thr Ser Ser Ala Gly Leu Ile Asn Leu Leu Pro Arg Gly Gly Pro Ile
   145                 150                 155                 160
   ggg cgg gtt gca agc gtg ttg ggc gca gat gtg ggc gaa ttg tat aaa    528
   Gly Arg Val Ala Ser Val Leu Gly Ala Asp Val Gly Glu Leu Tyr Lys
                   165                 170                 175
   cct ttg ttg acg gtg caa att atc ggt gtg gtg ttt atc ctt gtg ctg    576
   Pro Leu Leu Thr Val Gln Ile Ile Gly Val Val Phe Ile Leu Val Leu
               180                 185                 190
   tcc ctg ttt ttg ggt gtg cgt gaa aaa agg cgg att gtc cgg gag ttg    624
   Ser Leu Phe Leu Gly Val Arg Glu Lys Arg Arg Ile Val Arg Glu Leu
           195                 200                 205
   ggc gcg ttg ccc gcc gtg gcg gat ttg ata aag ccg gcg cct ttg tcg    672
   Gly Ala Leu Pro Ala Val Ala Asp Leu Ile Lys Pro Ala Pro Leu Ser
       210                 215                 220
   gaa gaa gaa caa aaa ttg gcg cgt ccg aaa ctg ttt tgg tgg aat gtc    720
   Glu Glu Glu Gln Lys Leu Ala Arg Pro Lys Leu Phe Trp Trp Asn Val
   225                 230                 235                 240
   ctg ctg ttt ttg gcg gcg atg agc ctg ctt ttt tcg ggc atc ttc ccg    768
   Leu Leu Phe Leu Ala Ala Met Ser Leu Leu Phe Ser Gly Ile Phe Pro
                   245                 250                 255
   ccg ggt tat gta ttt atg ctg gct gca acg gcg gcg ttg ctt ttg aat    816
   Pro Gly Tyr Val Phe Met Leu Ala Ala Thr Ala Ala Leu Leu Leu Asn
               260                 265                 270
   tac cgc agc ccg cag gaa cag atg gag cgg att tat gcc cac gcc ggc    864
   Tyr Arg Ser Pro Gln Glu Gln Met Glu Arg Ile Tyr Ala His Ala Gly
           275                 280                 285
   ggc gcg gtg atg atg gcg tcc att att ttg gcg gca ggt acg ttt ttg    912
   Gly Ala Val Met Met Ala Ser Ile Ile Leu Ala Ala Gly Thr Phe Leu
       290                 295                 300
   ggg att ttg aag ggc gcg ggg atg ttg gac gcg att tcc aaa gac ctt    960
   Gly Ile Leu Lys Gly Ala Gly Met Leu Asp Ala Ile Ser Lys Asp Leu
   305                 310                 315                 320
   gtg cat atc ctg ccg gac gcg ttg ctg cct tat ctg cat att gcc atc    1008
   Val His Ile Leu Pro Asp Ala Leu Leu Pro Tyr Leu His Ile Ala Ile
                   325                 330                 335
   ggt gtg ttg ggt att ccg ctt gag ttg gtt ttg agt acg gac gct tat    1056
   Gly Val Leu Gly Ile Pro Leu Glu Leu Val Leu Ser Thr Asp Ala Tyr
               340                 345                 350
   tat ttc gga ctg ttt ccg att gtg gaa cag att acc tcg cag gcg ggc    1104
   Tyr Phe Gly Leu Phe Pro Ile Val Glu Gln Ile Thr Ser Gln Ala Gly
           355                 360                 365
   gtt gca ccc gaa gcg gca ggc tat gcg atg ttg atc ggc agt atc gtc    1152
   Val Ala Pro Glu Ala Ala Gly Tyr Ala Met Leu Ile Gly Ser Ile Val
       370                 375                 380
   ggt act ttt gtt acg ccg ctt tcg ccg gct ttg tgg atg ggt ttg ggt    1200
   Gly Thr Phe Val Thr Pro Leu Ser Pro Ala Leu Trp Met Gly Leu Gly
   385                 390                 395                 400
   ttg gcg aaa ttg tcg atg ggc aaa cac atc cgt tat tcg ttt ttc tgg    1248
   Leu Ala Lys Leu Ser Met Gly Lys His Ile Arg Tyr Ser Phe Phe Trp
                   405                     410                 415
   gcg tgg ggt ttg tcg ctg gcg ata ttg gtc agt tcg ata gcg gca gga    1296
   Ala Trp Gly Leu Ser Leu Ala Ile Leu Val Ser Ser Ile Ala Ala Gly
               420                 425                     430
   atc gtg cct ctg ccg taa                                            1314
   Ile Val Pro Leu Pro
           435
<210> 59
   <211> 437
   <212> PRT
   <213> Neisseria gonorrhoeae
<400> 59
   Met Leu Thr Phe Ile Gly Leu Leu Ile Ile Gly Val Ile Val Trp Leu
   1               5                   10                  15
   Leu Leu Thr Glu Lys Val Ser Pro Ile Ile Ala Leu Ile Leu Val Pro
               20                  25                  30
   Leu Ile Gly Ala Leu Leu Ala Gly Phe Asp Val Ser Gln Leu Lys Glu
           35                  40                  45
   Phe Tyr Ser Gly Gly Thr Lys Ser Val Thr Gln Ile Val Ile Met Phe
       50                  55                  60
   Met Phe Ser Ile Leu Phe Phe Gly Ile Met Asn Asp Val Gly Leu Phe
   65                  70                  75                  80
   Arg Pro Met Ile Gly Gly Leu Ile Lys Leu Thr Arg Gly Asn Ile Val
                   85                  90                  95
   Ala Val Ser Val Gly Thr Val Leu Val Ser Val Val Ala Gln Leu Asp
               100                 105                 110
   Gly Ala Gly Ala Thr Thr Phe Leu Ser Val Val Pro Ala Leu Leu Pro
           115                 120                 125
   Leu Tyr Lys Arg Leu His Met Asn Pro Tyr Leu Leu Phe Leu Leu Leu
       130                 135                 140
   Thr Ser Ser Ala Gly Leu Ile Asn Leu Leu Pro Arg Gly Gly Pro Ile
   145                 150                 155                 160
   Gly Arg Val Ala Ser Val Leu Gly Ala Asp Val Gly Glu Leu Tyr Lys
                   165                 170                 175
   Pro Leu Leu Thr Val Gln Ile Ile Gly Val Val Phe Ile Leu Val Leu
               180                 185                 190
   Ser Leu Phe Leu Gly Val Arg Glu Lys Arg Arg Ile Val Arg Glu Leu
           195                 200                 205
   Gly Ala Leu Pro Ala Val Ala Asp Leu Ile Lys Pro Ala Pro Leu Ser
       210                 215                 220
   Glu Glu Glu Gln Lys Leu Ala Arg Pro Lys Leu Phe Trp Trp Asn Val
   225 230 235 240
   Leu Leu Phe Leu Ala Ala Met Ser Leu Leu Phe Ser Gly Ile Phe Pro
   245 250 255
   Pro Gly Tyr Val Phe Met Leu Ala Ala Thr Ala Ala Leu Leu Leu Asn
   260 265 270
   Tyr Arg Ser Pro Gln Glu Gln Met Glu Arg Ile Tyr Ala His Ala Gly
   275 280 285
   Gly Ala Val Met Met Ala Ser Ile Ile Leu Ala Ala Gly Thr Phe Leu
   290 295 300
   Gly Ile Leu Lys Gly Ala Gly Met Leu Asp Ala Ile Ser Lys Asp Leu
   305 310 315 320
   Val His Ile Leu Pro Asp Ala Leu Leu Pro Tyr Leu His Ile Ala Ile
   325 330 335
   Gly Val Leu Gly Ile Pro Leu Glu Leu Val Leu Ser Thr Asp Ala Tyr
   340 345 350
   Tyr Phe Gly Leu Phe Pro Ile Val Glu Gln Ile Thr Ser Gln Ala Gly
   355 360 365
   Val Ala Pro Glu Ala Ala Gly Tyr Ala Met Leu Ile Gly Ser Ile Val
   370 375 380
   Gly Thr Phe Val Thr Pro Leu Ser Pro Ala Leu Trp Met Gly Leu Gly
   385 390 395 400
   Leu Ala Lys Leu Ser Met Gly Lys His Ile Arg Tyr Ser Phe Phe Trp
   405 410 415
   Ala Trp Gly Leu Ser Leu Ala Ile Leu Val Ser Ser Ile Ala Ala Gly
   420 425 430
   Ile Val Pro Leu Pro
   435
<210> 60
   <211> 1155
   <212> ADN
   <213> Neisseria gonorrhoeae
<220>
   <221> CDS
   <222> (1)..(1152)
<400> 60
   atg ggc atc cat ctc gac ttc ggc att agt cct aaa acg ttc cga cag    48
   Met Gly Ile His Leu Asp Phe Gly Ile Ser Pro Lys Thr Phe Arg Gln
     1               5                  10                  15
   act tat ctg tat caa aag ccc aag ctc ttt aaa gga gcg gtt cgg aat    96
   Thr Tyr Leu Tyr Gln Lys Pro Lys Leu Phe Lys Gly Ala Val Arg Asn
               20                  25                  30
   ctc gaa gcc gca tct tgt aaa tat atc aac gag ata tac caa cga gca     144
   Leu Glu Ala Ala Ser Cys Lys Tyr Ile Asn Glu Ile Tyr Gln Arg Ala
           35                  40                  45
   gac cca acc gca ccg ctg ttt cat ctg cgt aaa aaa ggc gca atc gtt     192
   Asp Pro Thr Ala Pro Leu Phe His Leu Arg Lys Lys Gly Ala Ile Val
       50                  55                  60
   cct aaa gaa gaa tac gtc gaa agt ttc gac gat ttg ggc aaa act cgc     240
   Pro Lys Glu Glu Tyr Val Glu Ser Phe Asp Asp Leu Gly Lys Thr Arg
   65                  70                  75                  80
   tac cgt ttt att aaa tcc gtt atc tac gaa cat atg aag aat ggt gcg     288
   Tyr Arg Phe Ile Lys Ser Val Ile Tyr Glu His Met Lys Asn Gly Ala
                   85                  90                  95
   tcg tta gtc tat aac cat att aac aac gag ccg ttt tca gac cat atc     336
   Ser Leu Val Tyr Asn His Ile Asn Asn Glu Pro Phe Ser Asp His Ile
               100                 105                 110
   gcc cgt caa gtc gcc cgc ttt gcc ggc gca cat act att gtt agt gga     384
   Ala Arg Gln Val Ala Arg Phe Ala Gly Ala His Thr Ile Val Ser Gly
           115                 120                 125
   tat ctt gct ttt ggc agc gac gaa tct tat aaa aac cat tgg gat acc     432
   Tyr Leu Ala Phe Gly Ser Asp Glu Ser Tyr Lys Asn His Trp Asp Thr
       130                 135                 140
   cgc gat gtg tat gcc atc cag ctt ttc ggc aag aaa cgt tgg caa ctt     480
   Arg Asp Val Tyr Ala Ile Gln Leu Phe Gly Lys Lys Arg Trp Gln Leu
   145                 150                 155                 160
   act gcc cct gat ttc cct atg cca ttg tat atg caa cag act aaa gat     528
   Thr Ala Pro Asp Phe Pro Met Pro Leu Tyr Met Gln Gln Thr Lys Asp
                   165                 170                 175
   act gat att tcc att cct gaa cat atc gat atg gat att atc ctt gaa     576
   Thr Asp Ile Ser Ile Pro Glu His Ile Asp Met Asp Ile Ile Leu Glu
               180                 185                 190
   gca ggt gat gtc ctc tac atc cca cgc ggt tgg tgg cac aga cct atc     624
   Ala Gly Asp Val Leu Tyr Ile Pro Arg Gly Trp Trp His Arg Pro Ile
           195                 200                 205
   ccg ctc ggc tgt gaa acc ttc cac ttc gct gtc ggt acc ttc cca cca     672
   Pro Leu Gly Cys Glu Thr Phe His Phe Ala Val Gly Thr Phe Pro Pro
       210                 215                 220
   aac ggc tat aat tac ctc gag tgg cta atg aag aaa ttt ccc acc ata     720
   Asn Gly Tyr Asn Tyr Leu Glu Trp Leu Met Lys Lys Phe Pro Thr Ile
   225                 230                 235                 240
   gaa agt ctg cgc cac agt ttc tca gac tgg gag caa gat agg acg cgt     768
   Glu Ser Leu Arg His Ser Phe Ser Asp Trp Glu Gln Asp Arg Thr Arg
                   245                 250                 255
   atc aac gat act gcc gca caa att gct gcc atg att gcc gac ccc gtc     816
   Ile Asn Asp Thr Ala Ala Gln Ile Ala Ala Met Ile Ala Asp Pro Val
               260                 265                 270
   aat tat gaa gcc ttc agt gaa gac ttt ctc ggc aaa gaa cgt acc gat    864
   Asn Tyr Glu Ala Phe Ser Glu Asp Phe Leu Gly Lys Glu Arg Thr Asp
           275                 280                 285
   acc gct ttt cat ctc gaa cag ttc gcg aat ccc aac gct act ccg ctt    912
   Thr Ala Phe His Leu Glu Gln Phe Ala Asn Pro Asn Ala Thr Pro Leu
       290                 295                 300
   tca gac gac gtc agg ttg aga tta aat gcc aat aat ttg gat acg ttg    960
   Ser Asp Asp Val Arg Leu Arg Leu Asn Ala Asn Asn Leu Asp Thr Leu
   305                 310                 315                 320
   gaa aag gga tat ttg att ggg aat ggg atg aag ata agc gta gat gag    1008
   Glu Lys Gly Tyr Leu Ile Gly Asn Gly Met Lys Ile Ser Val Asp Glu
                   325                 330                 335
   ttg ggg aaa aaa gtg tta gaa cac atc ggt aag aat gaa ccg tta ttg    1056
   Leu Gly Lys Lys Val Leu Glu His Ile Gly Lys Asn Glu Pro Leu Leu
               340                 345                 350
   ttg aaa aat cta ctg gtt aac ttc aat cag gca aaa cat gaa gaa gtt    1104
   Leu Lys Asn Leu Leu Val Asn Phe Asn Gln Ala Lys His Glu Glu Val
           355                 360                 365
   agg aag ttg atc tat cag ttg ata gag tta gat ttt ctg gaa att ttg    1152
   Arg Lys Leu Ile Tyr Gln Leu Ile Glu Leu Asp Phe Leu Glu Ile Leu
       370                 375                 380
   tga                                                                1155
<210> 61
   <211> 384
   <212> PRT
   <213> Neisseria gonorrhoeae
<400> 61
   Met Gly Ile His Leu Asp Phe Gly Ile Ser Pro Lys Thr Phe Arg Gln
   1 5 10 15
   Thr Tyr Leu Tyr Gln Lys Pro Lys Leu Phe Lys Gly Ala Val Arg Asn
   20 25 30
   Leu Glu Ala Ala Ser Cys Lys Tyr Ile Asn Glu Ile Tyr Gln Arg Ala
   35 40 45
   Asp Pro Thr Ala Pro Leu Phe His Leu Arg Lys Lys Gly Ala Ile Val
   50 55 60
   Pro Lys Glu Glu Tyr Val Glu Ser Phe Asp Asp Leu Gly Lys Thr Arg
   65 70 75 80
   Tyr Arg Phe Ile Lys Ser Val Ile Tyr Glu His Met Lys Asn Gly Ala
   85 90 95
   Ser Leu Val Tyr Asn His Ile Asn Asn Glu Pro Phe Ser Asp His Ile
   100 105 110
   Ala Arg Gln Val Ala Arg Phe Ala Gly Ala His Thr Ile Val Ser Gly
           115                 120                 125
   Tyr Leu Ala Phe Gly Ser Asp Glu Ser Tyr Lys Asn His Trp Asp Thr
       130                 135                 140
   Arg Asp Val Tyr Ala Ile Gln Leu Phe Gly Lys Lys Arg Trp Gln Leu
   145                 150                 155                 160
   Thr Ala Pro Asp Phe Pro Met Pro Leu Tyr Met Gln Gln Thr Lys Asp
                   165                 170                 175
   Thr Asp Ile Ser Ile Pro Glu His Ile Asp Met Asp Ile Ile Leu Glu
               180                 185                 190
   Ala Gly Asp Val Leu Tyr Ile Pro Arg Gly Trp Trp His Arg Pro Ile
           195                 200                 205
   Pro Leu Gly Cys Glu Thr Phe His Phe Ala Val Gly Thr Phe Pro Pro
       210                 215                 220
   Asn Gly Tyr Asn Tyr Leu Glu Trp Leu Met Lys Lys Phe Pro Thr Ile
   225                 230                 235                 240
   Glu Ser Leu Arg His Ser Phe Ser Asp Trp Glu Gln Asp Arg Thr Arg
                   245                 250                 255
   Ile Asn Asp Thr Ala Ala Gln Ile Ala Ala Met Ile Ala Asp Pro Val
               260                 265                 270
   Asn Tyr Glu Ala Phe Ser Glu Asp Phe Leu Gly Lys Glu Arg Thr Asp
           275                 280                 285
   Thr Ala Phe His Leu Glu Gln Phe Ala Asn Pro Asn Ala Thr Pro Leu
       290                 295                 300
   Ser Asp Asp Val Arg Leu Arg Leu Asn Ala Asn Asn Leu Asp Thr Leu
   305                 310                 315                 320
   Glu Lys Gly Tyr Leu Ile Gly Asn Gly Met Lys Ile Ser Val Asp Glu
                   325                 330                 335
   Leu Gly Lys Lys Val Leu Glu His Ile Gly Lys Asn Glu Pro Leu Leu
               340                 345                 350
   Leu Lys Asn Leu Leu Val Asn Phe Asn Gln Ala Lys His Glu Glu Val
           355                 360                 365
   Arg Lys Leu Ile Tyr Gln Leu Ile Glu Leu Asp Phe Leu Glu Ile Leu
       370                 375                 380
<210> 62
   <211> 717
   <212> ADN
   <213> Néisseria meningitidis
<220>
   <221> CDS
   <222> (1)..(714)
<400> 62
   atg aat aga ccc aag caa ccc ttc ttc cgt ccc gaa gtc gcc gtt gcc      48
   Met Asn Arg Pro Lys Gln Pro Phe Phe Arg Pro Glu Val Ala Val Ala
   1               5                   10                  15
   cgc caa acc agc ctg acg ggt aaa gtg att ctg aca cga ccg ttg tca      96
   Arg Gln Thr Ser Leu Thr Gly Lys Val Ile Leu Thr Arg Pro Leu Ser
               20                  25                  30
   ttt tcc cta tgg acg aca ttt gca tcg ata tct gcg tta ttg att atc      144
   Phe Ser Leu Trp Thr Thr Phe Ala Ser Ile Ser Ala Leu Leu Ile Ile
           35                  40                  45
   ctg ttt ttg ata ttt ggt aac tat acg cga aag aca aca gtg gag gga      192
   Leu Phe Leu Ile Phe Gly Asn Tyr Thr Arg Lys Thr Thr Val Glu Gly
       50                  55                  60
   caa att tta cct gca tcg ggc gta atc agg gtg tat gca ccg gat acg      240
   Gln Ile Leu Pro Ala Ser Gly Val Ile Arg Val Tyr Ala Pro Asp Thr
   65                  70                  75                  80
   ggg aca att aca gcg aaa ttc gtg gaa gat gga gaa aag gtt aag gct      288
   Gly Thr Ile Thr Ala Lys Phe Val Glu Asp Gly Glu Lys Val Lys Ala
                   85                  90                  95
   ggc gac aag cta ttt gcg ctt tcg acc tca cgt ttc ggc gca gga gat      336
   Gly Asp Lys Leu Phe Ala Leu Ser Thr Ser Arg Phe Gly Ala Gly Asp
               100                 105                 110
   agc gtg cag cag cag ttg aaa acg gag gca gtt ttg aag aaa acg ttg      384
   Ser Val Gln Gln Gln Leu Lys Thr Glu Ala Val Leu Lys Lys Thr Leu
           115                 120                 125
   gca gaa cag gaa ctg ggt cgt ctg aag ctg ata cac ggg aat gaa acg      432
   Ala Glu Gln Glu Leu Gly Arg Leu Lys Leu Ile His Gly Asn Glu Thr
       130                 135                 140
   cgc agc ctt aaa gca act gtc gaa cgt ttg gaa aac cag gaa ctc cat      480
   Arg Ser Leu Lys Ala Thr Val Glu Arg Leu Glu Asn Gln Glu Leu His
   145                 150                 155                 160
   att tcg caa cag ata gac ggt cag aaa agg cgc att aga ctt gcg gaa      528
   Ile Ser Gln Gln Ile Asp Gly Gln Lys Arg Arg Ile Arg Leu Ala Glu
                   165                 170                 175
   gaa atg ttg cag aaa tat cgt ttc cta tcc gcc aat gat gca gtg cca      576
   Glu Met Leu Gln Lys Tyr Arg Phe Leu Ser Ala Asn Asp Ala Val Pro
               180                 185                 190
   aaa caa gaa atg atg aat gtc aag gca gag ctt tta gag cag aaa gcc      624
   Lys Gln Glu Met Met Asn Val Lys Ala Glu Leu Leu Glu Gln Lys Ala
           195                 200                 205
   aaa ctt gat gcc tac cgc cga gaa gaa gtc ggg ctg ctt cag gaa atc      672
   Lys Leu Asp Ala Tyr Arg Arg Glu Glu Val Gly Leu Leu Gln Glu Ile
       210                 215                 220
   cgc acg cag aat ctg aca ttg gcc agc ctc ccc caa gcg gca tga      717
   Arg Thr Gln Asn Leu Thr Leu Ala Ser Leu Pro Gln Ala Ala
   225                 230                 235
<210> 63
   <211> 238
   <212> PRT
   <213> Neisseria meningitidis
<400> 63
   Met Asn Arg Pro Lys Gln Pro Phe Phe Arg Pro Glu Val Ala Val Ala
   1               5                   10                  15
   Arg Gln Thr Ser Leu Thr Gly Lys Val Ile Leu Thr Arg Pro Leu Ser
               20                  25                  30
   Phe Ser Leu Trp Thr Thr Phe Ala Ser Ile Ser Ala Leu Leu Ile Ile
           35                  40                  45
   Leu Phe Leu Ile Phe Gly Asn Tyr Thr Arg Lys Thr Thr Val Glu Gly
       50                  55                  60
   Gln Ile Leu Pro Ala Ser Gly Val Ile Arg Val Tyr Ala Pro Asp Thr
   65                  70                  75                  80
   Gly Thr Ile Thr Ala Lys Phe Val Glu Asp Gly Glu Lys Val Lys Ala
                   85                  90                  95
   Gly Asp Lys Leu Phe Ala Leu Ser Thr Ser Arg Phe Gly Ala Gly Asp
               100                 105                 110
   Ser Val Gln Gln Gln Leu Lys Thr Glu Ala Val Leu Lys Lys Thr Leu
           115                 120                 125
   Ala Glu Gln Glu Leu Gly Arg Leu Lys Leu Ile His Gly Asn Glu Thr
       130                 135                 140
   Arg Ser Leu Lys Ala Thr Val Glu Arg Leu Glu Asn Gln Glu Leu His
   145                 150                 155                 160
   Ile Ser Gln Gln Ile Asp Gly Gln Lys Arg Arg Ile Arg Leu Ala Glu
                   165                 170                 175
   Glu Met Leu Gln Lys Tyr Arg Phe Leu Ser Ala Asn Asp Ala Val Pro
               180                 185                 190
   Lys Gln Glu Met Met Asn Val Lys Ala Glu Leu Leu Glu Gln Lys Ala
           195                 200                 205
   Lys Leu Asp Ala Tyr Arg Arg Glu Glu Val Gly Leu Leu Gln Glu Ile
       210                 215                 220
   Arg Thr Gln Asn Leu Thr Leu Ala Ser Leu Pro Gln Ala Ala
   225                 230                 235
<210> 64
   <211> 690
   <212> ADN
   <213> Neisseria gonorrhoeae
<220>
   <221> CDS
   <222> (1) .. (687)
<400> 64
   atg atg aat gtc gag gca gag ctt tta gag cag aaa gcc aaa ctt gat      48
   Met Met Asn Val Glu Ala Glu Leu Leu Glu Gln Lys Ala Lys Leu Asp
   1               5                   10                  15
   gcc tac ggc cga gaa gaa gcc ggg ctg ctt cag gaa atc cgc acg cag      96
   Ala Tyr Gly Arg Glu Glu Ala Gly Leu Leu Gln Glu Ile Arg Thr Gln
               20                  25                  30
   aat ctg aca ttg gcc agc ctc ccc aaa cgg cat gag aca gaa caa agc      144
   Asn Leu Thr Leu Ala Ser Leu Pro Lys Arg His Glu Thr Glu Gln Ser
           35                  40                  45
   cag ctt gaa cgc acc atg gcc gat att tct caa gaa gtt ttg gat ttt      192
   Gln Leu Glu Arg Thr Met Ala Asp Ile Ser Gln Glu Val Leu Asp Phe
       50                  55                  60
   gaa atg cgc tct gaa caa atc atc cgt gca gga cgg tcg ggt tat ata      240
   Glu Met Arg Ser Glu Gln Ile Ile Arg Ala Gly Arg Ser Gly Tyr Ile
   65                  70                  75                  80
   gca ata ccg aac gtc gaa gtc gga cgg cag gtt gat cct tcc aaa ctg      288
   Ala Ile Pro Asn Val Glu Val Gly Arg Gln Val Asp Pro Ser Lys Leu
                   85                  90                  95
   ctc ttg agc att gtt ccc gaa cgt acc gag tta tat gcc cat cta tat      336
   Leu Leu Ser Ile Val Pro Glu Arg Thr Glu Leu Tyr Ala His Leu Tyr
               100                 105                 110
   atc ccc agc agt gca gca ggc ttt atc aag ccg aaa gac aag gtt gtc      384
   Ile Pro Ser Ser Ala Ala Gly Phe Ile Lys Pro Lys Asp Lys Val Val
           115                 120                 125
   cta cgt tat cag gca tat ccc tat cag aaa ttc ggg ctt gct tcc ggc      432
   Leu Arg Tyr Gln Ala Tyr Pro Tyr Gln Lys Phe Gly Leu Ala Ser Gly
       130                 135                 140
   agt gtc gta tca gtg gca aaa acg gca ctg ggc aga cag gaa ttg tcg      480
   Ser Val Val Ser Val Ala Lys Thr Ala Leu Gly Arg Gln Glu Leu Ser
   145                 150                 155                 160
   gga ttg ggc atg gta tcc tcc gat ttg gcg aag agc aac gaa cct gtt      528
   Gly Leu Gly Met Val Ser Ser Asp Leu Ala Lys Ser Asn Glu Pro Val
                   165                 170                 175
   tat ctc gtg aaa ata aaa ccc gac aaa cca acc atc act gca tac ggt      576
   Tyr Leu Val Lys Ile Lys Pro Asp Lys Pro Thr Ile Thr Ala Tyr Gly
               180                 185                 190
   gag gaa aaa ccg ctg caa atc ggc atg acg ctg gaa gca gac atc cta      624
   Glu Glu Lys Pro Leu Gln Ile Gly Met Thr Leu Glu Ala Asp Ile Leu
           195                 200                 205
   cac gag aaa cgg cgg ctg tac gaa tgg gta ttg gag ccg att tac agt    672
   His Glu Lys Arg Arg Leu Tyr Glu Trp Val Leu Glu Pro Ile Tyr Ser
       210                 215                 220
   atg tcg ggc agg ttg taa                                            690
   Met Ser Gly Arg Leu
   225
<210> 65
   <211> 229
   <212> PRT
   <213> Neisseria gonorrhoeae
<400> 65
   Met Met Asn Val Glu Ala Glu Leu Leu Glu Gln Lys Ala Lys Leu Asp
   1               5                   10                  15
   Ala Tyr Gly Arg Glu Glu Ala Gly Leu Leu Gln Glu Ile Arg Thr Gln
               20                  25                  30
   Asn Leu Thr Leu Ala Ser Leu Pro Lys Arg His Glu Thr Glu Gln Ser
           35                  40                  45
   Gln Leu Glu Arg Thr Met Ala Asp Ile Ser Gln Glu Val Leu Asp Phe
       50                  55                  60
   Glu Met Arg Ser Glu Gln Ile Ile Arg Ala Gly Arg Ser Gly Tyr Ile
   65                  70                  75                  80
   Ala Ile Pro Asn Val Glu Val Gly Arg Gln Val Asp Pro Ser Lys Leu
                   85                  90                  95
   Leu Leu Ser Ile Val Pro Glu Arg Thr Glu Leu Tyr Ala His Leu Tyr
               100                 105                 110
   Ile Pro Ser Ser Ala Ala Gly Phe Ile Lys Pro Lys Asp Lys Val Val
           115                 120                 125
   Leu Arg Tyr Gln Ala Tyr Pro Tyr Gln Lys Phe Gly Leu Ala Ser Gly
       130                 135                 140
   Ser Val Val Ser Val Ala Lys Thr Ala Leu Gly Arg Gln Glu Leu Ser
   145                 150                 155                 160
   Gly Leu Gly Met Val Ser Ser Asp Leu Ala Lys Ser Asn Glu Pro Val
                   165                 170                 175
   Tyr Leu Val Lys Ile Lys Pro Asp Lys Pro Thr Ile Thr Ala Tyr Gly
               180                 185                 190
   Glu Glu Lys Pro Leu Gln Ile Gly Met Thr Leu Glu Ala Asp Ile Leu
           195                 200                 205
   His Glu Lys Arg Arg Leu Tyr Glu Trp Val Leu Glu Pro Ile Tyr Ser
       210                 215                 220
   Met Ser Gly Arg Leu
   225
<210> 66
   <211> 924
   <212> ADN
   <213> Neisseria gonorrhoeae
<220>
   <221> CDS
   <222> (1) .. (921)
<400> 66
   atg caa tac agc aca ctg gca gga caa acc gac aac tcc ctc gtt tcc      48
   Met Gln Tyr Ser Thr Leu Ala Gly Gln Thr Asp Asn Ser Leu Val Ser
   1               5                   10                  15
   aat aat ttc ggg ttt ttg cgc ctg ccg ctt aat ttt atg ccg tat gaa      96
   Asn Asn Phe Gly Phe Leu Arg Leu Pro Leu Asn Phe Met Pro Tyr Glu
               20                  25                  30
   agc cat gcc gat tgg gtt att acc ggc gtg cct tat gat atg gcg gtt      144
   Ser His Ala Asp Trp Val Ile Thr Gly Val Pro Tyr Asp Met Ala Val
           35                  40                  45
   tca ggg cgt tcc ggc gcg cgt ttc ggt cct gaa gcc atc cgg cgc gcc      192
   Ser Gly Arg Ser Gly Ala Arg Phe Gly Pro Glu Ala Ile Arg Arg Ala
       50                  55                  60
   tcc gtc aac ctc gct tgg gag cac cgc agg ttt ccg tgg aca ttt gat      240
   Ser Val Asn Leu Ala Trp Glu His Arg Arg Phe Pro Trp Thr Phe Asp
   65                  70                  75                  80
   gtg cgc gaa cgc ctg aac att att gat tgc ggc gac ttg gtt ttt tct      288
   Val Arg Glu Arg Leu Asn Ile Ile Asp Cys Gly Asp Leu Val Phe Ser
                   85                  90                  95
   ttt ggc gac agc agg gat ttt gtc gaa aaa atg gaa gcg cac gcc ggc      336
   Phe Gly Asp Ser Arg Asp Phe Val Glu Lys Met Glu Ala His Ala Gly
               100                 105                 110
   aaa tta ctt tct ttc ggc aaa cgc tgt ttg agt ttg ggc ggc gac cat      384
   Lys Leu Leu Ser Phe Gly Lys Arg Cys Leu Ser Leu Gly Gly Asp His
           115                 120                 125
   ttc att acc ctc ccg ttg ttg cgc gcc cac gcc cgc tat ttc ggc aaa      432
   Phe Ile Thr Leu Pro Leu Leu Arg Ala His Ala Arg Tyr Phe Gly Lys
       130                 135                 140
   ctc gca ctg att cat ttt gac gcg cac acc gac acc tac gac aac ggc      480
   Leu Ala Leu Ile His Phe Asp Ala His Thr Asp Thr Tyr Asp Asn Gly
   145                 150                 155                 160
   agc gaa tac gac cac ggc acg atg ttt tat acc gcc ccc aag gaa ggc      528
   Ser Glu Tyr Asp His Gly Thr Met Phe Tyr Thr Ala Pro Lys Glu Gly
                   165                 170                 175
   ctc atc gac ccg tcc cgt tcc gta caa atc ggc ata cgc acc gaa cac      576
   Leu Ile Asp Pro Ser Arg Ser Val Gln Ile Gly Ile Arg Thr Glu His
               180                 185                 190
   agt aaa aaa ttg cct ttt act gtg ttg tcc gcc ccc aaa gtc aat gaa    624
   Ser Lys Lys Leu Pro Phe Thr Val Leu Ser Ala Pro Lys Val Asn Glu
           195                 200                 205
   gac agt gtt gaa gag acc gtc cgt aaa atc aaa gaa acc gtc ggc aat    672
   Asp Ser Val Glu Glu Thr Val Arg Lys Ile Lys Glu Thr Val Gly Asn
       210                 215                 220
   atg ccc gtt tac ctg act ttc gac ata gac tgt ctc gac ccg tcg ttc    720
   Met Pro Val Tyr Leu Thr Phe Asp Ile Asp Cys Leu Asp Pro Ser Phe
   225                 230                 235                 240
   gcc ccc ggg acc ggt acg ccc gta tgc ggc ggc ttg agc agc gac agg    768
   Ala Pro Gly Thr Gly Thr Pro Val Cys Gly Gly Leu Ser Ser Asp Arg
                   245                 250                 255
   gca tta aaa atc cta cgt ggg ctg acg gat ctc gac atc gtc ggt atg    816
   Ala Leu Lys Ile Leu Arg Gly Leu Thr Asp Leu Asp Ile Val Gly Met
               260                 265                 270
   gat gtt gta gaa gtt gcc ccc tct tac gac caa tcc gac att acc gct    864
   Asp Val Val Glu Val Ala Pro Ser Tyr Asp Gln Ser Asp Ile Thr Ala
           275                 280                 285
   ttg gcc ggc gcc aca att gcc ttg gaa atg ctt tac ctt caa ggt gcg    912
   Leu Ala Gly Ala Thr Ile Ala Leu Glu Met Leu Tyr Leu Gln Gly Ala
       290                 295                 300
   aaa aag gac tga                                                    924
   Lys Lys Asp
   305
<210> 67
   <211> 307
   <212> PRT
   <213> Neisseria gonorrhoeae
<400> 67
   Met Gln Tyr Ser Thr Leu Ala Gly Gln Thr Asp Asn Ser Leu Val Ser
   1 5 10 15
   Asn Asn Phe Gly Phe Leu Arg Leu Pro Leu Asn Phe Met Pro Tyr Glu
   20 25 30
   Ser His Ala Asp Trp Val Ile Thr Gly Val Pro Tyr Asp Met Ala Val
   35 40 45
   Ser Gly Arg Ser Gly Ala Arg Phe Gly Pro Glu Ala Ile Arg Arg Ala
   50 55 60
   Ser Val Asn Leu Ala Trp Glu His Arg Arg Phe Pro Trp Thr Phe Asp
   65 70 75 80
   Val Arg Glu Arg Leu Asn Ile Ile Asp Cys Gly Asp Leu Val Phe Ser
   85 90 95
   Phe Gly Asp Ser Arg Asp Phe Val Glu Lys Met Glu Ala His Ala Gly
   100 105 110
   Lys Leu Leu Ser Phe Gly Lys Arg Cys Leu Ser Leu Gly Gly Asp His
   115 120 125
   Phe Ile Thr Leu Pro Leu Leu Arg Ala His Ala Arg Tyr Phe Gly Lys
   130 135 140
   Leu Ala Leu Ile His Phe Asp Ala His Thr Asp Thr Tyr Asp Asn Gly
   145 150 155 160
   Ser Glu Tyr Asp His Gly Thr Met Phe Tyr Thr Ala Pro Lys Glu Gly
   165 170 175
   Leu Ile Asp Pro Ser Arg Ser Val Gln Ile Gly Ile Arg Thr Glu His
   180 185 190
   Ser Lys Lys Leu Pro Phe Thr Val Leu Ser Ala Pro Lys Val Asn Glu
   195 200 205
   Asp Ser Val Glu Glu Thr Val Arg Lys Ile Lys Glu Thr Val Gly Asn
   210 215 220
   Met Pro Val Tyr Leu Thr Phe Asp Ile Asp Cys Leu Asp Pro Ser Phe
   225 230 235 240
   Ala Pro Gly Thr Gly Thr Pro Val Cys Gly Gly Leu Ser Ser Asp Arg
   245 250 255
   Ala Leu Lys Ile Leu Arg Gly Leu Thr Asp Leu Asp Ile Val Gly Met
   260 265 270
   Asp Val Val Glu Val Ala Pro Ser Tyr Asp Gln Ser Asp Ile Thr Ala
   275 280 285
   Leu Ala Gly Ala Thr Ile Ala Leu Glu Met Leu Tyr Leu Gln Gly Ala
   290 295 300
   Lys Lys Asp
   305
<210> 68
   <211> 1404
   <212> ADN
   <213> Neisseria meningitidis
<220>
   <221> CDS
   <222> (1) .. (1401)
<400> 68
   atg aca ttg ctc aat cta atg ata atg caa gat tac ggt att tcc gtt    48
   Met Thr Leu Leu Asn Leu Met Ile Met Gln Asp Tyr Gly Ile Ser Val
     1               5                  10                  15
   tgc ctg aca ctg acg ccc tat ttg caa cat gaa cta ttt tcg gct atg    96
   Cys Leu Thr Leu Thr Pro Tyr Leu Gln His Glu Leu Phe Ser Ala Met
               20                  25                  30
   aaa tcc tat ttt tcc aaa tat atc cta ccc gtt tca ctt ttt acc ttg   144
   Lys Ser Tyr Phe Ser Lys Tyr Ile Leu Pro Val Ser Leu Phe Thr Leu
           35                  40                  45
   cca cta tcc ctt tcc cca tcc gtt tcg gct ttt acg ctg cct gaa gca     192
   Pro Leu Ser Leu Ser Pro Ser Val Ser Ala Phe Thr Leu Pro Glu Ala
       50                  55                  60
   tgg cgg gcg gcg cag caa cat tcg gct gat ttt caa gcg tcc cat tac     240
   Trp Arg Ala Ala Gln Gln His Ser Ala Asp Phe Gln Ala Ser His Tyr
   65                  70                  75                  80
   cag cgt gat gca gtg cgc gca cgg caa caa caa gcc aag gcc gca ttc     288
   Gln Arg Asp Ala Val Arg Ala Arg Gln Gln Gln Ala Lys Ala Ala Phe
                   85                  90                  95
   ctt ccc cat gta tcc gcc aat gcc agc tac cag cgc cag ccg cca tcg     336
   Leu Pro His Val Ser Ala Asn Ala Ser Tyr Gln Arg Gln Pro Pro Ser
               100                 105                 110
   att tct tcc acc cgc gaa aca cag gga tgg agc gtg cag gtg gga caa     384
   Ile Ser Ser Thr Arg Glu Thr Gln Gly Trp Ser Val Gln Val Gly Gln
           115                 120                 125
   acc tta ttt gac gct gcc aaa ttt gca caa tac cgc caa agc agg ttc     432
   Thr Leu Phe Asp Ala Ala Lys Phe Ala Gln Tyr Arg Gln Ser Arg Phe
       130                 135                 140
   gat acg cag gct gca gaa cag cgt ttc gat gcg gca cgc gaa gaa ttg     480
   Asp Thr Gln Ala Ala Glu Gln Arg Phe Asp Ala Ala Arg Glu Glu Leu
   145                 150                 155                 160
   ctg ttg aaa gtt gcc gaa agt tat ttc aac gtt tta ctc agc cga gac     528
   Leu Leu Lys Val Ala Glu Ser Tyr Phe Asn Val Leu Leu Ser Arg Asp
                   165                 170                 175
   acc gtt gcc gcc cat gcg gcg gaa aaa gag gct tat gcc cag cag gta     576
   Thr Val Ala Ala His Ala Ala Glu Lys Glu Ala Tyr Ala Gln Gln Val
               180                 185                 190
   agg cag gcg cag gct tta ttc aat aaa ggt gct gcc acc gcg ctg gat     624
   Arg Gln Ala Gln Ala Leu Phe Asn Lys Gly Ala Ala Thr Ala Leu Asp
           195                 200                 205
   att cac gaa gcc aaa gcc ggt tac gac aat gcc ctg gcc caa gaa atc     672
   Ile His Glu Ala Lys Ala Gly Tyr Asp Asn Ala Leu Ala Gln Glu Ile
       210                 215                 220
   gcc gta ttg gct gag aaa caa acc tat gaa aac cag ttg aac gac tac     720
   Ala Val Leu Ala Glu Lys Gln Thr Tyr Glu Asn Gln Leu Asn Asp Tyr
   225                 230                 235                 240
   acc gac ctg gat agc aaa caa atc gag gcc ata gat acc gcc aac ctg     768
   Thr Asp Leu Asp Ser Lys Gln Ile Glu Ala Ile Asp Thr Ala Asn Leu
                   245                 250                 255
   ttg gca cgc tat ctg ccc aag ctg gaa cgt tac agt ctg gat gaa tgg     816
   Leu Ala Arg Tyr Leu Pro Lys Leu Glu Arg Tyr Ser Leu Asp Glu Trp
               260                 265                 270
   cag cgc att gcc tta tcc aac aat cat gaa tac cgg atg cag cag ctt     864
   Gln Arg Ile Ala Leu Ser Asn Asn His Glu Tyr Arg Met Gln Gln Leu
           275                 280                 285
   gcc ctg caa agc agc gga cag gcg ctt cgg gca gca cag aac agc cgc      912
   Ala Leu Gln Ser Ser Gly Gln Ala Leu Arg Ala Ala Gln Asn Ser Arg
       290                 295                 300
   tat ccc acc gtt tct gcc cat gtc ggc tat cag aat aac ctc tac act      960
   Tyr Pro Thr Val Ser Ala His Val Gly Tyr Gln Asn Asn Leu Tyr Thr
   305                 310                 315                 320
   tca tct gcg cag aat aat gac tac cac tat cgg ggc aaa ggg atg agc     1008
   Ser Ser Ala Gln Asn Asn Asp Tyr His Tyr Arg Gly Lys Gly Met Ser
                   325                 330                 335
   gtc ggc gta cag ttg aat ttg ccg ctt tat acc ggc gga gaa ttg tcg     1056
   Val Gly Val Gln Leu Asn Leu Pro Leu Tyr Thr Gly Gly Glu Leu Ser
               340                 345                 350
   ggc aaa atc cat gaa gcc gaa gcg caa tac ggg gcc gcc gaa gca cag     1104
   Gly Lys Ile His Glu Ala Glu Ala Gln Tyr Gly Ala Ala Glu Ala Gln
           355                 360                 365
   ctg acc gca acc gag cgg cac atc aaa ctc gcc gta cgc cag gct tat     1152
   Leu Thr Ala Thr Glu Arg His Ile Lys Leu Ala Val Arg Gln Ala Tyr
       370                 375                 380
   acc gaa agc ggt gcg gcg cgt tac caa atc atg gcg caa gaa cgg gtt     1200
   Thr Glu Ser Gly Ala Ala Arg Tyr Gln Ile Met Ala Gln Glu Arg Val
   385                 390                 395                 400
   ttg gaa agc agc cgt ttg aaa ctg aaa tcg acc gaa acc ggc caa caa     1248
   Leu Glu Ser Ser Arg Leu Lys Leu Lys Ser Thr Glu Thr Gly Gln Gln
                   405                 410                 415
   tac ggc atc cgc aac cgg ctg gaa gta ata cgg gcg cgg cag gaa gtc     1296
   Tyr Gly Ile Arg Asn Arg Leu Glu Val Ile Arg Ala Arg Gln Glu Val
               420                 425                 430
   gcc caa gca gaa cag aaa ctg gct caa gca cgg tat aaa ttc atg ctg     1344
   Ala Gln Ala Glu Gln Lys Leu Ala Gln Ala Arg Tyr Lys Phe Met Leu
           435                 440                 445
   gct tat ttg cgc ttg gtg aaa gag agc ggg tta ggg ttg gaa acg gta     1392
   Ala Tyr Leu Arg Leu Val Lys Glu Ser Gly Leu Gly Leu Glu Thr Val
       450                 455                 460
   ttt gcg gaa taa                                                     1404
   Phe Ala Glu
   465
<210 > 69
   <211> 467
   <212> PRT
   <213> Neisseria meningitidis
<400> 69
   Met Thr Leu Leu Asn Leu Met Ile Met Gln Asp Tyr Gly Ile Ser Val
   1               5                   10                  15
   Cys Leu Thr Leu Thr Pro Tyr Leu Gln His Glu Leu Phe Ser Ala Met
   20 25 30
   Lys Ser Tyr Phe Ser Lys Tyr Ile Leu Pro Val Ser Leu Phe Thr Leu
   35 40 45
   Pro Leu Ser Leu Ser Pro Ser Val Ser Ala Phe Thr Leu Pro Glu Ala
   50 55 60
   Trp Arg Ala Ala Gln Gln His Ser Ala Asp Phe Gln Ala Ser His Tyr
   65 70 75 80
   Gln Arg Asp Ala Val Arg Ala Arg Gln Gln Gln Ala Lys Ala Ala Phe
   85 90 95
   Leu Pro His Val Ser Ala Asn Ala Ser Tyr Gln Arg Gln Pro Pro Ser
   100 105 110
   Ile Ser Ser Thr Arg Glu Thr Gln Gly Trp Ser Val Gln Val Gly Gln
   115 120 125
   Thr Leu Phe Asp Ala Ala Lys Phe Ala Gln Tyr Arg Gln Ser Arg Phe
   130 135 140
   Asp Thr Gln Ala Ala Glu Gln Arg Phe Asp Ala Ala Arg Glu Glu Leu
   145 150 155 160
   Leu Leu Lys Val Ala Glu Ser Tyr Phe Asn Val Leu Leu Ser Arg Asp
   165 170 175
   Thr Val Ala Ala His Ala Ala Glu Lys Glu Ala Tyr Ala Gln Gln Val
   180 185 190
   Arg Gln Ala Gln Ala Leu Phe Asn Lys Gly Ala Ala Thr Ala Leu Asp
   195 200 205
   Ile His Glu Ala Lys Ala Gly Tyr Asp Asn Ala Leu Ala Gln Glu Ile
   210 215 220
   Ala Val Leu Ala Glu Lys Gln Thr Tyr Glu Asn Gln Leu Asn Asp Tyr
   225 230 235 240
   Thr Asp Leu Asp Ser Lys Gln Ile Glu Ala Ile Asp Thr Ala Asn Leu
   245 250 255
   Leu Ala Arg Tyr Leu Pro Lys Leu Glu Arg Tyr Ser Leu Asp Glu Trp
   260 265 270
   Gln Arg Ile Ala Leu Ser Asn Asn His Glu Tyr Arg Met Gln Gln Leu
   275 280 285
   Ala Leu Gln Ser Ser Gly Gln Ala Leu Arg Ala Ala Gln Asn Ser Arg
   290 295 300
   Tyr Pro Thr Val Ser Ala His Val Gly Tyr Gln Asn Asn Leu Tyr Thr
   305 310 315 320
   Ser Ser Ala Gln Asn Asn Asp Tyr His Tyr Arg Gly Lys Gly Met Ser
   325 330 335
   Val Gly Val Gln Leu Asn Leu Pro Leu Tyr Thr Gly Gly Glu Leu Ser
   340 345 350
   Gly Lys Ile His Glu Ala Glu Ala Gln Tyr Gly Ala Ala Glu Ala Gln
   355 360 365
   Leu Thr Ala Thr Glu Arg His Ile Lys Leu Ala Val Arg Gln Ala Tyr
   370 375 380
   Thr Glu Ser Gly Ala Ala Arg Tyr Gln Ile Met Ala Gln Glu Arg Val
   385 390 395 400
   Leu Glu Ser Ser Arg Leu Lys Leu Lys Ser Thr Glu Thr Gly Gln Gln
   405 410 415
   Tyr Gly Ile Arg Asn Arg Leu Glu Val Ile Arg Ala Arg Gln Glu Val
   420 425 430
   Ala Gln Ala Glu Gln Lys Leu Ala Gln Ala Arg Tyr Lys Phe Met Leu
   435 440 445
   Ala Tyr Leu Arg Leu Val Lys Glu Ser Gly Leu Gly Leu Glu Thr Val
   450 455 460
   Phe Ala Glu
   465
<210> 70
   <211> 696
   <212> ADN
   <213> Neisseria gonorrhoeae
<220>
   <221> CDS
   <222> (1) .. (693)
<400> 70
   atg aaa caa tcc gcc cga ata aaa aat atg gat cag aca tta aaa aat    48
   Met Lys Gln Ser Ala Arg Ile Lys Asn Met Asp Gln Thr Leu Lys Asn
    1               5                  10                  15
   aca ttg ggc att tgc gcg ctt tta gcc ttt tgt ttt ggc gcg gcc atc    96
   Thr Leu Gly Ile Cys Ala Leu Leu Ala Phe Cys Phe Gly Ala Ala Ile
               20                  25                  30
   gca tca ggt tat cac ttg gaa tat gaa tac ggc tac cgt tat tct gcc   144
   Ala Ser Gly Tyr His Leu Glu Tyr Glu Tyr Gly Tyr Arg Tyr Ser Ala
           35                  40                  45
   gtg ggc gct ttg gct tcg gtt gta ttt tta tta tta ttg gca cgc ggc   192
   Val Gly Ala Leu Ala Ser Val Val Phe Leu Leu Leu Leu Ala Arg Gly
       50                  55                  60
   ttc ccg cgc gtt tct tca gtt gtt tta ctg att tac gtc ggc aca acc   240
   Phe Pro Arg Val Ser Ser Val Val Leu Leu Ile Tyr Val Gly Thr Thr
   65                  70                  75                  80
   gcc cta tat ttg ccg gtc ggc tgg ctg tat ggt gcg cct tct tat cag    288
   Ala Leu Tyr Leu Pro Val Gly Trp Leu Tyr Gly Ala Pro Ser Tyr Gln
                    85                  90                  95
   ata gtc ggt tcg ata ttg gaa agc aat cct gcc gag gcg cgt gaa ttt    336
   Ile Val Gly Ser Ile Leu Glu Ser Asn Pro Ala Glu Ala Arg Glu Phe
               100                 105                 110
   gtc ggc aat ctt ccc ggg tcg ctt tat ttt gtg cag gca tta ttt ttc    384
   Val Gly Asn Leu Pro Gly Ser Leu Tyr Phe Val Gln Ala Leu Phe Phe
           115                 120                 125
   att ttt ggc ttg aca gtt tgg aaa tat tgt gta tct gtg ggg gta ttt    432
   Ile Phe Gly Leu Thr Val Trp Lys Tyr Cys Val Ser Val Gly Val Phe
       130                 135                 140
   gct gac gta aaa aac tat aaa cgt cgc agc aaa ata tgg ctg acc ata    480
   Ala Asp Val Lys Asn Tyr Lys Arg Arg Ser Lys Ile Trp Leu Thr Ile
   145                 150                 155                 160
   tta ttg act ttg att ttg tcc tgc gcg gtg atg gag aaa atc gcc ggc    528
   Leu Leu Thr Leu Ile Leu Ser Cys Ala Val Met Glu Lys Ile Ala Gly
                   165                 170                 175
   gat aaa gat tgg cga gaa cct gat gcc ggc ctg ttg ttg aat att ttc    576
   Asp Lys Asp Trp Arg Glu Pro Asp Ala Gly Leu Leu Leu Asn Ile Phe
               180                 185                 190
   gac ctg tat tac gac ttg gct ttc cgc gcc ggc aca ata tgc cgc caa    624
   Asp Leu Tyr Tyr Asp Leu Ala Phe Arg Ala Gly Thr Ile Cys Arg Gln
           195                 200                 205
   gcg cgc cca cat ttt gga agc agc aaa aaa agc gtc aac atg gca tat    672
   Ala Arg Pro His Phe Gly Ser Ser Lys Lys Ser Val Asn Met Ala Tyr
       210                 215                 220
   ccg cca act tgc gcc caa gta taa                                    696
   Pro Pro Thr Cys Ala Gln Val
   225                 230
<210> 71
   <211> 231
   <212> PRT
   <213> Neisseria gonorrhoeae
<400> 71
   Met Lys Gln Ser Ala Arg Ile Lys Asn Met Asp Gln Thr Leu Lys Asn
   1 5 10 15
   Thr Leu Gly Ile Cys Ala Leu Leu Ala Phe Cys Phe Gly Ala Ala Ile
   20 25 30
   Ala Ser Gly Tyr His Leu Glu Tyr Glu Tyr Gly Tyr Arg Tyr Ser Ala
   35 40 45
   Val Gly Ala Leu Ala Ser Val Val Phe Leu Leu Leu Leu Ala Arg Gly
   50 55 60
   Phe Pro Arg Val Ser Ser Val Val Leu Leu Ile Tyr Val Gly Thr Thr
   65 70 75 80
   Ala Leu Tyr Leu Pro Val Gly Trp Leu Tyr Gly Ala Pro Ser Tyr Gln
   85 90 95
   Ile Val Gly Ser Ile Leu Glu Ser Asn Pro Ala Glu Ala Arg Glu Phe
   100 105 110
   Val Gly Asn Leu Pro Gly Ser Leu Tyr Phe Val Gln Ala Leu Phe Phe
   115 120 125
   Ile Phe Gly Leu Thr Val Trp Lys Tyr Cys Val Ser Val Gly Val Phe
   130 135 140
   Ala Asp Val Lys Asn Tyr Lys Arg Arg Ser Lys Ile Trp Leu Thr Ile
   145 150 155 160
   Leu Leu Thr Leu Ile Leu Ser Cys Ala Val Met Glu Lys Ile Ala Gly
   165 170 175
   Asp Lys Asp Trp Arg Glu Pro Asp Ala Gly Leu Leu Leu Asn Ile Phe
   180 185 190
   Asp Leu Tyr Tyr Asp Leu Ala Phe Arg Ala Gly Thr Ile Cys Arg Gln
   195 200 205
   Ala Arg Pro His Phe Gly Ser Ser Lys Lys Ser Val Asn Met Ala Tyr
   210 215 220
   Pro Pro Thr Cys Ala Gln Val
   225 230
<210> 72
   <211> 2607
   < 212 > ADN
   <213> Neisseria meningitidis
<220>
   <221> CDS
   <222> (1)..(2604)
<400> 72
   atg gct gcc aac caa cgt tac cgc aaa ccg ctg ccc ggt acg gat ttg      48
   Met Ala Ala Asn Gln Arg Tyr Arg Lys Pro Leu Pro Gly Thr Asp Leu
     1               5                  10                  15
   gaa tac tac gac gcg cgt gcg gcg tgt gag ggc atc aaa ccc ggc tct      96
   Glu Tyr Tyr Asp Ala Arg Ala Ala Cys Glu Gly Ile Lys Pro Gly Ser
                20                  25                  30
   tac gac aag ctg cct tac acg agc cgc att ttg gcg gag aat ttg gtc     144
   Tyr Asp Lys Leu Pro Tyr Thr Ser Arg Ile Leu Ala Glu Asn Leu Val
            35                  40                  45
   aac cgc gcg gac aaa gtc gat ttg ccg acg ctg caa agc tgg ctg ggt     192
   Asn Arg Ala Asp Lys Val Asp Leu Pro Thr Leu Gln Ser Trp Leu Gly
        50                  55                  60
   cag ctg att gag gga aaa cag gaa atc gac ttt cct tgg tat ccg gcg     240
   Gln Leu Ile Glu Gly Lys Gln Glu Ile Asp Phe Pro Trp Tyr Pro Ala
    65                  70                  75                  80
   cgg gtg gtg tgc cac gat att ctg ggg cag acc gcg ttg gtg gat ttg     288
   Arg Val Val Cys His Asp Ile Leu Gly Gln Thr Ala Leu Val Asp Leu
                    85                  90                  95
   gca ggt ctg cgc gat gcg att gcc gaa aaa ggc ggc gat cct gcc aaa     336
   Ala Gly Leu Arg Asp Ala Ile Ala Glu Lys Gly Gly Asp Pro Ala Lys
               100                 105                 110
   gtg aat ccg gtg gtg caa acc cag ctc atc gtc gac cac tcg ctg gcg     384
   Val Asn Pro Val Val Gln Thr Gln Leu Ile Val Asp His Ser Leu Ala
           115                 120                 125
   gtg gaa tgc ggc ggc tac gac ccc gat gcg ttc cgc aaa aac cgc gaa     432
   Val Glu Cys Gly Gly Tyr Asp Pro Asp Ala Phe Arg Lys Asn Arg Glu
       130                 135                 140
   atc gaa gac aga cgt aac gaa gac cgt ttc cac ttc atc aac tgg aca     480
   Ile Glu Asp Arg Arg Asn Glu Asp Arg Phe His Phe Ile Asn Trp Thr
   145                 150                 155                 160
   aaa acc gct ttt gaa aat gtg gac gtg att ccg gcg ggc aac ggc atc     528
   Lys Thr Ala Phe Glu Asn Val Asp Val Ile Pro Ala Gly Asn Gly Ile
                   165                 170                 175
   atg cac caa atc aat cta gaa aaa atg tcg ccc gtc gtc caa gtc aaa     576
   Met His Gln Ile Asn Leu Glu Lys Met Ser Pro Val Val Gln Val Lys
               180                 185                 190
   aac ggc gtg gct ttc ccc gat acc tgc gtc ggc acg gat tcg cac acg     624
   Asn Gly Val Ala Phe Pro Asp Thr Cys Val Gly Thr Asp Ser His Thr
           195                 200                 205
   cca cac gtc gat gcg ctg ggc gtg att tcc gtg ggc gtg ggc gga ttg     672
   Pro His Val Asp Ala Leu Gly Val Ile Ser Val Gly Val Gly Gly Leu
       210                 215                 220
   gaa gcg gaa acc gta atg ctg gga cgc gcg tcc atg atg cgc ctg ccc     720
   Glu Ala Glu Thr Val Met Leu Gly Arg Ala Ser Met Met Arg Leu Pro
   225                 230                 235                 240
   gat att gtc ggc gtt gag ctg aac ggc aaa cgg aag gcg ggc att acg     768
   Asp Ile Val Gly Val Glu Leu Asn Gly Lys Arg Lys Ala Gly Ile Thr
                   245                 250                 255
   gcg acg gat att gtg ttg gca ctg acc gag ttt ctg cgc aaa gaa cgc     816
   Ala Thr Asp Ile Val Leu Ala Leu Thr Glu Phe Leu Arg Lys Glu Arg
               260                 265                 270
   gtg gtc ggg gcg ttt gtc gaa ttc ttc ggc gag ggc gcg aga agc ctg     864
   Val Val Gly Ala Phe Val Glu Phe Phe Gly Glu Gly Ala Arg Ser Leu
           275                 280                 285
   tct atc ggc gac cgc gcg acc att tcc aac atg acg ccg gag ttc ggc     912
   Ser Ile Gly Asp Arg Ala Thr Ile Ser Asn Met Thr Pro Glu Phe Gly
       290                 295                 300
   gcg act gcc gcg atg ttc gct att gat gag caa acc att gat tat ttg    960
   Ala Thr Ala Ala Met Phe Ala Ile Asp Glu Gln Thr Ile Asp Tyr Leu
   305                 310                 315                 320
   aaa ctg acc gga cgc gac gac gcg cag gtg aaa ttg gtg gaa acc tac   1008
   Lys Leu Thr Gly Arg Asp Asp Ala Gln Val Lys Leu Val Glu Thr Tyr
                   325                 330                 335
   gcc aaa acc gca ggc ttg tgg gca gat gcc ttg aaa acc gcc gtt tat   1056
   Ala Lys Thr Ala Gly Leu Trp Ala Asp Ala Leu Lys Thr Ala Val Tyr
               340                 345                 350
   ccg cgc gtt ttg aaa ttt gat ttg agc agc gta acg cgc aat atg gca   1104
   Pro Arg Val Leu Lys Phe Asp Leu Ser Ser Val Thr Arg Asn Met Ala
           355                 360                 365
   ggc ccg agc aac ccg cac gcg cgt ttt gcg acc gcc gat ttg gcc ggc   1152
   Gly Pro Ser Asn Pro His Ala Arg Phe Ala Thr Ala Asp Leu Ala Gly
       370                 375                 380
   aaa ggc ttg gct aaa cct tac gaa gag cct tca gac ggc caa atg cct   1200
   Lys Gly Leu Ala Lys Pro Tyr Glu Glu Pro Ser Asp Gly Gln Met Pro
   385                 390                 395                 400
   gac ggt gca gtg att att gcc gcg att act tcc tgt acc aat act tcc   1248
   Asp Gly Ala Val Ile Ile Ala Ala Ile Thr Ser Cys Thr Asn Thr Ser
                   405                 410                 415
   aat ccg cgc aac gtt gtc gcc gcc gcg ctg ttg gca cgc aat gcc aac   1296
   Asn Pro Arg Asn Val Val Ala Ala Ala Leu Leu Ala Arg Asn Ala Asn
               420                 425                 430
   cgc ctc ggc ttg caa cgc aaa cct tgg gtg aaa tct tcg ttt gcc ccg   1344
   Arg Leu Gly Leu Gln Arg Lys Pro Trp Val Lys Ser Ser Phe Ala Pro
           435                 440                 445
   ggt tca aaa gta gcc gaa atc tat ttg aaa gaa gca gat ctg ctg ccc   1392
   Gly Ser Lys Val Ala Glu Ile Tyr Leu Lys Glu Ala Asp Leu Leu Pro
       450                 455                 460
   gaa atg gaa aaa ctc ggc ttc ggt atc gtt gcc ttc gca tgt acc acc   1440
   Glu Met Glu Lys Leu Gly Phe Gly Ile Val Ala Phe Ala Cys Thr Thr
   465                 470                 475                 480
   tgt aac ggc atg agc ggc gcg ctg gat ccg aaa atc cag aaa gaa atc   1488
   Cys Asn Gly Met Ser Gly Ala Leu Asp Pro Lys Ile Gln Lys Glu Ile
                   485                 490                 495
   atc gac cgc gat ttg tac gcc acc gcc gta ttg tca ggc aac cgc aac   1536
   Ile Asp Arg Asp Leu Tyr Ala Thr Ala Val Leu Ser Gly Asn Arg Asn
               500                 505                 510
   ttt gac ggc cgt atc cat ccg tat gcg aaa cag gct ttc ctc gct tcg   1584
   Phe Asp Gly Arg Ile His Pro Tyr Ala Lys Gln Ala Phe Leu Ala Ser
           515                 520                 525
   cct ccg ttg gtc gtt gcc tac gcg ctg gca ggc agc atc cgt ttc gat   1632
   Pro Pro Leu Val Val Ala Tyr Ala Leu Ala Gly Ser Ile Arg Phe Asp
       530                 535                 540
   att gaa aac gac gta ctc ggc gtt gca gac ggc aaa gaa atc cgc ctg    1680
   Ile Glu Asn Asp Val Leu Gly Val Ala Asp Gly Lys Glu Ile Arg Leu
   545                 550                 555                 560
   aaa gac att tgg cct acc gat gaa gaa atc gat gcc atc gtt gcc gaa    1728
   Lys Asp Ile Trp Pro Thr Asp Glu Glu Ile Asp Ala Ile Val Ala Glu
                   565                 570                 575
   tat gtg aaa ccg cag caa ttt cgc gac gtt tat atc ccg atg ttc gac    1776
   Tyr Val Lys Pro Gln Gln Phe Arg Asp Val Tyr Ile Pro Met Phe Asp
               580                 585                 590
   acc ggc aca gcg caa aaa gca cca agc ccg ctg tac gac tgg cgt cca    1824
   Thr Gly Thr Ala Gln Lys Ala Pro Ser Pro Leu Tyr Asp Trp Arg Pro
           595                 600                 605
   atg tct acc tat atc cgc cgc cca cct tac tgg gaa ggc gca ctg gca    1872
   Met Ser Thr Tyr Ile Arg Arg Pro Pro Tyr Trp Glu Gly Ala Leu Ala
       610                 615                 620
   ggg gaa cgc aca tta agc ggt atg cgt ccg ctg gcg att ttg ccc gac    1920
   Gly Glu Arg Thr Leu Ser Gly Met Arg Pro Leu Ala Ile Leu Pro Asp
   625                 630                 635                 640
   aac atc acc acc gac cat ctc tcg cca tcc aat gcg att ttg gca agc    1968
   Asn Ile Thr Thr Asp His Leu Ser Pro Ser Asn Ala Ile Leu Ala Ser
                   645                 650                 655
   agt gcc gca ggc gaa tat ttg gca aaa atg ggt ttg cct gaa gaa gac    2016
   Ser Ala Ala Gly Glu Tyr Leu Ala Lys Met Gly Leu Pro Glu Glu Asp
               660                 665                 670
   ttc aac tct tac gca acc cac cgt ggc gac cac ttg acc gcc caa cgc    2064
   Phe Asn Ser Tyr Ala Thr His Arg Gly Asp His Leu Thr Ala Gln Arg
           675                 680                 685
   gca acc ttc gcc aat ccg aaa ctg ttt aac gaa atg gtg aga aac gaa    2112
   Ala Thr Phe Ala Asn Pro Lys Leu Phe Asn Glu Met Val Arg Asn Glu
       690                 695                 700
   gac ggc agc gta cgc caa ggt tcg ctg gca cgc gtt gaa ccc gaa ggc    2160
   Asp Gly Ser Val Arg Gln Gly Ser Leu Ala Arg Val Glu Pro Glu Gly
   705                 710                 715                 720
   caa acc atg cgc atg tgg gaa gcc atc gaa acc tat atg aac cgc aaa    2208
   Gln Thr Met Arg Met Trp Glu Ala Ile Glu Thr Tyr Met Asn Arg Lys
                   725                 730                 735
   cag ccg ctc atc atc att gcc ggc gcg gac tac ggt caa ggc tca agc    2256
   Gln Pro Leu Ile Ile Ile Ala Gly Ala Asp Tyr Gly Gln Gly Ser Ser
               740                 745                 750
   cgc gac tgg gct gca aaa ggc gta cgc ctc gcc ggc gtg gaa gcg att    2304
   Arg Asp Trp Ala Ala Lys Gly Val Arg Leu Ala Gly Val Glu Ala Ile
           755                 760                 765
   gtt gcc gaa ggc ttc gag cgt atc cac cgc acc aac ttg atc ggt atg    2352
   Val Ala Glu Gly Phe Glu Arg Ile His Arg Thr Asn Leu Ile Gly Met
       770                 775                 780
   ggc gtg ttg ccg ctg cag ttc aaa ccg ggt acc aac cgc cac acc ctg    2400
   Gly Val Leu Pro Leu Gln Phe Lys Pro Gly Thr Asn Arg His Thr Leu
   785                 790                 795                 800
   caa ctg gac ggt acg gaa acc tac gac gtt gtc ggc gaa cgc aca ccg    2448
   Gln Leu Asp Gly Thr Glu Thr Tyr Asp Val Val Gly Glu Arg Thr Pro
                   805                 810                 815
   cgc tgc gac ctg acc ctt gtg att cac cgt aaa aac ggc gag acc gtc    2496
   Arg Cys Asp Leu Thr Leu Val Ile His Arg Lys Asn Gly Glu Thr Val
               820                 825                 830
   gaa gtc ccc att acc tgc cgc ctc gat acc gca gaa gaa gtg ttg gta    2544
   Glu Val Pro Ile Thr Cys Arg Leu Asp Thr Ala Glu Glu Val Leu Val
           835                 840                 845
   tat gaa gcc ggt ggc gta ttg caa cgg ttt gca cag gat ttt ttg gaa    2592
   Tyr Glu Ala Gly Gly Val Leu Gln Arg Phe Ala Gln Asp Phe Leu Glu
       850                 855                 860
   ggg aac gcg gct tag                                                2607
   Gly Asn Ala Ala
   865
<210> 73
   <211> 868
   < 212 PRIT
   <213> Neisseria meningitidis
<400> 73
   Met Ala Ala Asn Gln Arg Tyr Arg Lys Pro Leu Pro Gly Thr Asp Leu
   1               5                   10                  15
   Glu Tyr Tyr Asp Ala Arg Ala Ala Cys Glu Gly Ile Lys Pro Gly Ser
               20                  25                  30
   Tyr Asp Lys Leu Pro Tyr Thr Ser Arg Ile Leu Ala Glu Asn Leu Val
           35                  40                  45
   Asn Arg Ala Asp Lys Val Asp Leu Pro Thr Leu Gln Ser Trp Leu Gly
       50                  55                  60
   Gln Leu Ile Glu Gly Lys Gln Glu Ile Asp Phe Pro Trp Tyr Pro Ala
   65                  70                  75                  80
   Arg Val Val Cys His Asp Ile Leu Gly Gln Thr Ala Leu Val Asp Leu
                   85                  90                  95
   Ala Gly Leu Arg Asp Ala Ile Ala Glu Lys Gly Gly Asp Pro Ala Lys
               100                 105                 110
   Val Asn Pro Val Val Gln Thr Gln Leu Ile Val Asp His Ser Leu Ala
           115                 120                 125
   Val Glu Cys Gly Gly Tyr Asp Pro Asp Ala Phe Arg Lys Asn Arg Glu
       130                 135                 140
   Ile Glu Asp Arg Arg Asn Glu Asp Arg Phe His Phe Ile Asn Trp Thr
   145                 150                 155                 160
   Lys Thr Ala Phe Glu Asn Val Asp Val Ile Pro Ala Gly Asn Gly Ile
   165 170 175
   Met His Gln Ile Asn Leu Glu Lys Met Ser Pro Val Val Gln Val Lys
   180 185 190
   Asn Gly Val Ala Phe Pro Asp Thr Cys Val Gly Thr Asp Ser His Thr
   195 200 205
   Pro His Val Asp Ala Leu Gly Val Ile Ser Val Gly Val Gly Gly Leu
   210 215 220
   Glu Ala Glu Thr Val Met Leu Gly Arg Ala Ser Met Met Arg Leu Pro
   225 230 235 240
   Asp Ile Val Gly Val Glu Leu Asn Gly Lys Arg Lys Ala Gly Ile Thr
   245 250 255
   Ala Thr Asp Ile Val Leu Ala Leu Thr Glu Phe Leu Arg Lys Glu Arg
   260 265 270
   Val Val Gly Ala Phe Val Glu Phe Phe Gly Glu Gly Ala Arg Ser Leu
   275 280 285
   Ser Ile Gly Asp Arg Ala Thr Ile Ser Asn Met Thr Pro Glu Phe Gly
   290 295 300
   Ala Thr Ala Ala Met Phe Ala Ile Asp Glu Gln Thr Ile Asp Tyr Leu
   305 310 315 320
   Lys Leu Thr Gly Arg Asp Asp Ala Gln Val Lys Leu Val Glu Thr Tyr
   325 330 335
   Ala Lys Thr Ala Gly Leu Trp Ala Asp Ala Leu Lys Thr Ala Val Tyr
   340 345 350
   Pro Arg Val Leu Lys Phe Asp Leu Ser Ser Val Thr Arg Asn Met Ala
   355 360 365
   Gly Pro Ser Asn Pro His Ala Arg Phe Ala Thr Ala Asp Leu Ala Gly
   370 375 380
   Lys Gly Leu Ala Lys Pro Tyr Glu Glu Pro Ser Asp Gly Gln Met Pro
   385 390 395 400
   Asp Gly Ala Val Ile Ile Ala Ala Ile Thr Ser Cys Thr Asn Thr Ser
   405 410 415
   Asn Pro Arg Asn Val Val Ala Ala Ala Leu Leu Ala Arg Asn Ala Asn
   420 425 430
   Arg Leu Gly Leu Gln Arg Lys Pro Trp Val Lys Ser Ser Phe Ala Pro
   435 440 445
   Gly Ser Lys Val Ala Glu Ile Tyr Leu Lys Glu Ala Asp Leu Leu Pro
   450 455 460
   Glu Met Glu Lys Leu Gly Phe Gly Ile Val Ala Phe Ala Cys Thr Thr
   465 470 475 480
   Cys Asn Gly Met Ser Gly Ala Leu Asp Pro Lys Ile Gln Lys Glu Ile
   485 490 495
   Ile Asp Arg Asp Leu Tyr Ala Thr Ala Val Leu Ser Gly Asn Arg Asn
   500 505 510
   Phe Asp Gly Arg Ile His Pro Tyr Ala Lys Gln Ala Phe Leu Ala Ser
   515 520 525
   Pro Pro Leu Val Val Ala Tyr Ala Leu Ala Gly Ser Ile Arg Phe Asp
   530 535 540
   Ile Glu Asn Asp Val Leu Gly Val Ala Asp Gly Lys Glu Ile Arg Leu
   545 550 555 560
   Lys Asp Ile Trp Pro Thr Asp Glu Glu Ile Asp Ala Ile Val Ala Glu
   565 570 575
   Tyr Val Lys Pro Gln Gln Phe Arg Asp Val Tyr Ile Pro Met Phe Asp
   580 585 590
   Thr Gly Thr Ala Gln Lys Ala Pro Ser Pro Leu Tyr Asp Trp Arg Pro
   595 600 605
   Met Ser Thr Tyr Ile Arg Arg Pro Pro Tyr Trp Glu Gly Ala Leu Ala
   610 615 620
   Gly Glu Arg Thr Leu Ser Gly Met Arg Pro Leu Ala Ile Leu Pro Asp
   625 630 635 640
   Asn Ile Thr Thr Asp His Leu Ser Pro Ser Asn Ala Ile Leu Ala Ser
   645 650 655
   Ser Ala Ala Gly Glu Tyr Leu Ala Lys Met Gly Leu Pro Glu Glu Asp
   660 665 670
   Phe Asn Ser Tyr Ala Thr His Arg Gly Asp His Leu Thr Ala Gln Arg
   675 680 685
   Ala Thr Phe Ala Asn Pro Lys Leu Phe Asn Glu Met Val Arg Asn Glu
   690 695 700
   Asp Gly Ser Val Arg Gln Gly Ser Leu Ala Arg Val Glu Pro Glu Gly
   705 710 715 720
   Gln Thr Met Arg Met Trp Glu Ala Ile Glu Thr Tyr Met Asn Arg Lys
   725 730 735
   Gln Pro Leu Ile Ile Ile Ala Gly Ala Asp Tyr Gly Gln Gly Ser Ser
   740 745 750
   Arg Asp Trp Ala Ala Lys Gly Val Arg Leu Ala Gly Val Glu Ala Ile
   755 760 765
   Val Ala Glu Gly Phe Glu Arg Ile His Arg Thr Asn Leu Ile Gly Met
   770 775 780
   Gly Val Leu Pro Leu Gln Phe Lys Pro Gly Thr Asn Arg His Thr Leu
   785 790 795 800
   Gln Leu Asp Gly Thr Glu Thr Tyr Asp Val Val Gly Glu Arg Thr Pro
                   805                 810                 815
   Arg Cys Asp Leu Thr Leu Val Ile His Arg Lys Asn Gly Glu Thr Val
               820                 825                 830
   Glu Val Pro Ile Thr Cys Arg Leu Asp Thr Ala Glu Glu Val Leu Val
           835                 840                 845
   Tyr Glu Ala Gly Gly Val Leu Gln Arg Phe Ala Gln Asp Phe Leu Glu
       850                 855                 860
   Gly Asn Ala Ala
   865
<210> 74
   <211> 1170
   <212> ADN
   <213> Neisseria meningitidis
<220>
   <221> CDS.
   <222> (1) .. (1167)
<400> 74
   atg ccg caa att aaa att ccc gcc gtt tac tac cgt ggc ggt aca tca      48
   Met Pro Gln Ile Lys Ile Pro Ala Val Tyr Tyr Arg Gly Gly Thr Ser
   1               5                   10                  15
   aaa ggc gtg ttt ttc aaa cgt tcc gac ctg ccc gag gcg gcg cgg gaa      96
   Lys Gly Val Phe Phe Lys Arg Ser Asp Leu Pro Glu Ala Ala Arg Glu
               20                  25                  30
   gcg gga agc gca cgc gac aaa atc ctc ttg cgc gta ctc ggc agc ccg     144
   Ala Gly Ser Ala Arg Asp Lys Ile Leu Leu Arg Val Leu Gly Ser Pro
           35                  40                  45
   gat ccc tac ggc aag cag ata gac ggt ttg ggc aac gcc agc tcg tcc     192
   Asp Pro Tyr Gly Lys Gln Ile Asp Gly Leu Gly Asn Ala Ser Ser Ser
       50                  55                  60
   acc agc aag gcg gtg att ttg gac aag tcc gaa cgc gcc gat cac gat     240
   Thr Ser Lys Ala Val Ile Leu Asp Lys Ser Glu Arg Ala Asp His Asp
   65                  70                  75                  80
   gtc gat tac ctt ttc ggg caa gtt tcc atc gac aaa cct ttt gtc gat     288
   Val Asp Tyr Leu Phe Gly Gln Val Ser Ile Asp Lys Pro Phe Val Asp
                   85                  90                  95
   tgg agt ggc aac tgc ggc aac ctc acc gcc gcc gtg ggc gca ttt gcc     336
   Trp Ser Gly Asn Cys Gly Asn Leu Thr Ala Ala Val Gly Ala Phe Ala
               100                 105                 110
   atc gag caa ggc ttg gtc gat aaa ggc aag att cct tca gac ggc atc     384
   Ile Glu Gln Gly Leu Val Asp Lys Gly Lys Ile Pro Ser Asp Gly Ile
           115                 120                 125
   tgc aca gtc aaa atc tgg cag aaa aac atc ggc aaa acc att att gcc     432
   Cys Thr Val Lys Ile Trp Gln Lys Asn Ile Gly Lys Thr Ile Ile Ala
       130                 135                 140
   cat gta ccg atg caa aac ggc gca gtt ttg gaa aca ggc gat ttt gag     480
   His Val Pro Met Gln Asn Gly Ala Val Leu Glu Thr Gly Asp Phe Glu
   145                 150                 155                 160
   ctc gac ggc gta acg ttc ccg gca gcc gaa gta caa atc gaa ttt ctt     528
   Leu Asp Gly Val Thr Phe Pro Ala Ala Glu Val Gln Ile Glu Phe Leu
                   165                 170                 175
   gat cca gcc gac ggc gaa ggc agt atg ttc cca acc ggc aat ttg gtc     576
   Asp Pro Ala Asp Gly Glu Gly Ser Met Phe Pro Thr Gly Asn Leu Val
               180                 185                 190
   gat gaa att gat gtg ccg aat ata ggc cgt ttg aaa gcc acg ctc atc     624
   Asp Glu Ile Asp Val Pro Asn Ile Gly Arg Leu Lys Ala Thr Leu Ile
           195                 200                 205
   aac gcg ggc att ccg acc gtt ttc ctg aat gcc gcc gac ttg ggc tac     672
   Asn Ala Gly Ile Pro Thr Val Phe Leu Asn Ala Ala Asp Leu Gly Tyr
       210                 215                 220
   acg ggc aaa gag ttg caa gac gac atc aac aac gat gcc gca gct ttg     720
   Thr Gly Lys Glu Leu Gln Asp Asp Ile Asn Asn Asp Ala Ala Ala Leu
   225                 230                 235                 240
   gaa aaa ttc gag aaa atc cgc gct tac ggt gcg ctg aaa atg ggt ctg     768
   Glu Lys Phe Glu Lys Ile Arg Ala Tyr Gly Ala Leu Lys Met Gly Leu
                   245                 250                 255
   atc agc gac gta tcc gaa gct gcc gcc cgc gcg cac acg ccg aaa gtc     816
   Ile Ser Asp Val Ser Glu Ala Ala Ala Arg Ala His Thr Pro Lys Val
               260                 265                 270
   gcc ttc gtc gcg ccc gcc gcc gat tac acc gcc tcc agt ggc aaa acc     864
   Ala Phe Val Ala Pro Ala Ala Asp Tyr Thr Ala Ser Ser Gly Lys Thr
           275                 280                 285
   gtg aat gcc gcc gac atc gat ttg ctg gta cgc gcc ctg agc atg ggc     912
   Val Asn Ala Ala Asp Ile Asp Leu Leu Val Arg Ala Leu Ser Met Gly
       290                 295                 300
   aaa ttg cac cac gcg atg atg ggt acc gcc tct gtt gcc att gcg acc     960
   Lys Leu His His Ala Met Met Gly Thr Ala Ser Val Ala Ile Ala Thr
   305                 310                 315                 320
   gcc gcc gcc gtg ccc ggt acg ctg gtc aac ctt gcc gca ggc ggc gga    1008
   Ala Ala Ala Val Pro Gly Thr Leu Val Asn Leu Ala Ala Gly Gly Gly
                   325                 330                 335
   acg cgt aaa gaa gtg cgc ttc ggg cat cct tcc ggc aca ttg cgc gtc    1056
   Thr Arg Lys Glu Val Arg Phe Gly His Pro Ser Gly Thr Leu Arg Val
               340                 345                 350
   ggt gca gcc gcc gaa tgt cag gac gga caa tgg acg gcc acc aaa gcg    1104
   Gly Ala Ala Ala Glu Cys Gln Asp Gly Gln Trp Thr Ala Thr Lys Ala
           355                 360                 365
   gtt atg agc cgc agc gca cgc gtg atg atg gaa ggt tgg gtc agg gtg    1152
   Val Met Ser Arg Ser Ala Arg Val Met Met Glu Gly Trp Val Arg Val
       370                 375                     380
   ccg gaa gat tgt ttt taa                                            1170
   Pro Glu Asp Cys Phe
   385
<210> 75
   <211> 389
   <212> PRT
   <213> Neisseria meningitidis
<400> 75
   Met Pro Gln Ile Lys Ile Pro Ala Val Tyr Tyr Arg Gly Gly Thr Ser
   1               5                   10                  15
   Lys Gly Val Phe Phe Lys Arg Ser Asp Leu Pro Glu Ala Ala Arg Glu
               20                  25                  30
   Ala Gly Ser Ala Arg Asp Lys Ile Leu Leu Arg Val Leu Gly Ser Pro
           35                  40                  45
   Asp Pro Tyr Gly Lys Gln Ile Asp Gly Leu Gly Asn Ala Ser Ser Ser
       50                  55                  60
   Thr Ser Lys Ala Val Ile Leu Asp Lys Ser Glu Arg Ala Asp His Asp
   65                  70                  75                  80
   Val Asp Tyr Leu Phe Gly Gln Val Ser Ile Asp Lys Pro Phe Val Asp
                   85                  90                  95
   Trp Ser Gly Asn Cys Gly Asn Leu Thr Ala Ala Val Gly Ala Phe Ala
               100                 105                 110
   Ile Glu Gln Gly Leu Val Asp Lys Gly Lys Ile Pro Ser Asp Gly Ile
           115                 120                 125
   Cys Thr Val Lys Ile Trp Gln Lys Asn Ile Gly Lys Thr Ile Ile Ala
       130                 135                 140
   His Val Pro Met Gln Asn Gly Ala Val Leu Glu Thr Gly Asp Phe Glu
   145                 150                 155                 160
   Leu Asp Gly Val Thr Phe Pro Ala Ala Glu Val Gln Ile Glu Phe Leu
                   165                 170                 175
   Asp Pro Ala Asp Gly Glu Gly Ser Met Phe Pro Thr Gly Asn Leu Val
               180                 185                 190
   Asp Glu Ile Asp Val Pro Asn Ile Gly Arg Leu Lys Ala Thr Leu Ile
           195                 200                 205
   Asn Ala Gly Ile Pro Thr Val Phe Leu Asn Ala Ala Asp Leu Gly Tyr
       210                 215                 220
   Thr Gly Lys Glu Leu Gln Asp Asp Ile Asn Asn Asp Ala Ala Ala Leu
   225                 230                 235                 240
   Glu Lys Phe Glu Lys Ile Arg Ala Tyr Gly Ala Leu Lys Met Gly Leu
   245 250 255
   Ile Ser Asp Val Ser Glu Ala Ala Ala Arg Ala His Thr Pro Lys Val
   260 265 270
   Ala Phe Val Ala Pro Ala Ala Asp Tyr Thr Ala Ser Ser Gly Lys Thr
   275 280 285
   Val Asn Ala Ala Asp Ile Asp Leu Leu Val Arg Ala Leu Ser Met Gly
   290 295 300
   Lys Leu His His Ala Met Met Gly Thr Ala Ser Val Ala Ile Ala Thr
   305 310 315 320
   Ala Ala Ala Val Pro Gly Thr Leu Val Asn Leu Ala Ala Gly Gly Gly
   325 330 335
   Thr Arg Lys Glu Val Arg Phe Gly His Pro Ser Gly Thr Leu Arg Val
   340 345 350
   Gly Ala Ala Ala Glu Cys Gln Asp Gly Gln Trp Thr Ala Thr Lys Ala
   355 360 365
   Val Met Ser Arg Ser Ala Arg Val Met Met Glu Gly Trp Val Arg Val
   370 375 380
   Pro Glu Asp Cys Phe
   385
<210> 76
   <211> 2094
   <212> ADN
   <213> Neisseria gonorrhoeae
<220>
   <221> CDS
   <222> (1) .. (2091)
<400> 76
   atg aat tcg acc gca agt aaa acc ctg aaa gga ttg tcg ctg gtg ttt     48
   Met Asn Ser Thr Ala Ser Lys Thr Leu Lys Gly Leu Ser Leu Val Phe
    1               5                  10                  15
   ttc gcc tct ggc ttc tgc gcc ctg att tac cag gtc agc tgg cag agg     96
   Phe Ala Ser Gly Phe Cys Ala Leu Ile Tyr Gln Val Ser Trp Gln Arg
               20                  25                  30
   ctt cta ttc agc cac ata ggt atc gat ttg agt tcg att act gtc att    144
   Leu Leu Phe Ser His Ile Gly Ile Asp Leu Ser Ser Ile Thr Val Ile
           35                  40                  45
   att tct gta ttt atg gtc ggc ttg ggt gta ggt gcg tat ttc ggc gga    192
   Ile Ser Val Phe Met Val Gly Leu Gly Val Gly Ala Tyr Phe Gly Gly
       50                  55                  60
   cgc att gct gac cgt ttt cct tca agt atc atc ccc ctg ttt tgc atc    240
   Arg Ile Ala Asp Arg Phe Pro Ser Ser Ile Ile Pro Leu Phe Cys Ile
   65                  70                  75                  80
   gct gaa gta tcc atc ggt ctg ttc ggt ttg gta agc aag ggt ctg att    288
   Ala Glu Val Ser Ile Gly Leu Phe Gly Leu Val Ser Lys Gly Leu Ile
                   85                  90                  95
   tcc ggc ttg ggg cat ctt tta gtt gag gct gat ttg ccc atc atc gct    336
   Ser Gly Leu Gly His Leu Leu Val Glu Ala Asp Leu Pro Ile Ile Ala
               100                 105                 110
   gct gcc aat ttc ctc tta ttg ctg ctt cct acc ttt atg atg ggc gcg    384
   Ala Ala Asn Phe Leu Leu Leu Leu Leu Pro Thr Phe Met Met Gly Ala
           115                 120                 125
   acc ttg ccc ttg ctg acc tgt ttt ttt aac cgg aaa ata cat aat gtt    432
   Thr Leu Pro Leu Leu Thr Cys Phe Phe Asn Arg Lys Ile His Asn Val
       130                 135                 140
   ggc gag tct atc ggt acc tta tat ttt ttc aac act ttg ggt gcg gca    480
   Gly Glu Ser Ile Gly Thr Leu Tyr Phe Phe Asn Thr Leu Gly Ala Ala
   145                 150                 155                 160
   ctc gga tcg ctt gcc gcc gcc gaa ttt ttc tac gtc ttt ttt acc ctc    528
   Leu Gly Ser Leu Ala Ala Ala Glu Phe Phe Tyr Val Phe Phe Thr Leu
                   165                 170                 175
   tcc caa acc att gcg ctg aca gcc tgc ctt aac ctt ctg att gct gct    576
   Ser Gln Thr Ile Ala Leu Thr Ala Cys Leu Asn Leu Leu Ile Ala Ala
               180                 185                 190
   tca gta tgc tgc gtt aca gaa agg atg gat atg gtg aac act aaa ccg    624
   Ser Val Cys Cys Val Thr Glu Arg Met Asp Met Val Asn Thr Lys Pro
           195                 200                 205
   aat act agt gtg att aat atg ctt tct ttc ctt acc gga tta ttg agc    672
   Asn Thr Ser Val Ile Asn Met Leu Ser Phe Leu Thr Gly Leu Leu Ser
       210                 215                 220
   ttg ggt ata gaa gtc ttg tgg gta agg atg ttt tcg ttc gca gca cag    720
   Leu Gly Ile Glu Val Leu Trp Val Arg Met Phe Ser Phe Ala Ala Gln
   225                 230                 235                 240
   tcc gtg cct cag gca ttt tca ttt att ctt gcc tgt ttt ctg acc ggt    768
   Ser Val Pro Gln Ala Phe Ser Phe Ile Leu Ala Cys Phe Leu Thr Gly
                   245                 250                 255
   atc gcc gtc ggc gcg tat ttt ggc aaa cgg att tgc cgc agc cgc ttt    816
   Ile Ala Val Gly Ala Tyr Phe Gly Lys Arg Ile Cys Arg Ser Arg Phe
               260                 265                 270
   gtt gat att ccc ttt atc ggg cag tgc ttc ttg tgg gcg ggt att gcc    864
   Val Asp Ile Pro Phe Ile Gly Gln Cys Phe Leu Trp Ala Gly Ile Ala
           275                 280                 285
   gat ttt ttg att ttg ggt gct gcg tgg ttg ttg acg ggt ttt tcc ggt    912
   Asp Phe Leu Ile Leu Gly Ala Ala Trp Leu Leu Thr Gly Phe Ser Gly
       290                 295                 300
   ttc gtc cac cac gcc ggt att ttc att acc ctg tct gcc gtc gtc agg    960
   Phe Val His His Ala Gly Ile Phe Ile Thr Leu Ser Ala Val Val Arg
   305                 310                 315                 320
   ggg ttg att ttc cca ctt gta cac cat gtg ggt acg gat ggc aac aaa      1008
   Gly Leu Ile Phe Pro Leu Val His His Val Gly Thr Asp Gly Asn Lys
                   325                 330                 335
   tcc gga cga cag gtt tcc aat gtt tat ttc gcc aac gtt gcc ggc agt      1056
   Ser Gly Arg Gln Val Ser Asn Val Tyr Phe Ala Asn Val Ala Gly Ser
               340                 345                 350
   gca ttg ggt ccg gtc ctt atc ggc ttt gtg ata ctt gat ttg ttg tcc      1104
   Ala Leu Gly Pro Val Leu Ile Gly Phe Val Ile Leu Asp Leu Leu Ser
           355                 360                 365
   acc caa cag att tac ctg ctc atc tgt ttg att tct gct gct gtc cct      1152
   Thr Gln Gln Ile Tyr Leu Leu Ile Cys Leu Ile Ser Ala Ala Val Pro
       370                 375                 380
   ttg ttt tgt aca ctg ttc caa aaa agt ctc cga ctg aat gca gtg tcg      1200
   Leu Phe Cys Thr Leu Phe Gln Lys Ser Leu Arg Leu Asn Ala Val Ser
   385                 390                 395                 400
   gta gca gtt tcc cta atg ttc ggc atc ctc atg ttc cta ctg ccg gat      1248
   Val Ala Val Ser Leu Met Phe Gly Ile Leu Met Phe Leu Leu Pro Asp
                   405                 410                 415
   tct gtc ttt caa aat att gct ggc cgt ccg gat agg ttg att gaa aac      1296
   Ser Val Phe Gln Asn Ile Ala Gly Arg Pro Asp Arg Leu Ile Glu Asn
               420                 425                 430
   aaa cac ggc att gtt gcg gtt tac cat aga gat ggt gat aag gtt gtt      1344
   Lys His Gly Ile Val Ala Val Tyr His Arg Asp Gly Asp Lys Val Val
           435                 440                 445
   tat ggg gcg aat gta tac gac ggc gca tac aat acc gat ata ttc aat      1392
   Tyr Gly Ala Asn Val Tyr Asp Gly Ala Tyr Asn Thr Asp Ile Phe Asn
       450                 455                 460
   agt gtc aac ggc atc gaa cgt gcc tat ctg cta ccc tcc ctg aag tcc      1440
   Ser Val Asn Gly Ile Glu Arg Ala Tyr Leu Leu Pro Ser Leu Lys Ser
   465                 470                 475                 480
   ggc ata cgc cgc att ttc gtc gtt gga ttg agt aca ggt tcg tgg gcg      1488
   Gly Ile Arg Arg Ile Phe Val Val Gly Leu Ser Thr Gly Ser Trp Ala
                   485                 490                 495
   cgc gtc ttg tct gcc att ccg gaa atg cag tcg atg atc gtt gcg gaa      1536
   Arg Val Leu Ser Ala Ile Pro Glu Met Gln Ser Met Ile Val Ala Glu
               500                 505                 510
   atc aat ccg gca tac cgt agc ctt atc gcg gac gag ccg caa atc gca      1584
   Ile Asn Pro Ala Tyr Arg Ser Leu Ile Ala Asp Glu Pro Gln Ile Ala
           515                 520                 525
   ccg ctt ttg cag gac aaa cgt gtt gaa att gta ttg gat gac ggt agg      1632
   Pro Leu Leu Gln Asp Lys Arg Val Glu Ile Val Leu Asp Asp Gly Arg
       530                 535                 540
   aaa tgg ctg cgt cgc cat cct gat gaa aaa ttc gac ctg att ttg atg      1680
   Lys Trp Leu Arg Arg His Pro Asp Glu Lys Phe Asp Leu Ile Leu Met
   545                 550                     555                     560
   aat tcg act tgg tac tgg cgt gcc tat tcc act aac ctg ttg agt gcg     1728
   Asn Ser Thr Trp Tyr Trp Arg Ala Tyr Ser Thr Asn Leu Leu Ser Ala
                   565                 570                 575
   gaa ttt tta aaa cag gtg caa agc cac ctt acc ccg gat ggt att gta     1776
   Glu Phe Leu Lys Gln Val Gln Ser His Leu Thr Pro Asp Gly Ile Val
               580                 585                 590
   atg ttt aat acc acg cac agc ccg cat gct ttt gct acc gcc gta cac     1824
   Met Phe Asn Thr Thr His Ser Pro His Ala Phe Ala Thr Ala Val His
           595                 600                 605
   agt att ccc tat gca tac cgc tac ggg cat atg gta gtc ggc tcg gca     1872
   Ser Ile Pro Tyr Ala Tyr Arg Tyr Gly His Met Val Val Gly Ser Ala
       610                 615                 620
   acc ccg gta gtt ttc cct aat aaa gaa ctg ctc aag caa cgc ctt tcc     1920
   Thr Pro Val Val Phe Pro Asn Lys Glu Leu Leu Lys Gln Arg Leu Ser
   625                 630                 635                 640
   cgg ttg att tgg ccg gaa agc ggc agg cac gta ttt gac agc agc acc     1968
   Arg Leu Ile Trp Pro Glu Ser Gly Arg His Val Phe Asp Ser Ser Thr
                   645                 650                 655
   gtg gat gct gca gca caa aag gtt gtc tct cgt atg ctg att cgg atg     2016
   Val Asp Ala Ala Ala Gln Lys Val Val Ser Arg Met Leu Ile Arg Met
               660                 665                 670
   acg gaa cct tcg gct ggg gcg gaa gtc att act gac gat aat atg att     2064
   Thr Glu Pro Ser Ala Gly Ala Glu Val Ile Thr Asp Asp Asn Met Ile
           675                 680                 685
   gta gaa tac aaa tac ggc aga ggg att taa                             2094
   Val Glu Tyr Lys Tyr Gly Arg Gly Ile
       690                 695
<210> 77
   <211> 697
   <212> PRT
   <213> Neisseria gonorrhoeae
<400> 77
   Met Asn Ser Thr Ala Ser Lys Thr Leu Lys Gly Leu Ser Leu Val Phe
   1               5                   10                  15
   Phe Ala Ser Gly Phe Cys Ala Leu Ile Tyr Gln Val Ser Trp Gln Arg
               20                  25                  30
   Leu Leu Phe Ser His Ile Gly Ile Asp Leu Ser Ser Ile Thr Val Ile
           35                  40                  45
   Ile Ser Val Phe Met Val Gly Leu Gly Val Gly Ala Tyr Phe Gly Gly
       50                  55                  60
   Arg Ile Ala Asp Arg Phe Pro Ser Ser Ile Ile Pro Leu Phe Cys Ile
   65                  70                  75                  80
   Ala Glu Val Ser Ile Gly Leu Phe Gly Leu Val Ser Lys Gly Leu Ile
   85 90 95
   Ser Gly Leu Gly His Leu Leu Val Glu Ala Asp Leu Pro Ile Ile Ala
   100 105 110
   Ala Ala Asn Phe Leu Leu Leu Leu Leu Pro Thr Phe Met Met Gly Ala
   115 120 125
   Thr Leu Pro Leu Leu Thr Cys Phe Phe Asn Arg Lys Ile His Asn Val
   130 135 140
   Gly Glu Ser Ile Gly Thr Leu Tyr Phe Phe Asn Thr Leu Gly Ala Ala
   145 150 155 160
   Leu Gly Ser Leu Ala Ala Ala Glu Phe Phe Tyr Val Phe Phe Thr Leu
   165 170 175
   Ser Gln Thr Ile Ala Leu Thr Ala Cys Leu Asn Leu Leu Ile Ala Ala
   180 185 190
   Ser Val Cys Cys Val Thr Glu Arg Met Asp Met Val Asn Thr Lys Pro
   195 200 205
   Asn Thr Ser Val Ile Asn Met Leu Ser Phe Leu Thr Gly Leu Leu Ser
   210 215 220
   Leu Gly Ile Glu Val Leu Trp Val Arg Met Phe Ser Phe Ala Ala Gln
   225 230 235 240
   Ser Val Pro Gln Ala Phe Ser Phe Ile Leu Ala Cys Phe Leu Thr Gly
   245 250 255
   Ile Ala Val Gly Ala Tyr Phe Gly Lys Arg Ile Cys Arg Ser Arg Phe
   260 265 270
   Val Asp Ile Pro Phe Ile Gly Gln Cys Phe Leu Trp Ala Gly Ile Ala
   275 280 285
   Asp Phe Leu Ile Leu Gly Ala Ala Trp Leu Leu Thr Gly Phe Ser Gly
   290 295 300
   Phe Val His His Ala Gly Ile Phe Ile Thr Leu Ser Ala Val Val Arg
   305 310 315 320
   Gly Leu Ile Phe Pro Leu Val His His Val Gly Thr Asp Gly Asn Lys
   325 330 335
   Ser Gly Arg Gln Val Ser Asn Val Tyr Phe Ala Asn Val Ala Gly Ser
   340 345 350
   Ala Leu Gly Pro Val Leu Ile Gly Phe Val Ile Leu Asp Leu Leu Ser
   355 360 365
   Thr Gln Gln Ile Tyr Leu Leu Ile Cys Leu Ile Ser Ala Ala Val Pro
   370 375 380
   Leu Phe Cys Thr Leu Phe Gln Lys Ser Leu Arg Leu Asn Ala Val Ser
   385 390 395 400
   Val Ala Val Ser Leu Met Phe Gly Ile Leu Met Phe Leu Leu Pro Asp
   405 410 415
   Ser Val Phe Gln Asn Ile Ala Gly Arg Pro Asp Arg Leu Ile Glu Asn
   420 425 430
   Lys His Gly Ile Val Ala Val Tyr His Arg Asp Gly Asp Lys Val Val
   435 440 445
   Tyr Gly Ala Asn Val Tyr Asp Gly Ala Tyr Asn Thr Asp Ile Phe Asn
   450 455 460
   Ser Val Asn Gly Ile Glu Arg Ala Tyr Leu Leu Pro Ser Leu Lys Ser
   465 470 475 480
   Gly Ile Arg Arg Ile Phe Val Val Gly Leu Ser Thr Gly Ser Trp Ala
   485 490 495
   Arg Val Leu Ser Ala Ile Pro Glu Met Gln Ser Met Ile Val Ala Glu
   500 505 510
   Ile Asn Pro Ala Tyr Arg Ser Leu Ile Ala Asp Glu Pro Gln Ile Ala
   515 520 525
   Pro Leu Leu Gln Asp Lys Arg Val Glu Ile Val Leu Asp Asp Gly Arg
   530 535 540
   Lys Trp Leu Arg Arg His Pro Asp Glu Lys Phe Asp Leu Ile Leu Met
   545 550 555 560
   Asn Ser Thr Trp Tyr Trp Arg Ala Tyr Ser Thr Asn Leu Leu Ser Ala
   565 570 575
   Glu Phe Leu Lys Gln Val Gln Ser His Leu Thr Pro Asp Gly Ile Val
   580 585 590
   Met Phe Asn Thr Thr His Ser Pro His Ala Phe Ala Thr Ala Val His
   595 600 605
   Ser Ile Pro Tyr Ala Tyr Arg Tyr Gly His Met Val Val Gly Ser Ala
   610 615 620
   Thr Pro Val Val Phe Pro Asn Lys Glu Leu Leu Lys Gln Arg Leu Ser
   625 630 635 640
   Arg Leu Ile Trp Pro Glu Ser Gly Arg His Val Phe Asp Ser Ser Thr
   645 650 655
   Val Asp Ala Ala Ala Gln Lys Val Val Ser Arg Met Leu Ile Arg Met
   660 665 670
   Thr Glu Pro Ser Ala Gly Ala Glu Val Ile Thr Asp Asp Asn Met Ile
   675 680 685
   Val Glu Tyr Lys Tyr Gly Arg Gly Ile
   690 695
<210> 78
   <211> 39
   <212> ADN
   <213> Séquence artificielle
<220>
   <223> Description de la séquence artificielle : amorce de PCR
<400> 78
   gctctagacc accatgtctg aagaaaaatt gaaaatgag 39
<210> 79
   <211> 32
   <212> ADN
   <213> Séquence artificielle
<220>
   <223> Description de la séquence artificielle: amorce de PCR
<400> 79
   cgggatccag aaatggctgg attcgctate ag 32
<210> 80
   <211> 32
   <212> ADN
   <213> Séquence artificielle
<220>
   <223> Description de la séquence artificielle : amorce de PCR
<400> 80
   gctctagacc accatgaaac acttactcat cg 32
<210> 81
   <211> 27
   <212> ADN
   <213> Séquence artificielle
<220>
   <223> Description de la séquence artificielle: amorce de PCR
<400> 81
   cgggatccaa tacgtaggac ttgggtc 27
<210> 82
   <211> 35
   <212> ADN
   <213> Séquence artificielle
<220>
   <223> Description de la séquence artificielle: amorce de PCR
<400> 82
   getetagacc accatgaaaa aatccctttt cgttc 35
<210> 83
   <211> 31
   <212> ADN
   <213> Séquence artificielle
<220>
   <223> Description de la séquence artificielle: amorce de PCR
<400> 83
   cgggatccat tgcggataaa catattccgc c 31
<210> 84
   <211> 34
   <212> ADN
   <213> Séquence artificielle.
<220>
   <223> Description de la séquence artificielle: amorce de PCR
<400> 84
   gctctagacc accacgcgaa cgaccccaac cttc 34
<210> 85
   <211> 30
   <212> ADN
   <213> Séquence artificielle
<220>
   <223> Description de la séquence artificielle: amorce de PCR
<400> 85
   cgggatccag aaccggtagc ctacgccgac 30
<210> 86
   <211> 36
   <212> ADN
   <213> Séquence artificielle
<220>
   <223> Description de la séquence artificielle: amorce de PCR
<400> 86
   gctctagacc accatgaaca cacgcatcat cgtttc 36
<210> 87
   <211> 30
   <212> ADN
   <213> Séquence artificielle
<220>
   <223> Description de la séquence artificielle: amorce de PCR
<400> 87
   cgggatccag caacggcctg ccgctttaag 30
<210> 88
   <211> 34
   <212> ADN
   <213> Séquence artificielle
<220>
   <223> Description de la séquence artificielle: amorce de PCR
<400> 88
   gctctagacc accatgctga cgtttatcgg actg 34
<210> 89
   <211> 27
   <212 ADN
   <213> Séquence artificielle
<220>
   <223> Description de la séquence artificielle: amorce de PCR
<400> 89
   cgggatccac ggcagaggca cgattcc 27
<210> 90
   <211> 34
   <212> ADN
   <213> Séquence artificielle
<220>
   <223> Description de la séquence artificielle: amorce de PCR
<400> 90
   gctctagacc accatgggca tccatctcga cttc 34
<210> 91
   <211> 33
   <212> ADN
   <213> Séquence artificielle
<220>
   <223> Description de la séquence artificielle: amorce de PCR
<400> 91
   cgggatccac aaaagttcca gaaaatctaa ctc 33
<210> 92
   <211> 33
   <212> ADN
   <213> Séquence artificielle
<220>
   <223> Description de la séquence artificielle: amorce de PCR
<400> 92
   gctctagacc accatgaata gacccaagca acc 33
<210> 93
   <211> 26
   <212> ADN
   <213> Séquence artificielle
<220>
   <223> Description de la séquence artificielle: amorce de PCR
<400> 93
   cgggatccat gccgcttggg ggaggc 26
<210> 94
   <211> 34
   <212> ADN
   <213> Séquence artificielle
<220>
   <223> Description de la séquence artificielle: amorce de PCR
<400> 94
   gctctagacc accatgatga atgtcgaggc agag 34
<210> 95
   <211> 26
   <212> ADN
   <213> Séquence artificielle
<220>
   <223> Description de la séquence artificielle: amorce de PCR
<400> 95
   cgggatccac agtttgcccg acatac 26
<210> 96
   <211> 33
   <212> ADN
   <213> Séquence artificielle
<220>
   <223> Description de la séquence artificielle: amorce de PCR
<400> 96
   gctctagacc accatgaaat tttttcctgc tcc 33
<210> 97
   <211> 62
   <212> ADN
   <213> Séquence artificielle
<220>
   <223> Description de la séquence artificielle: amorce de PCR
<400> 97
   gaagatctag aaactgtaat tcaagttgaa ggaagatcta gaaactgtaa ttcaagttga 60
   ag 62
<210> 98
   <211> 33
   <212> ADN
   <213> Séquence artificielle
<220>
   <223> Description de la séquence artificielle: amorce de PCR
<400> 98
   gctctagacc accatgattg aatttgtccg agc 33
<210> 99
   <211> 54
   <212 ADN
   <213> Séquence artificielle
<220>
   <223> Description de la séquence artificielle: amorce de PCR
<400> 99
   cgggatccaa ccctgcgacg agttgcgcgg gatccaaccc tgcgacgagt tgcg 54
<210> 100
   <211> 33
   <212> ADN
   <213> Séquence artificielle
<220>
   <223> Description de la séquence artificielle: amorce de PCR
<400> 100
   gctctagacc accatgcaat acagcacact ggc 33
<210> 101
   <211> 30
   <212> ADN
   <213> Séquence artificielle
<220>
   <223> Description de la séquence artificielle: amorce de PCR
<400> 101
   cgggatccag tcctttttcg caccttgaag 30
<210> 102
   <211> 34
   <212> ADN
   <213> Séquence artificielle
<220>
   <223> Description de la séquence artificielle: amorce de PCR
<400> 102
   gctctagacc accatggagc agtcgggcaa attc 34
<210> 103
   <211> 30
   <212> ADN
   <213> Séquence artificielle
<220>
   <223> Description de la séquence artificielle: amorce de PCR
<400> 103
   cgggatccaa gctgtttggc gatttcggtg 30
<210> 104
   <211> 34
   <212> ADN
   <213> Séquence artificielle
<220>
   <223> Description de la séquence artificielle: amorce de PCR
<400> 104
   gctctagacc accatgcaaa acggcggggg aaag 34
<210> 105
   <211> 30
   <212> ADN
   <213> Séquence artificielle
<220>
   <223> Description de la séquence artificielle: amorce de PCR
<400> 105
   cgggatccag tgcctgcgca gcttggaatc 30
<210> 106
   <211> 40
   <212> ADN
   <213> Séquence artificielle
<220>
   <223> Description de la séquence artificielle: amorce de PCR
<400> 106
   gctctagacc accatgacat tgctcaatct aatgataatg 40
<210> 107
   <211> 33
   <212> ADN
   <213> Séquence artificielle
<220>
   <223> Description de la séquence artificielle: amorce de PCR
<400> 107
   cgggatccat tccgcaaata cctgtttcca acc 33
<210> 108
   <211> 30
   <212> ADN
   <213> Séquence artificielle
<220>
   <223> Description de la séquence artificielle: amorce de PCR
<400> 108
   gctctagacc accatgaaac aatccgcccg 30
<210> 109
   <211> 28
   <212> ADN
   <213> Séquence artificielle
<220>
   <223> Description de la séquence artificielle: amorce de PCR
<400> 109
   cgggatccat acttgggcgc aacatgac 28
<210> 110
   <211> 33
   <212> ADN
   <213> Séquence artificielle
<220>
   <223> Description de la séquence artificielle: amorce de PCR
<400> 110
   gctctagacc accatgaatg tttacggttt ccc 33
<210> 111
   <211> 32
   <212> ADN
   <213> Séquence artificielle
<220>
   <223> Description de la séquence artificielle: amorce de PCR
<400> 111
   cgggatccat tttttagacg tatttttagt cg 32
<210> 112
   <211> 34
   <212> ADN
   <213> Séquence artificielle
<220>
   <223> Description de la séquence artificielle: amorce de PCR
<400> 112
   gctctagacc accatgatga gtcaacactc tgcc 34
<210> 113
   <211> 29
   <212> ADN
   <213> Séquence artificielle
<220>
   <223> Description de la séquence artificielle: amorce de PCR.
<400> 113
   cgggatccat ccagtttttg ctcgaaggc 29
<210> 114
   <211> 34
   <212> ADN
   <213> Séquence artificielle
<220>
   <223> Description de la séquence artificielle: amorce de PCR
<400> 114
   gctctagacc accatgcctt cgagcaaaaa ctgg 34
<210> 115
   <211> 32
   <212> ADN
   <213> Séquence artificielle
<220>
   <223> Description de la séquence artificielle: amorce de PCR
<400> 115
   cgggatccat cgttcttcaa tctccacaaa cg 32
<210> 116
   <211> 31
   <212> ADN
   <213> Séquence artificielle
<220>
   <223> Description de la séquence artificielle: amorce de PCR
<400> 116
   gctctagacc accacgcacc tatgtggaaa g 31
<210> 117
   <211> 29
   <212> ADN
   <213> Séquence artificielle
<220>
   <223> Description de la séquence artificielle: amorce de PCR
<400> 117
   cgggatccat tcaattcgct tcaacaatg 29
<210> 118
   <211> 36
   <212> ADN
   <213> Séquence artificielle
<220>
   <223> Description de la séquence artificielle: amorce de PCR
<400>
   ggactagtcc accatggctg ccaaccaacg ttaccg 36
<210> 119
   <211> 33
   <212> ADN
   <213> Séquence artificielle
<220>
   <223> Description de la séquence artificielle: amorce de PCR
<400> 119
   gaagatctaa gccgcgttcc cttccaaaaa atc 33
<210> 120
   <211> 34
   <212> ADN
   <213> Séquence artificielle
<220>
   <223> Description de la séquence artificielle: amorce de PCR
<400> 120
   gctctagacc accatgccgc aaattaaaat tccc 34
<210> 121
   <211> 29
   <212> ADN
   <213> Séquence artificielle
<220>
   <223> Description de la séquence artificielle: amorce de PCR
<400> 121
   cgggatccaa aaacaatctt:ccggcaccc 29
<210> 122
   <211> 33
   <212> ADN
   <213> Séquence artificielle
<220>
   <223> Description de la séquence artificielle: amorce de PCR
<400> 122
   gctctagacc accatgcgca cgccgttttg ttg 33
<210> 123 <211> 29
   <212> ADN
   <213> Séquence artificielle
<220>
   <223> Description de la séquence artificielle: amorce de PCR
<400> 123
   cgggatccat tgggcaacga cgaaggcac 29
<210> 124
   <211> 33
   <212> ADN
   <213> Séquence artificielle
<220>
   <223> Description de la séquence artificielle: amorce de PCR
<400> 124
   gctctagacc accatgagaa tagagatcac acc 33
<210> 125
   <211> 27
   <212> ADN
   <213> Séquence artificielle
<220>
   <223> Description de la séquence artificielle: amorce de PCR
<400> 125
   cgggatccat ggctcaatcc tttctgc 27
<210> 126
   <211> 34
   <212> ADN
   <213> Séquence artificielle
<220>
   <223> Description de la séquence artificielle: amorce de PCR
<400> 126
   gctctagacc accatgattc acgtttcggc agtg 34
<210> 127
   <211> 29
   <212> ADN
   <213> Séquence artificielle
<220>
   <223> Description de la séquence artificielle: amorce de PCR
<400> 127
   cgggatccaa cctgcttcat gggtgattc 29
<210> 128
   <211> 36
   <212> ADN
   <213> Séquence artificielle
<220>
   <223> Description de la séquence artificielle: amorce de PCR
<400> 128
   gctctagacc accatgaatt cgaccgcaag taaaac 36
<210> 129
   <211> 28
   <212> ADN
   <213> Séquence artificielle
<220>
   <223> Description de la séquence artificielle: amorce de PCR
<400> 129
   cgggatccaa atccctctgc cgtatttg 28

## Revendications

1. Acide nucléique sous forme isolée codant pour un polypeptide spécifique des souches pathogènes du genre *Neisseria* ou un fragment antigénique de celui-ci, à l'exclusion de l'acide nucléique représenté par la séquence SEQ ID n° 74A ou par la séquence SEQ ID n° 95A, dans lequel le polypeptide est représenté par la séquence SEQ ID NO: 8 ou par une séquence présentant au moins 75% d'identité avec la séquence SEQ ID NO: 8.

2. Acide nucléique selon la revendication 1, codant pour un polypeptide spécifique des souches pathogènes du genre *Neisseria,* dans lequel le polypeptide est représenté par la séquence SEQ ID NO: 8 ou par une séquence présentant au moins 75% d'identité avec la séquence SEQ ID NO: 8.

3. Polypeptide spécifique des souches pathogènes du genre *Neisseria* ou un fragment antigénique de celui-ci, codé par un acide nucléique selon la revendication 1 ou 2, la séquence d'acides aminés dudit polypeptide étant identique à la séquence SEQ ID n°8 ou présentant au moins 75 % d'identité de séquence avec SEQ ID n°8.

4. Vecteur d'expression comprenant une cassette d'expression dans laquelle une séquence nucléotidique telle que définie dans l'une des revendications 1 et 2 est placée dans des conditions permettant son expression dans une cellule hôte.

5. Cellule hôte transformée par le vecteur d'expression selon la revendication 4.

6. Composition pharmaceutique comprenant :
a) un acide nucléique selon l'une des revendications 1 et 2, sous forme nue ou en association avec au moins un agent facilitant la transfection ;
b) ou un vecteur vaccinal comprenant une séquence nucléotidique telle que définie dans l'une des revendications 1 et 2, tel que notamment un virus ou une bactérie ;
c) ou un polypeptide selon la revendication 3;
éventuellement en association avec un véhicule pharmaceutiquement acceptable.

7. Anticorps monospécifique dirigé contre un polypeptide selon la revendication 3.

8. Utilisation d'un acide nucléique selon la revendication 1 ou 2 ou d'un polypeptide spécifique des souches pathogènes de *Neisseria* ou de fragments antigéniques de celui-ci selon la revendication 3 pour la fabrication d'une composition pharmaceutique destinée à la vaccination contre *Neisseria.*

## Claims

1. Nucleic acid in isolated form encoding a polypeptide specific to the pathogenic strains of the genus *Neisseria* or an antigenic fragment thereof, with the exception of the nucleic acid represented by the sequence SEQ ID NO. 74A or by the sequence SEQ ID NO. 95A, wherein the polypeptide is represented by the sequence SEQ ID NO. 8 or by a sequence having at least 75% identity with the sequence SEQ ID NO. 8.

2. Nucleic acid according to claim 1 encoding a polypeptide specific to the pathogenic strains of the genus *Neisseria,* wherein the polypeptide is represented by the sequence SEQ ID NO. 8 or by a sequence having at least 75% identity with the sequence SEQ ID NO. 8.

3. Polypeptide specific to the pathogenic strains of the genus *Neisseria* or an antigenic fragment thereof, encoded by a nucleic acid according to claim 1 or 2, the amino acid sequence of said polypeptide being identical to the sequence SEQ ID NO. 8 or having at least 75% sequence identity with SEQ ID NO. 8.

4. Expression vector comprising an expression cassette wherein a nucleotide sequence as defined in one of claims 1 and 2 is placed under conditions that allow it to be expressed in a host cell.

5. Host cell transformed by the expression vector according to claim 4.

6. Pharmaceutical composition comprising:
a) a nucleic acid according to one of claims 1 and 2, in naked form or combined with at least transfection promoting agent;
b) or a vaccine vector comprising a nucleotide sequence as defined in one of claims 1 and 2, in particular such as a virus or a bacteria;
c) or a polypeptide according to claim 3;
optionally combined with a pharmaceutically acceptable carrier.

7. Monospecific antibody directed against a polypeptide according to claim 3.

8. Use of a nucleic acid according to claim 1 or 2 or a polypeptide specific to the pathogenic strains of the genus *Neisseria* or antigenic fragments thereof according to claim 3, for producing a pharmaceutical composition intended for vaccinating against *Neisseria.*

## Patentansprüche

1. Nucleinsäure in isolierter Form, welche für ein spezifisches Polypeptid von pathogenen Stämmen der Gattung *Neisseria* oder eines antigenen Fragments davon codiert, mit Ausnahme der durch die Sequenz SEQ ID No. 74A oder die Sequenz SEQ ID No. 95A wiedergegeben ist, worin das Polypeptid durch die Sequenz SEQ ID No. 8 wiedergegeben ist oder eine Sequenz, die eine Identität von mindestens 75 % mit der Sequenz SEQ ID No. 8 aufweist.

2. Nucleinsäure nach Anspruch 1, welche für ein spezifisches Polypeptid von pathogenen Stämmen der Gattung *Neisseria* codiert, worin das Polypeptid durch die Sequenz SEQ ID No. 8 wiedergegeben ist oder eine Sequenz, die mindestens 75 % Identität mit der Sequenz SEQ ID No. 8 aufweist.

3. Spezifisches Polypeptid von pathogenen Stämmen der Gattung *Neisseria* oder ein antigenes Fragment davon, welches durch eine Nucleinsäure nach Anspruch 1 oder 2 codiert wird, wobei die Aminosäuresequenz des Polypeptids identisch ist mit der Sequenz SEQ ID No. 8 oder zu mindestens 75 % mit der Sequenz SEQ ID No. 8 identisch ist.

4. Expressionsvektor, umfassend eine Expressionskassette, in die eine Nucleotidsequenz, wie sie in einem der Ansprüche 1 und 2 definiert worden ist, eingebracht worden ist unter Bedingungen, die ihre Expression in einer Wirtszelle ermöglicht.

5. Wirtszelle, die durch den Expressionsvektor nach Anspruch 4 transformiert ist.

6. Pharmazeutische Zubereitung, umfassend:
a) eine Nucleinsäure nach einem der Ansprüche 1 und 2 in reiner Form oder in Kombination mit mindestens einem Mittel, welches die Transfektion erleichtert;
b) oder einen Impfvektor, der eine Nucleotidsequenz umfasst, wie sie in einem der Ansprüche 1 und 2 definiert ist, wie insbesondere ein Virus oder eine Bakterie;
c) oder ein Polypeptid nach Anspruch 3;
gegebenenfalls in Kombination mit einem geeigneten pharmazeutischen Hilfsmittel.

7. Monospezifischer Antikörper, gerichtet gegen ein Polypeptid nach Anspruch 3.

8. Verwendung einer Nucleinsäure nach Anspruch 1 oder 2 oder eines spezifischen Polypeptids von pathogenen Stämmen von *Neisseria* oder von antigenen Fragmenten davon nach Anspruch 3 für die Herstellung einer pharmazeutischen Zubereitung, die als Impfstoff gegen *Neisseria* bestimmt ist.
